# EUROPEAN PATENT SPECIFICATION

(11) **EP 2 841 096 B1**
(45) Date of publication and mention of the grant of the patent: **30.09.2020**
(21) Application number: 13720312.1
(22) Date of filing: 24.04.2013
(51) Int. Cl.: A61K 39/102, C07K 19/00, C07K 14/285, C12N 15/63, G01N 33/569, A61K 39/00, A61K 38/00

(54) **ANTIGENS AND ANTIGEN COMBINATIONS**
ANTIGENEN UND KOMBINATIONENE VON ANTIGENEN
ANTIGÈNES ET LEURS COMBINAISONS

(30) Priority: 26.04.2012 GB 201207385; 21.12.2012 EP 12199079
(43) Date of publication of application: 04.03.2015
(73) Proprietor: GlaxoSmithKline Biologicals SA, 1330 Rixensart (BE)
(72) Inventor: SORIANI, Marco, I-53100 Siena (IT); SCARSELLI, Maria, I-53100 Siena (IT); NORAIS, Nathalie, I-53100 Siena (IT); GOMES MORIEL, Danilo, I-53100 Siena (IT); ROSSI PACCANI, Silvia, I-53100 Siena (IT)
(74) Representative: Dalton, Marcus Jonathan William
(86) International application number: PCT/EP2013/058459
(87) International publication number: WO 2013/160335

(56) References cited:
- WO-A2-02/28889
- WO-A2-2010/092176

### SEQUENCE LISTING

<110> Novartis AG
<120> ANTIGENS AND ANTIGEN COMBINATIONS
<130> PAT054802-WO-PCT
<140> PCT/EP2013/
   <141> Evendate Herewith
<150> GB1207385.4
   <151> 2012-04-26
<150> EP12199079.0
   <151> 2012-12-21
<160> 131
<170> BiSSAP 1.2
<210> 1
   <211> 277
   <212> PRT
   <213> Bacteria <prokaryote>
<220>
   <223> NT018
<400> 1
   Lys His Gly Gln Lys Arg Asp Asp Leu Asn Lys Ala Leu Tyr Phe Ser
   1               5                   10                  15
   Arg Leu Glu Glu Ile Glu Gln Asp Asn Ser Gln Gly Leu Val Glu Asn
               20                  25                  30
   Val Glu Gln Leu Lys Gln Glu Leu Gln Lys Thr Leu Leu Asp Asp Val
           35                  40                  45
   Pro Ser Lys Val Gln Glu Asn Val Asp Tyr Ser Gly Lys Ser Tyr Gly
       50                  55                  60
   Lys Ile Trp Phe Val Ser Gly Val Leu Ala Leu Gly Ile Ile Ala Gly
   65                  70                  75                  80
   Ser Ser Tyr Phe Met Val Gly Ser Trp Gln Ala Glu Ser Met Leu Glu
                   85                  90                  95
   Gln Thr Tyr Ala Lys Leu Pro Tyr Phe Phe Asp Arg Met Lys Asp Glu
               100                 105                 110
   Asp Lys Asn Pro Phe Ser Asp Ala Glu Met Gln Gln Phe Ser Ile Ala
           115                 120                 125
   Leu Arg Ile Asp Leu Gln Lys Asn Pro Thr Asp Ala Lys Lys Trp Trp
       130                 135                 140
   Met Leu Gly Gln Ile Gly Met Asn Leu Gly Asp Ala Arg Leu Ala Phe
   145                 150                 155                 160
   Asp Ser Tyr Gln Lys Ala Asn Lys Leu Glu Pro Asp Asn Val Gln Tyr
                   165                 170                 175
   Lys Leu Gly Tyr Ala Arg Ile Leu Met Phe Ser Glu Asp Ala Thr Asp
               180                 185                 190
   Lys Leu Lys Gly Gly Asn Leu Leu Arg Glu Val Ile Arg Gln Glu His
           195                 200                 205
   Thr Asn Ile Glu Ala Leu Ser Leu Leu Ala Phe Arg Tyr Phe Glu Thr
       210                 215                 220
   Glu Asp Tyr Lys Met Ala Ala Val Thr Trp Ala Met Met Leu Arg Leu
   225                 230                 235                 240
   Met Pro Lys Asp Asp Glu Arg Val Pro Leu Ile Glu Lys Ser Ile Arg
                   245                 250                 255
   Thr Ala Arg Asp Ala Leu Glu Ala Gln Asn Glu Glu Lys Ser Lys Ser
               260                 265                 270
   Ile Thr Pro Glu Lys
               275
<210> 2
   <211> 160
   <212> PRT
   <213> Bacteria <prokaryote>
<220>
   <223> NT024
<400> 2
   Met Lys Thr Ile Asp Ile Thr Ala Asn Ser Lys Met Asp Asp Gln Ala
   1               5                   10                  15
   Arg Met Asn Leu Ala Gln Glu Phe Ala Asn Lys Gln Gln Trp Ser Ser
               20                  25                  30
   Val Phe Asp Ile Met Tyr Pro Met Ala Leu Glu Gly Asn Thr Thr Ala
           35                  40                  45
   Gln Ser Asn Leu Gly Met Leu Tyr Asn Leu Gly Arg Gly Thr Val Arg
       50                  55                  60
   Asp Tyr Glu Lys Ala Tyr Trp Trp Phe Ser Glu Ala Ala Glu Lys Gly
   65                  70                  75                  80
   Ser Val Lys Gly Leu Asn Asn Leu Gly Val Met Tyr Leu Arg Gly Asp
                   85                  90                  95
   Tyr Val Lys Gln Asn Thr Glu Gln Ala Ile Lys Leu Phe Glu Arg Thr
               100                 105                 110
   Ala Arg Ala Lys Asp Thr Asp Ala Met Met Met Leu Ser Asn Ile Tyr
           115                 120                 125
   Arg Leu Gln Asn Gln Pro Glu Lys Ser Leu Glu Trp Leu Lys Lys Ala
       130                 135                 140
   Ala Glu Leu Gly Asn Lys Glu Ala Lys Gln Arg Leu Ser Ser Gln Pro
   145                 150                 155                 160
<210> 3
   <211> 102
   <212> PRT
   <213> Bacteria <prokaryote>
<220>
   <223> NT032
<400> 3
   Gly Phe Asn Gly Asn Asn Ser Gln Gly Gly Phe Gln Gln Thr Ala Pro
   1               5                   10                  15
   Ala Ala Ile Ser Val Lys Gln Ala Leu Ser Ala Ala Asp Asn Ser Met
               20                  25                  30
   Ile Thr Leu Val Gly Asn Ile Thr Gln Gln Ile Asp Asp Asp Glu Phe
           35                  40                  45
   Trp Phe Thr Asp Gly Thr Gly Gln Ile Lys Ile Glu Ile Lys Lys Arg
       50                  55                  60
   Val Trp Asn Gly Leu Asn Val Asp Ser Lys Asp Lys Val Lys Ile Tyr
   65                  70                  75                  80
   Gly Lys Leu Asp Asn Glu Ala Phe Glu Lys Ala Glu Leu Asp Val Leu
                   85                  90                  95
   Arg Val Glu Lys Ala Glu
               100
<210> 4
   <211> 259
   <212> PRT
   <213> Bacteria <prokaryote>
<220>
   <223> NT038 Tyr Gln Trp
<210> 5
   <211> 521
   <212> PRT
   <213> Bacteria <prokaryote>
<220>
   <223> NT067
<400> 5
   Val Ile Val Pro Glu Gly Thr Gln Leu Asp Glu Lys Gln His Ile Val
   1               5                   10                  15
   Ile Asn Asn Gly Ala Glu Pro Gln Ser Phe Asn Pro His Lys Thr Glu
               20                  25                  30
   Gly Val Pro Glu Ser Asn Val Ala Tyr Gln Leu Leu Glu Gly Leu Val
           35                  40                  45
   Thr Ser Asp Ser Glu Gly Lys Leu Gln Pro Gly Ala Ala Glu Ser Trp
       50                  55                  60
   Glu Asn Thr Pro Asp Phe Lys Thr Trp Thr Phe His Leu Arg Lys Asp
   65                  70                  75                  80
   Ala Lys Trp Ser Asn Gly Asp Pro Val Thr Ala His Asp Phe Val Phe
                   85                  90                  95
   Ala Trp Arg Arg Leu Val Asp Pro Ala Thr Ala Ala Pro Tyr Ala Ser
               100                 105                 110
   Tyr Leu Ser Tyr Leu Gln Val Glu Asn Ala Gln Asp Ile Ile Asp Gly
   115 120 125
   Lys Lys Lys Pro Ala Glu Leu Gly Val Glu Ala Lys Asp Asp Tyr Thr
   130 135 140
   Phe Val Val His Ala Thr Asn Pro Val Pro Tyr Ala Val Ser Leu Thr
   145 150 155 160
   Thr His Gln Ser Leu Leu Pro Leu Pro Gln Lys Val Val Glu Lys Leu
   165 170 175
   Gly Asp Ala Trp Val Lys Lys Glu Asn Tyr Val Gly Asn Gly Ala Tyr
   180 185 190
   Lys Leu Ala Asn His Ile Ile Asn Glu Lys Ile Glu Phe Glu Arg Asn
   195 200 205
   Pro Leu Tyr Trp Asn Asp Lys Glu Thr Val Ile Asn Ser Ala Thr Phe
   210 215 220
   Leu Ala Ile Glu Asn Pro Ser Thr Asp Val Ala Arg Tyr Arg Ala Gly
   225 230 235 240
   Asp Leu Asp Met Thr Ser Tyr Gly Leu Pro Pro Glu Gln Phe Ala Lys
   245 250 255
   Leu Lys Lys Glu Leu Leu Gly Glu Val Tyr Val Thr Arg Thr Leu Gly
   260 265 270
   Thr Tyr Ser Tyr Glu Leu Asn Asn Lys Lys Ala Pro Phe Asp Asn Val
   275 280 285
   Asn Ile Arg Lys Ala Leu Asn Leu Ser Leu Asp Arg Asn Val Ile Thr
   290 295 300
   Asp Lys Val Leu Gly Gln Gly Gln Thr Pro Thr Tyr Val Phe Thr Pro
   305 310 315 320
   Thr Tyr Ile Glu Glu Gly His Leu Ile Gln Gln Pro Ala Tyr Ser Lys
   325 330 335
   Glu Pro Met Ala Gln Arg Asn Glu Glu Ala Ile Lys Leu Leu Glu Glu
   340 345 350
   Ala Gly Tyr Ser Lys Ala Asn Pro Leu Lys Phe Ser Ile Leu Tyr Asn
   355 360 365
   Thr Asn Glu Asn His Lys Lys Val Ala Ile Ala Ala Ala Ser Met Trp
   370 375 380
   Lys Ala Asn Thr Lys Gly Leu Ile Asp Val Lys Leu Glu Asn Gln Glu
   385 390 395 400
   Trp Lys Thr Tyr Ile Asp Ser Arg Arg Ala Gly Arg Tyr Asp Val Ala
   405 410 415
   Arg Ala Gly Trp His Ala Asp Tyr Asn Gln Ala Thr Thr Phe Gly Asn
   420 425 430
   Tyr Phe Leu Ser Asn Ser Ser Asn Asn Thr Ala Lys Tyr Ala Asn Pro
   435 440 445
   Glu Tyr Asp Lys Ala Met Ala Glu Ser Tyr Ala Ala Thr Asp Ala Glu
   450 455 460
   Gly Arg Ala Lys Ala Tyr Ala Lys Ala Glu Glu Ile Leu Gly Lys Asp
   465 470 475 480
   Tyr Gly Ile Val Pro Ile Phe Asn Tyr Val Asn Pro Arg Leu Val Lys
   485 490 495
   Pro Tyr Val Lys Gly Tyr Ser Gly Lys Asp Pro Gln Asp His Ile Tyr
   500 505 510
   Leu Arg Asn Leu Tyr Ile Ile Lys His
   515 520
<210> 6
   <211> 252
   <212> PRT
   <213> Bacteria <prokaryote>
<220>
   <223> NT001
<400> 6
   Lys Glu Asp Lys Lys Pro Glu Ala Ala Val Ala Pro Leu Lys Ile Lys
<210> 7
   <211> 243
   <212> PRT
   <213> Bacteria <prokaryote>
<220>
   <223> NT016
<400> 7
   Ser Ser Gly Ser Lys Asp Val Glu Gln Ala Ser Val Asn Glu Leu Tyr
   1               5                   10                  15
   Thr Lys Gly Thr Thr Ser Leu Gln Glu Gly Ser Tyr Ser Glu Ala Ile
               20                  25                  30
   Arg Tyr Leu Lys Ala Thr Thr Glu Arg Phe Pro Gly Ser Val Tyr Gln
           35                  40                  45
   Glu Gln Ala Met Leu Asp Leu Ile Tyr Ala Asn Tyr Lys Thr Gln Asp
       50                  55                  60
   Tyr Thr Gln Val Leu Leu Met Val Asp Ser Phe Leu His Gln Phe Pro
   65                  70                  75                  80
   Gln Ser Pro Asn Gln Ala Tyr Ala Val Tyr Met Ala Gly Leu Thr Asn
                   85                  90                  95
   Ala Ala Thr Gly Asp Asn Phe Ile Gln Asp Phe Phe Gly Ile Asp Arg
               100                 105                 110
   Ala Thr Arg Glu Thr Thr Ser Met Arg Thr Ala Phe Ser Asn Phe Gln
           115                 120                 125
   Asn Leu Val Arg Val Phe Pro Asn Ser Pro Tyr Ser Gln Asp Ala Leu
       130                 135                 140
   Ala Arg Met Ala Tyr Ile Lys Asp Ala Leu Ala Arg His Glu Leu Glu
   145                 150                 155                 160
   Ile Ala Lys Phe Tyr Ala Lys Arg Lys Ala Trp Val Ala Val Ala Asn
                   165                 170                 175
   Arg Val Val Gly Met Leu Lys Gln Tyr Pro Asp Thr Lys Ala Thr Tyr
               180                 185                 190
   Glu Gly Leu Phe Leu Met Gln Ala Ala Tyr Glu Lys Met Gly Leu Thr
           195                 200                 205
   Ala Leu Ala Asn Asp Thr Gln Lys Ile Ile Asp Ala Asn Lys Asp Lys
       210                 215                 220
   Thr Phe Ala Pro Ile Glu Lys Pro Asn Glu Pro Asp Leu Lys Val Pro
   225                 230                 235                 240
   Ala Val Lys
<210> 8
   <211> 337
   <212> PRT
   <213> Bacteria <prokaryote>
<220>
   <223> NT052
<400> 8
   Asp Thr Leu Glu Gln Gln Phe Gln Gln Gly Leu Glu Ala Thr Lys Arg
   1               5                   10                  15
   Gly Asp Tyr Gln Thr Ala Phe Lys Leu Trp Leu Pro Leu Ala Glu Gln
               20                  25                  30
   Gly Asn Ala Ser Ile Gln Phe Asn Leu Gly Leu Met Tyr Lys Lys Gly
           35                  40                  45
   Gln Gly Ile Lys Gln Asp Asp Phe Glu Ala Val Lys Trp Tyr Arg Lys
       50                  55                  60
   Ala Ala Glu Gln Gly Val Ala Asp Ala Gln Leu Asn Leu Gly Asn Met
   65                  70                  75                  80
   Tyr Ala Lys Gly Leu Gly Val Lys Gln Asp Asp Val Glu Ala Val Lys
                   85                  90                  95
   Trp Tyr Arg Gln Ala Ala Glu Gln Gly Asn Ala Lys Ala Gln Phe Asn
               100                 105                 110
   Leu Gly Leu Met Tyr Asp Asn Gly Arg Gly Val Lys Gln Asp Tyr Phe
           115                 120                 125
   Glu Ala Val Lys Trp Phe Arg Lys Ala Ala Glu Gln Gly Tyr Ala Asp
       130                 135                 140
   Ala Gln Phe Asn Leu Gly Asn Met Tyr Tyr Asn Gly His Gly Val Lys
   145                 150                 155                 160
   Gln Asp Asp Phe Glu Ala Val Lys Trp Tyr Arg Lys Ala Ala Glu Gln
                   165                 170                 175
   Gly Tyr Ala Asp Ala Gln Phe Asn Leu Gly Asn Met Tyr Tyr Asn Gly
               180                 185                 190
   His Gly Val Lys Gln Asp Asp Phe Glu Ala Val Lys Trp Tyr Arg Lys
           195                 200                 205
   Ala Ala Glu Gln Gly His Ala Lys Ala Gln Tyr Asn Leu Gly Asn Met
       210                 215                 220
   Tyr Ala Asn Gly Arg Gly Val Lys Gln Asp Tyr Phe Glu Ala Val Lys
   225                 230                 235                 240
   Trp Tyr Arg Lys Ala Ala Glu Gln Gly Tyr Ala Asp Ala Gln Ala Asn
                   245                 250                 255
   Leu Gly Ser Ala Tyr Ser Ala Gly His Gly Val Arg Gln Asp Tyr Ile
               260                 265                 270
   Glu Ala Val Lys Trp Phe Lys Lys Ala Ala Glu Asn Gly Ser Ala Asp
           275                 280                 285
   Gly Gln Phe Lys Leu Gly Leu Val Tyr Leu Ile Gly Gln Gly Ile Gln
       290                 295                 300
   Lys Asp Arg Thr Leu Ala Lys Glu Trp Leu Gly Lys Ala Cys Asp Asn
   305                 310                 315                 320
   Gly Asn Gln Asn Gly Cys Glu Tyr Tyr Gly Glu Leu Asn Arg Gly Glu
                   325                 330                 335
   Arg
<210> 9
   <211> 143
   <212> PRT
   <213> Bacteria <prokaryote>
<220>
   <223> NT002
<400> 9
   Cys Ser Ser Phe Gln Asn Asp Asp Tyr Ala Met Asn Tyr Lys Gly Gln
   1               5                   10                  15
   Ile Gly Asp Pro Ile Met Ala Ile Ala Met Leu Ser Glu Gln Gln His
               20                  25                  30
   Glu Trp Ala Gly Thr Pro Tyr Val Leu Gly Gly Val Ser Arg Arg Gly
           35                  40                  45
   Val Asp Cys Ser Gly Phe Val Gln Lys Thr Phe Phe Asp Arg Phe Asn
       50                  55                  60
   Leu Arg Leu Pro Arg Ser Thr Val Glu Gln Ala Asn Tyr Gly Lys His
   65                  70                  75                  80
   Val Arg Lys Glu His Ile Gln Thr Gly Asp Leu Ile Phe Phe Lys Thr
                   85                  90                  95
   Gly Leu Gly Pro Asn Gly Tyr His Val Gly Ile Tyr Val Lys Glu Asp
               100                 105                 110
   Lys Phe Leu His Ala Ser Thr Arg Gly Gly Val Val Tyr Ser Ser Met
           115                 120                 125
   Asn Asn Pro Tyr Trp Ser Lys Ala Phe Trp Gln Val Arg Arg Ile
       130                 135                 140
<210> 10
   <211> 146
   <212> PRT
   <213> Bacteria <prokaryote>
<220>
   <223> NT026
<400> 10
   Val Pro Leu Trp Lys Thr Asp Ser Pro Lys Thr Ile Leu Ala Lys Glu
   1               5                   10                  15
   Gln His Arg Leu Tyr Leu Phe Leu Arg Gln Ile Gln Ala Arg Ala Glu
               20                  25                  30
   Asn Ser Ser Glu Val Trp Phe Leu Leu Ile Asn Arg Asn Leu Ala Thr
           35                  40                  45
   Gln Gln Trp Cys Leu Thr Ala Gln Val Lys Asn Asn Gln Thr Cys Asp
       50                  55                  60
   Cys Leu Asn Pro Ile Asn Cys Pro Lys Glu Val Tyr Ala His Phe Tyr
   65                  70                  75                  80
   Tyr Pro Tyr Phe Pro Asn Lys Thr Met Ile Gln Ser His His Ile Tyr
                   85                  90                  95
   Pro Lys Glu Ile Thr Arg Phe Asp Gly Ile Arg Asn Thr Ile Val Thr
               100                 105                 110
   Arg Cys Phe Ile Leu Gln Ala Glu Asn Glu Arg Thr Leu Phe Leu Phe
           115                 120                 125
   Phe Asn Val Gly Ser Ile Arg Leu Lys Thr Asn Gln Phe Asp Ser Ala
       130                 135                 140
   Cys Asn
   145
<210> 11
   <211> 556
   <212> PRT
   <213> Bacteria <prokaryote>
<220>
   <223> NT009
<400> 11
   Glu Gln Thr Val Asp Ile Glu Val Gln Gly Ile Arg Gly Phe Arg Ala
   1               5                   10                  15
   Val Arg Asn Thr Asp Leu Asn Val His Leu Ile Asn Lys Glu Glu Met
               20                  25                  30
   Asp Gly Ser Glu Arg Tyr Gln His Leu Val Thr Lys Ala Val Asp Arg
           35                  40                  45
   Gly Leu Arg Val Phe Gly Tyr Tyr Asp Ser Ser Val Arg Phe Glu Arg
       50                  55                  60
   Lys Gln Arg Gln Gly Lys Arg Asp Leu Leu Ile Ala His Val Thr Pro
   65                  70                  75                  80
   Gly Glu Pro Thr Lys Ile Ala Gly Thr Asp Val Gln Ile Glu Gly Glu
                   85                  90                  95
   Ala Ala Gln Asp Glu Asn Phe Asn Ala Leu Arg Lys Asn Leu Pro Lys
               100                 105                 110
   Asp Gly Val Leu Val Glu His Gln Thr Tyr Asp Asp Tyr Lys Thr Ala
           115                 120                 125
   Ile Ser Arg Leu Ala Leu Asn Arg Gly Tyr Phe Asp Gly Glu Phe Lys
       130                 135                 140
   Ile Ser Arg Leu Glu Ile Ser Pro Glu Thr His Gln Ala Trp Trp Arg
   145                 150                 155                 160
   Met Leu Phe Asp Ser Gly Val Arg Tyr His Tyr Gly Asn Ile Thr Phe
                   165                 170                 175
   Ser His Ser Gln Ile Arg Asp Asp Tyr Leu Asn Asn Ile Leu Asn Ile
               180                 185                 190
   Lys Ser Gly Asp Pro Tyr Leu Met Asn Asn Leu Ser Asp Leu Thr Ser
           195                 200                 205
   Asp Phe Ser Ser Ser Asn Trp Phe Asn Ser Val Leu Val Gln Pro Asn
       210                 215                 220
   Ile Asn His Lys Ser Lys Thr Val Asp Ile Glu Ile Ile Leu Tyr Pro
   225                 230                 235                 240
   Arg Lys Lys Asn Ala Met Glu Leu Gly Val Gly Phe Asp Thr Asp Gly
                   245                 250                 255
   Gly Val His Gly Gln Ile Gly Trp Thr Lys Pro Trp Ile Asn Ser Arg
               260                 265                 270
   Gly His Ser Leu Arg Ser Asn Leu Tyr Leu Ser Ala Pro Lys Gln Thr
           275                 280                 285
   Leu Glu Ala Thr Tyr Arg Ile Pro Leu Leu Lys Asn Pro Leu Asn Tyr
       290                 295                 300
   Tyr Tyr Asp Phe Ala Val Gly Trp Glu Gly Glu Lys Glu Asn Asp Thr
   305                 310                 315                 320
   Asn Thr Arg Ala Leu Thr Leu Ser Ala Leu Arg Tyr Trp Asn Asn Ala
                   325                 330                 335
   Arg Gly Trp Gln Tyr Phe Gly Gly Leu Arg Ala Arg Tyr Asp Ser Phe
               340                 345                 350
   Thr Gln Ala Asp Ile Thr Asp Lys Thr Leu Leu Leu Tyr Pro Thr Val
           355                 360                 365
   Gly Phe Thr Arg Thr Arg Leu Arg Gly Gly Ser Phe Ala Thr Trp Gly
       370                 375                 380
   Asp Val Gln Lys Ile Thr Phe Asp Leu Ser Lys Arg Ile Trp Leu Ser
   385                 390                 395                 400
   Glu Ser Ser Phe Ile Lys Val Gln Ala Ser Ser Ala Trp Ile Arg Thr
                   405                 410                 415
   Tyr Ala Glu Asn His Arg Ile Val Ala Arg Ala Glu Ile Gly Tyr Leu
               420                 425                 430
   His Thr Lys Asp Ile Glu Lys Ile Pro Pro Thr Leu Arg Phe Phe Ala
           435                 440                 445
   Gly Gly Asp Arg Ser Val Arg Gly Tyr Gly Tyr Lys Lys Ile Ala Pro
       450                 455                 460
   Lys Asn Lys Asn Gly Lys Leu Val Gly Gly Ser Arg Leu Leu Thr Gly
   465                 470                 475                 480
   Ser Leu Glu Tyr Gln Tyr Gln Val Tyr Pro Asn Trp Trp Ala Ala Thr
                   485                 490                 495
   Phe Val Asp Ser Gly Leu Val Ala Asp Asn Tyr Thr Ala Lys Glu Leu
               500                 505                 510
   Arg Tyr Gly Ala Gly Val Gly Val Arg Trp Ala Ser Pro Val Gly Ala
           515                 520                 525
   Ile Lys Phe Asp Ile Ala Thr Pro Ile Arg Asp Lys Asp Asn Ser Lys
       530                 535                 540
   Asn Ile Gln Phe Tyr Ile Gly Leu Gly Thr Glu Ile
   545                 550                 555
<210> 12
   <211> 126
   <212> PRT
   <213> Bacteria <prokaryote>
<220>
   <223> NT025
<400> 12
   Ile Ile Ala Ile Leu Ala Thr Ile Ala Ile Pro Ser Tyr Gln Asn Tyr
   1               5                   10                  15
   Thr Lys Lys Ala Ala Val Ser Glu Leu Leu Gln Ala Ser Ala Pro Tyr
               20                  25                  30
   Lys Ala Asp Val Glu Leu Cys Val Tyr Ser Thr Asn Glu Thr Thr Asn
           35                  40                  45
   Cys Thr Gly Gly Lys Asn Gly Ile Ala Ala Asp Ile Thr Thr Ala Lys
       50                  55                  60
   Gly Tyr Val Lys Ser Val Thr Thr Ser Asn Gly Ala Ile Thr Val Lys
   65                  70                  75                  80
   Gly Asp Gly Thr Leu Ala Asn Met Glu Tyr Ile Leu Gln Ala Thr Gly
                   85                  90                  95
   Asn Ala Ala Thr Gly Val Thr Trp Thr Thr Thr Cys Lys Gly Thr Asp
               100                 105                 110
   Ala Ser Leu Phe Pro Ala Asn Phe Cys Arg Ser Val Thr Lys
           115                 120                 125
<210> 13
   <211> 162
   <212> PRT
   <213> Bacteria <prokaryote>
<220>
   <223> NT028
<400> 13
   Pro Arg Thr Val Ser His Gln Val Ile Ser Glu Asn Asp Asp Ile Gln
   1               5                   10                  15
   Leu Thr Gly Leu Ile Asn Asn Leu Glu Lys Asp Asn Arg Thr Gly Ile
               20                  25                  30
   Phe His Lys Val Arg Thr Asn Arg Ser Ser Ala Leu Met Gly Asp Lys
           35                  40                  45
   Ala Leu Ala Ser Val Tyr Asn Glu Trp Val Gly Thr Arg Tyr Arg Met
       50                  55                  60
   Gly Gly Thr Thr Lys Arg Gly Ile Asp Cys Ser Ala Phe Met Gln Thr
   65                  70                  75                  80
   Thr Phe Ser Glu Val Phe Gly Ile Glu Leu Pro Arg Ser Thr Ala Glu
                   85                  90                  95
   Gln Arg His Leu Gly Arg Lys Ile Asn Lys Ser Glu Leu Lys Lys Gly
               100                 105                 110
   Asp Leu Val Phe Phe Arg Lys Asn Asn His Val Gly Val Tyr Ile Gly
           115                 120                 125
   Asn Asn Gln Phe Met His Ala Ser Thr Gly Gln Gly Val Thr Ile Ser
       130                 135                 140
   Ser Leu Asp Glu Lys Tyr Trp Ala Arg Thr Tyr Thr Gln Ser Arg Arg
   145                 150                 155                 160
   Ile Met
<210> 14
   <211> 895
   <212> PRT
   <213> Bacteria <prokaryote>
<220>
   <223> NT029
<400> 14
   Ser Thr Pro Asp Leu Pro Gln Asn His Lys Ile Ile Thr Gly Thr Ala
   1               5                   10                  15
   Thr Val Ser His Thr Glu Asn Glu Met Thr Ile Lys Gln Thr Thr Pro
               20                  25                  30
   Thr Thr Gln Ile Asn Trp Asp Ser Phe Asn Ile Gly Lys Asp Lys Glu
           35                  40                  45
   Val Lys Phe Glu Gln Pro Ser Thr Ser Ala Val Ala Tyr Asn Arg Val
       50                  55                  60
   Thr Gly Gly Asn Ala Ser His Ile Gln Gly Lys Leu Thr Ala Asn Gly
   65                  70                  75                  80
   Lys Val Tyr Leu Ala Asn Pro Asn Gly Val Ile Ile Thr Lys Gly Ala
                   85                  90                  95
   Glu Ile Asn Val Ala Gly Leu Leu Ala Thr Thr Lys Asp Leu Glu Arg
               100                 105                 110
   Ile Ser Glu Asn Gly Asn Thr Asn Thr Asn Lys Phe Thr Arg Lys Ala
           115                 120                 125
   Lys Glu Gly Lys Val Leu Thr Glu Gly Gln Val Ile Asn Glu Gly Glu
       130                 135                 140
   Ile Lys Ala Lys Asp Phe Val Val Leu Asn Gly Asp Glu Val Ile Asn
   145                 150                 155                 160
   Lys Gly Asn Ile Asn Val Glu Lys Asn Ser Thr Ile Asn Gly Glu Val
                   165                 170                 175
   Tyr Leu Ser Ser Ser Asn Asn Phe Thr Phe Thr Leu Ser Asp Ser Gly
               180                 185                 190
   Ile Ser Val Ala Leu Glu Asp Asn Thr Val Gln Gly Ile Val Lys Asn
           195                 200                 205
   Glu Gly Ile Val Lys Asn Glu Gly Ser Ile Lys Ala Gly Glu Ile Thr
       210                 215                 220
   Leu Ser Ala Lys Gly Arg Lys Glu Ala Leu Asp Ser Leu Val Val Asn
   225                 230                 235                 240
   Asn Gly Val Leu Glu Ala Thr Lys Val Ser Asn Arg Lys Gly Lys Ile
                   245                 250                 255
   Val Leu Ser Ala Asp Asp Val Gln Leu Asn Asn Asn Ser Asp Ile Lys
               260                 265                 270
   Gly Glu Ile Val Asn Phe Gly Thr Glu Val Thr Ser Asn Glu Asp Lys
           275                 280                 285
   Lys Leu Lys Ile Thr Ser Gln Thr Gly Ser Lys Val Thr Ser Pro Lys
       290                 295                 300
   Ile Asn Phe Lys Gly Lys Ser Val Asn Ile Lys Gly Asp Phe Gly Arg
   305                 310                 315                 320
   Glu Asp Asn Thr Thr Tyr Tyr Asp Asp Glu His Lys Lys Leu Lys Thr
                   325                 330                 335
   Glu Val Asn Ile Asp Val Pro Asn Thr Glu Asn Ile Gln Ile Ala Asp
               340                 345                 350
   Lys Asp Asn Ala Gly Thr Asp Ser Phe Ile Gln Thr Gly Ala Leu Ser
           355                 360                 365
   Ser Leu Leu Ala Asn Asn Gly Lys Val Asn Leu Lys Gly Lys Asp Val
       370                 375                 380
   Asn Ile Ser Gly Asn Ile Asn Ile Asn Ser Phe Arg Gly Thr Asp Ser
   385                 390                 395                 400
   Leu Leu Lys Leu Thr Asn Lys Gly His Ile Asn Ile Asn His Ala Asp
                   405                 410                 415
   Ile His Ser Lys Gly Arg Leu Phe Phe Ile Thr Ser Leu Gln Asn Asp
               420                 425                 430
   Val Asp Phe Gln Ser Asn Ile Thr Ile Thr Asp Ser Lys Ile Asn Leu
           435                 440                 445
   Gly Asn Gly Ala Met Gly Leu Gly Arg Ser Val Asn Glu Asn Asp Leu
       450                 455                 460
   Asp Arg Trp Arg Arg Thr Glu Tyr Ser Gln Arg Lys Lys Phe Asn Val
   465                 470                 475                 480
   Asn Met Arg Asn Val Val Phe Asp Gln Val Asp Asp Val Val Val Ala
                   485                 490                 495
   Gly Gly Phe Lys Glu Val Asn Leu Asn Asn Ile Val Ala Thr Gly Gln
               500                 505                 510
   Thr Asn Phe Tyr Ile Asp Gly Gly Val Ser Arg Asn Arg Asn Gly Val
           515                 520                 525
   Ser Ser Lys Tyr Glu Tyr Gly Val Leu Asp Leu Asp Lys Arg Thr Gln
       530                 535                 540
   Leu Ser Glu Leu Asp Gln Arg Arg Arg Arg Trp Gly Tyr Tyr Pro Asp
   545                 550                 555                 560
   Leu Asp Leu Asp Met Asn Lys Ala Tyr Trp His Arg Phe Asp Met Phe
                   565                 570                 575
   Ala Ser Lys Asn Thr Gly Arg Ser Thr Ile Lys Asp Thr Glu Ile Asn
               580                 585                 590
   Ile Ser Asn Ser Lys Ile Asn Leu Lys Asn Gly Phe Val His Leu Leu
           595                 600                 605
   Ala Glu Lys Ile Lys Leu Asp Asn Ser Lys Ile Asp Ile Thr Phe Asp
       610                 615                 620
   Lys Asp Asn Ser Gln Asp Ile Ser Thr Gln Ile Asn Arg Leu Gly Met
   625                 630                 635                 640
   Asn Gly Lys Val Ser Met Val Asn Ser His Ile Lys Ile Val Gly Asp
                   645                 650                 655
   Glu Lys Ile Asp Ile Ser Ala Lys Ala Pro Tyr Ala Thr Met Phe Leu
               660                 665                 670
   Ile Gly Glu Leu Ile Gly Glu Lys Ser Ser Ile Phe Val Lys Ser His
           675                 680                 685
   Gln Gly Tyr Thr Phe Arg Thr Asp Gly Asp Thr Lys Ile Ala Gly Lys
       690                 695                 700
   Asn Ser Lys Asp Asp Leu Lys Ile Thr Ala Ile Asn Thr Gly Gly Arg
   705                 710                 715                 720
   Thr Gly Lys Glu Val Ile Ile Asn Gly Ala Pro Gly Ser Ile Asp Asn
                   725                 730                 735
   Asp Ala Asn Ile Ala Asn Met Ala Phe Thr Ile Gly Asp Asn Ala Asn
               740                 745                 750
   Thr Lys Thr Thr Ile Glu Asn Ala Asp Ile Thr Ala Leu Ala Pro Asn
           755                 760                 765
   Gly Gly Thr Ala Tyr Leu Ser Ser Lys Gly Val Glu Ile Glu Val Asn
       770                 775                 780
   Pro Asn Ser Asn Phe Thr Phe Phe Glu Leu Pro Arg Glu Lys Asn Phe
   785                 790                 795                 800
   Asn Gln Thr Lys Ile Asn Gly Asp Ser Thr Lys Leu Ser Glu Arg Gly
                   805                 810                 815
   Phe Ala Arg Leu Tyr Asp Lys Ile Asn Gly Val Arg Ala Ser Asn Leu
               820                 825                 830
   Ser Ala Glu Gln Leu Asn Val Thr Asp Ser Ser Glu Lys Ile Ile Asn
           835                 840                 845
   Thr Lys Leu Val Ser Ser Leu Asp Val Glu Lys Leu Val Ser Val Ala
       850                 855                 860
   Val Cys Asp Ala Gly Lys Gly Cys Glu Glu Gln Gln Phe Gly Asp Lys
   865 870 875 880
   Gly Asn Asn Thr Lys Val Ser Val Gly Glu Leu Glu Ala Glu Gln
   885 890 895
<210> 15
   <211> 165
   <212> PRT
   <213> Bacteria <prokaryote>
<220>
   <223> NT031
<400> 15
   Cys Ile Ala Pro Pro Lys Gly Leu Glu Lys Glu Arg Phe Ser Ile Asn
   1 5 10 15
   Ser Tyr Arg Glu Ile Ser Pro Gln Asp Leu Thr Cys His Cys Asn Thr
   20 25 30
   Val Arg Leu Gly Gly Lys Ile Val Asn Thr Thr Val Leu Ala Asn Gln
   35 40 45
   Thr Lys Ile Glu Val Leu Ser Leu Pro Val Ser Ser Ile Ser Gly Lys
   50 55 60
   Pro Phe Val Glu Leu Gln Ser Asp Gly Arg Phe Ile Val Tyr Phe Asn
   65 70 75 80
   Gly Phe Val Glu Pro Glu Asn Leu Lys Glu Arg Tyr Ile Thr Val Gly
   85 90 95
   Gly Gln Leu Thr Gly Thr Glu Lys Gly Lys Ile Glu Gln Ala Asp Tyr
   100 105 110
   Thr Tyr Pro Val Val Gln Ala Asp Lys Tyr Arg Ile Trp Thr Leu Ser
   115 120 125
   Thr Thr Tyr Asn Tyr Pro Thr Asp Asp Trp Asp Glu Asp Asp Asp Trp
   130 135 140
   Gly Phe Phe Arg Trp Arg His Arg Pro Trp Tyr Val Gln Pro Glu Ile
   145 150 155 160
   His Tyr Tyr Leu Asn
   165
<210> 16
   <211> 117
   <212> PRT
   <213> Bacteria <prokaryote>
<220>
   <223> NT015
<400> 16
   Ala Gln Asn Ala Asn Val Thr Thr Pro Gln Thr Gln Lys Met Gln Val
   1               5                   10                  15
   Glu Lys Val Asp Lys Ala Leu Gln Lys Gly Glu Ala Asp Arg Tyr Leu
               20                  25                  30
   Cys Gln Asp Asp Lys Val Val Arg Val Val His Ala Thr His Lys Lys
           35                  40                  45
   Tyr Lys Lys Asn Leu His Tyr Val Thr Val Thr Phe Gln Gly Val Ser
       50                  55                  60
   Glu Lys Leu Thr Leu Met Ile Ser Glu Arg Gly Lys Asn Tyr Ala Asn
   65                  70                  75                  80
   Ile Arg Trp Met Trp Gln Glu Arg Asp Asp Phe Ser Thr Leu Lys Thr
                   85                  90                  95
   Asn Leu Gly Glu Ile Leu Ala Thr Gln Cys Val Ser Gln Thr Ser Glu
               100                 105                 110
   Arg Leu Ser Gly Gln
           115
<210> 17
   <211> 134
   <212> PRT
   <213> Bacteria <prokaryote>
<220>
   <223> NT023
<400> 17
   Asn Thr Asp Ile Phe Ser Gly Asp Val Tyr Ser Ala Ser Gln Ala Lys
   1               5                   10                  15
   Glu Ala Arg Ser Ile Thr Tyr Gly Thr Ile Val Ser Val Arg Pro Val
               20                  25                  30
   Lys Ile Gln Ala Asp Asn Gln Gly Val Val Gly Thr Leu Gly Gly Gly
           35                  40                  45
   Ala Leu Gly Gly Ile Ala Gly Ser Thr Ile Gly Gly Gly Arg Gly Gln
       50                  55                  60
   Ala Ile Ala Ala Val Val Gly Ala Ile Gly Gly Ala Ile Ala Gly Ser
   65                  70                  75                  80
   Lys Ile Glu Glu Lys Met Ser Gln Val Asn Gly Ala Glu Leu Val Ile
                   85                  90                  95
   Lys Lys Asp Asp Gly Gln Glu Ile Val Val Val Gln Lys Ala Asp Ser
               100                 105                 110
   Ser Phe Val Ala Gly Arg Arg Val Arg Ile Val Gly Gly Gly Ser Ser
           115                 120                 125
   Leu Asn Val Ser Val Leu
       130
<210> 18
   <211> 315
   <212> PRT
   <213> Bacteria <prokaryote>
<220>
   <223> NT100
<400> 18
   Ile Lys Lys Asn Glu Asn Asn Asn Ala Tyr Gln Phe Asn His Leu Lys
   1               5                   10                  15
   Thr Leu Gly Leu Tyr Ile Gln Asn Thr Thr Tyr Phe Thr Asp Asn Phe
               20                  25                  30
   Ile Ile Thr Gly Gly Leu Arg Tyr Glu Tyr Phe Asp Gln Val Val Gly
           35                  40                  45
   Arg Ser Thr Leu Lys Asn Ile Arg Ser Gly Tyr Leu Ala Gln Lys Asp
       50                  55                  60
   Gly Lys Leu Leu Tyr Gln Leu Gly Ser Val Tyr Lys Phe Thr Pro Asn
   65                  70                  75                  80
   Ile Ala Thr Phe Phe Asn His Ala Glu Ser Phe Arg Pro Gln Asn Asn
                   85                  90                  95
   Arg Thr Leu Ile Ile Asn Gly Glu Leu Pro Ala Glu Gln Gly Lys Ser
               100                 105                 110
   Phe Glu Thr Gly Leu Lys Tyr Glu Asn Ala Tyr Leu Asn Ala Thr Val
           115                 120                 125
   Ala Leu Phe Asn Ile Asn Lys Arg Asn Val Ala Glu Thr Val Asn Val
       130                 135                 140
   Asn Gly Thr Asn Glu Leu Gln Ile Val Gly Lys Gln Arg Ser Arg Gly
   145                 150                 155                 160
   Ile Glu Phe Asp Leu Asn Gly Gln Leu Thr Asp Asn Leu Ser Ile Ala
                   165                 170                 175
   Ala Asn Tyr Thr Tyr Thr Lys Val Lys Asn Leu Glu Asn His Asn Asn
               180                 185                 190
   Lys Leu Ala Val Gly Lys Gln Leu Ser Gly Val Pro Lys His Gln Ala
           195                 200                 205
   Ser Leu Phe Leu Ala Tyr Asn Ile Gly Glu Phe Asp Phe Gly Asn Ile
       210                 215                 220
   Arg Val Gly Gly Gly Ala Arg Tyr Leu Gly Ser Trp Tyr Ala Tyr Asn
   225                 230                 235                 240
   Asn Thr Tyr Thr Lys Ala Tyr Lys Leu Pro Gln Ala Ile Val Tyr Asp
                   245                 250                 255
   Ala Phe Ile Ala Tyr Asp Thr Lys Ile Ser Gly Lys Lys Val Ser Phe
               260                 265                 270
   Gln Leu Asn Gly Lys Asn Leu Ser Asn Lys Val Tyr Ser Pro Ser Thr
           275                 280                 285
   Ser Gly Asn Ala Ser Arg Thr Leu Ile Pro Val Ala Leu Gly Tyr Ala
       290                 295                 300
   Arg Glu Val Ile Leu Asn Thr Lys Ile Glu Phe
   305                 310                 315
<210> 19
   <211> 212
   <212> PRT
   <213> Bacteria <prokaryote>
<220>
   <223> NT040
<400> 19
   Ser Ile Ser His Phe Tyr Val Gln Ile Gln Thr Gln Asn Gln Gln Met
   1               5                   10                  15
   Leu Leu His Leu Lys Leu Gln Ala Glu Leu Gln Arg Thr Leu Gln Leu
               20                  25                  30
   Ile Gly Lys Asp Leu Arg Arg Leu Gly Phe Arg Ala Leu Asn Ala Lys
           35                  40                  45
   Leu Thr Glu Ser Asn Leu Ser Leu Phe Glu Leu Asp Glu Gln Gly Thr
       50                  55                  60
   Ala Ile Phe Ile Ser Gln Glu Asp Asn Ala Pro Pro Asn Ser Cys Val
   65                  70                  75                  80
   Leu Phe Phe Tyr Asp Leu Asn Lys Asn Gly Cys Ile Gly Lys Gly Ser
                   85                  90                  95
   Pro Lys Thr Cys Met Lys Lys Gly Lys Asn Thr Ser Lys Ser Ser Thr
               100                 105                 110
   Glu Glu Leu Phe Gly Tyr Lys Val Ser Asn Lys Met Ile Lys Thr Lys
           115                 120                 125
   Leu Thr Tyr Gln Ser Val Ile Pro Thr Asn Cys Thr Ala Glu Thr Cys
       130                 135                 140
   Lys Arg Ala Phe Gln Gln Thr Ala Cys Asn Ala Gly Gly Gly Trp Ala
   145                 150                 155                 160
   Asp Leu Leu Asp Asn Asn Glu Tyr Glu Ile Thr Arg Leu Gln Phe Asn
                   165                 170                 175
   Trp Leu Ile Glu Gly Lys Gly Leu Glu Ile Lys Leu Lys Gly Asn Leu
               180                 185                 190
   Lys Gln Thr Pro Asn Ile Ser Tyr Glu Thr Ser Leu Val Val Ala Leu
           195                 200                 205
   Trp Asn Gln Lys
       210
<210> 20
   <211> 630
   <212> PRT
   <213> Bacteria <prokaryote>
<220>
   <223> NT048
<400> 20
   Ser Gly Gly Gly Gly Ser Phe Asp Val Asp Asp Val Ser Asn Pro Ser
   1               5                   10                  15
   Ser Ser Lys Pro Arg Tyr Gln Asp Asp Thr Ser Ser Ser Arg Thr Lys
               20                  25                  30
   Ser Lys Leu Glu Asn Leu Ser Ile Pro Ser Leu Gly Gly Gly Met Lys
           35                  40                  45
   Leu Val Ala Gln Asn Leu Arg Asp Arg Thr Lys Pro Ser Leu Leu Asn
       50                  55                  60
   Glu Asp Asp Tyr Met Ile Phe Ser Ser Leu Ser Thr Ile Lys Ala Asp
   65                  70                  75                  80
   Val Glu Lys Glu Asn Lys His Tyr Thr Ser Pro Val Gly Ser Ile Asp
                   85                  90                  95
   Glu Pro Ser Thr Thr Asn Pro Lys Glu Asn Asp His Gly Gln Arg Tyr
               100                 105                 110
   Val Tyr Ser Gly Leu Tyr Tyr Ile Pro Ser Trp Asn Leu Asn Asp Leu
           115                 120                 125
   Lys Asn Asn Lys Tyr Tyr Tyr Ser Gly Tyr Tyr Gly Tyr Ala Tyr Tyr
       130                 135                 140
   Phe Gly Lys Gln Thr Ala Thr Thr Leu Pro Val Asn Gly Lys Val Thr
   145                 150                 155                 160
   Tyr Lys Gly Thr Trp Ser Phe Ile Thr Ala Ala Glu Asn Gly Lys Arg
                   165                 170                 175
   Tyr Pro Leu Leu Ser Asn Gly Ser Gln Ala Tyr Phe Arg Arg Ser Ala
               180                 185                 190
   Ile Pro Glu Asp Ile Asp Leu Glu Val Lys Asn Asp Glu Asn Arg Glu
           195                 200                 205
   Lys Gly Leu Val Ser Glu Phe Ser Ala Asp Phe Gly Thr Lys Lys Leu
       210                 215                 220
   Thr Gly Gly Leu Phe Tyr Thr Lys Arg Gln Thr His Ile Gln Asn His
   225                 230                 235                 240
   Glu Lys Lys Lys Leu Tyr Asp Ile Asp Ala His Ile Tyr Ser Asn Arg
                   245                 250                 255
   Phe Arg Gly Lys Val Asn Pro Thr Gln Lys Asp Ser Lys Glu His Pro
               260                 265                 270
   Phe Thr Ser Glu Gly Thr Leu Glu Gly Gly Phe Tyr Gly Pro Glu Gly
           275                 280                 285
   Gln Glu Leu Gly Gly Lys Phe Leu Ala Gly Asp Lys Lys Val Phe Gly
       290                 295                 300
   Val Phe Ser Ala Lys Gly Thr Glu Glu Asn Lys Lys Leu Pro Lys Glu
   305                 310                 315                 320
   Thr Leu Ile Asp Gly Lys Leu Thr Thr Phe Ser Thr Lys Thr Thr Asp
                   325                 330                 335
   Ala Lys Thr Asn Ala Thr Ala Asn Ala Thr Thr Ser Thr Ala Ala Asn
               340                 345                 350
   Thr Thr Thr Asp Thr Thr Ala Asn Thr Ile Thr Asp Ala Glu Asn Phe
           355                 360                 365
   Lys Thr Lys Asp Ile Ser Ser Phe Gly Glu Ala Asp Tyr Leu Leu Ile
       370                 375                 380
   Asp Asn Tyr Pro Val Pro Leu Leu Pro Glu Ser Gly Asp Phe Ile Ser
   385                 390                 395                 400
   Ser Lys His His Thr Val Gly Lys Lys Thr Tyr Gln Val Lys Ala Cys
                   405                 410                 415
   Cys Ser Asn Leu Ser Tyr Val Lys Phe Gly Met Tyr Tyr Glu Val Pro
               420                 425                 430
   Pro Lys Glu Glu Glu Lys Asp Lys Glu Lys Lys Glu Lys Glu Lys Glu
           435                 440                 445
   Lys Gln Ala Thr Asn Leu Ser Asn Thr Tyr Tyr Gln Phe Leu Leu Gly
       450                 455                 460
   Leu Arg Thr Pro Ser Ser Glu Ile Pro Lys Gly Gly Ser Ala Lys Tyr
   465                 470                 475                 480
   Leu Gly Ser Trp Phe Gly Tyr Leu Ser Asp Gly Ser Thr Ser Tyr Ser
                   485                 490                 495
   Pro Ser Gly Asp Lys Lys Arg Glu Asn Asn Ala Leu Ala Glu Phe Asn
               500                 505                 510
   Val Asn Phe Ala Asp Lys Thr Leu Lys Gly Gln Leu Lys Arg His Asp
   515 520 525
   Asn Gln Asn Thr Val Phe Thr Ile Asp Ala Thr Phe Lys Ser Gly Lys
   530 535 540
   Asn Asn Phe Thr Gly Thr Ala Thr Ala Asn Asn Val Ala Ile Asp Pro
   545 550 555 560
   Gln Ser Thr Gln Gly Thr Ser Asn Val Asn Phe Thr Ala Thr Val Asn
   565 570 575
   Gly Ala Phe Tyr Gly Pro Asn Ala Thr Glu Leu Gly Gly Tyr Phe Thr
   580 585 590
   Tyr Asn Gly Asn Pro Thr Asp Lys Ser Ser Ser Thr Val Pro Ser Ser
   595 600 605
   Ser Asn Ser Lys Asn Ala Arg Ala Ala Val Val Phe Gly Ala Arg Gln
   610 615 620
   Gln Val Glu Thr Thr Lys
   625 630
<210> 21
   <211> 157
   <212> PRT
   <213> Bacteria <prokaryote>
<220>
   <223> NT053
<210> 22
   <211> 505
   <212> PRT
   <213> Bacteria <prokaryote>
<220>
   <223> NT066
<400> 22
   Phe Lys Lys Ser Leu Ile Val Ala Ala Ser Phe Ala Ser Leu Ser Leu
   1               5                   10                  15
   Phe Asn Ser Ala Thr Ala Glu Leu Val Tyr Lys Pro Leu Glu Gln Pro
               20                  25                  30
   Val Glu Pro Ala Lys Pro Asp Leu Lys Ile Glu Ser Val Asn Glu Lys
           35                  40                  45
   Phe Ala Glu Lys Tyr Pro Asn Gln Tyr Asn Ser Trp Arg Ser Thr Ala
       50                  55                  60
   Asn Gly Asp Gly Glu Asn Ile Ile Tyr Ala Asp Glu Glu Asn Pro Arg
   65                  70                  75                  80
   Leu Ile Val Leu Trp Gly Gly Tyr Ala Phe Ala Lys Glu Tyr Asn Ala
                   85                  90                  95
   Pro Arg Gly His Phe Tyr Ala Val Thr Asp Val Arg Asn Ile Leu Arg
               100                 105                 110
   Thr Gly Ala Pro Lys Thr Ala Asn Asp Gly Pro Gln Ala Met Ala Cys
           115                 120                 125
   Trp Thr Cys Lys Gly Pro Asp Val Pro Arg Leu Ile Ala Glu Trp Gly
       130                 135                 140
   Glu Lys Asp Tyr Phe Asn Ala Lys Trp Ala Lys Gly Gly Pro Glu Ile
   145                 150                 155                 160
   Val Asn Ser Ile Gly Cys Ala Asp Cys His Asp Thr Thr Ser Lys Asp
                   165                 170                 175
   Phe Ala Glu Gly Lys Pro Ala Leu Arg Ile Ala Arg Pro His Ile Leu
               180                 185                 190
   Arg Ala Leu Asp Ala Leu Glu Lys Ala Thr Ala Glu Lys Asp Lys Ala
           195                 200                 205
   Glu Gly Arg Pro His Asn Asn Leu Ser Phe Asn Thr Ala Ala Arg Thr
       210                 215                 220
   Glu Lys Arg Ala Glu Ile Cys Ala Asn Cys His Val Glu Tyr Tyr Phe
   225                 230                 235                 240
   Ser Gly Asp Ile Lys Gln Val Thr Phe Pro Trp Asp Asn Gly Gln Thr
                   245                 250                 255
   Val Asp Asp Ile Glu Lys Tyr Tyr Asp Asp Ile Gly Phe Thr Asp Trp
               260                 265                 270
   Thr His Ser Leu Ser Lys Ala Pro Met Leu Lys Ala Gln His Pro Asp
           275                 280                 285
   Phe Glu Ile Trp Ser Leu Gly Met His Gly Lys Asn Gly Val Thr Cys
       290                 295                 300
   Val Asp Cys His Met Pro Lys Val Gln Gly Ala Asp Gly Lys Val Tyr
   305                 310                 315                 320
   Thr Asp His Gln Ile Gln Asn Pro Phe Glu Ala Phe Asp Ser Thr Cys
                   325                 330                 335
   Ala Asn Cys His Asp Gln Ser Lys Glu Lys Leu Arg Asp Ile Val Thr
               340                 345                 350
   Ser Arg Lys Lys Glu Val Lys Asp Val Met Gly Arg Leu Glu Asp Gln
           355                 360                 365
   Val Val Lys Ala His Phe Glu Ala Lys Glu Ala Trp Asp Ala Gly Ala
       370                 375                 380
   Thr Lys Lys Glu Met Glu Ala Ala Leu Met Asp Ile Arg His Ala Gln
   385                 390                 395                 400
   Trp Arg Trp Asp Tyr Thr Ala Ala Ser His Gly Gly His Met His Ala
                   405                 410                 415
   Pro Glu Val Val Leu Arg Val Leu Ala Ser Gly Leu Asp Lys Val Ala
               420                 425                 430
   Asp Ala Arg Thr Lys Leu Ala Val Ile Leu Thr Lys His Gly Val Lys
           435                 440                 445
   Thr Pro Val Gln Ile Pro Asp Ile Ser Thr Ala Asp Lys Ala Trp Lys
       450                 455                 460
   Val Met Gly Ile Asp Ile Glu Lys Glu Arg Lys Ala Lys Glu Glu Phe
   465                 470                 475                 480
   Leu Lys Thr Val Val Pro Gln Trp Glu Gln Gln Ala Arg Glu Lys Gly
                   485                 490                 495
   Leu Leu Val Asp Pro Pro Ala Gln Lys
               500                 505
<210> 23
   <211> 429
   <212> PRT
   <213> Bacteria <prokaryote>
<220>
   <223> NT097
<400> 23
   Ala Ala Phe Gln Leu Ala Glu Val Ser Thr Ser Gly Leu Gly Arg Ala
   1               5                   10                  15
   Tyr Ala Gly Glu Ala Ala Ile Ala Asp Asn Ala Ser Val Val Ala Thr
               20                  25                  30
   Asn Pro Ala Leu Met Ser Leu Phe Lys Thr Ala Gln Phe Ser Thr Gly
           35                  40                  45
   Gly Val Tyr Ile Asp Ser Arg Ile Asn Met Asn Gly Asp Val Asn Ser
       50                  55                  60
   Tyr Leu Asn Ser Gly Ser Met Ala Leu Thr Lys Tyr Gly Ser Ala Ser
   65                  70                  75                  80
   Gln Arg Asn Val Val Pro Gly Ala Phe Val Pro Asn Leu Tyr Phe Val
                   85                  90                  95
   Ala Pro Val Asn Asp Lys Phe Ala Leu Gly Ala Gly Met Asn Val Asn
               100                 105                 110
   Phe Gly Leu Lys Ser Glu Tyr Asp Asp Ser Tyr Asp Ala Gly Val Phe
           115                 120                 125
   Gly Gly Lys Thr Asp Leu Asn Ala Ile Asn Leu Asn Leu Ser Gly Ala
       130                 135                 140
   Tyr Arg Val Ile Glu Gly Leu Ser Leu Gly Leu Gly Val Asn Ala Val
   145                 150                 155                 160
   Tyr Ala Asn Ala Gln Val Glu Arg Asn Ala Gly Ile Ile Ala Asp Ser
                   165                 170                 175
   Leu Gln Asp Ser Gln Val Lys Gly Ala Leu Lys Ile Val Asp Ser Thr
               180                 185                 190
   Asn Lys Ala Pro Asp Arg Leu Thr Ser Lys Asp Lys Ser Val Val Ser
           195                 200                 205
   Leu Gln Asp Arg Ala Ala Trp Gly Phe Gly Trp Asn Ala Gly Val Met
       210                 215                 220
   Tyr Gln Phe Asn Glu Ala Asn Arg Ile Gly Leu Ala Tyr His Ser Lys
   225                 230                 235                 240
   Val Asp Ile Asp Phe Ala Asp Arg Thr Ala Thr Ser Phe Gly Lys Lys
                   245                 250                 255
   Asp Ile Val Ala Gly Lys Thr Gly Asn Leu Thr Phe Thr Leu Pro Asp
               260                 265                 270
   Tyr Leu Glu Leu Ser Gly Phe His Gln Leu Thr Asp Lys Phe Ala Val
           275                 280                 285
   His Tyr Ser Tyr Lys Tyr Thr His Trp Ser Arg Leu Thr Lys Leu His
       290                 295                 300
   Ala Ser Tyr Glu Asn Gly Glu Lys Ala Phe Asp Lys Glu Leu Gln Tyr
   305                 310                 315                 320
   Ser Asn Asn Ser Arg Val Ala Leu Gly Ala Ser Tyr Asn Leu Asp Glu
                   325                 330                 335
   Lys Leu Thr Leu Arg Ala Gly Ile Ala Tyr Asp Gln Ala Ala Ser Arg
               340                 345                 350
   His Gln Arg Ser Ala Ala Ile Pro Asp Thr Asp Arg Thr Trp Tyr Ser
           355                 360                 365
   Leu Gly Gly Thr Tyr Lys Phe Thr Pro Asn Leu Ser Val Asp Leu Gly
       370                 375                 380
   Tyr Ala Tyr Leu Lys Gly Lys Lys Val His Phe Lys Glu Val Gln Lys
   385                 390                 395                 400
   Ala Ala Gly Gly His Ile Thr Thr Thr Ala Asn Tyr Thr Ser Gln Ala
                   405                 410                 415
   His Ala Asn Leu Tyr Gly Leu Asn Leu Asn Tyr Ser Phe
               420                 425
<210> 24
   <211> 422
   <212> PRT
   <213> Bacteria <prokaryote>
<220>
   <223> P48 (NT007)
<400> 24
   Val Asn Gln Val Ala Ile Leu Gly Glu Glu Tyr Val Gly Met Arg Pro
   1 5 10 15
   Ser Met Lys Val Arg Glu Gly Asp Val Val Lys Lys Gly Gln Val Leu
   20 25 30
   Phe Glu Asp Lys Lys Asn Pro Gly Val Ile Phe Thr Ala Pro Ala Ser
   35 40 45
   Gly Thr Ile Thr Ala Ile Asn Arg Gly Glu Lys Arg Val Leu Gln Ser
   50 55 60
   Val Val Ile Asn Val Glu Gly Asp Glu Lys Ile Thr Phe Ala Lys Tyr
   65 70 75 80
   Ser Thr Glu Gln Leu Asn Thr Leu Ser Ser Glu Gln Val Lys Gln Asn
   85 90 95
   Leu Ile Glu Ser Gly Leu Trp Thr Ala Leu Arg Thr Arg Pro Phe Ser
   100 105 110
   Lys Val Pro Ser Ile Glu Ser Glu Ala Ser Ser Ile Phe Val Asn Ala
   115 120 125
   Met Asp Thr Asn Pro Leu Ala Ala Asp Pro Ser Val Val Leu Lys Glu
   130 135 140
   Tyr Ser Gln Asp Phe Thr Asn Gly Leu Thr Val Leu Ser Arg Leu Phe
   145 150 155 160
   Pro Ser Lys Pro Leu His Leu Cys Lys Ala Gly Asp Ser Asn Ile Pro
   165 170 175
   Thr Ala Asp Leu Glu Asn Leu Gln Ile His Asp Phe Thr Gly Val His
   180 185 190
   Pro Ala Gly Leu Val Gly Thr His Ile His Phe Ile Asp Pro Val Gly
   195 200 205
   Ile Gln Lys Thr Val Trp His Ile Asn Tyr Gln Asp Val Ile Ala Val
   210 215 220
   Gly Lys Leu Phe Thr Thr Gly Glu Leu Tyr Ser Glu Arg Val Ile Ser
   225 230 235 240
   Leu Ala Gly Pro Gln Val Lys Glu Pro Arg Leu Val Arg Thr Ile Ile
   245 250 255
   Gly Ala Asn Leu Ser Gln Leu Thr Gln Asn Glu Leu Ser Ala Gly Lys
   260 265 270
   Asn Arg Val Ile Ser Gly Ser Val Leu Cys Gly Gln Ile Ala Lys Asp
   275 280 285
   Ser His Asp Tyr Leu Gly Arg Tyr Ala Leu Gln Val Ser Val Ile Ala
   290 295 300
   Glu Gly Asn Glu Lys Glu Phe Phe Gly Trp Ile Met Pro Gln Ala Asn
   305 310 315 320
   Lys Tyr Ser Val Thr Arg Thr Val Leu Gly His Phe Ser Lys Lys Leu
   325 330 335
   Phe Asn Phe Thr Thr Ser Glu Asn Gly Gly Glu Arg Ala Met Val Pro
   340 345 350
   Ile Gly Ser Tyr Glu Arg Val Met Pro Leu Asp Ile Leu Pro Thr Leu
   355 360 365
   Leu Leu Arg Asp Leu Ile Val Gly Asp Thr Asp Gly Ala Gln Glu Leu
   370 375 380
   Gly Cys Leu Glu Leu Asp Glu Glu Asp Leu Ala Leu Cys Ser Phe Val
   385 390 395 400
   Cys Pro Gly Lys Tyr Glu Tyr Gly Ser Ile Leu Arg Gln Val Leu Asp
   405 410 415
   Lys Ile Glu Lys Glu Gly
   420
<210> 25
   <211> 437
   <212> PRT
   <213> Bacteria <prokaryote>
<220>
   <223> HtrA (NT006)
<400> 25
   Thr Leu Pro Ser Phe Val Ser Glu Gln Asn Ser Leu Ala Pro Met Leu
   1               5                   10                  15
   Glu Lys Val Gln Pro Ala Val Val Thr Leu Ser Val Glu Gly Lys Ala
               20                  25                  30
   Lys Gly Asp Ser Arg Ser Pro Phe Leu Asp Asp Ile Pro Glu Glu Phe
           35                  40                  45
   Lys Phe Phe Phe Gly Asp Arg Phe Ala Glu Gln Phe Gly Gly Arg Gly
       50                  55                  60
   Glu Ser Lys Arg Asn Phe Arg Gly Leu Gly Ser Gly Val Ile Ile Asn
   65                  70                  75                  80
   Ala Ser Lys Gly Tyr Val Leu Thr Asn Asn His Val Ile Asp Gly Ala
                   85                  90                  95
   Asp Lys Ile Thr Val Gln Leu Gln Asp Gly Arg Glu Phe Lys Ala Lys
               100                 105                 110
   Leu Val Gly Lys Asp Glu Gln Ser Asp Ile Ala Leu Val Gln Leu Glu
           115                 120                 125
   Lys Ser Ser Asn Leu Thr Glu Ile Lys Phe Ala Asp Ser Asp Lys Leu
       130                 135                 140
   Arg Val Gly Asp Phe Thr Val Ala Ile Gly Asn Pro Phe Gly Leu Gly
   145                 150                 155                 160
   Gln Thr Val Thr Ser Gly Val Val Ser Ala Leu Gly Arg Ser Thr Gly
                   165                 170                 175
   Ser Asp Ser Gly Thr Tyr Glu Asn Tyr Ile Gln Thr Asp Ala Ala Val
               180                 185                 190
   Asn Arg Gly Asn Ser Gly Gly Ala Leu Val Asn Leu Asn Gly Glu Leu
           195                 200                 205
   Ile Gly Ile Asn Thr Ala Ile Ile Ser Pro Ser Gly Gly Asn Ala Gly
       210                 215                 220
   Ile Ala Phe Ala Ile Pro Ser Asn Gln Ala Ser Asn Leu Val Gln Gln
   225                 230                 235                 240
   Ile Leu Glu Phe Gly Gln Val Arg Arg Gly Leu Leu Gly Ile Lys Gly
                   245                 250                 255
   Gly Glu Leu Asn Ala Asp Leu Ala Lys Ala Phe Asn Val Ser Ala Gln
               260                 265                 270
   Gln Gly Ala Phe Val Ser Glu Val Leu Pro Lys Ser Ala Ala Glu Lys
           275                 280                 285
   Ala Gly Leu Lys Ala Gly Asp Ile Ile Thr Ala Met Asn Gly Gln Lys
       290                 295                 300
   Ile Ser Ser Phe Ala Glu Met Arg Ala Lys Ile Ala Thr Thr Gly Ala
   305                 310                 315                 320
   Gly Lys Glu Ile Ser Leu Thr Tyr Leu Arg Asp Gly Lys Ser His Asp
                   325                 330                 335
   Val Lys Val Lys Leu Gln Ala Asp Asp Gly Ser Gln Leu Ser Ser Lys
               340                 345                 350
   Thr Glu Leu Leu Ala Leu Asp Gly Ala Thr Leu Lys Asp Tyr Asp Thr
           355                 360                 365
   Lys Gly Val Lys Gly Ile Glu Ile Thr Lys Ile Gln Pro Asn Ser Leu
       370                 375                 380
   Ala Ala Gln Arg Gly Leu Lys Ala Gly Asp Ile Ile Ile Gly Ile Asn
   385                 390                 395                 400
   Arg Gln Met Ile Glu Asn Ile Arg Glu Leu Asn Lys Val Leu Glu Thr
                   405                 410                 415
   Glu Pro Ser Ala Ile Ala Leu Asn Ile Leu Arg Gly Asn Ser Asn Phe
               420                 425                 430
   Tyr Leu Leu Val Gln
           435
<210> 26
   <211> 144
   <212> PRT
   <213> Bacteria <prokaryote>
<220>
   <223> PE (NT035)
<400> 26
   Ser Ala Gln Ile Gln Lys Ala Glu Gln Asn Asp Val Lys Leu Ala Pro
   1               5                   10                  15
   Pro Thr Asp Val Arg Ser Gly Tyr Ile Arg Leu Val Lys Asn Val Asn
               20                  25                  30
   Tyr Tyr Ile Asp Ser Glu Ser Ile Trp Val Asp Asn Gln Glu Pro Gln
           35                  40                  45
   Ile Val His Phe Asp Ala Val Val Asn Leu Asp Lys Gly Leu Tyr Val
       50                  55                  60
   Tyr Pro Glu Pro Lys Arg Tyr Ala Arg Ser Val Arg Gln Tyr Lys Ile
   65                  70                  75                  80
   Leu Asn Cys Ala Asn Tyr His Leu Thr Gln Val Arg Thr Asp Phe Tyr
                   85                  90                  95
   Asp Glu Phe Trp Gly Gln Gly Leu Arg Ala Ala Pro Lys Lys Gln Lys
               100                 105                 110
   Lys His Thr Leu Ser Leu Thr Pro Asp Thr Thr Leu Tyr Asn Ala Ala
           115                 120                 125
   Gln Ile Ile Cys Ala Asn Tyr Gly Lys Ala Phe Ser Val Asp Lys Lys
       130                 135                 140
<210> 27
   <211> 197
   <212> PRT
   <213> Bacteria <prokaryote>
<220>
   <223> P26 (NT010)
<400> 27
   Met Lys Asn Ile Ala Lys Val Thr Ala Leu Ala Leu Gly Ile Ala Leu
   1               5                   10                  15
   Ala Ser Gly Tyr Ala Ser Ala Glu Glu Lys Ile Ala Phe Ile Asn Ala
               20                  25                  30
   Gly Tyr Ile Phe Gln His His Pro Asp Arg Gln Ala Val Ala Asp Lys
           35                  40                  45
   Leu Asp Ala Glu Phe Lys Pro Val Ala Glu Lys Leu Ala Ala Ser Lys
       50                  55                  60
   Lys Glu Val Asp Asp Lys Ile Ala Ala Ala Arg Lys Lys Val Glu Ala
   65                  70                  75                  80
   Lys Val Ala Ala Leu Glu Lys Asp Ala Pro Arg Leu Arg Gln Ala Asp
                   85                  90                  95
   Ile Gln Lys Arg Gln Gln Glu Ile Asn Lys Leu Gly Ala Ala Glu Asp
               100                 105                 110
   Ala Glu Leu Gln Lys Leu Met Gln Glu Gln Asp Lys Lys Val Gln Glu
           115                 120                 125
   Phe Gln Ala Gln Asn Glu Lys Arg Gln Ala Glu Glu Arg Gly Lys Leu
       130                 135                 140
   Leu Asp Ser Ile Gln Thr Ala Thr Asn Asn Leu Ala Lys Ala Lys Gly
   145                 150                 155                 160
   Tyr Thr Tyr Val Leu Asp Ala Asn Ser Val Val Phe Ala Val Glu Gly
                   165                 170                 175
   Lys Asp Ile Thr Glu Glu Val Leu Lys Ser Ile Pro Ala Ser Glu Lys
               180                 185                 190
   Ala Gln Glu Lys Lys
               195
<210> 28
   <211> 346
   <212> PRT
   <213> Bacteria <prokaryote>
<220>
   <223> PHiD (NT080)
<400> 28
   Cys Ser Ser His Ser Ser Asn Met Ala Asn Thr Gln Met Lys Ser Asp
   1               5                   10                  15
   Lys Ile Ile Ile Ala His Arg Gly Ala Ser Gly Tyr Leu Pro Glu His
               20                  25                  30
   Thr Leu Glu Ser Lys Ala Leu Ala Phe Ala Gln Gln Ala Asp Tyr Leu
           35                  40                  45
   Glu Gln Asp Leu Ala Met Thr Lys Asp Gly Arg Leu Val Val Ile His
       50                  55                  60
   Asp His Phe Leu Asp Gly Leu Thr Asp Val Ala Lys Lys Phe Pro His
   65                  70                  75                  80
   Arg His Arg Lys Asp Gly Arg Tyr Tyr Val Ile Asp Phe Thr Leu Lys
                   85                  90                  95
   Glu Ile Gln Ser Leu Glu Met Thr Glu Asn Phe Glu Thr Lys Asp Gly
               100                 105                 110
   Lys Gln Ala Gln Val Tyr Pro Asn Arg Phe Pro Leu Trp Lys Ser His
           115                 120                 125
   Phe Arg Ile His Thr Phe Glu Asp Glu Ile Glu Phe Ile Gln Gly Leu
       130                 135                 140
   Glu Lys Ser Thr Gly Lys Lys Val Gly Ile Tyr Pro Glu Ile Lys Ala
   145                 150                 155                 160
   Pro Trp Phe His His Gln Asn Gly Lys Asp Ile Ala Ala Glu Thr Leu
                   165                 170                 175
   Lys Val Leu Lys Lys Tyr Gly Tyr Asp Lys Lys Thr Asp Met Val Tyr
               180                 185                 190
   Leu Gln Thr Phe Asp Phe Asn Glu Leu Lys Arg Ile Lys Thr Glu Leu
           195                 200                 205
   Leu Pro Gln Met Gly Met Asp Leu Lys Leu Val Gln Leu Ile Ala Tyr
       210                 215                 220
   Thr Asp Trp Lys Glu Thr Gln Glu Lys Asp Pro Lys Gly Tyr Trp Val
   225                 230                 235                 240
   Asn Tyr Asn Tyr Asp Trp Met Phe Lys Pro Gly Ala Met Ala Glu Val
                   245                 250                 255
   Val Lys Tyr Ala Asp Gly Val Gly Pro Gly Trp Tyr Met Leu Val Asn
               260                 265                 270
   Lys Glu Glu Ser Lys Pro Asp Asn Ile Val Tyr Thr Pro Leu Val Lys
           275                 280                 285
   Glu Leu Ala Gln Tyr Asn Val Glu Val His Pro Tyr Thr Val Arg Lys
       290                 295                 300
   Asp Ala Leu Pro Glu Phe Phe Thr Asp Val Asn Gln Met Tyr Asp Ala
   305                 310                 315                 320
   Leu Leu Asn Lys Ser Gly Ala Thr Gly Val Phe Thr Asp Phe Pro Asp
                   325                 330                 335
   Thr Gly Val Glu Phe Leu Lys Gly Ile Lys
               340                 345
<210> 29
   <211> 134
   <212> PRT
   <213> Bacteria <prokaryote>
<220>
   <223> P6 (NT081)
<400> 29
   Cys Ser Ser Ser Asn Asn Asp Ala Ala Gly Asn Gly Ala Ala Gln Thr
   1               5                   10                  15
   Phe Gly Gly Tyr Ser Val Ala Asp Leu Gln Gln Arg Tyr Asn Thr Val
               20                  25                  30
   Tyr Phe Gly Phe Asp Lys Tyr Asp Ile Thr Gly Glu Tyr Val Gln Ile
           35                  40                  45
   Leu Asp Ala His Ala Ala Tyr Leu Asn Ala Thr Pro Ala Ala Lys Val
       50                  55                  60
   Leu Val Glu Gly Asn Thr Asp Glu Arg Gly Thr Pro Glu Tyr Asn Ile
   65                  70                  75                  80
   Ala Leu Gly Gln Arg Arg Ala Asp Ala Val Lys Gly Tyr Leu Ala Gly
                   85                  90                  95
   Lys Gly Val Asp Ala Gly Lys Leu Gly Thr Val Ser Tyr Gly Glu Glu
               100                 105                 110
   Lys Pro Ala Val Leu Gly His Asp Glu Ala Ala Tyr Ser Lys Asn Arg
           115                 120                 125
   Arg Ala Val Leu Ala Tyr
       130
<210> 30
   <211> 433
   <212> PRT
   <213> Bacteria <prokaryote>
<220>
   <223> NT013
<400> 30
   Ser Glu Glu Asn Pro Ile Phe Ser Thr Ser Asp Ser Gly Glu Tyr His
   1               5                   10                  15
   Glu Leu Asn Thr Ser Pro Asn Lys Asn Ser Thr Ala Leu Gln Pro Asp
               20                  25                  30
   Glu Asp Ala Thr Ser Tyr Asp Asp Glu Leu Gln Ala Lys Asp Asp Glu
           35                  40                  45
   Val Asp Glu Val Lys Leu Ser Ser Asp Asp Leu Gly Thr Leu Pro Gln
       50                  55                  60
   His Ala Gln Asp Ala Leu Asn Gly Leu Leu Asp Ala Ala Asp Gln Ala
   65                  70                  75                  80
   Ile Arg Ile Thr Asp Gln Phe Ser Tyr Thr Val Thr Glu Gly Asp Thr
                   85                  90                  95
   Leu Lys Asp Val Leu Val Leu Ser Gly Leu Asp Asp Ser Ser Val Gln
               100                 105                 110
   Pro Leu Ile Lys Leu Asp Pro Glu Leu Ala His Leu Lys Ala Gly Gln
           115                 120                 125
   Gln Phe Tyr Trp Ile Leu Asn Lys Asn Asp Asn Leu Glu Tyr Leu Asn
       130                 135                 140
   Trp Leu Val Ser Glu Lys Glu Glu Arg Ile Tyr Glu Arg Leu Glu Asp
   145                 150                 155                 160
   Gly Lys Phe Lys Arg Gln Val Ile Glu Lys Lys Ser Ile Trp Arg Lys
                   165                 170                 175
   Glu Val Leu Lys Gly Glu Ile Gln Asn Ser Leu Asn Ser Ser Leu Arg
               180                 185                 190
   Glu Gln Gly Leu Asp Thr Arg Gln Ile Ser Gln Leu Ser Asn Ala Leu
           195                 200                 205
   Gln Trp Gln Val Ser Leu Arg Lys Leu Lys Lys Gly Thr Gln Phe Ala
       210                 215                 220
   Ile Leu Val Ser Arg Glu Tyr Leu Gly Asp Lys Leu Thr Gly Gln Gly
   225                 230                 235                 240
   Asn Val Glu Ala Leu Arg Ile Ser Ser Gly Gly Lys Asn Tyr Tyr Ala
                   245                 250                 255
   Val Gln Ala Ala Asn Gly Arg Tyr Tyr Asn Gln Gln Gly Glu Thr Leu
                   260                 265                 270
   Gly Lys Gly Phe Ala Arg Tyr Pro Leu Gln Arg Gln Ala Arg Val Ser
           275                 280                 285
   Ser Pro Phe Asn Pro Asn Arg Arg His Pro Val Thr Gly Arg Val Arg
       290                 295                 300
   Pro His Lys Gly Val Asp Phe Ser Val Ser Gln Gly Thr Pro Val Ile
   305                 310                 315                 320
   Ala Pro Ala Asp Gly Thr Val Glu Lys Val Ala Tyr Gln Ala Gly Gly
                   325                 330                 335
   Ala Gly Arg Tyr Val Met Leu Arg His Gly Arg Glu Tyr Gln Thr Val
               340                 345                 350
   Tyr Met His Leu Ser Lys Ser Leu Val Lys Ala Gly Gln Thr Val Lys
           355                 360                 365
   Lys Gly Glu Arg Ile Ala Leu Ser Gly Asn Thr Gly Ile Ser Thr Gly
       370                 375                 380
   Pro His Leu His Tyr Glu Phe Arg Ile Asn Gly Arg Ala Val Asn Pro
   385                 390                 395                 400
   Leu Thr Val Lys Leu Pro Gly Thr Ser Ser Gly Met Thr Ser Ala Glu
                   405                 410                 415
   Arg Lys Gln Phe Leu Val Arg Val Arg Glu Ala Glu Lys Met Leu Lys
               420                 425                 430
   Pro
<210> 31
   <211> 192
   <212> PRT
   <213> Bacteria <prokaryote>
<220>
   <223> NT106
<400> 31
   Ser Ser Asn Pro Glu Thr Leu Lys Ala Thr Asn Asp Ser Phe Gln Lys
   1               5                   10                  15
   Ser Glu Thr Ser Ile Pro His Phe Ser Pro Leu Ala Thr Gly Gly Val
               20                  25                  30
   Gln Leu Pro Lys Ala Asp Asp Ala Tyr Ser Leu Pro Asn Ile Glu Val
           35                  40                  45
   Lys Lys Gly Glu Asp Ile Asp Ile Arg Pro Pro Leu Ile Pro Leu Ala
       50                  55                  60
   Ile Ile Gln Asn Ser Ile Thr Lys Phe Asp Gly Glu Arg Ser Leu Ile
   65                  70                  75                  80
   Val Tyr Pro Lys Gln Gln Ala Lys Leu Tyr Asn Leu Gln Gln Val Lys
                   85                  90                  95
   Arg Leu Leu Lys Asp Glu Gly Ile Ser Ser Thr Thr Asp Gly Ser Ile
               100                 105                 110
   Leu Thr Thr Asp Trp Ala Lys Thr Glu Arg Ile Gly Asp Lys Ser Ile
           115                 120                 125
   Glu Ile Lys Tyr Gln Ile Glu Gln Val Met Thr Ala Asp Val Ser Val
       130                 135                 140
   Leu Thr Val Ser Ile Leu His Met Arg Arg Asp Gly Ile Ile Phe Thr
   145                 150                 155                 160
   Pro Asn Val Ser Asp Lys Gln Tyr Tyr Thr Ser Glu Arg Leu Asn Arg
                   165                 170                 175
   Ile Val Leu Thr Leu Thr Thr Ala Tyr Asn Lys Gln Leu Gln Asp Leu
               180                 185                 190
<210> 32
   <211> 537
   <212> PRT
   <213> Bacteria <prokaryote>
<220>
   <223> NT107
<400> 32
   Gln Pro Asp Thr Gly Ser Leu Asn Arg Glu Leu Glu Gln Arg Arg Ile
   1               5                   10                  15
   Gln Pro Glu Ala Lys Pro Ser Gly Glu Leu Phe Asn Gln Ala Ala Lys
               20                  25                  30
   Ser Pro Tyr Thr Ala Gln Tyr Lys Gln Glu Leu Lys Phe Pro Leu Thr
           35                  40                  45
   Gln Val Gln Ile Leu Asp Arg Asn Asn Gln Glu Val Val Thr Asp Glu
       50                  55                  60
   Leu Ala His Ile Leu Lys Asn Tyr Val Gly Lys Glu Val Ser Leu Ser
   65                  70                  75                  80
   Asp Leu Ser Asn Leu Ala Asn Glu Ile Ser Glu Phe Tyr Arg Asn Asn
                   85                  90                  95
   Asn Tyr Leu Val Ala Lys Ala Ile Leu Pro Pro Gln Glu Ile Glu Gln
               100                 105                 110
   Gly Thr Val Lys Ile Leu Leu Leu Lys Gly Asn Val Gly Glu Ile Arg
           115                 120                 125
   Leu Gln Asn His Ser Ala Leu Ser Asn Lys Phe Val Ser Arg Leu Ser
       130                 135                 140
   Asn Thr Thr Val Asn Thr Ser Glu Phe Ile Leu Lys Asp Glu Leu Glu
   145                 150                 155                 160
   Lys Phe Ala Leu Thr Ile Asn Asp Val Pro Gly Val Asn Ala Gly Leu
                   165                 170                 175
   Gln Leu Ser Ala Gly Lys Lys Val Gly Glu Ala Asn Leu Leu Ile Lys
               180                 185                 190
   Ile Asn Asp Ala Lys Arg Phe Ser Ser Tyr Val Ser Val Asp Asn Gln
           195                 200                 205
   Gly Asn Lys Tyr Thr Gly Arg Tyr Arg Leu Ala Ala Gly Thr Lys Val
       210                 215                 220
   Asn Asn Leu Thr Gly Trp Gly Asp Glu Leu Lys Leu Asp Leu Leu Ser
   225                 230                 235                 240
   Ser Asn Gln Ala Asn Leu Lys Asn Ala Arg Ile Asp Tyr Ser Ser Leu
                   245                 250                 255
   Ile Asp Gly Tyr Ser Thr Arg Phe Gly Val Thr Ala Asn Tyr Leu His
               260                 265                 270
   Tyr Lys Leu Gly Gly Asn Phe Lys Ser Leu Gln Ser Gln Gly His Ser
           275                 280                 285
   His Asn Leu Gly Ala Tyr Leu Leu His Pro Thr Ile Arg Thr Pro Asn
       290                 295                 300
   Phe Arg Leu Ser Thr Lys Val Ser Phe Asn His Gln Asn Leu Thr Asp
   305                 310                 315                 320
   Glu Gln Gln Ala Val Thr Val Lys Gln Lys Arg Lys Ile Asn Ser Leu
                   325                 330                 335
   Thr Val Gly Ile Asp Gly Ser Trp Asn Leu Ile Lys Asp Gly Thr Thr
               340                 345                 350
   Tyr Phe Ser Leu Ser Thr Leu Phe Gly Asn Leu Ala Asn Gln Thr Asn
           355                 360                 365
   Glu Lys Lys His Asn Ala Lys Glu Asp Phe Gln Pro Gln Ser His Phe
       370                 375                 380
   Thr Val Tyr Asn Tyr Arg Leu Ser His Glu Gln Ile Leu Pro Lys Ser
   385                 390                 395                 400
   Phe Ala Phe Asn Ile Gly Ile Asn Gly Gln Phe Ala Asp Lys Thr Leu
                   405                 410                 415
   Glu Ser Ser Gln Lys Met Leu Leu Gly Gly Leu Ser Gly Val Arg Gly
               420                 425                 430
   His Gln Ala Gly Ala Ala Ser Val Asp Glu Gly His Leu Ile Gln Thr
           435                 440                 445
   Glu Phe Lys His Tyr Leu Pro Val Phe Ser Gln Ser Val Leu Val Ser
       450                 455                 460
   Ser Leu Phe Tyr Asp Tyr Gly Phe Gly Lys Tyr Tyr Lys Asn Ser Gln
   465                 470                 475                 480
   Ser Leu Ala Gln Ser Val Lys Asn Ser Val Lys Leu Gln Ser Val Gly
                   485                 490                 495
   Ala Gly Leu Ser Phe Ser Asp Ala Gly Ser Tyr Ala Ile Asn Val Ser
               500                 505                 510
   Val Ala Lys Pro Leu Asp Asn Asn Ile Asp Asn Ala Asp Lys His Gln
           515                 520                 525
   Phe Trp Leu Ser Met Ile Lys Thr Phe
       530                 535
<210> 33
   <211> 345
   <212> PRT
   <213> Bacteria <prokaryote>
<220>
   <223> NT108
<400> 33
   Thr Ser Asn Ile Lys Asn Ile Gln Ile Pro Thr Thr Leu Asn Gly Ser
   1               5                   10                  15
   Asp Pro Gln Gln Phe Gly Ala Lys Tyr Thr Asn Arg Thr Tyr Gln Gln
               20                  25                  30
   Ala Ala Leu Val Pro Val Ser Asn Ile Glu Asn Gln Ser Ala Val Ile
           35                  40                  45
   Asn Gln Gly Asp Phe Leu Thr Gln Leu Ser Asn Ile Lys Asn Tyr Ser
       50                  55                  60
   Ser Lys Leu Ser Thr Asn Phe Tyr Asp Asn Tyr Glu Lys Ile Thr Asn
   65                  70                  75                  80
   Trp Val Leu Ser Gly Ala Asn Ile Asn Glu Leu Thr Gln Phe Asn Ile
                   85                  90                  95
   His Pro Gln Ile Met Arg Gly Phe Asp Gly Tyr Gln Asn Val Leu Met
               100                 105                 110
   Thr Gly Tyr Tyr Ser Pro Ile Leu Tyr Ala Arg His Thr Pro Gln Gly
           115                 120                 125
   Gln Phe Lys Asn Pro Ile Tyr Arg Met Pro Val Lys Lys Arg Leu Ser
       130                 135                 140
   Arg Ala Gln Ile Tyr Ala Gly Ala Leu Thr Gly Lys Arg Leu Glu Leu
   145                 150                 155                 160
   Ala Tyr Ser Asp Ser Met Leu Glu Asn Phe Leu Leu Gly Val Gln Gly
                   165                 170                 175
   Ser Gly Tyr Val Asp Phe Gly Asp Gly Asn Leu Asn Tyr Phe Ala Tyr
               180                 185                 190
   Ala Gly Gln Asn Gly Tyr Pro Tyr Thr Ala Ile Gly Arg Leu Leu Val
           195                 200                 205
   Glu Asp Gly Glu Ile Pro Lys Glu Lys Met Ser Ile Gln Ala Ile Arg
       210                 215                 220
   Glu Trp Gly Asn Arg Asn Pro Ser Arg Val Gln Ser Leu Leu Glu Arg
   225                 230                 235                 240
   Asn Glu Ala Tyr Val Phe Phe Lys Asn Asp Pro Ser Gly Lys Val Lys
                   245                 250                 255
   Gly Ser Ala Gly Val Pro Leu Val Ala Met Ala Ser Val Ala Ser Asp
               260                 265                 270
   Arg Asn Ile Ile Pro Ser Gly Ser Val Leu Leu Val Glu Val Pro Asp
           275                 280                 285
   Ile Asp Asn Asn Gly Asn Trp Ile Gly Thr His Lys Leu His Leu Met
       290                 295                 300
   Val Ala Leu Asp Val Gly Gly Ala Val Lys Gly His His Phe Asp Leu
   305                 310                 315                 320
   Tyr Arg Gly Ile Gly Ala Arg Ala Gly His Ile Ala Gly Leu Ser Lys
                   325                 330                 335
   His Tyr Gly Arg Val Trp Val Leu Arg
               340                 345
<210> 34
   <211> 534
   <212> PRT
   <213> Bacteria <prokaryote>
<220>
   <223> NT109
<400> 34
   Ser Ser Asn Lys Tyr Asp Ala Glu Ala Ile Asn Ile Ser Gly Ser Leu
   1               5                   10                  15
   Arg Met Gln Ser Tyr Arg Leu Leu Tyr Glu Met Gln Glu Gln Pro Glu
               20                  25                  30
   Ser Val Glu Thr Asn Leu Arg Arg Tyr His Ile Ser Leu His Ser Ser
           35                  40                  45
   Ala Leu Leu Glu Val Gln Asn Gln Phe Phe Thr Pro Asn Val Leu Lys
       50                  55                  60
   His Ser Tyr Gln Asn Ile Leu Gln Arg Trp Thr Asn Met Glu Lys Tyr
   65                  70                  75                  80
   Ala Arg Gln Gln Asp Val Lys Asn Tyr Ser Lys Gln Leu Thr Asn Tyr
                   85                  90                  95
   Val Ala Asp Val Asp Tyr Phe Val Phe Glu Leu Gln Arg Phe Ser Glu
               100                 105                 110
   Gln Lys Trp Ile Leu Gly Val Ser Val Leu Gly Phe Ala Met Leu Leu
           115                 120                 125
   Ile Leu Leu Met Val Ser Tyr Val Ile Trp Tyr Thr Asn Arg Glu Val
       130                 135                 140
   Val Lys Pro Leu His Leu Met Thr Lys Ala Ser Met Gln Val Gln Met
   145                 150                 155                 160
   Arg Gln Phe Asn His Ile Pro Leu Asp Thr Arg Lys Gln Asn Glu Leu
                   165                 170                 175
   Gly Thr Leu Ala Arg Val Phe Thr Gln Met Ser Thr Glu Leu Gly Gln
               180                 185                 190
   Leu Tyr Ser Arg Leu Glu Glu Ala Val Asn Glu Lys Thr Gln Lys Leu
           195                 200                 205
   Arg Gln Thr Asn Arg Thr Leu Ser Thr Leu Tyr Gln Ser Ala Gln Leu
       210                 215                 220
   Leu Asn Thr Asn Thr Ile Asn Asp Lys Ile Leu Asn Gln Val Leu His
   225                 230                 235                 240
   Tyr Ile Phe Ile Ser Asp His Leu Asn Phe Val Lys Val Glu Val Met
                   245                 250                 255
   Gly Ala Glu His Trp Asp Ile Thr Leu Gly Lys Gln Asp Ala Asn Asn
               260                 265                 270
   Glu Leu Gln Ile Glu Thr Leu Ser Val Asp Asn Glu Glu Leu Gly Val
           275                 280                 285
   Leu Ser Trp Gln Ala Gly Leu Pro Cys Pro Asp Pro Arg Ile Met Gln
       290                 295                 300
   Asn Leu Ala Gln Met Leu Ala Arg Ala Leu Tyr Phe His Lys Asn Leu
   305                 310                 315                 320
   Arg Gln Lys Glu Gln Ile Leu Leu Met Glu Glu Arg Ser Ile Ile Ala
                   325                 330                 335
   Arg Glu Leu His Asp Ser Leu Ala Gln Val Leu Ser Phe Leu Gln Ile
               340                 345                 350
   Gln Leu Thr Leu Leu Lys His Asn Leu Lys Lys Glu Asp Glu Gln Ser
           355                 360                 365
   Lys Glu Lys Ser Leu Ala Ile Ile Ala Asn Phe Glu Gln Ala Leu Ser
       370                 375                 380
   Gly Gly Tyr Ala Gln Leu Arg Glu Leu Leu Ala Thr Phe Arg Leu Thr
   385                 390                 395                 400
   Ile Gln Glu Ala Asn Leu Gln Leu Ala Leu Lys Gln Val Ile Asp Ser
                   405                 410                 415
   Leu Arg Ser Gln Thr Thr Met Gln Met Asn Val Asn Cys Gln Leu Pro
               420                 425                 430
   Ser Gln Ser Leu Asn Pro Gln Gln Leu Val His Val Leu Gln Ile Val
           435                 440                 445
   Arg Glu Ala Thr Thr Asn Ala Ile Lys His Ser Gln Gly Thr Val Ile
       450                 455                 460
   Glu Ile Ser Ala Arg Ile Asn Ala Glu Gly Glu Tyr Glu Ile Leu Val
   465                 470                 475                 480
   Glu Asp Asp Gly Val Gly Ile Pro Asn Leu Glu Glu Pro Glu Gly His
                   485                 490                 495
   Tyr Gly Leu Asn Ile Met Ala Glu Arg Cys Arg Gln Leu Asn Ala Gln
               500                 505                 510
   Leu His Ile His Arg Arg Glu Gln Gly Gly Thr Gln Val Lys Ile Thr
           515                 520                 525
   Leu Pro His Thr Leu Tyr
       530
<210> 35
   <211> 402
   <212> PRT
   <213> Bacteria <prokaryote>
<220>
   <223> NT110
<400> 35
   Ser Ser Arg Lys Leu Lys Leu Gln Ser Leu Ala Asn Lys Gly Asp Val
   1               5                   10                  15
   Arg Ala Leu Gln Val Leu Lys Leu Gln Glu His Pro Gly Arg Phe Ile
               20                  25                  30
   Thr Val Val Gln Ile Leu Leu Asn Met Val Ala Ile Leu Gly Gly Gly
           35                  40                  45
   Ile Gly Glu Ser Ala Leu Ser Pro Tyr Ile Ala Asp Ile Leu Asn Arg
       50                  55                  60
   Ser Phe Glu Gly Ser Trp Ile Glu Pro Thr Ala Ser Thr Ile Ala Phe
   65                  70                  75                  80
   Ile Leu Val Thr Cys Leu Phe Ile Leu Phe Ala Asp Leu Ile Pro Lys
                   85                  90                  95
   Arg Ile Ala Ile Thr Tyr Pro Glu Met Val Ala Leu Ser Val Val Gly
               100                 105                 110
   Ile Met Asn Phe Ser Met Tyr Val Phe Lys Pro Leu Val Trp Phe Phe
           115                 120                 125
   Asp Thr Ile Ala Asn Val Phe Phe Arg Leu Phe Arg Ile Ser Thr Val
       130                 135                 140
   Arg Glu Asp Gly Met Thr Ser Glu Asp Ile Phe Ala Val Val Glu Ala
   145                 150                 155                 160
   Gly Ala Glu Ala Gly Val Leu Lys Thr Gln Glu His Tyr Leu Ile Glu
                   165                 170                 175
   Asn Ile Phe Asp Met Gln Ala Arg Thr Val Thr Ser Thr Met Thr Thr
               180                 185                 190
   Arg Glu Asn Ile Val Tyr Leu Asp Arg Thr Phe Ser Arg Gln Glu Val
           195                 200                 205
   Met Asp Thr Leu Ser Arg Asp Ser His Ser Lys Ile Val Ile Cys Asp
       210                 215                 220
   Asn Gly Leu Asp Lys Ile Leu Gly Tyr Ile Glu Ser His Thr Leu Leu
   225                 230                 235                 240
   Thr Met Tyr Leu Gln Asn Glu Asn Val Val Leu Thr Asp Pro Lys Leu
                   245                 250                 255
   Leu Arg Lys Ala Leu Phe Val Pro Asp Thr Leu Ser Leu Tyr Glu Val
               260                 265                 270
   Leu Glu Leu Phe Lys Ser Thr Gly Glu Asp Phe Ala Ile Ile Val Asn
           275                 280                 285
   Glu Tyr Ala Leu Val Val Gly Ile Val Thr Leu Asn Asp Val Met Ser
       290                 295                 300
   Ile Val Met Gly Glu Leu Val Ser Asn Glu Glu Glu Tyr Ile Val Ser
   305                 310                 315                 320
   Arg Asp Glu Asn Ser Trp Leu Ile Asp Gly Ala Thr Pro Leu Lys Glu
                   325                 330                 335
   Val Thr Arg Val Leu Asp Ile Ala Tyr Phe Pro Asp Glu Glu Asn Tyr
               340                 345                 350
   Glu Thr Ile Ser Gly Phe Met Met Tyr Met Leu Arg Lys Ile Pro Lys
           355                 360                 365
   Lys Thr Asp Ser Val Val Tyr Gly Lys Tyr Lys Phe Glu Val Ile Asp
       370                 375                 380
   Thr Glu Asn Phe Lys Ile Asp Gln Ile Leu Val Ser Leu Val Lys Glu
   385                 390                 395                 400
   Gln Glu
<210> 36
   <211> 265
   <212> PRT
   <213> Bacteria <prokaryote>
<220>
   <223> NT111
<400> 36
   Gln Asp Lys Asp Thr Glu Ala Lys Ile Lys Gln Leu Asn Gln Thr Val
   1               5                   10                  15
   Ala Gln Leu Ser Ala Glu Asn Thr Lys Leu Lys Glu Gln Ile Glu Lys
               20                  25                  30
   Thr Val Pro Ala Ile Ile Val Glu Asn Asp Glu Ile Phe Asn Gln Ser
           35                  40                  45
   Glu Ile Ile Lys His Pro Lys Ser Lys Glu Asp Tyr Gln Pro Glu Glu
       50                  55                  60
   Thr Lys Ile Glu Tyr Ser Ile Ser Thr Ile Lys Thr Asn Ile Asp Trp
   65                  70                  75                  80
   Leu Asn Asp Leu Leu Trp Lys Lys Leu Thr Glu Asn Glu Glu Thr Lys
                   85                  90                  95
   Asn Ile Ser Arg Glu Gln Phe Val Ala Arg Tyr Gln Thr Ala Phe Glu
               100                 105                 110
   Glu Asp Lys Lys Glu Ala Lys Glu Thr Pro Ser Phe Gly Ile Ser His
           115                 120                 125
   Ser Ile Trp Thr Asn Phe Ile Gly Gln Lys Glu Lys Leu Ala Thr Phe
       130                 135                 140
   Ala Ile Ser Phe Tyr Asp Tyr Glu Gly Gly Ala His Gly Ile Glu Gly
   145                 150                 155                 160
   Asn Arg Tyr Phe Thr Ile Asp Leu Thr Thr Arg His Ile Leu Thr Leu
                   165                 170                 175
   Asn Asp Leu Phe Asn Glu Lys Asp Leu Pro Lys Val Lys Thr Leu Leu
               180                 185                 190
   Trp Glu Gln Tyr Asn Asn Ser Asn Lys Glu Tyr Glu Pro Ile Ile Glu
           195                 200                 205
   Ala Asp Ser Phe Asn Leu Ser Asn Asn Ile Tyr Leu Asp Ser Lys Gly
       210                 215                 220
   Val His Phe Ile Tyr Asp Val Tyr Glu Ile Ala Pro Tyr Ala Ala Gly
   225                 230                 235                 240
   Glu Gln Asp Leu Met Leu His Phe Gly Gln Leu Glu Glu Leu Phe Lys
                   245                 250                 255
   Pro Glu Phe Arg Gln Gly Asn Asp Ile
               260                 265
<210> 37
   <211> 231
   <212> PRT
   <213> Bacteria <prokaryote>
<220>
   <223> NT112
<400> 37
   Ala Asn His Asn Ala Thr Lys Gln Ala Ser Glu Arg Asn Asp Ser Leu
   1 5 10 15
   Glu Asp Phe Asn Arg Thr Met Trp Lys Phe Asn Tyr Asn Val Ile Asp
   20 25 30
   Arg Tyr Val Leu Glu Pro Ala Ala Lys Gly Trp Asn Asn Tyr Val Pro
   35 40 45
   Lys Pro Ile Ser Ser Gly Leu Ala Gly Ile Ala Asn Asn Leu Asp Glu
   50 55 60
   Pro Val Ser Phe Ile Asn Arg Leu Ile Glu Gly Glu Pro Lys Lys Ala
   65 70 75 80
   Phe Val His Phe Asn Arg Phe Trp Ile Asn Thr Val Phe Gly Leu Gly
   85 90 95
   Gly Phe Ile Asp Phe Ala Ser Ala Ser Lys Glu Leu Arg Ile Asp Asn
   100 105 110
   Gln Arg Gly Phe Gly Glu Thr Leu Gly Ser Tyr Gly Val Asp Ala Gly
   115 120 125
   Thr Tyr Ile Val Leu Pro Ile Tyr Asn Ala Thr Thr Pro Arg Gln Leu
   130 135 140
   Thr Gly Ala Val Val Asp Ala Ala Tyr Met Tyr Pro Phe Trp Gln Trp
   145 150 155 160
   Val Gly Gly Pro Trp Ala Leu Val Lys Tyr Gly Val Gln Ala Val Asp
   165 170 175
   Ala Arg Ala Lys Asn Leu Asn Asn Ala Glu Leu Leu Arg Gln Ala Gln
   180 185 190
   Asp Pro Tyr Ile Thr Phe Arg Glu Ala Tyr Tyr Gln Asn Leu Gln Phe
   195 200 205
   Lys Val Asn Asp Gly Lys Leu Val Glu Ser Lys Glu Ser Leu Pro Asp
   210 215 220
   Asp Ile Leu Lys Glu Ile Asp
   225 230
<210> 38
   <211> 341
   <212> PRT
   <213> Bacteria <prokaryote>
<220>
   <223> NT113
<400> 38
   Ser Asn Ser Ser Asn Gln Gly Ile Asn Tyr Asp Glu Ala Phe Ala Lys
   1 5 10 15
   Asp Thr Gln Gly Leu Asp Ile Leu Thr Gly Gln Phe Ser His Asn Ile
   20 25 30
   Asp Arg Ile Trp Gly Val Asn Glu Leu Leu Val Ala Ser Arg Lys Asp
   35 40 45
   Tyr Val Lys Tyr Thr Asp Ser Phe Tyr Thr Arg Ser His Val Ser Phe
   50 55 60
   Asp Glu Gly Asn Ile Val Ile Glu Thr Gln Gln Asp Leu Asn Arg Leu
   65 70 75 80
   His Asn Ala Ile Val His Thr Leu Leu Met Gly Ala Asp Ala Lys Gly
   85 90 95
   Ile Asp Leu Phe Thr Ser Gly Asp Val Pro Ile Ser Ser Arg Pro Phe
   100 105 110
   Leu Leu Gly Gln Val Val Asp His Gln Gly Gln Gln Ile Ala Asn Gln
           115                 120                 125
   Val Ile Ala Ser Asn Phe Ala Thr Tyr Leu Ile Gln Asn Lys Leu Gln
       130                 135                 140
   Thr Arg Arg Leu Gln Asn Gly His Thr Val Gln Phe Val Ser Val Pro
   145                 150                 155                 160
   Met Ile Ala Asn His Val Glu Val Arg Ala Arg Lys Tyr Leu Pro Leu
                   165                 170                 175
   Ile Arg Lys Ala Ala Gln Arg Tyr Gly Ile Asp Glu Ser Leu Ile Leu
               180                 185                 190
   Gly Ile Met Gln Thr Glu Ser Ser Phe Asn Pro Tyr Ala Ile Ser Tyr
           195                 200                 205
   Ala Asn Ala Ile Gly Leu Met Gln Val Val Pro His Thr Ala Gly Arg
       210                 215                 220
   Asp Val Phe Ala Met Lys Gly Lys Gly Gly Gln Pro Ser Thr Arg Tyr
   225                 230                 235                 240
   Leu Tyr Asp Pro Ala Asn Asn Ile Asp Ala Gly Val Ser Tyr Leu Trp
                   245                 250                 255
   Ile Leu Gln Asn Gln Tyr Leu Asp Gly Ile Thr Asn Pro Thr Ser Lys
               260                 265                 270
   Arg Phe Ala Met Ile Ser Ala Tyr Asn Ser Gly Ala Gly Ala Val Leu
           275                 280                 285
   Arg Val Phe Asp Asn Asp Lys Asp Thr Ala Ile Tyr Lys Ile Asn Gln
       290                 295                 300
   Met Tyr Pro Glu Gln Val Tyr Arg Ile Leu Thr Thr Val His Pro Ser
   305                 310                 315                 320
   Ser Gln Ala Arg Asn Tyr Leu Leu Lys Val Asp Lys Ala Gln Lys Lys
                   325                 330                 335
   Phe Arg Val Arg Arg
               340
<210> 39
   <211> 574
   <212> PRT
   <213> Bacteria <prokaryote>
<220>
   <223> NT114
<400> 39
   Asp Ser Pro Asn Thr Ala Thr Ala Ser Ile Asn Leu Glu Gln Glu Lys
   1               5                   10                  15
   Gln Asn Trp Ala Ser Ile Gln His Gln Asp Tyr Leu Lys Arg Leu Lys
               20                  25                  30
   Gln Arg Glu Val Phe Leu Gln Val Glu Gly Leu Leu Lys Ser Ala Val
           35                  40                  45
   Lys Lys Gln Gln Phe Ser Glu Ala Ile Gln Asn Ile Thr Lys Thr Leu
       50                  55                  60
   Ile Asp Ser Leu Gln Gly Tyr Pro Leu Gln Tyr Asp Leu Leu Ala Arg
   65                  70                  75                  80
   Phe Trp Glu Thr Lys Ile Ala Phe Leu Gln Asn Asp Asp Ile Gln Gly
                   85                  90                  95
   Lys Gln Gln Ala Ile Asn Glu Val Asn Ala Leu Val Gln Gln Asn Tyr
               100                 105                 110
   Pro Phe Val Thr Pro Ala Phe Gln Ala Leu Leu Gln Lys Leu Ser Thr
           115                 120                 125
   Leu Asn Glu Gln Gln Thr Ser Ala Thr Ser Asp Asn Ala Lys Glu Asn
       130                 135                 140
   Asn Arg Val Gln Lys Glu Gln Asn Gln Val Glu Asn Pro Lys Gln Leu
   145                 150                 155                 160
   Ala Glu Ile Val Arg Lys Ser Asp Pro Asn Thr Leu Asp Lys Thr Val
                   165                 170                 175
   Leu Ile Asp Ala Phe Pro Arg Tyr Leu Lys Thr Leu Pro Glu Gln Met
               180                 185                 190
   Asn Asn Leu Ser Phe Glu Ser Tyr Gln Lys Trp Ala Asn Thr Trp Gln
           195                 200                 205
   Leu Ser Glu Asp Glu Ile Lys Gln Trp Lys Ile Ala Phe Leu Asn Arg
       210                 215                 220
   Phe Phe Asp Asn Glu Asn Thr Asp Phe Gln Lys Trp Arg Asp Glu Gln
   225                 230                 235                 240
   Ile Arg Gln Leu Gln Thr Asp Asn Leu Thr Glu Arg Arg Leu Arg Met
                   245                 250                 255
   Ala Ile Trp Gln Lys Thr Glu Leu Thr Ser Trp Leu Asn Leu Leu Ser
               260                 265                 270
   Ala Glu Ser Lys Ser Lys Gln Glu Trp Arg Tyr Trp Glu Ala Lys Gln
           275                 280                 285
   Asp Ile Leu Lys Asn Thr Lys Lys Leu Thr Ala Leu Ser Lys Glu Arg
       290                 295                 300
   Gly Phe Tyr Pro Met Leu Ala Ala Thr Gln Leu Lys Gln Ala Tyr Gln
   305                 310                 315                 320
   Leu Asn Val Pro Ile Ala Ser Ser Phe Thr Gln Ala Glu Gln Leu Pro
                   325                 330                 335
   Phe Lys Gln Val Phe Ala Met Ile Thr Glu Leu Arg Glu Leu Gly Arg
               340                 345                 350
   Asn Gly Leu Ala Lys Gln Arg Trp Arg Ile Leu Leu Asp Asn Val Asp
           355                 360                 365
   Phe Thr Thr Gln Leu Lys Leu Ser Glu Tyr Ala Lys Asn Gln Gln Trp
       370                 375                 380
   Phe Glu Leu Ala Val Asp Ala Ser Ile Val Ala Lys Ala Trp Gly Tyr
   385                 390                 395                 400
   Leu Ser Leu Arg Leu Pro Asn Ala Tyr Ser Glu Tyr Phe Asn Ala Ala
                   405                 410                 415
   Leu Gln Asn Leu Asn Ile Ser Lys Thr Phe Ala Met Ala Ile Ala Arg
               420                 425                 430
   Gln Glu Ser Ala Trp Asn Pro Met Ala Gln Ser Ser Ala Asn Ala Arg
           435                 440                 445
   Gly Leu Met Gln Leu Leu Pro Ser Thr Ala Lys Leu Thr Ala Glu Asn
       450                 455                 460
   Asn Gln Leu Pro Tyr Gln Gly Glu Gln Asp Leu Phe Lys Pro Leu Asn
   465                 470                 475                 480
   Asn Ile Leu Leu Gly Thr Ala His Leu Asn Glu Leu Asn Gly Lys Tyr
                   485                 490                 495
   Pro Asn Asn Arg Ile Leu Ile Ala Ala Ala Tyr Asn Ala Gly Ala Asn
               500                 505                 510
   Arg Val Glu Lys Trp Leu Ser Arg Ala Ser Gly Lys Leu Ala Leu Asp
           515                 520                 525
   Glu Phe Val Ala Ser Ile Pro Phe Tyr Glu Thr Arg Gly Tyr Val Gln
       530                 535                 540
   Asn Val Val Ala Tyr Asp Phe Tyr Tyr Gln Ile Leu Gln Asn Lys Glu
   545                 550                 555                 560
   Asn Pro Gln Ile Phe Ser Gln Glu Glu Leu Asn Arg Leu Tyr
                   565                 570
<210> 40
   <211> 147
   <212> PRT
   <213> Bacteria <prokaryote>
<220>
   <223> NT115
<400> 40
   Gly Trp His Phe Gln Gln Ser Val Thr Met Pro Asn Glu Trp Arg Thr
   1               5                   10                  15
   Leu Ala Leu Glu Ser Asp Asp Ser Tyr Asn Asp Phe Thr Val Ile Met
               20                  25                  30
   Arg Arg Lys Leu Gln Glu Asn Gln Val Asn Val Val Asn Leu Glu Gln
           35                  40                  45
   Asn Ile Pro Ile Leu Arg Ile Asn Lys Gln Ile Thr Ser Asp Gln Val
       50                  55                  60
   Ala Ser Ile Phe Lys His Gly Arg Glu Ala Glu Lys Leu Leu Met Leu
   65                  70                  75                  80
   Glu Val Glu Ala Thr Phe Arg Leu Ala Asn Gly Glu Ser Tyr Pro Ile
                   85                  90                  95
   Asn Ala Lys Val Asn Arg Thr Phe Phe Asp Asn Ala Arg Ala Ala Leu
               100                 105                 110
   Ala Lys Ser Glu Glu Arg Glu Val Ile Trp Asn Asp Met Arg Glu Gln
           115                 120                 125
   Val Ala Arg Gln Leu Ile Val Lys Ile Ile Ala Leu Gln Asn Gln Ile
       130                 135                 140
   Lys Arg Lys
   145
<210> 41
   <211> 612
   <212> PRT
   <213> Bacteria <prokaryote>
<220>
   <223> NT116
<400> 41
   Ser Gly Gly Gly Gly Ser Phe Asp Val Asp Asp Val Ser Asn Pro Ser
   1               5                   10                  15
   Ser Ser Lys Pro Arg Tyr Gln Asp Asp Thr Ser Ser Ser Arg Thr Lys
               20                  25                  30
   Ser Asn Leu Glu Lys Leu Ser Ile Pro Ser Leu Gly Gly Gly Met Lys
           35                  40                  45
   Leu Val Ala Gln Asn Leu Ser Gly Asn Lys Glu Pro Ser Phe Leu Asn
       50                  55                  60
   Glu Asn Gly Tyr Ile Ser Tyr Phe Ser Ser Pro Ser Thr Ile Glu Asp
   65                  70                  75                  80
   Asp Val Lys Asn Val Lys Thr Glu Asn Lys Ile His Thr Asn Pro Ile
                   85                  90                  95
   Gly Leu Glu Pro Asn Arg Ala Leu Gln Asp Pro Asn Leu Gln Lys Tyr
               100                 105                 110
   Val Tyr Ser Gly Leu Tyr Tyr Ile Glu Asn Trp Lys Asp Phe Ser Lys
           115                 120                 125
   Leu Ala Thr Glu Lys Lys Ala Tyr Ser Gly His Tyr Gly Tyr Ala Phe
       130                 135                 140
   Tyr Tyr Gly Asn Lys Thr Ala Thr Asp Leu Pro Val Ser Gly Val Ala
   145                 150                 155                 160
   Thr Tyr Lys Gly Thr Trp Asp Phe Ile Thr Ala Thr Lys Tyr Gly Gln
                   165                 170                 175
   Asn Tyr Ser Leu Phe Ser Asn Ala Arg Gly Gln Ala Tyr Phe Arg Arg
               180                 185                 190
   Ser Ala Thr Arg Gly Asp Ile Asp Leu Glu Asn Asn Ser Lys Asn Gly
           195                 200                 205
   Asp Ile Gly Leu Ile Ser Glu Phe Ser Ala Asp Phe Gly Thr Lys Lys
       210                 215                 220
   Leu Thr Gly Gln Leu Ser Tyr Thr Lys Arg Lys Thr Asp Ile Gln Gln
   225                 230                 235                 240
   Tyr Glu Lys Glu Lys Leu Tyr Asp Ile Asp Ala His Ile Tyr Ser Asn
                   245                 250                 255
   Arg Phe Arg Gly Lys Val Thr Pro Thr Lys Ser Thr Ser Asp Glu His
               260                 265                 270
   Pro Phe Thr Ser Glu Gly Thr Leu Glu Gly Gly Phe Tyr Gly Pro Asn
           275                 280                 285
   Ala Glu Glu Leu Gly Gly Lys Phe Leu Ala Arg Asp Lys Arg Val Phe
       290                 295                 300
   Gly Val Phe Ser Ala Lys Glu Thr Pro Glu Thr Glu Lys Glu Lys Leu
   305                 310                 315                 320
   Ser Lys Glu Thr Leu Ile Asp Gly Lys Leu Ile Thr Phe Ser Thr Lys
                   325                 330                 335
   Thr Ala Asp Ala Thr Thr Ser Thr Thr Ala Ser Thr Thr Ala Asp Val
               340                 345                 350
   Lys Thr Asp Glu Lys Asn Phe Thr Thr Lys Asp Ile Ser Ser Phe Gly
           355                 360                 365
   Glu Ala Asp Tyr Leu Leu Ile Asp Asn Tyr Pro Val Pro Leu Phe Pro
       370                 375                 380
   Glu Gly Asp Thr Asp Asp Phe Val Thr Ser Lys His His Asp Ile Gly
   385                 390                 395                 400
   Asn Lys Thr Tyr Lys Val Glu Ala Cys Cys Lys Asn Leu Ser Tyr Val
                   405                 410                 415
   Lys Phe Gly Met Tyr Tyr Glu Asp Lys Glu Lys Lys Asn Thr Asn Gln
               420                 425                 430
   Thr Gly Gln Tyr His Gln Phe Leu Leu Gly Leu Arg Thr Pro Ser Ser
           435                 440                 445
   Gln Ile Pro Val Thr Gly Asn Val Lys Tyr Leu Gly Ser Trp Phe Gly
       450                 455                 460
   Tyr Ile Gly Asp Asp Lys Thr Ser Tyr Ser Thr Thr Gly Asn Lys Gln
   465                 470                 475                 480
   Gln Asp Lys Asn Ala Pro Ala Glu Phe Asp Val Asn Phe Asp Asn Lys
                   485                 490                 495
   Thr Leu Thr Gly Lys Leu Lys Arg Ala Asp Ser Gln Asn Thr Val Phe
               500                 505                 510
   Asn Ile Glu Ala Thr Phe Lys Asn Gly Ser Asn Ala Phe Glu Gly Lys
           515                 520                 525
   Ala Thr Ala Asn Val Val Ile Asp Pro Lys Asn Thr Gln Ala Thr Ser
       530                 535                 540
   Lys Val Asn Phe Thr Thr Thr Val Asn Gly Ala Phe Tyr Gly Pro His
   545                 550                 555                 560
   Ala Thr Glu Leu Gly Gly Tyr Phe Thr Tyr Asn Gly Asn Asn Pro Thr
                   565                 570                 575
   Ala Thr Asn Ser Glu Ser Ser Ser Thr Val Pro Ser Pro Pro Asn Ser
               580                 585                 590
   Pro Asn Ala Arg Ala Ala Val Val Phe Gly Ala Lys Arg Gln Val Glu
           595                 600                 605
   Lys Thr Asn Lys
       610
<210> 42
   <211> 350
   <212> PRT
   <213> Bacteria <prokaryote>
<220>
   <223> NT117
<400> 42
   Asn Thr Ile Ile Pro Asn Tyr Asn Thr Asp Ala His Leu Tyr Glu Phe
   1               5                   10                  15
   Thr Gln Thr Tyr Asp Leu Val Val Pro Lys Gly Ser Gln Gly Gln Thr
               20                  25                  30
   Asn Leu Trp Val Pro Leu Pro Phe Asn Gly Glu Tyr Gln Gln Val Lys
           35                  40                  45
   Ser Ile His Phe Glu Gly Asn Tyr Met Asn Ala Tyr Val Thr Glu Asn
       50                  55                  60
   Asn Lys Tyr Gly Ala Lys Thr Leu Phe Ala Thr Trp Asp Lys Asp Ala
   65                  70                  75                  80
   Gln Lys Arg Asp Leu Lys Val Thr Met Val Ile Glu Thr Lys Asp Arg
                   85                  90                  95
   Glu Pro Met Val Lys Gly Ala Leu Glu Asn Tyr Thr Pro Pro Lys Asp
               100                 105                 110
   Ile Gln Tyr Ser Val Asp Val Gln Glu Tyr Leu Lys Ala Thr Pro His
           115                 120                 125
   Ile Lys Thr Asp Gly Ile Val Lys Glu Phe Ala Asp Lys Ile Leu Gly
       130                 135                 140
   Lys Glu Thr Asn Pro Leu Lys Lys Ala Glu Leu Ile His His Trp Ile
   145                 150                 155                 160
   Val Lys Asn Met Glu Arg Asp Asn Ser Val Leu Gly Cys Gly Asp Gly
                   165                 170                 175
   Asp Val Glu Lys Ile Leu Thr Thr Gly Val Leu Lys Gly Lys Cys Thr
               180                 185                 190
   Asp Ile Asn Ser Val Phe Val Ala Leu Ala Arg Ala Ala Gly Ile Pro
           195                 200                 205
   Ala Arg Glu Ile Phe Gly Ile Arg Leu Gly Thr Ala Glu Lys Met Gly
       210                 215                 220
   Lys Tyr Ser Lys Gly Ala Phe Gly Ser Ala Asn Glu Gln Gly Ile Val
   225                 230                 235                 240
   Asn Val Ser Gly Gly Gln His Cys Arg Ala Glu Phe Tyr Leu Ala Gly
                   245                 250                 255
   Phe Gly Trp Val Pro Val Asp Ser Ala Asp Val Ala Lys Met Arg Leu
               260                 265                 270
   Ala Glu Lys Lys Ser Val Glu Asp Lys Asp Thr Gln Ala Val Ala Lys
           275                 280                 285
   Tyr Leu Phe Gly Asn Trp Glu Ala Asn Trp Val Gly Phe Asn His Ala
       290                 295                 300
   Arg Asp Phe Asp Leu Tyr Pro Gln Pro Glu Leu Ala Pro Ile Asn Asn
   305                 310                 315                 320
   Phe Gly Tyr Pro Tyr Ala Glu Val Gly Gly Asp Pro Leu Asn Ser Phe
                   325                 330                 335
   Asp Pro Lys Glu Phe Lys Tyr Asp Tyr Val Ser Lys Lys Leu
               340                 345                 350
<210> 43
   <211> 184
   <212> PRT
   <213> Bacteria <prokaryote>
<220>
   <223> NT118
<400> 43
   Trp Phe Tyr Ser Leu Asn Gln Glu Thr Ala Asp Leu Ser Glu Leu Val
   1               5                   10                  15
   Lys Lys Pro Asp Ser Pro Asp Tyr Val Gly Tyr Lys Met Glu Thr Thr
               20                  25                  30
   Val Phe Ser Pro Glu Gly Lys Lys Gln Tyr Leu Ala Leu Ser Asp Lys
           35                  40                  45
   Ile Glu His Tyr Thr Val Asn Glu Gln Thr Leu Phe Thr Ala Pro Leu
       50                  55                  60
   Val Tyr Leu Tyr Pro Thr Thr Ser Asn Glu Lys Glu Gln Asn Pro Asn
   65                  70                  75                  80
   Gln Asn Val Asp Phe Phe Ser Thr Gln Asn Trp Lys Leu Ser Ala Asn
                   85                  90                  95
   Gln Ala Arg Leu Thr Lys Asp Gln Ile Leu Tyr Leu Glu Gly Asn Val
               100                 105                 110
   Val Val Gln Ser Leu Thr Ser Asp Ser Arg Leu Gln Arg Ile Glu Thr
           115                 120                 125
   Glu Ser Ala Val Val Asn Leu Lys Thr Gln Asp Met Thr Ser Glu Thr
       130                 135                 140
   Gln Val Lys Ile Lys Gly Lys Asn Phe Ser Ser Thr Gly Leu Lys Leu
   145                 150                 155                 160
   Val Gly Asn Leu Arg Gln Gln Val Ala Thr Leu Lys Glu Gln Val Lys
                   165                 170                 175
   Thr Tyr Tyr Glu Val Ser Lys Gln
               180
<210> 44
   <211> 909
   <212> PRT
   <213> Bacteria <prokaryote>
<220>
   <223> NT123
<400> 44
   Gln Ser Leu Glu Leu Ser Pro Asn Asn Asp Leu Pro Phe Asp Pro Asn
   1               5                   10                  15
   Ile Gln His Gly Lys Leu Ser Asn Gly Leu Gln Tyr Phe Val Leu Lys
               20                  25                  30
   Asn Thr Glu Pro Lys Glu Arg Val Tyr Ile Arg Leu Val Ile Asn Ala
           35                  40                  45
   Gly Ser Met His Glu Asp Asp Asp Gln Lys Gly Ile Ala His Leu Val
       50                  55                  60
   Glu His Met Ala Phe Asn Gly Ser Lys Lys Tyr Pro Glu Asn Gln Ile
   65                  70                  75                  80
   Ile Asn Ala Leu Glu Lys Leu Gly Met Lys Phe Ala Arg Asp Ile Asn
                   85                  90                  95
   Ala Phe Thr Asp Phe Glu Asn Thr Val Tyr Thr Leu Asn Leu Asp Ser
               100                 105                 110
   Asn Asn Gln Gln Lys Leu Glu Leu Ala Phe Asp Val Ile Asn Glu Trp
           115                 120                 125
   Met Asn Asn Ile Thr Phe Leu Pro Lys Asp Val Asp Gly Glu Arg Gly
       130                 135                 140
   Val Val Gln Glu Glu Trp Arg Arg Arg Leu Ser Pro Met Leu Arg Ile
   145                 150                 155                 160
   Gly Asn Lys Lys Ser Ala Ile Glu Met Ala Gly Ser Arg Tyr Val Leu
                   165                 170                 175
   Arg Asp Pro Ile Gly Asp Met Asp Ile Ile Lys Thr Ile Ser Ala Lys
               180                 185                 190
   Arg Val Ala Asp Phe Tyr His Lys Trp Tyr Arg Pro Asp Asn Met Ser
           195                 200                 205
   Val Ile Ile Val Gly Asp Ile Asp Thr Lys Gln Val Val Lys Leu Leu
       210                 215                 220
   Lys Gln Asn Leu Ser Gln Glu Asn Pro Ile Thr Lys Thr Thr Leu Glu
   225                 230                 235                 240
   Lys Ile Asp Phe Asn Ile Pro Leu Ile Asn Lys Trp Arg Leu Asp Ser
                   245                 250                 255
   Ile Ser Glu Gln Gly Thr Thr Ile Pro Ser Ile Glu Leu Ser Phe Phe
               260                 265                 270
   Glu Asn Thr Ile Glu Thr Asn Thr Leu Ala Ser Tyr Lys Gln Glu Leu
           275                 280                 285
   Ile Gln Gln Ile Thr Thr Arg Leu Leu Asn Leu Arg Leu Gln Gln Trp
       290                 295                 300
   Glu Lys Glu Thr Glu Asn Gly Val Asp Ser Ala Asn Phe Tyr Arg Thr
   305                 310                 315                 320
   His Leu Gly Lys Glu Thr Leu Gln Ser Ile Phe Ser Leu Gln Leu Ile
                   325                 330                 335
   Asp Thr Gln Tyr Ser Lys Thr Ile Asp Lys Leu Phe Ala Phe Ile Ala
               340                 345                 350
   Ser Ile Lys Gln Gln Gly Phe Thr Gln Asn Glu Leu Asn Gly Glu Ile
           355                 360                 365
   Lys Arg Leu Thr Gln Leu Asn Glu Lys Gln Leu Asn Ile Arg Ser Gly
       370                 375                 380
   Ser Leu Lys Ile Ala Asp Asp Leu Ile Thr Ser Val Ala Asn Lys Gln
   385                 390                 395                 400
   Val Val Leu Ser Val Asn Asp Arg Tyr Glu Leu Asn Lys Arg Phe Leu
                   405                 410                 415
   Ser Gln Ile Thr Leu Ala Asp Leu Gln Arg Thr Leu Asn Gln Thr Leu
               420                 425                 430
   Ala Leu Lys Ala Lys Leu Leu Leu Ile Thr Gln Pro Leu Pro Gln Lys
           435                 440                 445
   Ala Leu Pro Phe Asp Val Val Glu Ile Glu Thr Arg Trp Asn Asn Ala
       450                 455                 460
   Met Lys Met Gln Gln His Gln Trp Asp Glu Lys Lys Gln Ile Glu Lys
   465                 470                 475                 480
   Leu Pro His Leu Thr Phe Asn Thr Gly Ser Leu Ser Gln Glu Lys Tyr
                   485                 490                 495
   Trp Asp Arg Gly Asp Ile Tyr Glu Phe Arg Leu Ser Asn Gly Ser Lys
               500                 505                 510
   Leu Ile Tyr His Tyr Ser Asp Lys Thr Pro Asn Gln Val His Phe Arg
           515                 520                 525
   Ala Val Thr Gln Gly Gly Leu Arg Ser Ile Pro Asp Lys Asp Tyr His
       530                 535                 540
   Leu Leu Arg Ala Ala Val Ser Val Val Asp Glu Thr Gly Val Gly Glu
   545                 550                 555                 560
   Leu Ser Leu Ser Ala Val Asn Gln Ile Phe Ser Arg Asp Pro Leu Val
                   565                 570                 575
   Ile Ala Thr Val Ile Asp Asp Asp Lys Gln Gly Phe Thr Gly Val Ser
               580                 585                 590
   Lys Pro Lys Asp Leu Glu Asn Leu Leu Thr Leu Phe Arg Leu Lys Leu
           595                 600                 605
   Arg Ser Ser Pro Ile Ser Asp Leu Ala Leu Glu Lys Tyr Arg Arg Glu
       610                 615                 620
   Thr Arg Asp Tyr Phe Lys Gln Ile Asp Leu Glu Thr Gln Phe Met Gln
   625                 630                 635                 640
   Ala Val Ser Lys Leu Arg Phe Pro Asn Ile Glu Thr Val Tyr Thr Gln
                   645                 650                 655
   Lys Gln Ala Gln Gln Leu Ala Phe Asp Lys Asn Gln Leu Ser Asn Ala
               660                 665                 670
   Tyr Gln Arg Tyr Ile Leu Asn Lys Thr Asp Phe Thr Tyr Phe Ile Ile
           675                 680                 685
   Gly Asp Ile Glu Leu Asn Gln Val Lys Lys Leu Ala Glu Arg Tyr Leu
       690                 695                 700
   Ala Ser Val Glu Ser Lys Thr Gln Ile Arg His Phe Val Pro Thr Ile
   705                 710                 715                 720
   Ile His Thr Pro Thr Gln Ser Phe Ile Met Asn Gly Leu Lys Glu Pro
                   725                 730                 735
   Arg Ala Asp Val Glu Ile Tyr Leu Thr Ala Asp Asn Thr Trp Arg Ala
               740                 745                 750
   Glu Gln Lys Tyr Leu Phe Asn Ile Leu Ala Asp Ile Val Gln Glu Lys
           755                 760                 765
   Leu Arg Leu Ile Leu Arg Glu Lys Val Ser Gly Val Tyr Ser Val Asn
       770                 775                 780
   Ser Trp Phe Met Gln Asp Val His Thr Pro Gln Ile Glu Gly Lys Ile
   785                 790                 795                 800
   Glu Phe Ser Cys Asp Pro Lys Arg Val Glu Glu Leu Thr His Leu Thr
                   805                 810                 815
   Asn Gln Val Leu Asp Asp Ile Val Lys Asn Gly Ile Asp Glu Asn Leu
               820                 825                 830
   Leu Arg Lys Lys Leu Ala Glu Gln His Thr Gln Ile Arg Arg Glu Phe
           835                 840                 845
   Asp Ser Leu Val Ser Val Ala Ser Ile Ile Glu Glu Ser Tyr Trp Gln
       850                 855                 860
   Gln Asp Asn Pro Asp Ala Ile Tyr Thr Tyr Gln His Leu Asp Gln Leu
   865                 870                 875                 880
   Ala Thr Lys Ala Thr Ile Asp Ala Leu Ala Gln Lys Ala Leu Lys Lys
                   885                 890                 895
   Ser Gly Arg Phe Val Ser Val Leu Lys Ala Ala Thr Tyr
               900                 905
<210> 45
   <211> 458
   <212> PRT
   <213> Bacteria <prokaryote>
<220>
   <223> NT124
<400> 45
   Trp Phe Thr Gly Lys Lys Ala Glu Glu Glu Tyr Leu His Gln Leu Lys
   1               5                   10                  15
   Gln Leu Asn Gln Leu Phe Thr Lys Thr Glu Ala Leu Glu Glu Ser Lys
               20                  25                  30
   Ile Phe Tyr Lys Asn Ile Lys Phe Glu Arg Gly Leu Phe Ala Ser His
           35                  40                  45
   Ile Gln Asp Gln Ile Glu Ile His Lys Ala Asn Glu Thr Ile Ile Ile
       50                  55                  60
   Pro Leu Ser Ser Thr Leu Tyr His Gly Pro Leu Pro Leu Asp Arg Val
   65                  70                  75                  80
   Ala Lys Leu Asn Phe Val Pro Ala Ile Phe Ser Ser Gln Thr Leu Leu
                   85                  90                  95
   Gly Lys Asn Ala Thr Thr Gln Ala Phe Phe Asp Ile Thr Glu Ser Glu
               100                 105                 110
   Lys Pro Leu Gln Leu Asn Phe Ala Met Asn Tyr Ser Leu Ser Gly Asn
           115                 120                 125
   Ala Glu Leu Lys Leu Ala Ser Gly Gln Tyr His Asn Glu Gln Ser Lys
       130                 135                 140
   Thr Asp Phe Asp Trp Ser Asn Val Val Leu Asn Ile Asp Leu Asn Gln
   145                 150                 155                 160
   Asn Thr Pro Asn Asn Tyr Val Leu Ser Val Asp Thr Phe Asn Ser Asn
                   165                 170                 175
   Ala Pro Asn His Ala Val Ser Thr Ala Ser Ser Ile Lys Ile Lys Asp
               180                 185                 190
   Leu Val Val Gln Gly Ser Leu Gln Ser Thr Lys Trp Pro Phe Ile Tyr
           195                 200                 205
   Ser Gly Asn Ile Asn Ser Lys Ile Gly Tyr Phe Glu Gln Asn Thr Glu
       210                 215                 220
   Ser Pro Glu Thr Gly Glu Lys Phe Ser Leu Ile Gln Lys Asn Ser Gln
   225                 230                 235                 240
   Ala Asn Leu Thr Thr Gln Val Glu Gly Asp Thr Val Asn Ile Ile Asn
                   245                 250                 255
   Lys Thr Asn Leu Asp Glu Leu His Ile Asn Gly Asn Asn Leu Gly Lys
               260                 265                 270
   Val Thr Asn Asn Val Glu Phe Asn His Ile Asp Gly Asn Ala Leu Gln
           275                 280                 285
   Glu Leu Leu Asn Ile Leu Val Ala Ile Gly Lys Ala Asp Ser Asp Met
       290                 295                 300
   Pro Leu Ser Lys Thr Leu Val Gln Lys Leu Gln Gln Ala Gly Met Ile
   305                 310                 315                 320
   Ile Ala Asn Asn Gln Pro Gln Ile Lys Phe Thr Pro Leu Ser Ile Ser
                   325                 330                 335
   Asp Glu Lys Gly Lys Val Ala Leu Asp Leu Asn Ile Ala Leu Val Pro
               340                 345                 350
   Asn Pro Lys Phe Asp Leu Met Arg Ser Gly Leu Tyr Lys Gln Phe Lys
           355                 360                 365
   Asp Phe Ser Ile Asn Phe Asp Val Asn Lys Glu Thr Ala Ile Ser Leu
       370                 375                 380
   Leu Ser Lys Phe Val Pro Glu Asn Gln Lys Gln Asp Phe Val His Arg
   385                 390                 395                 400
   Leu Asn Glu Leu Val Thr Glu Gly Glu Val Asn Asp Ile Ile Val Asn
                   405                 410                 415
   Thr Asp Lys Thr Val Thr Leu Thr Leu Ala Leu Glu Asn Asn Asp Leu
               420                 425                 430
   Lys Leu Asn Gly Lys Pro Ile Pro Glu Glu Gln Leu Lys Val Val Leu
           435                 440                 445
   Phe Ile Leu Val Met Gly Gly Phe Gly Arg
       450                 455
<210> 46
   <211> 6
   <212> PRT
   <213> Bacteria <prokaryote>
<220>
   <223> Linker 1
<400> 46
   Gly Ser Gly Gly Gly Gly
   1               5
<210> 47
   <211> 8
   <212> PRT
   <213> Bacteria <prokaryote>
<220>
   <223> Linker 2
<400> 47
   Gly Ser Gly Ser Gly Gly Gly Gly
   1               5
<210> 48
   <211> 17
   <212> PRT
   <213> Bacteria <prokaryote>
<220>
   <223> Histidine Tag
<400> 48
   Met Gly Ser Ser His His His His His His Glu Asn Leu Tyr Phe Gln
   1               5                   10                  15
   Gly
<210> 49
   <211> 277
   <212> PRT
   <213> Bacteria <prokaryote>
<220>
   <223> NT018 Variant from Fi176
<400> 49
   Lys His Gly Gln Lys Arg Asp Asp Leu Asn Lys Ala Leu Tyr Phe Ser
   1               5                   10                  15
   Arg Leu Glu Glu Ile Glu Gln Asp Asn Ser Gln Gly Leu Val Glu Asn
               20                  25                  30
   Val Glu Gln Leu Lys Gln Glu Leu Gln Lys Thr Leu Leu Asp Asp Val
           35                  40                  45
   Pro Ser Lys Val Gln Glu Asn Val Asp Tyr Ser Gly Lys Ser Tyr Gly
       50                  55                  60
   Lys Ile Trp Phe Val Ser Gly Val Leu Ala Leu Gly Ile Ile Ala Gly
   65                  70                  75                  80
   Ser Pro Tyr Phe Met Val Gly Ser Trp Gln Ala Glu Ser Met Leu Glu
                   85                  90                  95
   Gln Thr Tyr Ala Lys Leu Pro Tyr Phe Phe Asp Arg Met Lys Asp Glu
               100                 105                 110
   Asp Lys Asn Pro Phe Ser Asp Thr Glu Met Gln Gln Phe Ser Thr Ala
           115                 120                 125
   Leu Arg Ile Asp Leu Gln Lys Asn Pro Thr Asp Ala Lys Lys Trp Trp
       130                 135                 140
   Met Leu Gly Gln Ile Gly Met Asn Leu Gly Asp Ala Arg Leu Ala Phe
   145                 150                 155                 160
   Asp Ser Tyr Gln Lys Ala Asn Lys Leu Glu Pro Asp Asn Val Gln Tyr
                   165                 170                 175
   Lys Leu Gly Tyr Ala Arg Ile Leu Met Phe Ser Glu Asp Ala Thr Asp
               180                 185                 190
   Lys Leu Lys Gly Gly Asn Leu Leu Arg Glu Val Ile Arg Gln Glu His
           195                 200                 205
   Thr Asn Ile Glu Ala Leu Ser Leu Leu Ala Phe Arg Tyr Phe Glu Thr
       210                 215                 220
   Glu Asp Tyr Lys Met Ala Ala Val Thr Trp Ala Met Met Leu Arg Leu
   225                 230                 235                 240
   Met Pro Lys Asp Asp Glu Arg Val Pro Leu Ile Glu Lys Ser Ile Arg
                   245                 250                 255
   Thr Ala Arg Asp Ala Leu Glu Ala Gln Asn Glu Glu Lys Ser Lys Ser
               260                 265                 270
   Ile Thr Pro Glu Lys
           275
<210> 50
   <211> 160
   <212> PRT
   <213> Bacteria <prokaryote>
<220>
   <223> NT024Variant from Fi176
<400> 50
   Met Lys Thr Ile Asp Ile Thr Ala Asn Ser Lys Met Asp Asp Gln Ala
   1               5                   10                  15
   Arg Met Asn Leu Ala Gln Glu Phe Ala Asn Lys Gln Gln Trp Ser Ser
               20                  25                  30
   Val Phe Asp Ile Met Tyr Pro Met Ala Leu Glu Gly Asn Thr Thr Ala
           35                  40                  45
   Gln Ser Asn Leu Gly Met Leu Tyr Asn Leu Gly Arg Gly Thr Val Arg
       50                  55                  60
   Asp Tyr Glu Lys Ala Tyr Trp Trp Phe Ser Glu Ala Ala Glu Lys Gly
   65                  70                  75                  80
   Ser Val Lys Gly Leu Asn Asn Leu Gly Val Met Tyr Leu Arg Gly Asp
                   85                  90                  95
   Tyr Val Lys Gln Asn Thr Glu Gln Ala Ile Lys Leu Phe Glu Arg Thr
               100                 105                 110
   Ala Arg Ala Lys Asp Thr Asp Ala Met Met Met Leu Ser Asn Ile Tyr
           115                 120                 125
   Arg Leu Gln Asn Gln Pro Glu Lys Ser Leu Glu Trp Leu Lys Lys Ala
       130                 135                 140
   Ala Glu Leu Gly Asn Lys Glu Ala Lys Gln Arg Leu Ser Ser Gln Pro
   145                 150                 155                 160
<210> 51
   <211> 102
   <212> PRT
   <213> Bacteria <prokaryote>
<220>
   <223> NT032 Variant from Fi176
<400> 51
   Gly Phe Asn Gly Asn Asn Ser Gln Gly Gly Phe Gln Gln Thr Ala Pro
   1               5                   10                  15
   Ala Ala Ile Ser Val Lys Gln Ala Leu Ser Ala Ala Asp Asn Ser Met
               20                  25                  30
   Ile Thr Leu Val Gly Asn Ile Thr Gln Gln Ile Asp Asp Asp Glu Phe
           35                  40                  45
   Trp Phe Thr Asp Gly Thr Gly Gln Ile Lys Ile Glu Ile Lys Lys Arg
       50                  55                  60
   Val Trp Asn Gly Leu Asn Val Asp Ser Lys Asp Lys Val Lys Ile Tyr
   65                  70                  75                  80
   Gly Lys Leu Asp Asn Glu Ala Phe Glu Lys Ala Glu Leu Asp Val Leu
                   85                  90                  95
   Arg Val Glu Lys Ala Glu
               100
<210> 52
   <211> 521
   <212> PRT
   <213> Bacteria <prokaryote>
<220>
   <223> NT067 Variant from Fi176
<400> 52
   Val Ile Val Pro Glu Gly Thr Gln Leu Asp Glu Lys Gln His Ile Val
   1               5                   10                  15
   Phe Asn Asn Gly Ala Glu Pro Gln Ser Phe Asp Pro His Lys Thr Glu
               20                  25                  30
   Gly Val Pro Glu Ser Asn Val Ala Tyr Gln Leu Leu Glu Gly Leu Val
           35                  40                  45
   Thr Ser Asp Ser Glu Gly Lys Leu Gln Pro Gly Val Ala Glu Ser Trp
       50                  55                  60
   Glu Asn Thr Pro Asp Phe Lys Thr Trp Thr Phe His Leu Arg Lys Asp
   65                  70                  75                  80
   Ala Lys Trp Ser Asn Gly Asp Pro Val Thr Ala His Asp Phe Val Phe
                   85                  90                  95
   Ala Trp Arg Arg Leu Val Asp Pro Ala Thr Ala Ala Pro Tyr Ala Ser
               100                 105                 110
   Tyr Leu Ser Tyr Leu Gln Val Glu Asn Ala Gln Asp Ile Ile Asp Gly
           115                 120                 125
   Lys Lys Lys Pro Ala Glu Leu Gly Val Glu Ala Lys Asp Asp Tyr Thr
       130                 135                 140
   Phe Val Val His Ala Ile Asn Pro Val Pro Tyr Ala Val Ser Leu Thr
   145                 150                 155                 160
   Thr His Gln Ser Leu Leu Pro Leu Pro Gln Lys Val Val Glu Lys Leu
                   165                 170                 175
   Gly Asp Ala Trp Val Lys Lys Glu Asn Tyr Val Gly Asn Gly Ala Tyr
               180                 185                 190
   Lys Leu Ala Asn His Ile Ile Asn Glu Lys Ile Glu Phe Glu Arg Asn
           195                 200                 205
   Pro Leu Tyr Trp Asn Asp Lys Glu Thr Val Ile Asn Ser Ala Thr Phe
       210                 215                 220
   Leu Ala Ile Glu Asn Pro Ser Thr Asp Val Ala Arg Tyr Arg Ala Gly
   225                 230                 235                 240
   Asp Leu Asp Met Thr Ser Tyr Gly Leu Pro Pro Glu Gln Phe Ala Lys
                   245                 250                 255
   Leu Lys Lys Glu Leu Leu Gly Glu Val Tyr Val Thr Arg Thr Leu Gly
               260                 265                 270
   Thr Tyr Ser Tyr Glu Leu Asn Asn Lys Lys Ala Pro Phe Asp Asn Val
   275 280 285
   Asn Ile Arg Lys Ala Leu Asn Leu Ser Leu Asp Arg His Val Ile Thr
   290 295 300
   Asp Lys Val Leu Gly Gln Gly Gln Thr Pro Thr Tyr Val Phe Thr Pro
   305 310 315 320
   Thr Tyr Ile Glu Glu Gly His Leu Ile Gln Gln Pro Ala Tyr Ser Lys
   325 330 335
   Glu Pro Met Ala Gln Arg Asn Glu Glu Ala Ile Lys Leu Leu Glu Glu
   340 345 350
   Ala Gly Tyr Ser Lys Ala Asn Pro Leu Lys Phe Ser Ile Leu Tyr Asn
   355 360 365
   Thr Asn Glu Asn His Lys Lys Val Ala Ile Ala Ala Ala Ser Met Trp
   370 375 380
   Lys Ala Asn Thr Lys Gly Leu Ile Asp Val Lys Leu Glu Asn Gln Glu
   385 390 395 400
   Trp Lys Thr Tyr Ile Asp Ser Arg Arg Ala Gly Arg Tyr Asp Ala Ala
   405 410 415
   Arg Ala Gly Trp Ser Ala Asp Tyr Asn Gln Ala Thr Thr Phe Gly Asn
   420 425 430
   Tyr Phe Leu Ser Asn Ser Ser Asn Asn Thr Ala Lys Tyr Ala Asn Pro
   435 440 445
   Glu Tyr Asp Lys Ala Met Ala Glu Ser Tyr Ala Ala Thr Asp Ala Glu
   450 455 460
   Gly Arg Ala Lys Ala Tyr Ala Lys Ala Glu Glu Ile Leu Ala Lys Asp
   465 470 475 480
   Tyr Gly Ile Val Pro Ile Phe Asn Tyr Val Asn Pro Arg Leu Val Lys
   485 490 495
   Pro Tyr Val Lys Gly Tyr Ser Gly Lys Asp Pro Gln Asp His Ile Tyr
   500 505 510
   Leu Arg Asn Leu Tyr Ile Ile Lys His
   515 520
<210> 53
   <211> 157
   <212> PRT
   <213> Bacteria <prokaryote>
<220>
   <223> NT038 Variant from R2846
<400> 53
   Lys Gln Asp Gly Ser Ala Asp Met Asp Lys Lys Val Lys Asn Gly Glu
   1               5                   10                  15
   Leu Val Lys Thr Lys Val Lys Leu Val Ser Ala Asn Gly Thr Asn Pro
               20                  25                  30
   Val Lys Ile Ser Asn Val Ala Glu Gly Thr Glu Asp Thr Asp Ala Val
           35                  40                  45
   Ser Phe Lys Gln Leu Lys Ala Leu Gln Asn Lys Gln Val Thr Leu Ser
       50                  55                  60
   Ala Ser Asn Ala Tyr Ala Asn Gly Gly Ser Asp Ala Asp Val Gly Lys
   65                  70                  75                  80
   Val Thr Gln Thr Leu Ser Asn Gly Leu Asn Phe Lys Phe Lys Ser Thr
                   85                  90                  95
   Asp Gly Glu Leu Leu Asn Ile Lys Ala Asp Lys Asp Thr Val Thr Ile
               100                 105                 110
   Thr Arg Ala Ser Gly Ala Asn Gly Ala Ala Ala Thr Asp Ala Asp Lys
           115                 120                 125
   Ile Lys Val Ala Ser Asp Gly Ile Ser Ala Gly Asn Lys Ala Val Lys
       130                 135                 140
   Asn Val Ala Ala Gly Glu Ile Ser Ala Thr Ser Thr Asp
   145                 150                 155
<210> 54
   <211> 252
   <212> PRT
   <213> Bacteria <prokaryote>
<220>
   <223> NT001 Variant from Fi176
<210> 55
   <211> 243
   <212> PRT
   <213> Bacteria <prokaryote>
<220>
   <223> NT016 Variant from Fi176
<400> 55
   Ser Ser Gly Ser Lys Asp Val Glu Gln Ala Ser Val Asn Glu Leu Tyr
   1               5                   10                  15
   Thr Lys Gly Thr Thr Ser Leu Gln Glu Gly Ser Tyr Ser Glu Ala Ile
               20                  25                  30
   Arg Tyr Leu Lys Ala Thr Thr Glu Arg Phe Pro Gly Ser Val Tyr Gln
           35                  40                  45
   Glu Gln Ala Met Leu Asp Leu Ile Tyr Ala Asn Tyr Lys Thr Gln Asp
       50                  55                  60
   Tyr Thr Gln Val Leu Leu Met Val Asp Ser Phe Leu His Gln Phe Pro
   65                  70                  75                  80
   Gln Ser Pro Asn Gln Ala Tyr Ala Val Tyr Met Ala Gly Leu Thr Asn
                   85                  90                  95
   Ala Ala Thr Gly Asp Asn Phe Ile Gln Asp Phe Phe Gly Ile Asp Arg
               100                 105                 110
   Ala Thr Arg Glu Thr Thr Ser Met Arg Thr Ala Phe Ser Asn Phe Gln
           115                 120                 125
   Asn Leu Val Arg Val Phe Pro Asn Ser Pro Tyr Ser Gln Asp Ala Leu
       130                 135                 140
   Ala Arg Met Ala Tyr Ile Lys Asp Ala Leu Ala Arg His Glu Leu Glu
   145                 150                 155                 160
   Ile Ala Lys Phe Tyr Ala Lys Arg Lys Ala Trp Val Ala Val Ala Asn
                   165                 170                 175
   Arg Val Val Gly Met Leu Lys Gln Tyr Pro Asp Thr Lys Ala Thr Tyr
               180                 185                 190
   Glu Gly Leu Phe Leu Met Gln Glu Ala Tyr Glu Lys Met Gly Leu Thr
           195                 200                 205
   Ala Leu Ala Asn Asp Thr Gln Lys Ile Ile Asp Ala Asn Lys Asp Lys
       210                 215                 220
   Thr Phe Ala Pro Ile Glu Lys Pro Asn Glu Pro Asp Leu Lys Val Pro
   225                 230                 235                 240
   Ala Val Lys
<210> 56
   <211> 337
   <212> PRT
   <213> Bacteria <prokaryote>
<220>
   <223> NT052 Variant from R2846
<400> 56
   Asp Thr Leu Glu Gln Gln Phe Gln Gln Gly Leu Glu Ala Thr Lys Arg
   1 5 10 15
   Gly Asp Tyr Gln Thr Ala Phe Lys Leu Trp Leu Pro Leu Ala Glu Gln
   20 25 30
   Gly Asn Ala Ser Ile Gln Phe Asn Leu Gly Leu Met Tyr Lys Lys Gly
   35 40 45
   Gln Gly Ile Lys Gln Asp Asp Phe Glu Ala Val Lys Trp Tyr Arg Lys
   50 55 60
   Ala Ala Glu Gln Gly Val Ala Asp Ala Gln Leu Asn Leu Gly Asn Met
   65 70 75 80
   Tyr Ala Lys Gly Leu Gly Val Lys Gln Asp Asp Val Glu Ala Val Lys
   85 90 95
   Trp Tyr Arg Gln Ala Ala Glu Gln Gly Asn Ala Lys Ala Gln Phe Asn
   100 105 110
   Leu Gly Leu Met Tyr Asp Asn Gly Arg Gly Val Lys Gln Asp Tyr Phe
   115 120 125
   Glu Ala Val Lys Trp Phe Arg Lys Ala Ala Glu Gln Gly Tyr Ala Asp
   130 135 140
   Ala Gln Phe Asn Leu Gly Asn Met Tyr Tyr Asn Gly His Gly Val Lys
   145 150 155 160
   Gln Asp Asp Phe Glu Ala Val Lys Trp Tyr Arg Lys Ala Ala Glu Gln
   165 170 175
   Gly Tyr Ala Asp Ala Gln Phe Asn Leu Gly Asn Met Tyr Tyr Asn Gly
   180 185 190
   His Gly Val Lys Gln Asp Asp Phe Glu Ala Val Lys Trp Tyr Arg Lys
   195 200 205
   Ala Ala Glu Gln Gly His Ala Lys Ala Gln Tyr Asn Leu Gly Asn Met
   210 215 220
   Tyr Ala Asn Gly Arg Gly Val Lys Gln Asp Tyr Phe Glu Ala Val Lys
   225 230 235 240
   Trp Tyr Arg Lys Ala Ala Glu Gln Gly Tyr Ala Asp Ala Gln Ala Asn
   245 250 255
   Leu Gly Ser Ala Tyr Ser Ala Gly His Gly Val Arg Gln Asp Tyr Ile
   260 265 270
   Glu Ala Val Lys Trp Phe Lys Lys Ala Ala Glu Asn Gly Ser Ala Asp
   275 280 285
   Gly Gln Phe Lys Leu Gly Leu Val Tyr Leu Ile Gly Gln Gly Ile Gln
   290 295 300
   Lys Asp Arg Thr Leu Ala Lys Glu Trp Leu Gly Lys Ala Cys Asp Asn
   305 310 315 320
   Gly Asn Gln Asn Gly Cys Glu Tyr Tyr Gly Glu Leu Asn Arg Gly Glu
   325 330 335
   Arg
<210> 57
   <211> 143
   <212> PRT
   <213> Bacteria <prokaryote>
<220>
   <223> NT002 Variant from Fi176
<400> 57
   Cys Ser Ser Phe Gln Asn Asp Asp Tyr Ala Met Asn Tyr Lys Gly Gln
   1               5                   10                  15
   Ile Gly Asp Pro Ile Met Ala Ile Ala Met Leu Ser Glu Gln Gln His
               20                  25                  30
   Glu Trp Ala Gly Thr Pro Tyr Val Leu Gly Gly Val Ser Arg Arg Gly
           35                  40                  45
   Val Asp Cys Ser Gly Phe Val Gln Lys Thr Phe Phe Asp Arg Phe Asn
       50                  55                  60
   Leu Arg Leu Pro Arg Ser Thr Val Glu Gln Ala Asn Tyr Gly Lys His
   65                  70                  75                  80
   Val Arg Lys Glu Asp Ile Gln Thr Gly Asp Leu Ile Phe Phe Lys Thr
                   85                  90                  95
   Gly Arg Gly Pro Asn Gly Tyr His Val Gly Ile Tyr Val Lys Glu Asp
               100                 105                 110
   Lys Phe Leu His Ala Ser Thr Arg Gly Gly Val Val Tyr Ser Ser Met
           115                 120                 125
   Asn Asn Pro Tyr Trp Ser Lys Ala Phe Trp Gln Val Arg Arg Ile
       130                 135                 140
<210> 58
   <211> 146
   <212> PRT
   <213> Bacteria <prokaryote>
<220>
   <223> NT026 Variant from FI176
<400> 58
   Val Pro Leu Trp Lys Thr Asp Ser Pro Lys Thr Ile Leu Ala Lys Glu
   1               5                   10                  15
   Gln His Arg Leu Tyr Leu Phe Leu Arg Gln Ile Gln Ala Arg Ala Glu
               20                  25                  30
   Asn Ser Ser Glu Val Trp Phe Leu Leu Ile Asn Arg Asn Leu Ala Thr
           35                  40                  45
   Gln Gln Trp Cys Leu Thr Ala Gln Val Lys Asn Asn Gln Thr Cys Asp
       50                  55                  60
   Cys Leu Asn Pro Ile Asn Cys Pro Lys Glu Val Tyr Val His Phe Tyr
   65                  70                  75                  80
   Tyr Pro Tyr Phe Pro Asn Lys Thr Ile Ile Gln Ser His His Ile Tyr
                   85                  90                  95
   Pro Lys Glu Ile Thr Arg Phe Asp Gly Ile Arg Asn Thr Ile Val Thr
               100                 105                 110
   Arg Cys Phe Ile Leu Gln Ala Glu Asn Glu Arg Thr Leu Phe Leu Phe
           115                 120                 125
   Phe Asn Val Gly Ser Ile Arg Leu Lys Thr Asn Gln Phe Asp Ser Ala
       130                 135                 140
   Cys Asn
   145
<210> 59
   <211> 556
   <212> PRT
   <213> Bacteria <prokaryote>
<220>
   <223> NT009Variant from Fi176
<400> 59
   Glu Gln Thr Val Asp Ile Glu Val Gln Gly Ile Arg Gly Phe Arg Ala
   1               5                   10                  15
   Val Arg Asn Thr Asp Leu Asn Val Asn Leu Ile Asn Lys Glu Glu Met
               20                  25                  30
   Asp Gly Ser Glu Arg Tyr Gln His Leu Val Thr Lys Ala Val Asp Arg
           35                  40                  45
   Gly Leu Arg Val Phe Gly Tyr Tyr Asp Ser Ser Val Arg Phe Glu Arg
       50                  55                  60
   Lys Gln Arg Gln Gly Lys Arg Asp Leu Leu Ile Ala His Val Thr Pro
   65                  70                  75                  80
   Gly Glu Pro Thr Lys Ile Ala Gly Thr Asp Val Gln Ile Glu Gly Glu
                   85                  90                  95
   Ala Ala Gln Asp Glu Asn Phe Asn Ala Leu Arg Lys Asn Leu Pro Lys
               100                 105                 110
   Asp Gly Val Leu Val Glu His Gln Thr Tyr Asp Asp Tyr Lys Thr Ala
           115                 120                 125
   Ile Ser Arg Leu Ala Leu Asn Arg Gly Tyr Phe Asp Gly Glu Phe Lys
       130                 135                 140
   Ile Ser Arg Leu Glu Ile Ser Pro Glu Thr His Gln Ala Trp Trp Arg
   145                 150                 155                 160
   Met Leu Phe Asp Ser Gly Val Arg Tyr His Tyr Gly Asn Ile Thr Phe
                   165                 170                 175
   Ser His Ser Gln Ile Arg Asp Asp Tyr Leu Asn Asn Ile Leu Asn Ile
               180                 185                 190
   Lys Ser Gly Asp Pro Tyr Leu Met Asn Asn Leu Ser Asp Leu Thr Ser
           195                 200                 205
   Asp Phe Ser Ser Ser Asn Trp Phe Ser Ser Val Leu Val Gln Pro Asn
       210                 215                 220
   Ile Asn His Lys Ser Lys Thr Val Asp Ile Glu Ile Ile Leu Tyr Pro
   225                 230                 235                 240
   Arg Lys Lys Asn Ala Met Glu Leu Gly Val Gly Phe Asp Thr Asp Gly
                   245                 250                 255
   Gly Val His Gly Gln Ile Gly Trp Thr Lys Pro Trp Ile Asn Ser Arg
               260                 265                 270
   Gly His Ser Leu Arg Ser Asn Leu Tyr Leu Ser Ala Pro Lys Gln Thr
           275                 280                 285
   Leu Glu Ala Thr Tyr Arg Ile Pro Leu Leu Lys Asn Pro Leu Asn Tyr
       290                 295                 300
   Tyr Tyr Asp Phe Ala Val Gly Trp Glu Gly Glu Lys Glu Asn Asp Thr
   305                 310                 315                 320
   Asn Thr Arg Ala Leu Thr Leu Ser Ala Leu Arg Tyr Trp Asn Asn Ala
                   325                 330                 335
   His Gly Trp Gln Tyr Phe Gly Gly Leu Arg Thr Arg Tyr Asp Ser Phe
               340                 345                 350
   Thr Gln Ala Asp Ile Thr Asp Lys Thr Leu Leu Leu Tyr Pro Thr Val
           355                 360                 365
   Gly Phe Thr Arg Thr Arg Leu Arg Gly Gly Ser Phe Ala Thr Trp Gly
   370 375 380
   Asp Val Gln Lys Ile Thr Phe Asp Leu Ser Lys Arg Ile Trp Leu Ser
   385 390 395 400
   Glu Ser Ser Phe Ile Lys Val Gln Ala Ser Ser Ala Trp Ile Arg Thr
   405 410 415
   Tyr Ala Glu Asn His Arg Ile Val Ala Arg Ala Glu Ile Gly Tyr Leu
   420 425 430
   His Thr Lys Asp Ile Glu Lys Ile Pro Pro Thr Leu Arg Phe Phe Ala
   435 440 445
   Gly Gly Asp Arg Ser Val Arg Gly Tyr Gly Tyr Lys Lys Ile Ala Pro
   450 455 460
   Lys Asn Arg Asn Gly Lys Leu Val Gly Gly Ser Arg Leu Leu Thr Thr
   465 470 475 480
   Ser Leu Glu Tyr Gln Tyr Gln Val Tyr Pro Asn Trp Trp Ala Ala Thr
   485 490 495
   Phe Ala Asp Ser Gly Leu Ala Ala Asp Asn Tyr Thr Ala Lys Glu Leu
   500 505 510
   Arg Tyr Gly Ala Gly Val Gly Val Arg Trp Ala Ser Pro Val Gly Ala
   515 520 525
   Ile Lys Phe Asp Ile Ala Thr Pro Ile Arg Asp Lys Asp Asn Ser Lys
   530 535 540
   Asn Ile Gln Phe Tyr Ile Gly Leu Gly Thr Glu Ile
   545 550 555
<210> 60
   <211> 126
   <212> PRT
   <213> Bacteria <prokaryote>
<220>
   <223> NT025Variant from Fi176
<400> 60
   Ile Ile Ala Ile Leu Ala Thr Ile Ala Ile Pro Ser Tyr Gln Asn Tyr
   1 5 10 15
   Thr Lys Lys Ala Ala Val Ser Glu Leu Leu Gln Ala Ser Ala Pro Tyr
   20 25 30
   Lys Ala Asp Val Glu Leu Cys Val Tyr Ser Thr Asn Glu Thr Thr Asn
   35 40 45
   Cys Thr Gly Gly Lys Asn Gly Ile Ala Ala Asp Ile Thr Thr Ala Lys
   50 55 60
   Gly Tyr Val Lys Ser Val Thr Thr Ser Asn Gly Ala Ile Thr Val Lys
   65 70 75 80
   Gly Asp Gly Thr Leu Ala Asn Met Glu Tyr Ile Leu Gln Ala Thr Gly
   85 90 95
   Asn Ala Ala Thr Gly Val Thr Trp Thr Thr Thr Cys Lys Gly Thr Asp
   100 105 110
   Ala Ser Leu Phe Pro Ala Asn Phe Cys Arg Ser Val Thr Lys
   115 120 125
<210> 61
   <211> 162
   <212> PRT
   <213> Bacteria <prokaryote>
<220>
   <223> NT028Variant from Fi176
<400> 61
   Pro Arg Thr Val Ser His Gln Val Ile Ser Glu Asn Asp Asp Ile Gln
   1               5                   10                  15
   Leu Thr Gly Leu Ile Asn Asn Leu Glu Lys Asp Asn Arg Thr Gly Ile
               20                  25                  30
   Phe His Lys Val Arg Thr Asn Arg Ser Ser Ala Leu Met Gly Asp Lys
           35                  40                  45
   Ala Leu Ala Ser Val Tyr Asn Glu Trp Val Gly Thr Arg Tyr Arg Met
       50                  55                  60
   Gly Gly Thr Thr Lys Arg Gly Ile Asp Cys Ser Ala Phe Met Gln Thr
   65                  70                  75                  80
   Thr Phe Ser Glu Val Phe Gly Ile Glu Leu Pro Arg Ser Thr Ala Glu
                   85                  90                  95
   Gln Arg His Leu Gly Arg Lys Ile Asn Lys Ser Glu Leu Lys Lys Gly
               100                 105                 110
   Asp Leu Val Phe Phe Arg Lys Asn Asn His Val Gly Val Tyr Ile Gly
           115                 120                 125
   Asn Asn Gln Phe Met His Ala Ser Thr Gly Gln Gly Val Thr Ile Ser
       130                 135                 140
   Ser Leu Asp Glu Lys Tyr Trp Ala Arg Thr Tyr Thr Gln Ser Arg Arg
   145                 150                 155                 160
   Ile Met
<210> 62
   <211> 895
   <212> PRT
   <213> Bacteria <prokaryote>
<220>
   <223> NT029 Variant from R2846
<400> 62
   Ser Thr Pro Asp Leu Pro Gln Asn His Lys Ile Ile Thr Gly Thr Ala
   1               5                   10                  15
   Thr Val Ser His Thr Glu Asn Glu Met Thr Ile Lys Gln Thr Thr Pro
               20                  25                  30
   Thr Thr Gln Ile Asn Trp Asp Ser Phe Asn Ile Gly Lys Asp Lys Glu
           35                  40                  45
   Val Lys Phe Glu Gln Pro Ser Thr Ser Ala Val Ala Tyr Asn Arg Val
       50                  55                  60
   Thr Gly Gly Asn Ala Ser His Ile Gln Gly Lys Leu Thr Ala Asn Gly
   65                  70                  75                  80
   Lys Val Tyr Leu Ala Asn Pro Asn Gly Val Ile Ile Thr Lys Gly Ala
                   85                  90                  95
   Glu Ile Asn Val Ala Gly Leu Leu Ala Thr Thr Lys Asp Leu Glu Arg
               100                 105                 110
   Ile Ser Glu Asn Gly Asn Thr Asn Thr Asn Lys Phe Thr Arg Lys Ala
           115                 120                 125
   Lys Glu Gly Lys Val Leu Thr Glu Gly Gln Val Ile Asn Glu Gly Glu
       130                 135                 140
   Ile Lys Ala Lys Asp Phe Val Val Leu Asn Gly Asp Glu Val Ile Asn
   145                 150                 155                 160
   Lys Gly Asn Ile Asn Val Glu Lys Asn Ser Thr Ile Asn Gly Glu Val
                   165                 170                 175
   Tyr Leu Ser Ser Ser Asn Asn Phe Thr Phe Thr Leu Ser Asp Ser Gly
               180                 185                 190
   Ile Ser Val Ala Leu Glu Asp Asn Thr Val Gln Gly Ile Val Lys Asn
           195                 200                 205
   Glu Gly Ile Val Lys Asn Glu Gly Ser Ile Lys Ala Gly Glu Ile Thr
       210                 215                 220
   Leu Ser Ala Lys Gly Arg Lys Glu Ala Leu Asp Ser Leu Val Val Asn
   225                 230                 235                 240
   Asn Gly Val Leu Glu Ala Thr Lys Val Ser Asn Arg Lys Gly Lys Ile
                   245                 250                 255
   Val Leu Ser Ala Asp Asp Val Gln Leu Asn Asn Asn Ser Asp Ile Lys
               260                 265                 270
   Gly Glu Ile Val Asn Phe Gly Thr Glu Val Thr Ser Asn Glu Asp Lys
           275                 280                 285
   Lys Leu Lys Ile Thr Ser Gln Thr Gly Ser Lys Val Thr Ser Pro Lys
       290                 295                 300
   Ile Asn Phe Lys Gly Lys Ser Val Asn Ile Lys Gly Asp Phe Gly Arg
   305                 310                 315                 320
   Glu Asp Asn Thr Thr Tyr Tyr Asp Asp Glu His Lys Lys Leu Lys Thr
                   325                 330                 335
   Glu Val Asn Ile Asp Val Pro Asn Thr Glu Asn Ile Gln Ile Ala Asp
               340                 345                 350
   Lys Asp Asn Ala Gly Thr Asp Ser Phe Ile Gln Thr Gly Ala Leu Ser
           355                 360                 365
   Ser Leu Leu Ala Asn Asn Gly Lys Val Asn Leu Lys Gly Lys Asp Val
       370                 375                 380
   Asn Ile Ser Gly Asn Ile Asn Ile Asn Ser Phe Arg Gly Thr Asp Ser
   385                 390                 395                 400
   Leu Leu Lys Leu Thr Asn Lys Gly His Ile Asn Ile Asn His Ala Asp
                   405                 410                 415
   Ile His Ser Lys Gly Arg Leu Phe Phe Ile Thr Ser Leu Gln Asn Asp
               420                 425                 430
   Val Asp Phe Gln Ser Asn Ile Thr Ile Thr Asp Ser Lys Ile Asn Leu
           435                 440                 445
   Gly Asn Gly Ala Met Gly Leu Gly Arg Ser Val Asn Glu Asn Asp Leu
       450                 455                 460
   Asp Arg Trp Arg Arg Thr Glu Tyr Ser Gln Arg Lys Lys Phe Asn Val
   465                 470                 475                 480
   Asn Met Arg Asn Val Val Phe Asp Gln Val Asp Asp Val Val Val Ala
                   485                 490                 495
   Gly Gly Phe Lys Glu Val Asn Leu Asn Asn Ile Val Ala Thr Gly Gln
               500                 505                 510
   Thr Asn Phe Tyr Ile Asp Gly Gly Val Ser Arg Asn Arg Asn Gly Val
           515                 520                 525
   Ser Ser Lys Tyr Glu Tyr Gly Val Leu Asp Leu Asp Lys Arg Thr Gln
       530                 535                 540
   Leu Ser Glu Leu Asp Gln Arg Arg Arg Arg Trp Gly Tyr Tyr Pro Asp
   545                 550                 555                 560
   Leu Asp Leu Asp Met Asn Lys Ala Tyr Trp His Arg Phe Asp Met Phe
                   565                 570                 575
   Ala Ser Lys Asn Thr Gly Arg Ser Thr Ile Lys Asp Thr Glu Ile Asn
               580                 585                 590
   Ile Ser Asn Ser Lys Ile Asn Leu Lys Asn Gly Phe Val His Leu Leu
           595                 600                 605
   Ala Glu Lys Ile Lys Leu Asp Asn Ser Lys Ile Asp Ile Thr Phe Asp
       610                 615                 620
   Lys Asp Asn Ser Gln Asp Ile Ser Thr Gln Ile Asn Arg Leu Gly Met
   625                 630                 635                 640
   Asn Gly Lys Val Ser Met Val Asn Ser His Ile Lys Ile Val Gly Asp
                   645                 650                 655
   Glu Lys Ile Asp Ile Ser Ala Lys Ala Pro Tyr Ala Thr Met Phe Leu
               660                 665                 670
   Ile Gly Glu Leu Ile Gly Glu Lys Ser Ser Ile Phe Val Lys Ser His
           675                 680                 685
   Gln Gly Tyr Thr Phe Arg Thr Asp Gly Asp Thr Lys Ile Ala Gly Lys
       690                 695                 700
   Asn Ser Lys Asp Asp Leu Lys Ile Thr Ala Ile Asn Thr Gly Gly Arg
   705                 710                 715                 720
   Thr Gly Lys Glu Val Ile Ile Asn Gly Ala Pro Gly Ser Ile Asp Asn
                   725                 730                 735
   Asp Ala Asn Ile Ala Asn Met Ala Phe Thr Ile Gly Asp Asn Ala Asn
               740                 745                 750
   Thr Lys Thr Thr Ile Glu Asn Ala Asp Ile Thr Ala Leu Ala Pro Asn
           755                 760                 765
   Gly Gly Thr Ala Tyr Leu Ser Ser Lys Gly Val Glu Ile Glu Val Asn
       770                 775                 780
   Pro Asn Ser Asn Phe Thr Phe Phe Glu Leu Pro Arg Glu Lys Asn Phe
   785                 790                 795                 800
   Asn Gln Thr Lys Ile Asn Gly Asp Ser Thr Lys Leu Ser Glu Arg Gly
                   805                 810                 815
   Phe Ala Arg Leu Tyr Asp Lys Ile Asn Gly Val Arg Ala Ser Asn Leu
               820                 825                 830
   Ser Ala Glu Gln Leu Asn Val Thr Asp Ser Ser Glu Lys Ile Ile Asn
           835                 840                 845
   Thr Lys Leu Val Ser Ser Leu Asp Val Glu Lys Leu Val Ser Val Ala
       850                 855                 860
   Val Cys Asp Ala Gly Lys Gly Cys Glu Glu Gln Gln Phe Gly Asp Lys
   865                 870                 875                 880
   Gly Asn Asn Thr Lys Val Ser Val Gly Glu Leu Glu Ala Glu Gln
                   885                 890                 895
<210> 63
   <211> 183
   <212> PRT
   <213> Bacteria <prokaryote>
<220>
   <223> NT031Variant from R2846
<210> 64
   <211> 117
   <212> PRT
   <213> Bacteria <prokaryote>
<220>
   <223> NT015Variant from Fi176
<400> 64
   Ala Gln Asn Ala Asn Val Thr Thr Pro Gln Ala Gln Lys Met Gln Val
   1 5 10 15
   Glu Lys Val Asp Lys Ala Leu Gln Lys Gly Glu Ala Asp Arg Tyr Leu
   20 25 30
   Cys Gln Asp Asp Lys Val Val Arg Val Val His Ala Thr His Lys Lys
   35 40 45
   Tyr Lys Lys Asn Leu His Tyr Val Thr Val Thr Phe Gln Gly Val Ser
   50 55 60
   Glu Lys Leu Thr Leu Met Ile Ser Glu Arg Gly Lys Asn Tyr Ala Asn
   65 70 75 80
   Ile Arg Trp Met Trp Gln Glu Arg Asp Asp Phe Ser Thr Leu Lys Thr
   85 90 95
   Asn Leu Gly Glu Ile Leu Ala Thr Gln Cys Val Ser Gln Thr Ser Glu
   100 105 110
   Arg Leu Ser Gly Gln
   115
<210> 65
   <211> 134
   <212> PRT
   <213> Bacteria <prokaryote>
<220>
   <223> NT023Variant from Fi176
<400> 65
   Asn Thr Asp Ile Phe Ser Gly Asp Val Tyr Ser Ala Ser Gln Ala Lys
   1 5 10 15
   Glu Ala Arg Ser Ile Thr Tyr Gly Thr Ile Val Ser Val Arg Pro Val
   20 25 30
   Lys Ile Gln Ala Asp Asn Gln Gly Val Val Gly Thr Leu Gly Gly Gly
   35 40 45
   Ala Leu Gly Gly Ile Ala Gly Ser Ala Ile Gly Gly Gly Arg Gly Gln
   50 55 60
   Ala Ile Ala Ala Val Val Gly Ala Ile Gly Gly Ala Ile Ala Gly Ser
   65 70 75 80
   Lys Ile Glu Glu Lys Met Ser Gln Val Asn Gly Ala Glu Leu Val Ile
   85 90 95
   Lys Lys Asp Asp Gly Gln Glu Ile Val Val Val Gln Lys Ala Asp Ser
   100 105 110
   Ser Phe Val Ala Gly Arg Arg Val Arg Ile Val Gly Gly Gly Ser Ser
   115 120 125
   Leu Asn Val Ser Val Leu
   130
<210> 66
   <211> 345
   <212> PRT
   <213> Bacteria <prokaryote>
<220>
   <223> NT100 Variant from R2846
<400> 66
   Met Asp Leu Gly Pro Ile Tyr Asn Thr Arg Asp Ile Asn Asp Gly Lys
   1               5                   10                  15
   Val Ile Asn Ile Asp Asn Pro Asn Tyr Thr Asn Pro Val Ala Ile Lys
               20                  25                  30
   Lys Asn Glu Asn Asn Asn Ala Tyr Gln Phe Asn His Leu Lys Thr Leu
           35                  40                  45
   Gly Leu Tyr Ile Gln Asn Thr Thr Tyr Phe Thr Asp Asn Phe Ile Ile
       50                  55                  60
   Thr Gly Gly Leu Arg Tyr Glu Tyr Phe Asp Gln Val Val Gly Arg Ser
   65                  70                  75                  80
   Thr Leu Lys Asn Ile Arg Ser Gly Tyr Leu Ala Gln Lys Asp Gly Lys
                   85                  90                  95
   Leu Leu Tyr Gln Leu Gly Ser Val Tyr Lys Phe Thr Pro Asn Ile Ala
               100                 105                 110
   Thr Phe Phe Asn His Ala Glu Ser Phe Arg Pro Gln Asn Asn Arg Thr
           115                 120                 125
   Leu Ile Ile Asn Gly Glu Leu Pro Ala Glu Gln Gly Lys Ser Phe Glu
       130                 135                 140
   Thr Gly Leu Lys Tyr Glu Asn Ala Tyr Leu Asn Ala Thr Val Ala Leu
   145                 150                 155                 160
   Phe Asn Ile Asn Lys Arg Asn Val Ala Glu Thr Val Asn Val Asn Gly
                   165                 170                 175
   Thr Asn Glu Leu Gln Ile Val Gly Lys Gln Arg Ser Arg Gly Ile Glu
               180                 185                 190
   Phe Asp Leu Asn Gly Gln Leu Thr Asp Asn Leu Ser Ile Ala Ala Asn
           195                 200                 205
   Tyr Thr Tyr Thr Lys Val Lys Asn Leu Glu Asn His Asn Asn Lys Leu
       210                 215                 220
   Ala Val Gly Lys Gln Leu Ser Gly Val Pro Lys His Gln Ala Ser Leu
   225                 230                 235                 240
   Phe Leu Ala Tyr Asn Ile Gly Glu Phe Asp Phe Gly Asn Ile Arg Val
                   245                 250                 255
   Gly Gly Gly Ala Arg Tyr Leu Gly Ser Trp Tyr Ala Tyr Asn Asn Thr
               260                 265                 270
   Tyr Thr Lys Ala Tyr Lys Leu Pro Gln Ala Ile Val Tyr Asp Ala Phe
           275                 280                 285
   Ile Ala Tyr Asp Thr Lys Ile Ser Gly Lys Lys Val Ser Phe Gln Leu
       290                 295                 300
   Asn Gly Lys Asn Leu Ser Asn Lys Val Tyr Ser Pro Ser Thr Ser Gly
   305                 310                 315                 320
   Asn Ala Ser Arg Thr Leu Ile Pro Val Ala Leu Gly Tyr Ala Arg Glu
                   325                 330                 335
   Val Ile Leu Asn Thr Lys Ile Glu Phe
               340                 345
<210> 67
   <211> 505
   <212> PRT
   <213> Bacteria <prokaryote>
<220>
   <223> NT066 variant from Fi176
<400> 67
   Phe Lys Lys Ser Leu Ile Val Ala Ala Ser Phe Ala Ser Leu Ser Leu
   1               5                   10                  15
   Phe Asn Ser Ala Thr Ala Glu Leu Val Tyr Lys Pro Leu Glu Gln Pro
               20                  25                  30
   Val Glu Pro Ala Lys Pro Asp Leu Lys Ile Glu Ser Val Asn Glu Lys
           35                  40                  45
   Phe Ala Glu Lys Tyr Pro Asn Gln Tyr Asn Ser Trp Arg Ser Thr Ala
       50                  55                  60
   Asn Gly Asp Gly Glu Asn Ile Ile Tyr Ala Asp Glu Glu Asn Pro Arg
   65                  70                  75                  80
   Leu Ile Val Leu Trp Gly Gly Tyr Ala Phe Ala Lys Glu Tyr Asn Ala
                   85                  90                  95
   Pro Arg Gly His Phe Tyr Ala Val Thr Asp Val Arg Asn Ile Leu Arg
               100                 105                 110
   Thr Gly Ala Pro Lys Thr Ala Asn Asp Gly Pro Gln Ala Met Ala Cys
           115                 120                 125
   Trp Thr Cys Lys Gly Pro Asp Val Pro Arg Leu Ile Ala Glu Trp Gly
       130                 135                 140
   Glu Lys Asp Tyr Phe Asn Ala Lys Trp Ala Lys Gly Gly Pro Glu Ile
   145                 150                 155                 160
   Val Asn Ser Ile Gly Cys Ala Asp Cys His Asp Thr Thr Ser Lys Asp
                   165                 170                 175
   Phe Ala Glu Gly Lys Pro Ala Leu Arg Ile Ala Arg Pro His Ile Leu
   180 185 190
   Arg Ala Leu Asp Ala Leu Glu Lys Ala Thr Ala Glu Lys Asp Lys Ala
   195 200 205
   Glu Gly Arg Pro His Asn Asn Leu Ser Phe Asn Thr Ala Ala Arg Thr
   210 215 220
   Glu Lys Arg Ala Glu Ile Cys Ala Asn Cys His Val Glu Tyr Tyr Phe
   225 230 235 240
   Ala Gly Asp Ile Lys Gln Val Thr Phe Pro Trp Asp Asn Gly Gln Thr
   245 250 255
   Val Asp Asp Ile Glu Lys Tyr Tyr Asp Asp Ile Gly Phe Thr Asp Trp
   260 265 270
   Thr His Ser Leu Ser Lys Ala Pro Met Leu Lys Ala Gln His Pro Asp
   275 280 285
   Phe Glu Ile Trp Ser Leu Gly Met His Gly Lys Asn Gly Val Thr Cys
   290 295 300
   Val Asp Cys His Met Pro Lys Val Gln Gly Ala Asp Gly Lys Val Tyr
   305 310 315 320
   Thr Asp His Gln Ile Gln Asn Pro Phe Glu Ala Phe Asp His Thr Cys
   325 330 335
   Ala Asn Cys His Asp Gln Ser Lys Glu Lys Leu Arg Asp Ile Val Thr
   340 345 350
   Ser Arg Lys Lys Glu Val Lys Asp Val Met Gly Arg Leu Glu Asp Gln
   355 360 365
   Val Val Lys Ala His Phe Glu Ala Lys Ala Ala Trp Asp Ala Gly Ala
   370 375 380
   Thr Lys Glu Glu Met Glu Ala Ala Leu Met Asp Ile Arg His Ala Gln
   385 390 395 400
   Trp Arg Trp Asp Tyr Thr Ala Ala Ser His Gly Gly His Met His Ala
   405 410 415
   Pro Glu Val Val Leu Arg Val Leu Ala Ser Gly Leu Asp Lys Val Ala
   420 425 430
   Asp Ala Arg Thr Lys Leu Ala Val Ile Leu Thr Lys His Gly Val Lys
   435 440 445
   Thr Pro Val Gln Ile Pro Asp Ile Ser Thr Ala Asp Lys Ala Trp Lys
   450 455 460
   Val Met Gly Ile Asp Ile Glu Lys Glu Arg Lys Ala Lys Glu Glu Phe
   465 470 475 480
   Leu Lys Thr Val Val Pro Gln Trp Glu Gln Gln Ala Arg Glu Lys Gly
   485 490 495
   Leu Leu Val Asp Pro Pro Ala Gln Lys
   500 505
<210> 68
   <211> 6
   <212> PRT
   <213> Bacteria <prokaryote>
<220>
   <223> His tag
<400> 68
   His His His His His His
   1 5
<210> 69
   <211> 28
   <212> PRT
   <213> Bacteria <prokaryote>
<220>
   <223> NT018 N-terminus
<400> 69
   Met Asn Phe Thr Leu Ile Phe Ile Leu Thr Thr Leu Val Val Ala Leu
   1               5                   10                  15
   Ile Cys Phe Tyr Pro Leu Leu Arg Gln Phe Lys Ala
               20                  25
<210> 70
   <211> 20
   <212> PRT
   <213> Bacteria <prokaryote>
<220>
   <223> NT024 N-Terminus
<400> 70
   Met Lys Leu Lys Leu Phe Phe His Ile Val Leu Leu Cys Phe Ser Leu
   1               5                   10                  15
   Pro Val Trp Ala
               20
<210> 71
   <211> 19
   <212> PRT
   <213> Bacteria <prokaryote>
<220>
   <223> NT032 N-terminus
<400> 71
   Met Lys Lys Phe Ala Leu Ala Thr Ile Phe Ala Leu Ala Thr Thr Ser
   1               5                   10                  15
   Ala Phe Ala
<210> 72
   <211> 20
   <212> PRT
   <213> Bacteria <prokaryote>
<220>
   <223> NT067 N terminus
<400> 72
   Met Gln His Lys Leu Leu Phe Ser Ala Ile Ala Leu Ala Leu Ser Tyr
   1               5                   10                  15
   Ser Val Gln Ala
               20
<210> 73
   <211> 23
   <212> PRT
   <213> Bacteria <prokaryote>
<220>
   <223> NT038 N-terminus
<400> 73
   Met Pro Phe Gln Tyr Val Thr Glu Asp Gly Lys Thr Val Val Lys Val
   1               5                   10                  15
   Gly Asn Gly Tyr Tyr Glu Ala
               20
<210> 74
   <211> 21
   <212> PRT
   <213> Bacteria <prokaryote>
<220>
   <223> NT001 N-terminus
<400> 74
   Met Lys Leu Lys Gln Leu Phe Ala Ile Thr Ala Ile Ala Ser Ala Leu
   1               5                   10                  15
   Val Leu Thr Gly Cys
               20
<210> 75
   <211> 19
   <212> PRT
   <213> Bacteria <prokaryote>
<220>
   <223> NT016 N-terminus
<400> 75
   Met Arg Lys Ile Lys Ser Leu Ala Leu Leu Ala Val Ala Ala Leu Val
   1               5                   10                  15
   Ile Gly Cys
<210> 76
   <211> 11
   <212> PRT
   <213> Bacteria <prokaryote>
<220>
   <223> NT052 N-terminus
<400> 76
   Met Leu Leu Phe Ile Leu Ser Ile Ala Trp Ala
   1               5                   10
<210> 77
   <211> 18
   <212> PRT
   <213> Bacteria <prokaryote>
<220>
   <223> NT002 N-terminus
<400> 77
   Met Lys Val Tyr Lys Ser Phe Leu Ile Ala Thr Ala Ser Leu Phe Leu
   1               5                   10                  15
   Phe Ala
<210> 78
   <211> 24
   <212> PRT
   <213> Bacteria <prokaryote>
<220>
   <223> NT026 N-terminus
<400> 78
   Met Gln Lys Gly Met Thr Leu Val Glu Leu Leu Ile Gly Leu Ala Ile
   1               5                   10                  15
   Ile Ser Ile Val Leu Asn Phe Ala
               20
<210> 79
   <211> 22
   <212> PRT
   <213> Bacteria <prokaryote>
<220>
   <223> NT009 N-terminus
<400> 79
   Met Asn Lys Thr Leu Leu Lys Leu Thr Ala Leu Phe Leu Ala Leu Asn
   1               5                   10                  15
   Cys Phe Pro Ala Phe Ala
               20
<210> 80
   <211> 23
   <212> PRT
   <213> Bacteria <prokaryote>
<220>
   <223> NT025 N-terminus
<400> 80
   Met Lys Leu Thr Thr Gln Gln Thr Leu Lys Lys Gly Phe Thr Leu Ile
   1               5                   10                  15
   Glu Leu Met Ile Val Ile Ala
               20
<210> 81
   <211> 21
   <212> PRT
   <213> Bacteria <prokaryote>
<220>
   <223> NT028 N-terminus
<400> 81
   Met Leu Lys Arg Ile Leu Val Ile Ile Gly Leu Ala Val Leu Ala Thr
   1               5                   10                  15
   Ala Cys Ser Asn Ala
               20
<210> 82
   <211> 21
   <212> PRT
   <213> Bacteria <prokaryote>
<220>
   <223> NT029 N-terminus
<400> 82
   Met Tyr Lys Leu Asn Val Ile Ser Leu Ile Ile Leu Thr Thr Tyr Thr
   1               5                   10                  15
   Gly Ala Thr Tyr Ala
               20
<210> 83
   <211> 18
   <212> PRT
   <213> Bacteria <prokaryote>
<220>
   <223> NT031 N-terminus
<400> 83
   Met Lys Gly Lys Ile Thr Leu Phe Phe Thr Ala Leu Cys Phe Gly Leu
   1               5                   10                  15
   Thr Gly
<210> 84
   <211> 17
   <212> PRT
   <213> Bacteria <prokaryote>
<220>
   <223> NT015 N-terminus
<400> 84
   Met Leu Lys Lys Thr Ser Leu Ile Phe Thr Ala Leu Leu Leu Ala Gly
   1               5                   10                  15
   Cys
<210> 85
   <211> 20
   <212> PRT
   <213> Bacteria <prokaryote>
<220>
   <223> NT023 N-terminus
<400> 85
   Met Lys Lys Thr Asn Met Ala Leu Ala Leu Leu Val Ala Phe Ser Val
   1               5                   10                  15
   Thr Gly Cys Ala
               20
<210> 86
   <211> 30
   <212> PRT
   <213> Bacteria <prokaryote>
<220>
   <223> NT100 N-terminus
<400> 86
   Met Asp Leu Gly Pro Ile Tyr Asn Thr Arg Asp Ile Asn Asp Gly Lys
   1               5                   10                  15
   Val Ile Asn Ile Asp Asn Pro Asn Tyr Thr Asn Pro Val Ala
               20                  25                  30
<210> 87
   <211> 26
   <212> PRT
   <213> Bacteria <prokaryote>
<220>
   <223> NT040 N-terminus
<400> 87
   Met Met Lys Thr Leu Leu Lys Gly Gln Thr Leu Leu Ala Leu Met Ile
   1               5                   10                  15
   Ser Leu Thr Leu Ser Ser Leu Leu Leu Leu
               20                  25
<210> 88
   <211> 18
   <212> PRT
   <213> Bacteria <prokaryote>
<220>
   <223> NT048 N-terminus
<400> 88
   Met Lys Ser Val Pro Leu Ile Thr Gly Gly Leu Ser Phe Leu Leu Ser
   1               5                   10                  15
   Ala Cys
<210> 89
   <211> 22
   <212> PRT
   <213> Bacteria <prokaryote>
<220>
   <223> NT124 N-terminus
<400> 89
   Met Lys Lys Ser Lys Ile Ala Ala Gly Val Val Ile Ser Leu Ala Ala
   1               5                   10                  15
   Val Trp Cys Ala Gly Ala
               20
<210> 90
   <211> 33
   <212> PRT
   <213> Bacteria <prokaryote>
<220>
   <223> NT066 N-terminus
<400> 90
   Met Lys Ile Tyr Leu Arg Phe Val Trp Ile Leu Ile Ile Ile Leu Asn
   1               5                   10                  15
   Phe Leu Leu Asn Leu Phe Ile Thr Thr Asn Gly Val Ile Ile Val Asn
               20                  25                  30
   Ala
<210> 91
   <211> 22
   <212> PRT
   <213> Bacteria <prokaryote>
<220>
   <223> NT097 N-terminus
<400> 91
   Met Lys Lys Phe Asn Gln Ser Ile Leu Ala Thr Ala Met Leu Leu Ala
   1               5                   10                  15
   Ala Gly Gly Ala Asn Ala
               20
<210> 92
   <211> 25
   <212> PRT
   <213> Bacteria <prokaryote>
<220>
   <223> P48 N-terminus
<400> 92
   Met Ile Thr Ile Lys Lys Gly Leu Asp Leu Pro Ile Ala Gly Lys Pro
   1               5                   10                  15
   Ala Gln Val Ile His Ser Gly Asn Ala
               20                  25
<210> 93
   <211> 26
   <212> PRT
   <213> Bacteria <prokaryote>
<220>
   <223> HtrA N-terminus
<400> 93
   Met Lys Lys Thr Arg Phe Val Leu Asn Ser Ile Ala Leu Gly Leu Ser
   1               5                   10                  15
   Val Leu Ser Thr Ser Phe Val Ala Gln Ala
               20                  25
<210> 94
   <211> 16
   <212> PRT
   <213> Bacteria <prokaryote>
<220>
   <223> PE N-terminus
<400> 94
   Met Lys Lys Ile Ile Leu Thr Leu Ser Leu Gly Leu Leu Thr Ala Cys
   1               5                   10                  15
<210> 95
   <211> 18
   <212> PRT
   <213> Bacteria <prokaryote>
<220>
   <223> PhiD N-terminus
<400> 95
   Met Lys Leu Lys Thr Leu Ala Leu Ser Leu Leu Ala Ala Gly Val Leu
   1               5                   10                  15
   Ala Gly
<210> 96
   <211> 19
   <212> PRT
   <213> Bacteria <prokaryote>
<220>
   <223> P6 N-terminus
<400> 96
   Met Asn Lys Phe Val Lys Ser Leu Leu Val Ala Gly Ser Val Ala Ala
   1               5                   10                  15
   Leu Ala Ala
<210> 97
   <211> 42
   <212> PRT
   <213> Bacteria <prokaryote>
<220>
   <223> NT013 N-terminus
<400> 97
   Met Pro Val Gln His Val Lys Leu Ala Arg Asp Arg Arg Lys Lys Arg
   1               5                   10                  15
   Thr Tyr Ile Lys Val Gly Val Phe Phe Val Ala Ile Leu Leu Ile Leu
               20                  25                  30
   Thr Gly Ile Leu Leu Thr Ile Lys Asp Lys
           35                  40
<210> 98
   <211> 17
   <212> PRT
   <213> Bacteria <prokaryote>
<220>
   <223> NT106 N-terminus
<400> 98
   Met Lys Lys Ile Ile Leu Asn Leu Val Thr Ala Ile Ile Leu Ala Gly
   1               5                   10                  15
   Cys
<210> 99
   <211> 28
   <212> PRT
   <213> Bacteria <prokaryote>
<220>
   <223> NT107 N-terminus
<400> 99
   Met Lys Met Arg Pro Arg Tyr Ser Val Ile Ala Ser Ala Val Ser Leu
   1               5                   10                  15
   Gly Phe Val Leu Ser Lys Ser Val Met Ala Leu Gly
               20                  25
<210> 100
   <211> 24
   <212> PRT
   <213> Bacteria <prokaryote>
<220>
   <223> NT108 N-terminus
<400> 100
   Met Ser Val Cys Lys Pro Phe Trp Phe Lys Thr Phe Ser Ile Ser Ile
   1               5                   10                  15
   Ile Thr Ala Leu Leu Val Ser Cys
               20
<210> 101
   <211> 30
   <212> PRT
   <213> Bacteria <prokaryote>
<220>
   <223> NT109 N-terminus
<400> 101
   Met Ile Met Glu Leu Phe His Thr Ile Leu Ala Ile Val Ala Leu Ile
   1               5                   10                  15
   Leu Ser Ser Ala Val Val Ser Ser Ala Glu Ile Ser Leu Ala
               20                  25                  30
<210> 102
   <211> 30
   <212> PRT
   <213> Bacteria <prokaryote>
<220>
   <223> NT110 N-terminus
<400> 102
   Met Ile Met Glu Leu Phe His Thr Ile Leu Ala Ile Val Ala Leu Ile
   1               5                   10                  15
   Leu Ser Ser Ala Val Val Ser Ser Ala Glu Ile Ser Leu Ala
               20                  25                  30
<210> 103
   <211> 19
   <212> PRT
   <213> Bacteria <prokaryote>
<220>
   <223> NT111 N-terminus
<400> 103
   Met Lys Lys Thr Leu Val Ala Ala Leu Ile Ser Ser Val Ile Leu Leu
   1               5                   10                  15
   Thr Gly Cys
<210> 104
   <211> 19
   <212> PRT
   <213> Bacteria <prokaryote>
<220>
   <223> NT112 N-terminus
<400> 104
   Met Lys Thr Lys Val Ile Leu Thr Ala Leu Leu Ser Ala Ile Ala Leu
   1               5                   10                  15
   Thr Gly Cys
<210> 105
   <211> 16
   <212> PRT
   <213> Bacteria <prokaryote>
<220>
   <223> NT113 N-terminus
<400> 105
   Met Lys Lys Tyr Leu Leu Leu Ala Leu Leu Pro Phe Leu Tyr Ala Cys
   1               5                   10                  15
<210> 106
   <211> 19
   <212> PRT
   <213> Bacteria <prokaryote>
<220>
   <223> NT114 N-terminus
<400> 106
   Met Lys Lys Val Ala Leu Ile Ser Leu Cys Ile Phe Thr Ala Leu Ser
   1               5                   10                  15
   Ala Phe Ala
<210> 107
   <211> 35
   <212> PRT
   <213> Bacteria <prokaryote>
<220>
   <223> NT115 N-terminus
<400> 107
   Met Lys Tyr Leu His Phe Thr Arg Pro Thr Ile Lys Val Ile Phe Met
   1               5                   10                  15
   Ile Asn Ser Ile Lys Thr Leu Leu Leu Ile Ala Thr Leu Ala Ile Leu
               20                  25                  30
   Ser Ala Cys
           35
<210> 108
   <211> 18
   <212> PRT
   <213> Bacteria <prokaryote>
<220>
   <223> NT116 N-terminus
<400> 108
   Met Lys Ser Val Pro Leu Ile Thr Gly Gly Leu Ser Phe Leu Leu Ser
   1               5                   10                  15
   Ala Cys
<210> 109
   <211> 19
   <212> PRT
   <213> Bacteria <prokaryote>
<220>
   <223> NT117 N-terminus
<400> 109
   Met Lys Lys Leu Ile Ala Val Ala Val Phe Ser Ala Cys Gly Ser Leu
   1               5                   10                  15
   Ala His Ala
<210> 110
   <211> 18
   <212> PRT
   <213> Bacteria <prokaryote>
<220>
   <223> NT118 N-terminus
<400> 110
   Met Asn Ile Arg Trp Asn Val Ile Leu Gly Val Ile Ala Leu Cys Ala
   1               5                   10                  15
   Leu Ala
<210> 111
   <211> 17
   <212> PRT
   <213> Bacteria <prokaryote>
<220>
   <223> NT123 N-terminus
<400> 111
   Met Lys Lys Thr Thr Ala Leu Phe Leu Leu Ile Phe Ser Leu Ile Ala
   1               5                   10                  15
   Cys
<210> 112
   <211> 26
   <212> PRT
   <213> Bacteria <prokaryote>
<220>
   <223> Immunostimulatory oligonucleotide
<400> 112
   Ile Cys Ile Cys Ile Cys Ile Cys Ile Cys Ile Cys Ile Cys Ile Cys
   1               5                   10                  15
   Ile Cys Ile Cys Ile Cys Ile Cys Ile Cys
               20                  25
<210> 113
   <211> 11
   <212> PRT
   <213> Bacteria <prokaryote>
<220>
   <223> Polycationicoligopeptide
<400> 113
   Lys Leu Lys Leu Leu Leu Leu Leu Lys Leu Lys
   1               5                   10
<210> 114
   <211> 122
   <212> PRT
   <213> Bacteria <prokaryote>
<220>
   <223> NT119
<400> 114
   Asp Thr Leu Glu Gln Gln Phe Gln Gln Gly Ser Glu Ala Thr Thr Arg
   1               5                   10                  15
   Gly Asp Tyr Gln Thr Thr Phe Lys Phe Leu Leu Pro Leu Ala Glu Gln
               20                  25                  30
   Gly Asn Ala Glu Ala Gln Leu Met Leu Gly Val Met Tyr Ala Arg Gly
           35                  40                  45
   Ile Gly Val Lys Gln Asp Asp Phe Glu Ala Val Lys Trp Tyr Arg Gln
       50                  55                  60
   Ala Ala Glu Gln Gly Tyr Ala Asn Ala Gln Ala Ile Leu Gly Phe Ser
   65                  70                  75                  80
   Tyr Leu Leu Gly Gln Ser Gly Val Gln Val Asn Lys Ser Leu Ala Lys
                   85                  90                  95
   Glu Trp Phe Gly Lys Ala Cys Asp Asn Gly Asp Gln Asn Gly Cys Glu
               100                 105                 110
   Tyr Tyr Gly Lys Leu Asn Arg Gly Glu Leu
           115                 120
<210> 115
   <211> 158
   <212> PRT
   <213> Bacteria <prokaryote>
<220>
   <223> NT120
<400> 115
   Asp Thr Leu Glu Gln Gln Phe Gln Gln Gly Leu Thr Ala Tyr Glu Gln
   1               5                   10                  15
   Ser Asn Tyr Gln Thr Ala Phe Lys Leu Trp Leu Pro Met Ala Glu Gln
               20                  25                  30
   Gly Tyr Ala Lys Ala Gln Phe Asn Leu Gly Val Met Tyr Ala Lys Gly
           35                  40                  45
   Gln Gly Val Lys Gln Asp Asp Phe Glu Ala Val Lys Trp Phe Arg Lys
       50                  55                  60
   Ala Ala Glu Gln Gly Tyr Ala Glu Ala Lys Phe Asn Leu Gly His Met
   65                  70                  75                  80
   Tyr Ser Lys Gly Arg Gly Val Lys Gln Asp Asp Phe Glu Ala Val Asn
                   85                  90                  95
   Trp Tyr Arg Lys Ala Ala Glu Gln Gly Asp Ala Asp Ala Gln Ala Ile
               100                 105                 110
   Leu Gly Phe Leu Tyr Leu Leu Gly Glu Arg Gly Val Gln Val Asn Lys
           115                 120                 125
   Ser Leu Ala Lys Glu Trp Phe Gly Lys Ala Cys Asp Asn Gly Asn Gln
       130                 135                 140
   Asn Gly Cys Glu Tyr Tyr Gly Lys Leu Asn Arg Gly Glu Leu
   145                 150                 155
<210> 116
   <211> 157
   <212> PRT
   <213> Bacteria <prokaryote>
<220>
   <223> NT121
<400> 116
   Asp Thr Leu Glu Gln Gln Phe Gln Gln Gly Leu Thr Ala Tyr Glu Gln
   1               5                   10                  15
   Ser Asn Tyr Gln Thr Ala Phe Lys Leu Trp Leu Pro Leu Ala Glu Gln
               20                  25                  30
   Gly Asp Ala Asn Val Gln Phe Asn Leu Gly Val Met Tyr Ala Glu Gly
           35                  40                  45
   Gln Gly Val Lys Gln Asp Asp Phe Glu Ala Val Lys Trp Tyr Arg Lys
       50                  55                  60
   Ala Ala Glu Gln Gly Asp Ala Asn Ala Gln Ala Tyr Leu Gly Leu Ala
   65                  70                  75                  80
   Tyr Thr Glu Gly Arg Gly Val Arg Gln Asp Tyr Thr Glu Ala Val Lys
                   85                  90                  95
   Trp Phe Arg Lys Ala Ala Glu Gln Gly His Ala Asn Ala Gln Ala Ile
               100                 105                 110
   Leu Gly Phe Ser Tyr Leu Leu Gly Lys Gly Val Gln Val Asn Lys Ser
           115                 120                 125
   Leu Ala Lys Glu Trp Phe Gly Lys Ala Cys Asp Asn Gly Asp Gln Gly
       130                 135                 140
   Gly Cys Lys Tyr Tyr Gly Lys Leu Asn Arg Gly Glu Arg
   145                 150                 155
<210> 117
   <211> 168
   <212> PRT
   <213> Bacteria <prokaryote>
<220>
   <223> NT122
<400> 117
   Phe Asp Lys Gln Glu Ala Lys Gln Lys Val Glu Asp Thr Lys Gln Thr
   1               5                   10                  15
   Val Ala Ser Val Ala Ser Glu Thr Lys Asp Ala Ala Ala Asn Thr Met
               20                  25                  30
   Thr Glu Val Lys Glu Lys Ala Gln Gln Leu Ser Thr Asp Val Lys Asn
           35                  40                  45
   Lys Val Ala Glu Lys Val Glu Asp Ala Lys Glu Val Ile Lys Ser Ala
       50                  55                  60
   Thr Glu Thr Ala Ser Glu Lys Ala Thr Glu Ile Lys Glu Ala Val Ser
   65                  70                  75                  80
   Glu Lys Ala Ser Glu Met Lys Glu Ala Ala Ser Glu Lys Ala Ser Glu
                   85                  90                  95
   Met Lys Glu Ala Ala Ser Glu Lys Ala Ser Glu Met Lys Glu Ala Ala
               100                 105                 110
   Ser Glu Lys Ala Ser Glu Met Lys Glu Ala Ala Ser Glu Lys Ala Ser
           115                 120                 125
   Glu Met Lys Glu Ala Ala Ser Glu Lys Val Gly Glu Met Lys Glu Lys
       130                 135                 140
   Ala Thr Glu Met Lys Glu Ala Val Ser Glu Lys Ala Thr Gln Ala Val
   145                 150                 155                 160
   Asp Ala Val Lys Glu Ala Thr Lys
                   165
<210> 118
   <211> 26
   <212> PRT
   <213> Bacteria <prokaryote>
<220>
   <223> NT119encoded N-terminus
<400> 118
   Met Arg Phe Thr Lys Thr Leu Phe Thr Thr Ala Leu Leu Gly Ala Ser
   1               5                   10                  15
   Ile Phe Ser Phe Gln Ser Thr Ala Trp Ala
               20                  25
<210> 119
   <211> 26
   <212> PRT
   <213> Bacteria <prokaryote>
<220>
   <223> NT120encoded N-terminus
<400> 119
   Met Lys Leu Thr Lys Thr Leu Leu Thr Thr Ala Leu Phe Gly Ala Ser
   1               5                   10                  15
   Val Phe Ser Phe Gln Ser Thr Ala Trp Ala
               20                  25
<210> 120
   <211> 26
   <212> PRT
   <213> Bacteria <prokaryote>
<220>
   <223> NT121encoded N-terminus
<400> 120
   Met Lys Leu Thr Lys Thr Leu Leu Thr Thr Ala Leu Leu Gly Ala Ser
   1               5                   10                  15
   Val Phe Ser Phe Gln Ser Thr Ala Trp Ala
               20                  25
<210> 121
   <211> 23
   <212> PRT
   <213> Bacteria <prokaryote>
<220>
   <223> NT122encoded N-terminus
<400> 121
   Met Glu Lys Ile Met Lys Lys Leu Thr Leu Ala Leu Val Leu Gly Ser
   1               5                   10                  15
   Ala Leu Ala Val Thr Gly Cys
               20
<210> 122
   <211> 173
   <212> PRT
   <213> Bacteria <prokaryote>
<220>
   <223> NT004
<400> 122
   Ser Glu Glu Gln Val Gln Arg Asp Val Tyr Gln Ser Leu Asp Asp Cys
   1               5                   10                  15
   Leu Ala Asp Trp Lys Lys Ile Glu Leu Cys Glu Ala Asp Lys Asn Thr
               20                  25                  30
   Glu Ser Thr Gln Lys Thr Glu Thr Thr Pro Gln Gln Gly Leu Gly Leu
           35                  40                  45
   Asn Ile Arg Asp Asn Gly Asn Ala Glu Ser Ala Val Lys Asn Pro Ala
       50                  55                  60
   Glu Asn Asn Val Gln Ala Asn Gln Ser Glu Asn Asn Ala Glu Ser Thr
   65                  70                  75                  80
   Thr Lys Ala Glu Ser Thr Asp Pro Ser Leu Gly Ala Ala Ile Ala Gly
                   85                  90                  95
   Gly Val Met Gly Tyr Leu Ala Ala Arg Ala Ile Ser Ser Phe Leu Gly
                   100                 105                 110
   Pro Ser Tyr His Pro Gly Asn Arg Ala Val Thr Thr Pro Thr Gly Gln
           115                 120                 125
   Val Val Gln Pro Gln Thr Asn Arg Ser Val Gly Lys Pro Met Leu Val
       130                 135                 140
   Lys Gly Asn Ala Gly Ser Met Asn Ser Lys Pro Val Ser Arg Gly Gly
   145                 150                 155                 160
   Phe Ser Ser Pro Asn Asn Thr His Arg Ser Ser Gly Gly
                   165                 170
<210> 123
   <211> 171
   <212> PRT
   <213> Bacteria <prokaryote>
<220>
   <223> NT014
<400> 123
   Val Ala Ala Val Ile Gly Gly Gly Ala Val Ala Ala Lys Val Ala Thr
   1               5                   10                  15
   Asp Pro Arg Thr Thr Gly Thr Gln Ile Asp Asp Glu Thr Leu Glu Phe
               20                  25                  30
   Lys Val Glu Asn Ala Val Glu Lys Asp Ala Gln Ile Lys Ala Glu Gly
           35                  40                  45
   Arg Val Asn Ala Val Ser Tyr Asn Gly Arg Val Leu Leu Ile Gly Gln
       50                  55                  60
   Val Pro Asn Ser Asp Val Lys Asp Thr Ala Thr Ala Leu Ala Lys Gly
   65                  70                  75                  80
   Val Lys Gly Val Asn Glu Val Tyr Asn Glu Leu Thr Val Ser Ser Lys
                   85                  90                  95
   Ile Ser Phe Ala Gln Ile Ser Lys Asp Ser Trp Leu Thr Thr Gln Val
               100                 105                 110
   Lys Ser Lys Met Phe Val Asp Gly Arg Val Lys Ala Thr Asp Val Lys
           115                 120                 125
   Val Ile Ser Glu Asn Gly Glu Val Phe Leu Leu Gly Asn Val Thr Gln
       130                 135                 140
   Ser Gln Ala Asn Ala Ala Ala Asp Ile Ala Ser Lys Ile Ser Gly Val
   145                 150                 155                 160
   Lys Lys Val Ile Lys Val Phe Lys Tyr Leu Asp
                   165                 170
<210> 124
   <211> 387
   <212> PRT
   <213> Bacteria <prokaryote>
<220>
   <223> NT022
<400> 124
   Thr Ser Asn Phe Pro Ala Pro Ile Ser Asp Ala Asp Gly Asn Leu Ser
   1               5                   10                  15
   Pro Ser Val Ile Gln Ser Val Asn Gly Ser Asn Val Gly Gly Ala Trp
               20                  25                  30
   Gln Pro Glu Ile Gln Lys Asn Ser Leu Pro Thr Thr Gly Asn Met Val
           35                  40                  45
   Thr Pro Gln Pro Asn Phe Gln Pro Ile Asn Gln Gln Pro Thr Met Pro
       50                  55                  60
   Thr Ala Pro Ala Gln Pro Ala Phe Gln Pro Ser Pro Lys Thr Val Val
   65                  70                  75                  80
   Ser Ala Pro Thr Val Gln Thr Lys Thr Val Thr Lys Thr Val Ala Asp
                   85                  90                  95
   Cys Val Asp Gly Gln His Ile Asn Ile Pro Arg Asn Pro Asn Thr Asn
               100                 105                 110
   Val Pro Asp Tyr Ser Lys Ile Ser Lys Gly Ser Tyr Lys Gly Asn Thr
           115                 120                 125
   Tyr Lys Val Asn Lys Gly Asp Thr Met Phe Leu Ile Ala Tyr Leu Ala
       130                 135                 140
   Gly Ile Asp Val Lys Glu Leu Ala Ala Leu Asn Asn Leu Ser Glu Pro
   145                 150                 155                 160
   Tyr Asn Leu Ser Leu Gly Gln Val Leu Lys Ile Ser Asn Cys Ser Thr
                   165                 170                 175
   Lys Thr Val Thr Thr Thr Val Ser Val Lys Gln Pro Ala Val Thr Thr
               180                 185                 190
   Ser Thr Ala Thr Pro Val Lys Pro Ala Val Thr Tyr Thr Pro Gly Ala
           195                 200                 205
   Asn Gly Thr Gln Ile Gly Ser Asp Gly Thr Ile Ile Gly Pro Ile Lys
       210                 215                 220
   Ser Glu Ala Gly Thr Ser Pro Ser Val Pro Val Ala Thr Ser Ser Thr
   225                 230                 235                 240
   Gln Val Thr Ser Ser Val Asn Asn Ala Asn Ser Thr Pro Ile Asn Ser
                   245                 250                 255
   Asn Val Val Ala Pro Ile Ala Ser His Val Val Trp Gln Trp Pro Thr
               260                 265                 270
   Ser Gly Asn Ile Ile Gln Gly Phe Ser Ser Thr Asp Gly Gly Asn Lys
           275                 280                 285
   Gly Ile Asp Ile Ser Gly Ser Arg Gly Gln Ala Val Lys Ala Ala Ala
       290                 295                 300
   Ala Gly Arg Ile Val Tyr Ala Gly Asn Ala Leu Arg Gly Tyr Gly Asn
   305                 310                 315                 320
   Leu Ile Ile Ile Lys His Asn Asp Asp Phe Leu Ser Ala Tyr Ala His
                   325                 330                 335
   Asn Asp Lys Ile Leu Val Ala Asp Gln Gln Glu Val Lys Ala Gly Gln
               340                 345                 350
   Asp Ile Ala Lys Met Gly Ser Ser Gly Thr Asn Thr Val Lys Leu His
           355                 360                 365
   Phe Glu Ile Arg Tyr Lys Gly Lys Ser Val Asp Pro Val Arg Tyr Leu
       370                 375                 380
   Pro Arg His
   385
<210> 125
   <211> 20
   <212> PRT
   <213> Bacteria <prokaryote>
<220>
   <223> NT004 N-terminus
<400> 125
   Met Lys Lys Lys Asn Gln Ile Leu Val Ser Leu Ser Ile Val Ala Leu
   1               5                   10                  15
   Leu Gly Gly Cys
               20
<210> 126
   <211> 22
   <212> PRT
   <213> Bacteria <prokaryote>
<220>
   <223> NT014 N-terminus
<400> 126
   Met Thr Leu Ser Pro Leu Lys Lys Leu Ala Ile Leu Leu Gly Ala Thr
<210> 127
   <211> 22
   <212> PRT
   <213> Bacteria <prokaryote>
<220>
   <223> NT022 N-terminus
<400> 127
   Met Thr Leu Ser Pro Leu Lys Lys Leu Ala Ile Leu Leu Gly Ala Thr
   1               5                   10                  15
   Ile Phe Leu Gln Gly Cys
               20
<210> 128
   <211> 286
   <212> PRT
   <213> Artificial Sequence
<220>
   <223> NT061
<400> 128
   Glu Glu Arg Val Val Ala Thr Val Asp Gly Ile Pro Ile Leu Glu Ser
   1               5                   10                  15
   Gln Val Arg Ala Asn Met Gly Lys Lys Gly Asp Arg Gln Ser Ala Leu
               20                  25                  30
   Asp Lys Ile Ile Asp Asp Leu Leu Val Gln Lys Ala Ile Gln Glu Ser
           35                  40                  45
   Gly Val Lys Ile Asp Pro Arg Glu Ile Asp Arg Val Val Glu Asp Thr
       50                  55                  60
   Ala Ala Arg Asn Gly Leu Thr Tyr Gly Gln Phe Leu Asp Ala Leu Asp
   65                  70                  75                  80
   Tyr Gln Gly Ile Ser Leu Asn Thr Phe Arg Gln Gln Ile Ala Asn Gln
                   85                  90                  95
   Met Val Met Gly Ala Val Arg Asn Lys Ala Ile Gln Glu Ser Ile Asp
               100                 105                 110
   Val Thr Arg Glu Glu Val Val Ala Leu Gly Gln Lys Met Leu Asp Glu
           115                 120                 125
   Ala Lys Ser Gln Gly Thr Ala Gln Lys Val Thr Gly Lys Glu Tyr Glu
       130                 135                 140
   Val Arg His Ile Leu Leu Lys Leu Asn Pro Leu Leu Asn Asp Ala Gln
   145                 150                 155                 160
   Ala Lys Lys Gln Leu Ala Lys Ile Arg Ser Asp Ile Ile Ala Gly Lys
                   165                 170                 175
   Thr Thr Phe Ala Asp Ala Ala Leu Lys Tyr Ser Lys Asp Tyr Leu Ser
               180                 185                 190
   Gly Ala Asn Gly Gly Ser Leu Gly Tyr Ala Phe Pro Glu Thr Tyr Ala
           195                 200                 205
   Pro Gln Phe Ala Gln Thr Val Met Lys Ser Lys Gln Gly Val Ile Ser
       210                 215                 220
   Ala Pro Phe Lys Thr Glu Phe Gly Trp His Ile Leu Glu Val Thr Gly
   225                 230                 235                 240
   Val Ser Asp Gly Asp Leu Thr Ala Glu Ala Tyr Thr Gln Lys Ala Tyr
                   245                 250                 255
   Glu Arg Leu Val Asn Thr Gln Leu Gln Asp Ala Thr Asn Asp Trp Val
               260                 265                 270
   Lys Ala Leu Arg Lys Arg Ala Asn Ile Gln Tyr Phe Asn Lys
           275                 280                 285
<210> 129
   <211> 27
   <212> PRT
   <213> Artificial Sequence
<220>
   <223> NT061 N-terminus
<400> 129
   Met Lys Met Lys Lys Phe Ile Leu Lys Ser Phe Leu Leu Ala Thr Leu
   1               5                   10                  15
   Gly Cys Val Ala Phe Thr Ser Met Ala Gln Ala
               20                  25
<210> 130
   <211> 390
   <212> PRT
   <213> Artificial Sequence
<220>
   <223> NT017
<400> 130
   Gly Leu Leu Ile Phe Ser Pro Val Ser Gln Ser Ser Asp Leu Asn Gln
   1               5                   10                  15
   Ile Gln Lys Gln Ile Lys Gln Gln Glu Ser Lys Ile Glu Lys Gln Lys
               20                  25                  30
   Arg Glu Gln Ala Lys Leu Gln Ala Asn Leu Lys Lys His Glu Ser Lys
           35                  40                  45
   Ile Asn Thr Val Glu Gly Glu Leu Leu Glu Thr Glu Ile Ser Leu Lys
       50                  55                  60
   Glu Ile Arg Lys Gln Ile Ala Asp Ala Asp Lys Gln Phe Lys Gln Leu
   65                  70                  75                  80
   Glu Lys Gln Glu Arg Glu Gln Lys Ala Arg Leu Ala Lys Gln Met Asp
                   85                  90                  95
   Ile Ile Tyr Arg Ser Gly Ile Asn Pro Ser Leu Ile Glu Arg Met Phe
               100                 105                 110
   Ala Gln Asp Pro Thr Lys Ala Glu Arg Met Lys Val Tyr Tyr Gln His
           115                 120                 125
   Leu Asn Gln Val Arg Ile Glu Met Ile Asp Asn Leu Lys Ala Thr Gln
       130                 135                 140
   Ala Gln Ile Ala Val Gln Lys Glu Ala Ile Leu Ala Gln Gln Lys Asn
   145                 150                 155                 160
   His Arg Asn Gln Leu Ser Thr Gln Lys Lys Gln Gln Gln Ala Leu Gln
                   165                 170                 175
   Lys Ala Gln Gln Glu His Gln Ser Thr Leu Asn Glu Leu Asn Lys Asn
               180                 185                 190
   Leu Ala Leu Asp Gln Asp Lys Leu Asn Ala Leu Lys Ala Asn Glu Gln
           195                 200                 205
   Ala Leu Arg Gln Glu Ile Gln Arg Ala Glu Gln Ala Ala Arg Glu Gln
       210                 215                 220
   Glu Lys Arg Glu Arg Glu Ala Leu Ala Gln Arg Gln Lys Ala Glu Glu
   225                 230                 235                 240
   Lys Arg Thr Ser Lys Pro Tyr Gln Pro Thr Val Gln Glu Arg Gln Leu
                   245                 250                 255
   Ile Asn Ser Thr Ser Gly Leu Gly Ala Ala Lys Lys Gln Tyr Ser Leu
               260                 265                 270
   Pro Val Ser Gly Ser Ile Leu His Thr Phe Gly Ser Ile Gln Ala Gly
           275                 280                 285
   Glu Val Arg Trp Lys Gly Met Val Ile Gly Ala Ser Ala Gly Thr Pro
       290                 295                 300
   Val Lys Ala Ile Ala Ala Gly Arg Val Ile Leu Ala Gly Tyr Leu Asn
   305                 310                 315                 320
   Gly Tyr Gly Tyr Met Val Ile Val Lys His Gly Glu Thr Asp Leu Ser
                   325                 330                 335
   Leu Tyr Gly Phe Asn Gln Ala Val Ser Val Lys Val Gly Gln Leu Val
               340                 345                 350
   Ser Ala Gly Gln Val Ile Ala Gln Val Gly Asn Thr Gly Glu Ile Ser
           355                 360                 365
   Arg Ser Ala Leu Tyr Phe Gly Ile Ser Arg Lys Gly Thr Pro Val Asn
       370                 375                 380
   Pro Ala Gly Trp Val Arg
   385                 390
<210> 131
   <211> 20
   <212> PRT
   <213> Artificial Sequence
<220>
   <223> NT017 N-terminus
<400> 131
   Met Leu Arg Phe Gly Val Asn Gln Lys Thr Ser Leu Leu Leu Thr Ala
   1               5                   10                  15
   Leu Leu Ser Cys
               20

## Description

### TECHNICAL FIELD

This invention is in the field of *Haemophilus influenzae* immunology and vaccinology, in particular non-typeable *H. influenzae* (NTHI). The invention provides immunogenic compositions comprising antigen polypeptides and combinations of antigen polypeptides for raising antibodies and immune responses and against *H. influenzae* strains. The invention also provides compositions containing such antigens, and the use thereof as vaccines or medicaments against *H. influenzae.* The invention also provides immunogenic compositions containing such antigens used alone or in combination or used together with other vaccines. The invention also provides methods for raising immune responses against *H. influenzae*, and methods for the treatment and prevention of infections by *H. influenzae.*

### BACKGROUND ART

*Haemophilus influenzae* is a small, non-motile, Gram-negative coccobacillus. It is a respiratory pathogen that causes a wide spectrum of human infections, including: asymptomatic colonization of the upper respiratory tract (*i.e.* carriage); infections that extend from colonized mucosal surfaces to cause otitis media (inflammation of the middle ear), bronchitis, conjunctivitis, sinusitis, urinary tract infections and pneumonia; and invasive infections, such as bacteremia, septic arthritis, epiglottitis, pneumonia, empyema, pericarditis, cellulitis, osteomyelitis and meningitis. *H. influenzae* was the first bacterium for which a complete genome sequence was published [1].

*H. influenzae* strains are either capsulated (typeable) or non-capsulated (non-typeable), and there are six major serological types of capsulated strains (a to f). 95% of *H. influenzae-caused* invasive diseases are caused by *H. influenzae* type b ('Hib') strains. The most serious manifestation of Hib disease is meningitis, but the introduction in the 1980s of vaccines based on conjugated Hib capsular saccharides has hugely reduced incidence of this disease.

Although Hib infections can now be controlled by vaccination, other pathogenic *H. influenzae* strains remain a risk. For instance, non-typeable *H. influenzae* (NTHI) is responsible for otitis media (OM), particularly chronic and acute OM. While OM is rarely associated with mortality, it is associated with significant morbidity. Hearing loss is the most common complication of OM, with behavioural, educational and language development delays being additional consequences of early onset OM with effusion. Acute OM is the most common bacterial infection in children in the USA. The non-typeable *H. influenzae* biogroup aegyptius causes epidemic conjunctivitis and Brazilian purpuric fever (BPF) [2], with BPF having a mortality of up to 70%.

To date, antibiotics are the main tool against the spectrum of clinical entities known collectively as OM, but widespread use of antibiotics for OM has met with controversy due to the emergence of multiple-antibiotic resistant microorganisms. Progress towards a vaccine is slow due to an incomplete understanding of both the pathogenesis of OM and the immune response to it.

The genome sequence of the serotype d strain KW20 [1,3] has been useful for understanding basic *H. influenzae* biology, but it has not been so useful in countering pathogenic *H. influenzae* strains, as serotype d strains are generally not pathogens. Polypeptides from pathogenic non-typeable *H. influenzae* have been identified and investigated as vaccine candidates. Reference 4 discloses immunogenic polypeptides from a pathogenic non-typeable *H. influenzae* strain.

However, there remains a need for providing a vaccine that protects against a broad spectrum of *Haemophilus influenzae* strains. *H. influenzae* is a versatile microorganism with an improved ability to adapt to new niches and to cause a broad spectrum of disease. Fitness, virulence and colonization factors can change in order to allow the microorganism to adapt to different tissues and hosts. Therefore, potential antigens are subject to high selective pressure and, as a result, may have sequence variability among different strains.

Thus there remains a need to identify further and improved antigens for use in non-typeable *Haemophilus influenzae* vaccines, and in particular for vaccines which are useful against multiple NTHI-caused pathologies.

The database of genomes available at ncbi.nlm.nih.gov under genomes listed pathogenic and non-pathogenic *Haemophilus influenzae* genomes with as few as 2,500 proteins to as many as 4,000 proteins. However, such listings do not identify which are conserved across a significant fraction of the pathogenic NTHI, what are the conserved regions in the proteins that are so conserved, or which proteins among the thousands of potential proteins can be used in a vaccine to produce a sufficient immune response to protect against pathogenic NTHI which requires screening large numbers of proteins to identify the best candidates.

WO2010/092176 discloses a number of *H. influenzae* antigens with protective effects. It is an object of the invention to provide further and better antigens and/or combinations which are efficacious in raising immune responses against different strains of *H. influenzae,* for use in the development of vaccines for preventing and/or treating infections caused by *H. influenzae* pathogens, in particular non-typeable *H. influenzae.* In particular, it is an object to provide polypeptides and combinations of polypeptides for use in improved immunogenic compositions and vaccines for preventing and/or in treating such infections, and in particular acute otitis media and chronic obstructive pulmonary disease (COPD). The polypeptides may also be useful for diagnostic purposes, and as targets for antibiotics.

### DISCLOSURE OF THE INVENTION

Present invention describes non-typeable *Haemophilus influenzae* (NTHI) polypeptides that are useful for immunisation, for use either alone or in combination. These polypeptides may be combined with other NTHI polypeptides as well as. The antigens are useful in *NTHI* vaccines but may also be used as components in vaccines for immunising against multiple pathogens.

By using two parallel approaches, namely reverse vaccinology and proteomic analysis of outer membrane vesicles (OMVs) it has been possible to identify antigens which are conserved amongst 86 different NTHI strains. Reverse vaccinology uses *in silico* analysis to identify proteins conserved in the genomes of different NTHI strains and potentially surface-exposed. The second approach is instead focused on the identification of antigens by analysing mass spectrometry of the proteins contained in the outer membrane vesicles produced by NTHI.

The genome of a NTHI strain includes about 1800 genes. The inventors have identified 274 conserved antigens from 15 complete genomes plus 39 strains selected on the basis of geographical distribution and 32 strains derived from an otitis media Finnish collection which are all currently publicly available. From these 274 the inventors have selected 53 polypeptides of particular interest. These antigens were selected from the strain NP86-028, with the exception of CGSHiGG_00130 being selected from PittG, CGSHiGG_02400 selected from PittG, gi-145633184 selected from 3655 strain and gi-145628236 selected from 22.1-21 strain.

Amongst the group of 53 antigens the following further selection has been generated considering immunogenicity and conservation criteria:
- A set of 26 antigens referred herein as "the first antigen group"
- A set of 6 antigens referred herein as "the second antigen group"
- A set of 21 antigens referred herein as "the third antigen group"

Most preferred set of antigens is referred to herein as 'the first antigen group'. Thus the disclosure provides an immunogenic composition comprising at least one antigen, preferably comprising one or more (*i.e.* 1, 2, 3, 4, 5, 6 or more) antigens selected from the group consisting of: (1) NTHI0915 (NT018), (2) NTHI1416 (NT024), (3) NTHI2017 (NT032), (4) CGSHiGG_02400 (NT038), (5) NTHI1292 (NT067), (6) NTHI0877 (NT001), (7) NTHI0266 (NT016), (8) CGSHiGG_00130 (NT052), (9) NTHI1627 (NT002), (10) NTHI1109 (NT026), (11) NTHI0821 (NT009), (12) NTHI0409 (NT025), (13) NTHI1954 (NT028), (14) NTHI0371 (NT029), (15) NTHI0509 (NT031), (16) NTHI0449 (NT015), (17) NTHI1473 (NT023), (18) gi-145633184 (NT100), (19) NTHI1110 (NT040), (20) gi-46129075 (NT048), (21) gi-145628236 (NT053), (22) NTHI1230 (NT066), (23) NTHI0522 (NT097), (24) NT004, (25) NT014, (26) NT022. These antigens show a positive bactericidal activity as shown in Table III and Table IV.

Within the first antigen group, preferred antigens are selected from a subset of any of (1) NTHI0915 (NT018) antigen, (2) NTHI1416 (NT024) antigen, (3) NTHI2017 (NT032) antigen, (4) CGSHiGG_02400 (NT038), (5) NTHI1292 antigen (NT067), (6) NTHI0877 (NT001) antigen, (8) NT052 antigen, (24) NT004 antigen, (25) NT014 antigen, (26) NT022 antigen, (7) NTHI0266 NT016 antigen. These antigens are all showing a good level of purification as shown in Table II and immunogenicity efficacy is reported in tables III and IV.

Particularly preferred antigens were NT067, NT014, NT016, NT022.

Thus the invention provides an immunogenic composition comprising a NT067 non-typeable *H. influenzae* antigen for use in a method of raising a protective immune response against *H. influenzae* infection in a mammal, wherein said NT067 antigen is a polypeptide that comprises an amino acid sequence having 95% or more identity to SEQ ID NO: 5.

Also disclosed is an immunogenic composition comprising one or more (*i.e.* 1, 2, 3, 4, 5, 6 or more) antigens selected from the group consisting from the "first antigen group".

The inventors have also identified the following 6 polypeptides: (24) P48 (NTHI0254 also defined as NT007), (25) HtrA (NTHI1905 also defined as NT006), (26) PE (NTHI0267 also defined as NT035), (27) P26 (NTHI0501 also defined as NT010), (28) PHiD (NTHI0811 also defined as NT080), (29) P6 (NTHI0501, also defined as NT081). This set of 6 antigens is referred to herein as 'the second antigen group'.

The inventors have also identified the following 22 polypeptides: (30) NTHI0532 (NT013), (31) NTHI0363 (NT106), (32) NTHI0370 (NT107), (33) NTHI0205 (NT108), (34) NTHI0374 (NT109), (35) NTHI0579 (NT110), (36) NTHI0837 (NT111), (37) NTHI0849 (NT112), (38) NTHI0921 (NT113), (39) NTHI0995 (NT114), (40) NTHI1091 (NT115), (41) NTHI1169 (NT116), (42) NTHI1208 (NT117), (43) NTHI1318 (NT118), (44) NTHI1796 (NT123), (45) NTHI1930 (NT124), (46) NTHI1565 (NT119), (47) NTHI1569 (NT120), (48) NTHI1571 (NT121), (49) NTHI1667 (NT122), (50) NTHI0588 (NT061), (51) NTHI0915 (NT017). This set of 22 antigens is referred to herein as 'the third antigen group'.

Disclosed herein is a composition including at least one antigen (*i.e.* 1, 2, 3, 4, 5, 6 or more) selected from the first antigen group and/or at least one antigen (*i.e.* 1, 2, 3, 4, 5, 6 or more) selected from the second antigen group and/or at least one antigen (i.e. 1, 2, 3, 4, 5, 6 or more) selected from the third antigen group. Antigens from the first antigen group can be selected from the most preferred subset of antigens.

Also disclosed is an immunogenic composition comprising one antigen selected from any of the first antigen group or second antigen group or third antigen group.

Thus disclosed herein is an immunogenic composition comprising a combination of antigens, said combination comprising two or more (*i.e.* 2, 3, 4, 5, 6 or more) antigens selected from the group consisting of the "first antigen group" and/or the "second antigen group" and/or the "third antigen group".

Where a composition includes an antigen from the "second antigen group", it is preferred that the composition should also include (i) at least one further antigen from the "second antigen group" or (ii) at least one antigen from the "first antigen group" or the "third antigen group". Thus the invention would not encompass a composition including as its sole antigenic component a single antigen from the "second antigen group". Where a composition includes two or more antigens from the "second antigen group", it is preferred that the composition should include at least one antigen which is not (a) a P48 antigen (b) a HtrA antigen (c) a PE antigen or (d) a P26 antigen. Thus the invention does not encompass combinations only of P48, HtrA, PE and/or P26. Similarly, the invention does not encompass hybrid antigens which include 'X' moieties only from P48, HtrA, PE and/or P26.

Within the 11 preferred antigens of the first antigen group there are 55 possible pairs of different antigens. All such pairs are disclosed herein. Thus disclosed herein is an immunogenic composition comprising a pair of antigens, wherein said pair is one of said 55 pairs.

In one example, a composition includes at least one antigen (*i.e.* 1, 2, 3, 4, 5, 6 or more) selected from the first antigen group and/or at least one antigen (*i.e.* 1, 2, 3, 4, 5, 6 or more) selected from the second antigen group, and/or at least one antigen (i.e. 1, 2, 3, 4, 5, 6 or more) selected from the third antigen group.

In all cases, antigens from the first antigen group can be advantageously selected from the most preferred subset of any of (1) NTHI0915 (NT018), (2) NTHI1416 (NT024), (3) NTHI2017 (NT032), (4) CGSHiGG_02400 (NT038), (5) NTHI1292 (NT067), (6) NTHI0877 (NT001), (8) NT052, (24) NT004, (25) NT014, (26) NT022, (7) NT016.

Also disclosed herein is an immunogenic composition comprising a combination of antigens, said combination comprising two or more (*i.e.* 2, 3, 4 or 5) antigens selected from the group consisting of: (1) NTHI0915 (NT018), (2) NTHI1416 (NT024), (3) NTHI2017 (NT032), (4) CGSHiGG_02400 (NT038), (5) NTHI1292 (NT067), (6) NTHI0877 (NT001), (8) NT052, (24) NT004, (25) NT014, (26) NT022, (7) NT016. The composition can also include an adjuvant *e.g*. an adjuvant comprising an oil-in-water emulsion or an aluminium salt.

Reference 5 discusses non-typeable *H. influenzae* antigens, *inter alia* as candidates for potential use in vaccines. References 6 to 10, are concerned, individually, with non-typeable *H. influenzae* polypeptides P48, HtrA, PE and P26, respectively, and *inter alia* with their immunogenic potential. Reference 5 also mentions HtrA, PE and P26 individually amongst a larger number of vaccine candidates, and *e.g.* reference 10 is concerned with polypeptide PE. However, these antigens, belonging to the "second antigen group" and were not described for use in combination. It has now surprisingly been found that a combination of one or more of these antigens (second antigen group) with at least one of the antigen listed in the "first antigen group" is particularly suitable for generating a protective immune response against non-typeable *H. influenzae.*

Advantageous combinations are those in which two or more antigens act synergistically. Thus the protection against NTHI pathogen achieved by their combined administration exceeds that expected by mere addition of their individual protective efficacy.

### FIRST ANTIGEN GROUP

### NT018 antigen

The "NT018" antigen is annotated as TPR repeat-containing protein and also as cytochrome c maturation heme lyase subunit CcmH2. It has been annotated as NTHI0915 in the strain 86-028NP. Said sequence is highly conserved amongst all the strains analyzed and is predicted to be a membrane-bound metal-peptidase. NT018 is surface exposed as shown in Table III. NT018 has been cloned and expressed from another non-typeable strain, Fi176, which is a strain isolated form the Finland otitis media collection.

Useful NT018 antigens can elicit an antibody (*e.g*. when administered to a human) that recognises SEQ ID NO: 1 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 1; and/or (b) comprising a fragment of at least 'n' consecutive amino acids of SEQ ID NO: 1, wherein 'n' is 7 or more *(e.g.* 8, 10, 12, 14, 16, 18, 20, 25, 30, 35, 40, 50, 60, 70, 80, 90, 100, 150, 200, 250 or more). These NT018 proteins include variants of SEQ ID NO: 1, such as SEQ ID NO: 49 which has been cloned and expressed and tested in immunogenicity (Table III, IV). Preferred fragments of (b) comprise an epitope from SEQ ID NO: 1. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25, 26, 27, 28 or more) from the N-terminus of SEQ ID NO: 1 while retaining at least one epitope of SEQ ID NO: 1. Other fragments omit one or more protein domains.

A NT018 antigen can be expressed with its native 28 N-terminal amino acids of NT018 (MNFTLIFILTTLVVALICFYPLLRQFKA; SEQ ID NO: 69) or can be expressed with an alternative N-terminal sequence e.g. with a simple N-terminus methionine, or Met-Ala-, or a leader peptide which targets or traffics the expressed protein in a desired fashion.

### NT024 antigen

The "NT024" antigen is annotated as "hypothetical protein" and has been annotated as NTHI1416 in the genome 86-028NP. This antigen has been cloned and expressed from Fi176 strain.

Useful NT024 antigens can elicit an antibody (*e.g*. when administered to a human) that recognises SEQ ID NO: 2 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 2; and/or (b) comprising a fragment of at least 'n' consecutive amino acids of SEQ ID NO: 2 wherein 'n' is 7 or more (*e.g.* 8, 10, 12, 14, 16, 18, 20, 25, 30, 35, 40, 50, 60, 70, 80, 90, 100, 150, 200, 250 or more). These NT024 proteins include variants of SEQ ID NO: 2, such as SEQ ID NO: 50 cloned from strain Fi176. Preferred fragments of (b) comprise an epitope from SEQ ID NO: 2. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 2 while retaining at least one epitope of SEQ ID NO: 2. Other fragments omit one or more protein domains.

A NT024 antigen can be expressed with the native 20 N-terminal amino acids of NT024 (MKLKLFFHIVLLCFSLPVWA; SEQ ID NO: 70) or can be expressed with an alternative N-terminal sequence *e.g*. with a simple N-terminus methionine, or Met-Ala-, or a leader peptide which targets or traffics the expressed protein in a desired fashion.

### NT032 antigen

The "NT032" antigen is annotated as "hypothetical protein" and has been annotated as NTHI2017 in the genome 86-028NP. Domain most conserved amongst strains tested is described as "Bacterial OB fold (BOF) protein". This antigen has been cloned and expressed from Fi176 strain.

Useful NT032 antigens can elicit an antibody (*e.g*. when administered to a human) that recognises SEQ ID NO: 3 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 3; and/or (b) comprising a fragment of at least 'n' consecutive amino acids of SEQ ID NO: 3, wherein 'n' is 7 or more (*e.g.* 8, 10, 12, 14, 16, 18, 20, 25, 30, 35, 40, 50, 60, 70, 80, 90, 100, 150, 200, 250 or more). These NT032 proteins include variants of SEQ ID NO: 3, such as SEQ ID NO: 51 cloned from Fi176 strain. Preferred fragments of (b) comprise an epitope from SEQ ID NO: 3. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 3 while retaining at least one epitope of SEQ ID NO: 3. Other fragments omit one or more protein domains.

A NT032 antigen can be expressed with the native 19 N-terminal amino acids of NT032 (MKKFALATIFALATTSAFA; SEQ ID NO: 71) or can be expressed with an alternative N-terminal sequence e.g. with a simple N-terminus methionine, or Met-Ala-, or a leader peptide which targets or traffics the expressed protein in a desired fashion.

### NT067 antigen

The "NT067" antigen is annotated as ABC transporter protein and it has been proposed its hypothetical function as periplasmic oligopeptide-binding protein OppA. In the strain 86-028NP has been annotated as NTHI1292. This antigen has been cloned and expressed from Fi176 strain.

Useful NT067 antigens can elicit an antibody (*e.g*. when administered to a human) that recognises SEQ ID NO: 5 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 5; and/or (b) comprising a fragment of at least 'n' consecutive amino acids of SEQ ID NO: 5, wherein 'n' is 7 or more *(e.g.* 8, 10, 12, 14, 16, 18, 20, 25, 30, 35, 40, 50, 60, 70, 80, 90, 100, 150, 200, 250 or more). These NT067 proteins include variants of SEQ ID NO: 5, such as SEQ ID NO: 52 cloned from Fi176 strain. Preferred fragments of (b) comprise an epitope from SEQ ID NO: 5. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 5 while retaining at least one epitope of SEQ ID NO: 5. Other fragments omit one or more protein domains.

A NT067 antigen of the invention can be expressed with the native 20 N-terminal amino acids of NT067 (MQHKLLFSAIALALSYSVQA; SEQ ID NO: 72) or can be expressed with an alternative N-terminal sequence *e.g*. with a simple N-terminus methionine, or Met-Ala-, or a leader peptide which targets or traffics the expressed protein in a desired fashion.

### NT038 antigen

This antigen is known as Hia (Haemophilus influenzae adhesin) protein [11] and has been identified in the strain CGSHiGG_02400 as a 282 aa in length, however it is a truncated form of Hia (616 aa) as originally described in the strain 86-028NP or in other NTHi strains. This antigen has been cloned from R2846 strain.

Useful NT038 antigens can elicit an antibody (*e.g*. when administered to a human) that recognises SEQ ID NO: 4 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 4; and/or (b) comprising a fragment of at least 'n' consecutive amino acids of SEQ ID NO: 4, wherein 'n' is 7 or more *(e.g.* 8, 10, 12, 14, 16, 18, 20, 25, 30, 35, 40, 50, 60, 70, 80, 90, 100, 150, 200, 250 or more). These NT038 proteins include variants of SEQ ID NO: 4, such as SEQ ID NO: 53, which is lacking the first 23 native N-terminal amino acids and 102 amino acids at the C-terminal. Preferred fragments of (b) comprise an epitope from SEQ ID NO: 4. Other preferred fragments lack one or more amino acids (*e.g.* 1, 2, 3, 4, 5, 6, 7, 8, 9, 10, 15, 20, 25 or more) from the C-terminus (even up to 102aa) and/or one or more amino acids (*e.g.* 1, 2, 3, 4, 5, 6, 7, 8, 9, 10, 15, 20, 23, 25 or more) from the N-terminus of SEQ ID NO: 4 while retaining at least one epitope of SEQ ID NO: 4. Other fragments omit one or more protein domains.

A NT038 antigen can be expressed with the native 23 N-terminal amino acids of NT038 (MPFQYVTEDGKTVVKVGNGYYEA; SEQ ID NO: 73) or can be expressed with an alternative N-terminal sequence *e.g*. with a simple N-terminus methionine, or Met-Ala-, or a leader peptide which targets or traffics the expressed protein in a desired fashion.

### NT001 antigen

This antigen has been annotated as NTHI0877 in the genome 86-028NP and is known as D-methionine-binding lipoprotein MetQ. MetD is an ABC transporter encoding a DL methionine uptake system. This antigen has been previously disclosed as BASB202 (28 Kda) [12, 13], and its use as vaccine against NTHI has been proposed. This antigen shares 99,63% alignment ID with an homologue antigen as described in Ref (4) and it has been found well conserved amongst all the strains considered in the present disclosure. In present disclosure it is cloned and expressed from Fi176 strain.

Useful NT001 antigens can elicit an antibody (*e.g*. when administered to a human) that recognises SEQ ID NO: 6 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 6; and/or (b) comprising a fragment of at least 'n' consecutive amino acids of SEQ ID NO: 6, wherein 'n' is 7 or more (*e.g.* 8, 10, 12, 14, 16, 18, 20, 25, 30, 35, 40, 50, 60, 70, 80, 90, 100, 150, 200, 250 or more). These NT001 proteins include variants of SEQ ID NO: 4, such as SEQ ID NO: 54. Preferred fragments of (b) comprise an epitope from SEQ ID NO: 6. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 21, 25 or more) from the N-terminus of SEQ ID NO: 6 while retaining at least one epitope of SEQ ID NO: 6. Other fragments omit one or more protein domains.

A NT001 antigen can be expressed with the native 21 N-terminal amino acids of NT001 (MKLKQLFAITAIASALVLTGC; SEQ ID NO: 74) or can be expressed with an alternative N-terminal sequence *e.g*. with a simple N-terminus methionine, or Met-Ala-, or a leader peptide which targets or traffics the expressed protein in a desired fashion.

### NT016 antigen

This antigen has been annotated as NTHI0266 in the strain 86-028NP and described as Hypothetical lipoprotein. This antigen has been cloned and expressed from Fi176 strain.

Useful NT016 antigens can elicit an antibody (*e.g*. when administered to a human) that recognises SEQ ID NO: 7 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 7; and/or (b) comprising a fragment of at least 'n' consecutive amino acids of SEQ ID NO: 7, wherein 'n' is 7 or more (*e.g.* 8, 10, 12, 14, 16, 18, 20, 25, 30, 35, 40, 50, 60, 70, 80, 90, 100, 150, 200, 250 or more). These NT016 proteins include variants of SEQ ID NO: 7, such as SEQ ID NO: 55. Preferred fragments of (b) comprise an epitope from SEQ ID NO: 7. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 19, 20, 25 or more) from the N-terminus of SEQ ID NO: 7 while retaining at least one epitope of SEQ ID NO: 7. Other fragments omit one or more protein domains.

A NT016 antigen can be expressed with the native 16 N-terminal amino acids of NT016 (MRKIKSLALLAVAALVIGC; SEQ ID NO: 75) or can be expressed with an alternative N-terminal sequence *e.g*. with a simple N-terminus methionine, or Met-Ala-, or a leader peptide which targets or traffics the expressed protein in a desired fashion.

### NT052 antigen

This antigen has been annotated as CGSHiGG_00130 from PittGG strain. It is part of Sell-domain containing protein families. It has been cloned from R2846 strain and the cloned sequence is reported as SEQ ID NO: 8. Despite the sequence cloned from R2846 is sharing only 64.16% identity over the sequence as annotated CGSHiGG_00130, it has been shown that there are conserved Sell domains which are repeated along the sequence which are useful to provide an efficacious antigenicity. Consensus for this repeats is SEQ ID NO: EAVKWYRKAAEQ.

Useful NT052 antigens can elicit an antibody (*e.g*. when administered to a human) that recognises SEQ ID NO: 8 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 8; and/or (b) comprising a fragment of at least 'n' consecutive amino acids of SEQ ID NO: 8, wherein 'n' is 7 or more (*e.g.* 8, 10, 12, 14, 16, 18, 20, 25, 30, 35, 40, 50, 60, 70, 80, 90, 100, 150, 200, 250 or more). These NT052 proteins include variants of SEQ ID NO: 8, such as SEQ ID NO: 56. Preferred fragments of (b) comprise an epitope from SEQ ID NO: 8. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 11, 15, 20, 25 or more) from the N-terminus of SEQ ID NO: 8 while retaining at least one epitope of SEQ ID NO: 8. Other fragments omit one or more protein domains.

A NT052 antigen can be expressed with the native 11 N-terminal amino acids of NT052 (MLLFILSIAWA; SEQ ID NO: 76) or can be expressed with an alternative N-terminal sequence *e.g*. with a simple N-terminus methionine, or Met-Ala-, or a leader peptide which targets or traffics the expressed protein in a desired fashion.

### NT002 antigen

This antigen has been annotated as NTHI1627 in 86-026NP strain and as lipoprotein. It has been cloned and expressed from Fi176 strain.

Useful NT002 antigens can elicit an antibody (*e.g*. when administered to a human) that recognises SEQ ID NO: 9 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 9; and/or (b) comprising a fragment of at least 'n' consecutive amino acids of SEQ ID NO: 9, wherein 'n' is 7 or more (*e.g.* 8, 10, 12, 14, 16, 18, 20, 25, 30, 35, 40, 50, 60, 70, 80, 90, 100, 150, 200, 250 or more). These NT002 proteins include variants of SEQ ID NO: 9, such as SEQ ID NO: 57. Preferred fragments of (b) comprise an epitope from SEQ ID NO: 9. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 9 while retaining at least one epitope of SEQ ID NO: 9. Other fragments omit one or more protein domains.

A NT002 antigen can be expressed with the native 18 N-terminal amino acids of NT002 (MKVYKSFLIATASLFLFA; SEQ ID NO: 77) or can be expressed with an alternative N-terminal sequence *e.g*. with a simple N-terminus methionine, or Met-Ala-, or a leader peptide which targets or traffics the expressed protein in a desired fashion.

A NT002 antigen can be a lipoprotein *e.g*. lipidated at a N-terminus cysteine.

### NT026 antigen

This antigen has been annotated as hypothetical protein NTHI1109 in 86-026NP strain. It has been predicted to be a cytoplasmic membrane protein. It has been cloned and expressed from strain Fi176.

Useful NT026 antigens can elicit an antibody (*e.g*. when administered to a human) that recognises SEQ ID NO: 10 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 10; and/or (b) comprising a fragment of at least 'n' consecutive amino acids of SEQ ID NO:10 wherein 'n' is 7 or more (*e.g.* 8, 10, 12, 14, 16, 18, 20, 25, 30, 35, 40, 50, 60, 70, 80, 90, 100, 150, 200, 250 or more). These NT026 proteins include variants of SEQ ID NO: 10, such as SEQ ID NO: 58, cloned from Fi176 strain. Preferred fragments of (b) comprise an epitope from SEQ ID NO: 10. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 10 while retaining at least one epitope of SEQ ID NO: 10. Other fragments omit one or more protein domains.

A NT026 antigen can be expressed with the native 24 N-terminal amino acids of NT026 (MQKGMTLVELLIGLAIISIVLNFA; SEQ ID NO: 78) or can be expressed with an alternative N-terminal sequence e.g. with a simple N-terminus methionine, or Met-Ala-, or a leader peptide which targets or traffics the expressed protein in a desired fashion.

### NT009 antigen

This antigen has been annotated as NTHI0821in 86-026NP strain and is part of OMP85 family protein. It is located in the outer membrane of the bacteria. It has been cloned and expressed from Fi176 strain.

Useful NT009 antigens can elicit an antibody (*e.g*. when administered to a human) that recognises SEQ ID NO: 11 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 11; and/or (b) comprising a fragment of at least 'n' consecutive amino acids of SEQ ID NO: 11, wherein 'n' is 7 or more (*e.g.* 8, 10, 12, 14, 16, 18, 20, 25, 30, 35, 40, 50, 60, 70, 80, 90, 100, 150, 200, 250 or more). These NT009 proteins include variants of SEQ ID NO: 11, such as SEQ ID NO: 59 cloned from Fi176. Preferred fragments of (b) comprise an epitope from SEQ ID NO: 11. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 22, 25 or more) from the N-terminus of SEQ ID NO: 11 while retaining at least one epitope of SEQ ID NO: 11. Other fragments omit one or more protein domains.

A NT009 antigen can be expressed with the native 22 N-terminal amino acids of NT009 (MNKTLLKLTALFLALNCFPAFA; SEQ ID NO: 79) or can be expressed with an alternative N-terminal sequence *e.g*. with a simple N-terminus methionine, or Met-Ala-, or a leader peptide which targets or traffics the expressed protein in a desired fashion.

### NT025 antigen

This antigen has been annotated as NTHI0409 in 86-026NP strain and belongs to the type IV pilin subunit protein family. It has been cloned and expressed from Fi176 strain.

Useful NT025 antigens can elicit an antibody (*e.g*. when administered to a human) that recognises SEQ ID NO: 12 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 12; and/or (b) comprising a fragment of at least 'n' consecutive amino acids of SEQ ID NO: 12, wherein 'n' is 7 or more (*e.g.* 8, 10, 12, 14, 16, 18, 20, 25, 30, 35, 40, 50, 60, 70, 80, 90, 100, 150, 200, 250 or more). These NT025 proteins include variants of SEQ ID NO: 12, such as SEQ ID NO: 60 as cloned from Fi176. Preferred fragments of (b) comprise an epitope from SEQ ID NO: 12. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 23, 25 or more) from the N-terminus of SEQ ID NO: 12 while retaining at least one epitope of SEQ ID NO: 12. Other fragments omit one or more protein domains.

A NT025 antigen can be expressed with the native 23 N-terminal amino acids of NT025 (MKLTTQQTLKKGFTLIELMIVIA; SEQ ID NO: 80) or can be expressed with an alternative N-terminal sequence *e.g*. with a simple N-terminus methionine, or Met-Ala-, or a leader peptide which targets or traffics the expressed protein in a desired fashion.

### NT028 antigen

This antigen has been annotated as NTHI1954 in 86-026NP strain and as lipoprotein NlpC. It has been cloned and expressed from Fi176 strain.

Useful NT028 antigens can elicit an antibody (*e.g*. when administered to a human) that recognises SEQ ID NO: 13 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 13; and/or (b) comprising a fragment of at least 'n' consecutive amino acids of SEQ ID NO: 13, wherein 'n' is 7 or more (*e.g.* 8, 10, 12, 14, 16, 18, 20, 25, 30, 35, 40, 50, 60, 70, 80, 90, 100, 150, 200, 250 or more). These NT028 proteins include variants of SEQ ID NO: 13, such as SEQ ID NO: 61 as cloned from Fi176. Preferred fragments of (b) comprise an epitope from SEQ ID NO: 13. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 13 while retaining at least one epitope of SEQ ID NO: 13. Other fragments omit one or more protein domains.

A NT028 antigen can be expressed with the native 21 N-terminal amino acids of NT028 (MLKRILVIIGLAVLATACSNA; SEQ ID NO: 81) or can be expressed with an alternative N-terminal sequence *e.g*. with a simple N-terminus methionine, or Met-Ala-, or a leader peptide which targets or traffics the expressed protein in a desired fashion.

A NT028 antigen can be a lipoprotein *e.g*. lipidated at a N-terminus cysteine.

### NT029 antigen

This antigen has been annotated as NTHI0371 in 86-026NP strain and as heme/hemopexin binding protein A, belonging to the outer membrane protein family. It has been cloned and expressed from R2846 strain.

Useful NT029 antigens can elicit an antibody (*e.g*. when administered to a human) that recognises SEQ ID NO: 14 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 14; and/or (b) comprising a fragment of at least 'n' consecutive amino acids of SEQ ID NO: 14, wherein 'n' is 7 or more (*e.g.* 8, 10, 12, 14, 16, 18, 20, 25, 30, 35, 40, 50, 60, 70, 80, 90, 100, 150, 200, 250 or more). These NT029 proteins include variants of SEQ ID NO: 14, such as SEQ ID NO 62 cloned from R2846 strain. Preferred fragments of (b) comprise an epitope from SEQ ID NO: 14. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 14 while retaining at least one epitope of SEQ ID NO: 14. Other fragments omit one or more protein domains.

A NT029 antigen can be expressed with the native 21 N-terminal amino acids of NT029 (MYKLNVISLIILTTYTGATYA; SEQ ID NO: 82) or can be expressed with an alternative N-terminal sequence *e.g*. with a simple N-terminus methionine, or Met-Ala-, or a leader peptide which targets or traffics the expressed protein in a desired fashion.

### NT031 antigen

This antigen has been annotated as starvation inducible outer membrane lipoprotein NTHI0509 in 86-026NP strain. It has been cloned and expressed from R2846 strain.

Useful NT031 antigens can elicit an antibody (*e.g*. when administered to a human) that recognises SEQ ID NO: 15 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 15; and/or (b) comprising a fragment of at least 'n' consecutive amino acids of SEQ ID NO: 15, wherein 'n' is 7 or more (*e.g.* 8, 10, 12, 14, 16, 18, 20, 25, 30, 35, 40, 50, 60, 70, 80, 90, 100, 150, 200, 250 or more). These NT031 proteins include variants of SEQ ID NO: 15, such as SEQ ID NO: 63 cloned and expressed from R2846 strain. Preferred fragments of (b) comprise an epitope from SEQ ID NO: 15. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 18, 20, 25 or more) from the N-terminus of SEQ ID NO: 15 while retaining at least one epitope of SEQ ID NO: 15. Other fragments omit one or more protein domains.

A NT031 antigen can be expressed with the native 18 N-terminal amino acids of NT031 (MKGKITLFFTALCFGLTG; SEQ ID NO: 83) or can be expressed with an alternative N-terminal sequence *e.g*. with a simple N-terminus methionine, or Met-Ala-, or a leader peptide which targets or traffics the expressed protein in a desired fashion.

A NT031 antigen can be a lipoprotein *e.g*. lipidated at a N-terminus cysteine.

### NT015 antigen

This antigen has been annotated as opacity associated protein OapB NTHI0449 in 86-026NP strain. It has been cloned and expressed from Fi176 strain.

Useful NT015 antigens can elicit an antibody (*e.g*. when administered to a human) that recognises SEQ ID NO: 16 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 16; and/or (b) comprising a fragment of at least 'n' consecutive amino acids of SEQ ID NO: 16, wherein 'n' is 7 or more (*e.g.* 8, 10, 12, 14, 16, 18, 20, 25, 30, 35, 40, 50, 60, 70, 80, 90, 100, 150, 200, 250 or more). These NT015 proteins include variants of SEQ ID NO: 16, such as SEQ ID NO: 64 cloned and expressed from Fi176. Preferred fragments of (b) comprise an epitope from SEQ ID NO: 16. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 16 while retaining at least one epitope of SEQ ID NO: 16. Other fragments omit one or more protein domains.

A NT015 antigen can be expressed with the native 17 N-terminal amino acids of NT015 (MLKKTSLIFTALLLAGC; SEQ ID NO: 84) or can be expressed with an alternative N-terminal sequence *e.g*. with a simple N-terminus methionine, or Met-Ala-, or a leader peptide which targets or traffics the expressed protein in a desired fashion.

### NT023 antigen

This antigen has been annotated as outer membrane lipoprotein PCP, NTHI1473 in 86-026NP strain. It has been cloned and expressed from Fi176 strain.

Useful NT023 antigens can elicit an antibody (*e.g*. when administered to a human) that recognises SEQ ID NO: 17 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 17; and/or (b) comprising a fragment of at least 'n' consecutive amino acids of SEQ ID NO: 17, wherein 'n' is 7 or more (*e.g.* 8, 10, 12, 14, 16, 18, 20, 25, 30, 35, 40, 50, 60, 70, 80, 90, 100, 150, 200, 250 or more). These NT023 proteins include variants of SEQ ID NO: 17, such as SEQ ID NO: 65 cloned and expressed from strain Fi176. Preferred fragments of (b) comprise an epitope from SEQ ID NO: 17. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 17 while retaining at least one epitope of SEQ ID NO: 17. Other fragments omit one or more protein domains.

A NT023 antigen can be expressed with the native 20 N-terminal amino acids of NT023 (MKKTNMALALLVAFSVTGCA; SEQ ID NO: 85) or can be expressed with an alternative N-terminal sequence *e.g*. with a simple N-terminus methionine, or Met-Ala-, or a leader peptide which targets or traffics the expressed protein in a desired fashion.

A NT023 antigen can be a lipoprotein *e.g*. lipidated at a N-terminus cysteine.

### NT100 antigen

This antigen has been annotated as "putative hydroxamate-type ferric siderophore receptor" and in NCBI as gi-145633184 from strain 3655. It has been cloned from R246 strain.

Useful NT100 antigens can elicit an antibody (*e.g*. when administered to a human) that recognises SEQ ID NO: 18 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 18; and/or (b) comprising a fragment of at least 'n' consecutive amino acids of SEQ ID NO: 18, wherein 'n' is 7 or more (*e.g.* 8, 10, 12, 14, 16, 18, 20, 25, 30, 35, 40, 50, 60, 70, 80, 90, 100, 150, 200, 250 or more). These NT100 proteins include variants of SEQ ID NO: 18, such as SEQ ID NO: 66. Preferred fragments of (b) comprise an epitope from SEQ ID NO: 18. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 18 while retaining at least one epitope of SEQ ID NO: 18. Other fragments omit one or more protein domains.

A NT100 antigen can be expressed with the native 30 N-terminal amino acids of NT100 (MDLGPIYNTRDINDGKVINIDNPNYTNPVA; SEQ ID NO: 86) or can be expressed with an alternative N-terminal sequence e.g. with a simple N-terminus methionine, or Met-Ala-, or a leader peptide which targets or traffics the expressed protein in a desired fashion.

### NT040 antigen

This antigen has been annotated as hypothetical protein NTHI1110 in 86-026NP strain. It has been cloned and expressed from R2846 strain
Useful NT040 antigens can elicit an antibody (*e.g*. when administered to a human) that recognises SEQ ID NO: 19 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 19; and/or (b) comprising a fragment of at least 'n' consecutive amino acids of SEQ ID NO: 19, wherein 'n' is 7 or more (*e.g.* 8, 10, 12, 14, 16, 18, 20, 25, 30, 35, 40, 50, 60, 70, 80, 90, 100, 150, 200, 250 or more). These NT040 proteins include variants of SEQ ID NO: 19. Preferred fragments of (b) comprise an epitope from SEQ ID NO: 19. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 19 while retaining at least one epitope of SEQ ID NO: 19. Other fragments omit one or more protein domains.

A NT040 antigen can be expressed with the native 26 N-terminal amino acids of NT040 (MMKTLLKGQTLLALMISLTLSSLLLL; SEQ ID NO: 87) or can be expressed with an alternative N-terminal sequence *e.g*. with a simple N-terminus methionine, or Met-Ala-, or a leader peptide which targets or traffics the expressed protein in a desired fashion.

### NT048 antigen

This antigen has been annotated as NTHI1169 in strain 86-028NP. It has been cloned and expressed from R2846 strain.

Useful NT048 antigens can elicit an antibody (*e.g*. when administered to a human) that recognises SEQ ID NO: 20 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 20; and/or (b) comprising a fragment of at least 'n' consecutive amino acids of SEQ ID NO: 20, wherein 'n' is 7 or more (*e.g.* 8, 10, 12, 14, 16, 18, 20, 25, 30, 35, 40, 50, 60, 70, 80, 90, 100, 150, 200, 250 or more). These NT048 proteins include variants of SEQ ID NO: 20. Preferred fragments of (b) comprise an epitope from SEQ ID NO: 20. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 20 while retaining at least one epitope of SEQ ID NO: 20. Other fragments omit one or more protein domains.

A NT048 antigen can be expressed with the native 18 N-terminal amino acids of NT048 (MKSVPLITGGLSFLLSAC; SEQ ID NO: 88) or can be expressed with an alternative N-terminal sequence *e.g*. with a simple N-terminus methionine, or Met-Ala-, or a leader peptide which targets or traffics the expressed protein in a desired fashion.

### NT053 antigen

The antigen has been annotated as gi-145628236 in R2846 strain and cloned from said strain.

Useful NT053 antigens can elicit an antibody (*e.g*. when administered to a human) that recognises SEQ ID NO: 21 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 21; and/or (b) comprising a fragment of at least 'n' consecutive amino acids of SEQ ID NO: 21, wherein 'n' is 7 or more (*e.g.* 8, 10, 12, 14, 16, 18, 20, 25, 30, 35, 40, 50, 60, 70, 80, 90, 100, 150, 200, 250 or more). These NT053 proteins include variants of SEQ ID NO: 21. Preferred fragments of (b) comprise an epitope from SEQ ID NO: 21. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 21 while retaining at least one epitope of SEQ ID NO: 21. Other fragments omit one or more protein domains.

A NT053 antigen can be expressed with the native N-terminal Met of NT053 or can be expressed with an alternative N-terminal sequence *e.g*. with Met-Ala-, or a leader peptide which targets or traffics the expressed protein in a desired fashion.

### NT066 antigen

The antigen has been annotated as NTHI1230 in NP86-028 strain and localized in the periplasm of the bacteria. It has been cloned and expressed from Fi176 strain.

Useful NT066 antigens can elicit an antibody (*e.g*. when administered to a human) that recognises SEQ ID NO: 22 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 22; and/or (b) comprising a fragment of at least 'n' consecutive amino acids of SEQ ID NO: 22, wherein 'n' is 7 or more (*e.g.* 8, 10, 12, 14, 16, 18, 20, 25, 30, 35, 40, 50, 60, 70, 80, 90, 100, 150, 200, 250 or more). These NT066 proteins include variants of SEQ ID NO: 22, such as SEQ ID NO: 67. Preferred fragments of (b) comprise an epitope from SEQ ID NO: 22. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25, 27, 30, 33 or more) from the N-terminus of SEQ ID NO: 22 while retaining at least one epitope of SEQ ID NO: 22. Other fragments omit one or more protein domains.

A NT066 antigen can be expressed with the native 33 N-terminal amino acids of NT066 (MKIYLRFVWILIIILNFLLNLFITTNGVIIVNA; SEQ ID NO: 90) or can be expressed with an alternative N-terminal sequence *e.g*. with a simple N-terminus methionine, or Met-Ala-, or a leader peptide which targets or traffics the expressed protein in a desired fashion.

### NT097 antigen

The antigen has been annotated as NTHI0522 in NP86-028 strain and described as long-chain fatty acid FadL like transporter protein predicted to be present in the outer membrane milieu. It has been cloned and expressed from R2846 strain.

Useful NT097 antigens can elicit an antibody (*e.g*. when administered to a human) that recognises SEQ ID NO: 23 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 23; and/or (b) comprising a fragment of at least 'n' consecutive amino acids of SEQ ID NO: 23, wherein 'n' is 7 or more (*e.g.* 8, 10, 12, 14, 16, 18, 20, 25, 30, 35, 40, 50, 60, 70, 80, 90, 100, 150, 200, 250 or more). These NT097 proteins include variants of SEQ ID NO: 23. Preferred fragments of (b) comprise an epitope from SEQ ID NO: 23. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 22, 25 or more) from the N-terminus of SEQ ID NO: 23 while retaining at least one epitope of SEQ ID NO: 23. Other fragments omit one or more protein domains.

A NT097 antigen can be expressed with the native 22 N-terminal amino acids of NT097 (MKKFNQSILATAMLLAAGGANA; SEQ ID NO: 91) or can be expressed with an alternative N-terminal sequence *e.g*. with a simple N-terminus methionine, or Met-Ala-, or a leader peptide which targets or traffics the expressed protein in a desired fashion.

### NT004 antigen

The antigen has been annotated as hypothetical protein CGSHiGG_08215 from strain PittGG in the outer membrane milieu. It has been cloned and expressed from Fi 176 strain.

Useful NT004 antigens can elicit an antibody (*e.g*. when administered to a human) that recognises SEQ ID NO: 122 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 122; and/or (b) comprising a fragment of at least 'n' consecutive amino acids of SEQ ID NO: 122, wherein 'n' is 7 or more (*e.g.* 8, 10, 12, 14, 16, 18, 20, 25, 30, 35, 40, 50, 60, 70, 80, 90, 100, 150, 200, 250 or more). These NT004 proteins include variants of SEQ ID NO: 122. Preferred fragments of (b) comprise an epitope from SEQ ID NO: 122. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 22, 25 or more) from the N-terminus of SEQ ID NO: 122 while retaining at least one epitope of SEQ ID NO: 122. Other fragments omit one or more protein domains.

A NT004 antigen can be expressed with the native 20 N-terminal amino acids of NT004 (MKKKNQILVSLSIVALLGGC; SEQ ID NO: 125) or can be expressed with an alternative N-terminal sequence *e.g*. with a simple N-terminus methionine, or Met-Ala-, or a leader peptide which targets or traffics the expressed protein in a desired fashion.

### NT014 antigen

The antigen has been annotated as hypothetical protein HI1658 from strain Rd KW20. It has been cloned and expressed from Fi176 strain.

Useful NT014 antigens can elicit an antibody (*e.g*. when administered to a human) that recognises SEQ ID NO: 123 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 123; and/or (b) comprising a fragment of at least 'n' consecutive amino acids of SEQ ID NO: 123, wherein 'n' is 7 or more (*e.g.* 8, 10, 12, 14, 16, 18, 20, 25, 30, 35, 40, 50, 60, 70, 80, 90, 100, 150, 200, 250 or more). These NT014 proteins include variants of SEQ ID NO: 123. Preferred fragments of (b) comprise an epitope from SEQ ID NO: 123. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 22, 25 or more) from the N-terminus of SEQ ID NO: 123 while retaining at least one epitope of SEQ ID NO: 123. Other fragments omit one or more protein domains.

A NT014 antigen can be expressed with the native 22 N-terminal amino acids of NT014 (MTLSPLKKLAILLGATIFLQGC; SEQ ID NO: 126) or can be expressed with an alternative N-terminal sequence *e.g*. with a simple N-terminus methionine, or Met-Ala-, or a leader peptide which targets or traffics the expressed protein in a desired fashion.

### NT022 antigen

The antigen has been annotated as NTHI0830 from strain NP86-028 and identified to be a possible outer membrane antigenic lipoprotein B. It has been cloned and expressed from Fi176 strain. It has been also found to contain a LytM catalytic domain and to be surface exposed and secreted.

Useful NT022 antigens can elicit an antibody (*e.g*. when administered to a human) that recognises SEQ ID NO: 124 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 124; and/or (b) comprising a fragment of at least 'n' consecutive amino acids of SEQ ID NO: 124, wherein 'n' is 7 or more (*e.g.* 8, 10, 12, 14, 16, 18, 20, 25, 30, 35, 40, 50, 60, 70, 80, 90, 100, 150, 200, 250 or more). These NT022 proteins include variants of SEQ ID NO: 124. Preferred fragments of (b) comprise an epitope from SEQ ID NO: 124. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 22, 25 or more) from the N-terminus of SEQ ID NO: 124 while retaining at least one epitope of SEQ ID NO: 124. Other fragments omit one or more protein domains.

A NT022 antigen can be expressed with the native 18 N-terminal amino acids of NT022 (MKKSFLLLPLSLVVLSAC; SEQ ID NO: 127) or can be expressed with an alternative N-terminal sequence *e.g*. with a simple N-terminus methionine, or Met-Ala-, or a leader peptide which targets or traffics the expressed protein in a desired fashion.

### SECOND ANTIGEN GROUP

### Antigen P48

The P48 polypeptide has been annotated in the literature as a Na(+)-translocating NADH-quinone reductase subunit A. For reference purposes, a full-length amino acid sequence of P48 is given as SEQ ID NO: 24 herein.

Preferred P48 polypeptides comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 24, *e.g.* 90% identity or more, or 95% identity or more, or 99% identity or more; and/or (b) comprising a fragment of at least 'n' consecutive amino acids of SEQ ID NO: 24, wherein 'n' is 7 or more (*e.g.* 8, 10, 12, 14, 16, 18, 20, 25, 30, 35, 40, 50, 60, 70, 80, 90, 100, 150, 200, 250 or more; *e.g.* 20 or more; or *e.g.* 50 or more; or *e.g.* 80 or more). These P48 polypeptides include variants of SEQ ID NO: 24. Preferred fragments of (b) comprise an epitope from SEQ ID NO: 24. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 24 while retaining at least one epitope of SEQ ID NO: 24. Other fragments omit one or more protein domains.

A P48 antigen ideally does not have the native 25 N-terminal amino acids of P48 (MITIKKGLDLPIAGKPAQVIHSGNA; SEQ ID NO: 92) and so it should be expressed with an alternative N-terminal sequence e.g. with a simple N-terminus methionine, or Met-Ala-, or a leader peptide which targets or traffics the expressed protein in a desired fashion.

T P48 antigen may advantageously be combined with one or more (*e.g.* 1, 2 or 3) of antigens HtrA, PE, P26, PHiD antigen and/or P6 as described herein, in particular, *e.g*. with HtrA.

### Antigen HtrA

The HtrA polypeptide has been annotated in the literature as a periplasmic serine protease do/HhoA-like precursor, and has been described as a heat-shock protein or chaperone. For reference purposes, a full-length amino acid sequence of HtrA is given as SEQ ID NO: 25 herein.

Preferred HtrA polypeptides comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 25, *e.g.* 90% identity or more, or 95% identity or more, or 99% identity or more; and/or (b) comprising a fragment of at least 'n' consecutive amino acids of SEQ ID NO: 25, wherein 'n' is 7 or more (*e.g.* 8, 10, 12, 14, 16, 18, 20, 25, 30, 35, 40, 50, 60, 70, 80, 90, 100, 150, 200, 250 or more; *e.g.* 20 or more; or *e.g.* 50 or more; or *e.g.* 80 or more). These HtrA polypeptides include variants of SEQ ID NO: 25. Preferred fragments of (b) comprise an epitope from SEQ ID NO: 25. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 25 while retaining at least one epitope of SEQ ID NO: 25. Other fragments omit one or more polypeptide domains.

A HtrA antigen ideally does not have the native 26 N-terminal amino acids of HtrA (MKKTRFVLNSIALGLSVLSTSFVAQA; SEQ ID NO: 93) and so it should be expressed with an alternative N-terminal sequence *e.g*. with a simple N-terminus methionine, or Met-Ala-, or a leader peptide which targets or traffics the expressed protein in a desired fashion.

TheHtrA antigen may advantageously be combined with one or more (*e.g*. 1, 2, or 3) of antigens P48, PE, P26, P6 and/or PHiD, in particular, e.g. with P48.

### Antigen PE

The PE polypeptide has been annotated as Lipoprotein - Vitronectin binding protein, or as binding IgD and acting as an adhesion to type 2 alveolar cells. For reference purposes, a full-length amino acid sequence of PE is given as SEQ ID NO: 26 herein.

Preferred PE polypeptides comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 26, *e.g.* 90% identity or more, or 95% identity or more, or 99% identity or more; and/or (b) comprising a fragment of at least 'n' consecutive amino acids of SEQ ID NO: 26, wherein 'n' is 7 or more (*e.g.* 8, 10, 12, 14, 16, 18, 20, 25, 30, 35, 40, 50, 60, 70, 80, 90, 100, 150 or more; *e.g.* 20 or more; or *e.g.* 50 or more; or *e.g.* 80 or more). These PE polypeptides include variants of SEQ ID NO: 26. Preferred fragments of (b) comprise an epitope from SEQ ID NO: 26. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 26 while retaining at least one epitope of SEQ ID NO: 26. Other fragments omit one or more polypeptide domains.

A PE antigen ideally does not have the native 16 N-terminal amino acids of PE (MKKIILTLSLGLLTAC; SEQ ID NO: 94) and so it should be expressed with an alternative N-terminal sequence *e.g*. with a simple N-terminus methionine, or Met-Ala-, or a leader peptide which targets or traffics the expressed protein in a desired fashion.

The PE antigen may advantageously be combined with one or more (e.g. 1, 2 or 3) of antigens P48, HtrA, P26, P6 and/or PHiD as described herein.

### Antigen P26

The P26 polypeptide is also known as outer membrane protein 26. It has been annotated as a member of the Skp family of proteins, whose putative function is translocation of outer membrane proteins [5]. For reference purposes, a full-length amino acid sequence of P26 is given as SEQ ID NO: 27 herein.

Preferred P26 polypeptides comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 27, *e.g.* 90% identity or more, or 95% identity or more, or 99% identity or more; and/or (b) comprising a fragment of at least 'n' consecutive amino acids of SEQ ID NO: 27, wherein 'n' is 7 or more (*e.g.* 8, 10, 12, 14, 16, 18, 20, 25, 30, 35, 40, 50, 60, 70, 80, 90, 100, 150 or more; *e.g.* 20 or more; or *e.g.* 50 or more; or *e.g.* 80 or more). These P26 polypeptides include variants of SEQ ID NO:27. Preferred fragments of (b) comprise an epitope from SEQ ID NO: 27. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 27 while retaining at least one epitope of SEQ ID NO: 27. Other fragments omit one or more protein domains.

The P26 antigen may advantageously be combined with one or more (*e.g*. 1, 2, or 3) of the antigens P48, HtrA, PE, PHiD and/or P6 as described herein, in particular with either or all of P48, HtrA and or PE as described herein.

### PHiD antigen

PHiD antigen is known also as "protein D" and has been used primarily as carrier protein in glycoconjugate NTHi vaccine approaches [95]. This antigen has been cloned and expressed from Fi176 strain.

Preferred PHiD polypeptides comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 28, *e.g.* 90% identity or more, or 95% identity or more, or 99% identity or more; and/or (b) comprising a fragment of at least 'n' consecutive amino acids of SEQ ID NO: 28, wherein 'n' is 7 or more (*e.g.* 8, 10, 12, 14, 16, 18, 20, 25, 30, 35, 40, 50, 60, 70, 80, 90, 100, 150 or more; *e.g.* 20 or more; or *e.g.* 50 or more; or *e.g.* 80 or more). These PHiD polypeptides include variants of SEQ ID NO: 28. Preferred fragments of (b) comprise an epitope from SEQ ID NO: 28. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 28 while retaining at least one epitope of SEQ ID NO: 28. Other fragments omit one or more protein domains.

A PhiD antigen can be expressed with the native 18 N-terminal amino acids of PhiD (MKLKTLALSLLAAGVLAG; SEQ ID NO: 95) or can be expressed with an alternative N-terminal sequence *e.g*. with a simple N-terminus methionine, or Met-Ala-, or a leader peptide which targets or traffics the expressed protein in a desired fashion. The PHiD antigen may advantageously combined with one or more (*e.g*. 1, 2, or 3) of any of the antigens P48, HtrA, PE, P26, and/or P6 as described herein.

A PhiD antigen can be a lipoprotein *e.g*. lipidated at a N-terminus cysteine.

### P6 antigen

P6 antigen is known also as OMP 6 (Outer membrane protein 6) [14]. This antigen was cloned and expressed from Fi176 strain.

Preferred P6 polypeptides comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 29, *e.g.* 90% identity or more, or 95% identity or more, or 99% identity or more; and/or (b) comprising a fragment of at least 'n' consecutive amino acids of SEQ ID NO: 29, wherein 'n' is 7 or more (*e.g.* 8, 10, 12, 14, 16, 18, 20, 25, 30, 35, 40, 50, 60, 70, 80, 90, 100, 150 or more; *e.g.* 20 or more; or *e.g.* 50 or more; or *e.g.* 80 or more). These P6 polypeptides include variants of SEQ ID NO: 29. Preferred fragments of (b) comprise an epitope from SEQ ID NO: 29. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 29 while retaining at least one epitope of SEQ ID NO: 29. Other fragments omit one or more protein domains.

A P6 antigen can be expressed with the native 19 N-terminal amino acids of P6 (MNKFVKSLLVAGSVAALAA; SEQ ID NO: 96) or can be expressed with an alternative N-terminal sequence *e.g*. with a simple N-terminus methionine, or Met-Ala-, or a leader peptide which targets or traffics the expressed protein in a desired fashion.

The P6 antigen may advantageously combined with one or more (*e.g*. 1, 2, or 3) of any of the antigens P48, HtrA, PE, P26, and/or PHiD as described herein.

A P6 antigen can be a lipoprotein e.g. lipidated at a N-terminus cysteine.

### THIRD ANTIGEN GROUP

### NT013 antigen

The "NT013" antigen is annotated as TPR repeat-containing protein and also as cytochrome c maturation heme lyase subunit CcmH2. It has been released as NTHI0532 in the strain 86-028NP. NT013 has been annotated as belonging to the metalloprotease protein family and it has a LytM catalytic domain.

Useful NT013 antigens can elicit an antibody (*e.g*. when administered to a human) that recognises SEQ ID NO: 30 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 30; and/or (b) comprising a fragment of at least 'n' consecutive amino acids of SEQ ID NO: 30, wherein 'n' is 7 or more (*e.g.* 8, 10, 12, 14, 16, 18, 20, 25, 30, 35, 40, 50, 60, 70, 80, 90, 100, 150, 200, 250 or more). These NT013 proteins include variants of SEQ ID NO: 30. Preferred fragments of (b) comprise an epitope from SEQ ID NO: 30. Other preferred fragments lack one or more amino acids (*e.g.* 1, 2, 3, 4, 5, 6, 7, 8, 9, 10, 15, 20, 25 or more) from the C-terminus and/or one or more amino acids *(e.g.* 1, 2, 3, 4, 5, 6, 7, 8, 9, 10, 15, 20, 25 or more) from the N-terminus of SEQ ID NO: 30 while retaining at least one epitope of SEQ ID NO: 30. Other fragments omit one or more protein domains.

A NT013 antigen can be expressed with the native 42 N-terminal amino acids of NT013 (MPVQHVKLARDRRKKRTYIKVGVFFVAILLILTGILLTIKDK; SEQ ID NO: 97) or can be expressed with an alternative N-terminal sequence *e.g*. with a simple N-terminus methionine, or Met-Ala-, or a leader peptide which targets or traffics the expressed protein in a desired fashion.

### NT106 antigen

The "NT106" antigen is annotated as lipoprotein and has been released as NTHI0363 in the genome 86-028NP.

Useful NT106 antigens can elicit an antibody (*e.g*. when administered to a human) that recognises SEQ ID NO: 31 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 31; and/or (b) comprising a fragment of at least 'n' consecutive amino acids of SEQ ID NO: 31, wherein 'n' is 7 or more (*e.g.* 8, 10, 12, 14, 16, 18, 20, 25, 30, 35, 40, 50, 60, 70, 80, 90, 100, 150, 200, 250 or more). These NT106 proteins include variants of SEQ ID NO: 31. Preferred fragments of (b) comprise an epitope from SEQ ID NO: 31. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 31 while retaining at least one epitope of SEQ ID NO: 31. Other fragments omit one or more protein domains.

A NT106 antigen can be expressed with the native 17 N-terminal amino acids of NT106 (MKKIILNLVTAIILAGC; SEQ ID NO: 98) or can be expressed with an alternative N-terminal sequence *e.g*. with a simple N-terminus methionine, or Met-Ala-, or a leader peptide which targets or traffics the expressed protein in a desired fashion.

A NT106 antigen can be a lipoprotein *e.g*. lipidated at a N-terminus cysteine.

### NT107 antigen

The "NT107" antigen is annotated as "Heme/hemopexin-binding protein B" and has been released as NTHI0370 in the genome 86-028NP.

Useful NT107 antigens can elicit an antibody (*e.g*. when administered to a human) that recognises SEQ ID NO: 32 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 32; and/or (b) comprising a fragment of at least 'n' consecutive amino acids of SEQ ID NO: 32, wherein 'n' is 7 or more (*e.g.* 8, 10, 12, 14, 16, 18, 20, 25, 30, 35, 40, 50, 60, 70, 80, 90, 100, 150, 200, 250 or more). These NT107 proteins include variants of SEQ ID NO: 32. Preferred fragments of (b) comprise an epitope from SEQ ID NO: 32. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 32 while retaining at least one epitope of SEQ ID NO: 32. Other fragments omit one or more protein domains.

A NT107 antigen can be expressed with the native 28 N-terminal amino acids of NT107 (MKMRPRYSVIASAVSLGFVLSKSVMALG; SEQ ID NO: 99) or can be expressed with an alternative N-terminal sequence *e.g*. with a simple N-terminus methionine, or Met-Ala-, or a leader peptide which targets or traffics the expressed protein in a desired fashion.

### NT108 antigen

The "NT108" antigen is annotated as murein transglycosylase A lipoprotein. In the strain 86-028NP has been annotated as NTHI0205.

Useful NT108 antigens can elicit an antibody (*e.g*. when administered to a human) that recognises SEQ ID NO: 33 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 33; and/or (b) comprising a fragment of at least 'n' consecutive amino acids of SEQ ID NO: 33, wherein 'n' is 7 or more *(e.g.* 8, 10, 12, 14, 16, 18, 20, 25, 30, 35, 40, 50, 60, 70, 80, 90, 100, 150, 200, 250 or more). These NT108 proteins include variants of SEQ ID NO: 33. Preferred fragments of (b) comprise an epitope from SEQ ID NO: 33. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 33 while retaining at least one epitope of SEQ ID NO: 33. Other fragments omit one or more protein domains.

A NT108 antigen can be expressed with the native 24 N-terminal amino acids of NT108 (MSVCKPFWFKTFSISIITALLVSC; SEQ ID NO: 100) or can be expressed with an alternative N-terminal sequence *e.g*. with a simple N-terminus methionine, or Met-Ala-, or a leader peptide which targets or traffics the expressed protein in a desired fashion.

A NT108 antigen can be a lipoprotein *e.g*. lipidated at a N-terminus cysteine.

### NT109 antigen

This antigen is annotated as nitrate/nitrite sensor protein NarQand has been identified as NTHI0374 in the strain 86-028NP and found to be conserved in the strains analysed.

Useful NT109 antigens can elicit an antibody (*e.g*. when administered to a human) that recognises SEQ ID NO: 34 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 34; and/or (b) comprising a fragment of at least 'n' consecutive amino acids of SEQ ID NO: 34, wherein 'n' is 7 or more (*e.g.* 8, 10, 12, 14, 16, 18, 20, 25, 30, 35, 40, 50, 60, 70, 80, 90, 100, 150, 200, 250 or more). These NT109 proteins include variants of SEQ ID NO: 34. Preferred fragments of (b) comprise an epitope from SEQ ID NO: 34. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 34 while retaining at least one epitope of SEQ ID NO: 34. Other fragments omit one or more protein domains.

A NT109 antigen can be expressed with the native 33 N-terminal amino acids of NT109 (MYTKGSVSTRIAKYLFIILIVAGVISSLSLAIM; SEQ ID NO: 101) or can be expressed with an alternative N-terminal sequence *e.g*. with a simple N-terminus methionine, or Met-Ala-, or a leader peptide which targets or traffics the expressed protein in a desired fashion.

### NT110 antigen

This antigen has been annotated as NTHI0579 in the genome 86-028NP and is known as putative haemolysis TlyC. IT has been found associated to the outer membrane.

Useful NT110 antigens can elicit an antibody (*e.g*. when administered to a human) that recognises SEQ ID NO: 35.

Preferred fragments of (b) comprise an epitope from SEQ ID NO: 35. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 35 while retaining at least one epitope of SEQ ID NO: 35. Other fragments omit one or more protein domains.

A NT110 antigen can be expressed with the native 30 N-terminal amino acids of NT110 (MIMELFHTILAIVALILSSAVVSSAEISLA; SEQ ID NO: 102) or can be expressed with an alternative N-terminal sequence *e.g*. with a simple N-terminus methionine, or Met-Ala-, or a leader peptide which targets or traffics the expressed protein in a desired fashion.

### NT111 antigen

This antigen has been annotated as NTHI0837 in the strain 86-028NP and described as putative lipoprotein.

Useful NT111 antigens can elicit an antibody (*e.g*. when administered to a human) that recognises SEQ ID NO: 36 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 36; and/or (b) comprising a fragment of at least 'n' consecutive amino acids of SEQ ID NO: 36, wherein 'n' is 7 or more (*e.g.* 8, 10, 12, 14, 16, 18, 20, 25, 30, 35, 40, 50, 60, 70, 80, 90, 100, 150, 200, 250 or more). These NT111 proteins include variants of SEQ ID NO: 36. Preferred fragments of (b) comprise an epitope from SEQ ID NO: 36. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 36 while retaining at least one epitope of SEQ ID NO: 36. Other fragments omit one or more protein domains.

A NT111 antigen can be expressed with the native 19 N-terminal amino acids of NT111 (MKKTLVAALISSVILLTGC; SEQ ID NO: 103) or can be expressed with an alternative N-terminal sequence e.g. with a simple N-terminus methionine, or Met-Ala-, or a leader peptide which targets or traffics the expressed protein in a desired fashion.

A NT110 antigen can be a lipoprotein *e.g*. lipidated at a N-terminus cysteine.

### NT112 antigen

This antigen has been annotated as NTHI0849 from NP86-028strain. It is annotated as VacJ lipoprotein.

Useful NT112 antigens can elicit an antibody (*e.g*. when administered to a human) that recognises SEQ ID NO: 37 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 37; and/or (b) comprising a fragment of at least 'n' consecutive amino acids of SEQ ID NO: 37, wherein 'n' is 7 or more (*e.g.* 8, 10, 12, 14, 16, 18, 20, 25, 30, 35, 40, 50, 60, 70, 80, 90, 100, 150, 200, 250 or more). These NT112 proteins include variants of SEQ ID NO: 37. Preferred fragments of (b) comprise an epitope from SEQ ID NO: 37. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 37 while retaining at least one epitope of SEQ ID NO: 37. Other fragments omit one or more protein domains.

A NT112 antigen can be expressed with the native 19 N-terminal amino acids of NT112 (MKTKVILTALLSAIALTGC; SEQ ID NO: 104) or can be expressed with an alternative N-terminal sequence *e.g*. with a simple N-terminus methionine, or Met-Ala-, or a leader peptide which targets or traffics the expressed protein in a desired fashion.

A NT112 antigen can be a lipoprotein *e.g*. lipidated at a N-terminus cysteine.

### NT113 antigen

This antigen has been annotated as NTHI0921 in 86-026NP strain and as lipoprotein.

Useful NT113 antigens can elicit an antibody (*e.g*. when administered to a human) that recognises SEQ ID NO: 38 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 38; and/or (b) comprising a fragment of at least 'n' consecutive amino acids of SEQ ID NO: 38, wherein 'n' is 7 or more (*e.g.* 8, 10, 12, 14, 16, 18, 20, 25, 30, 35, 40, 50, 60, 70, 80, 90, 100, 150, 200, 250 or more). These NT113 proteins include variants of SEQ ID NO: 38.

Preferred fragments of (b) comprise an epitope from SEQ ID NO: 38. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 38 while retaining at least one epitope of SEQ ID NO: 38. Other fragments omit one or more protein domains.

A NT113 antigen can be expressed with the native 16 N-terminal amino acids of NT113 (MKKYLLLALLPFLYAC; SEQ ID NO: 105) or can be expressed with an alternative N-terminal sequence *e.g*. with a simple N-terminus methionine, or Met-Ala-, or a leader peptide which targets or traffics the expressed protein in a desired fashion.

A NT113 antigen can be a lipoprotein *e.g*. lipidated at a N-terminus cysteine.

### NT114 antigen

This antigen has been annotated as soluble lytic murein transglycosylase protein and as NTHI0995 in 86-026NP strain.

Useful NT114 antigens can elicit an antibody (*e.g*. when administered to a human) that recognises SEQ ID NO: 39 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 39; and/or (b) comprising a fragment of at least 'n' consecutive amino acids of SEQ ID NO: 39, wherein 'n' is 7 or more (*e.g.* 8, 10, 12, 14, 16, 18, 20, 25, 30, 35, 40, 50, 60, 70, 80, 90, 100, 150, 200, 250 or more). These NT114 proteins include variants of SEQ ID NO: 39. Preferred fragments of (b) comprise an epitope from SEQ ID NO: 39. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 39 while retaining at least one epitope of SEQ ID NO: 39. Other fragments omit one or more protein domains.

A NT114 antigen can be expressed with the native 19 N-terminal amino acids of NT114 (MKKVALISLCIFTALSAFA; SEQ ID NO: 106) or can be expressed with an alternative N-terminal sequence *e.g*. with a simple N-terminus methionine, or Met-Ala-, or a leader peptide which targets or traffics the expressed protein in a desired fashion.

### NT115 antigen

This antigen has been annotated as NTHI1091 in 86-026NP strain and as putative LptE lipoprotein. It is located in the extracellular milieu.

Useful NT115 antigens can elicit an antibody (*e.g*. when administered to a human) that recognises SEQ ID NO: 40 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 40; and/or (b) comprising a fragment of at least 'n' consecutive amino acids of SEQ ID NO: 40, wherein 'n' is 7 or more (*e.g.* 8, 10, 12, 14, 16, 18, 20, 25, 30, 35, 40, 50, 60, 70, 80, 90, 100, 150, 200, 250 or more). These NT115 proteins include variants of SEQ ID NO: 40. Preferred fragments of (b) comprise an epitope from SEQ ID NO: 40. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 40 while retaining at least one epitope of SEQ ID NO: 40. Other fragments omit one or more protein domains.

A NT115 antigen can be expressed with the native 35 N-terminal amino acids of NT115 (MKYLHFTRPTIKVIFMINSIKTLLLIATLAILSAC; SEQ ID NO: 107) or can be expressed with an alternative N-terminal sequence *e.g*. with a simple N-terminus methionine, or Met-Ala-, or a leader peptide which targets or traffics the expressed protein in a desired fashion.

A NT115 antigen can be a lipoprotein *e.g*. lipidated at a N-terminus cysteine.

### NT116 antigen

This antigen has been described as NTHI1169 in 86-026NP strain and belongs to the transferrin-binding protein family.

Useful NT116 antigens can elicit an antibody (*e.g*. when administered to a human) that recognises SEQ ID NO: 41 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 41; and/or (b) comprising a fragment of at least 'n' consecutive amino acids of SEQ ID NO: 41, wherein 'n' is 7 or more (*e.g.* 8, 10, 12, 14, 16, 18, 20, 25, 30, 35, 40, 50, 60, 70, 80, 90, 100, 150, 200, 250 or more). These NT116 proteins include variants of SEQ ID NO: 41. Preferred fragments of (b) comprise an epitope from SEQ ID NO: 41. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 41 while retaining at least one epitope of SEQ ID NO: 41. Other fragments omit one or more protein domains.

A NT116 antigen can be expressed with the native 18 N-terminal amino acids of NT116 (MKSVPLITGGLSFLLSAC; SEQ ID NO: 108) or can be expressed with an alternative N-terminal sequence *e.g*. with a simple N-terminus methionine, or Met-Ala-, or a leader peptide which targets or traffics the expressed protein in a desired fashion.

### NT117 antigen

This antigen has been annotated as NTHI1208 in 86-026NP strain and putative transglutaminase. It has been located in the outer membrane.

Useful NT117 antigens can elicit an antibody (*e.g*. when administered to a human) that recognises SEQ ID NO: 42 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 42; and/or (b) comprising a fragment of at least 'n' consecutive amino acids of SEQ ID NO: 42, wherein 'n' is 7 or more (*e.g.* 8, 10, 12, 14, 16, 18, 20, 25, 30, 35, 40, 50, 60, 70, 80, 90, 100, 150, 200, 250 or more). These NT117 proteins include variants of SEQ ID NO: 42 Preferred fragments of (b) comprise an epitope from SEQ ID NO: 42 Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 42 while retaining at least one epitope of SEQ ID NO: 42. Other fragments omit one or more protein domains.

A NT117 antigen can be expressed with the native 19 N-terminal amino acids of NT117 (MKKLIAVAVFSACGSLAHA; SEQ ID NO: 109) or can be expressed with an alternative N-terminal sequence e.g. with a simple N-terminus methionine, or Met-Ala-, or a leader peptide which targets or traffics the expressed protein in a desired fashion.

### NT118 antigen

This antigen has been annotated as NTHI1318 in 86-026NP strain and as hypothetical protein.

Useful NT118 antigens can elicit an antibody (*e.g*. when administered to a human) that recognises SEQ ID NO: 43 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 43; and/or (b) comprising a fragment of at least 'n' consecutive amino acids of SEQ ID NO: 43, wherein 'n' is 7 or more (*e.g.* 8, 10, 12, 14, 16, 18, 20, 25, 30, 35, 40, 50, 60, 70, 80, 90, 100, 150, 200, 250 or more). These NT118 proteins include variants of SEQ ID NO: 43. Preferred fragments of (b) comprise an epitope from SEQ ID NO: 43. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 43 while retaining at least one epitope of SEQ ID NO: 43. Other fragments omit one or more protein domains.

A NT118 antigen can be expressed with the native 18 N-terminal amino acids of NT118 (MNIRWNVILGVIALCALA; SEQ ID NO: 110) or can be expressed with an alternative N-terminal sequence e.g. with a simple N-terminus methionine, or Met-Ala-, or a leader peptide which targets or traffics the expressed protein in a desired fashion.

### NT119 antigen

This antigen has been annotated as NTHI1565 hypothetical protein in 86-028NP strain.

Useful NT119 antigens can elicit an antibody (*e.g*. when administered to a human) that recognises SEQ ID NO: 114 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 114; and/or (b) comprising a fragment of at least 'n' consecutive amino acids of SEQ ID NO: 114, wherein 'n' is 7 or more (*e.g.* 8, 10, 12, 14, 16, 18, 20, 25, 30, 35, 40, 50, 60, 70, 80, 90, 100, 150, 200, 250 or more). These NT119 proteins include variants of SEQ ID NO: 114. Preferred fragments of (b) comprise an epitope from SEQ ID NO: 114. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 114 while retaining at least one epitope of SEQ ID NO: 114. Other fragments omit one or more protein domains.

A NT119 antigen can be expressed with the native 26 N-terminal amino acids of NT119 (MRFTKTLFTTALLGASIFSFQSTAWA; SEQ ID NO: 118) or can be expressed with an alternative N-terminal sequence *e.g*. with a simple N-terminus methionine, or Met-Ala-, or a leader peptide which targets or traffics the expressed protein in a desired fashion.

### NT120 antigen

This antigen has been annotated as NTHI1569 hypothetical protein in 86-028NP strain.

Useful NT120 antigens can elicit an antibody (*e.g*. when administered to a human) that recognises SEQ ID NO: 115 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 115; and/or (b) comprising a fragment of at least 'n' consecutive amino acids of SEQ ID NO: 115, wherein 'n' is 7 or more (*e.g.* 8, 10, 12, 14, 16, 18, 20, 25, 30, 35, 40, 50, 60, 70, 80, 90, 100, 150, 200, 250 or more). These NT120 proteins include variants of SEQ ID NO: 115. Preferred fragments of (b) comprise an epitope from SEQ ID NO: 115. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 115 while retaining at least one epitope of SEQ ID NO: 115. Other fragments omit one or more protein domains.

A NT120 antigen can be expressed with the native 26 N-terminal amino acids of NT120 (MKLTKTLLTTALFGASVFSFQSTAWA; SEQ ID NO: 119) or can be expressed with an alternative N-terminal sequence *e.g*. with a simple N-terminus methionine, or Met-Ala-, or a leader peptide which targets or traffics the expressed protein in a desired fashion.

### NT121 antigen

This antigen has been annotated as NTHI1571 hypothetical protein in 86-028NP strain.

Useful NT121 antigens can elicit an antibody (*e.g*. when administered to a human) that recognises SEQ ID NO: 116 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 116; and/or (b) comprising a fragment of at least 'n' consecutive amino acids of SEQ ID NO: 116, wherein 'n' is 7 or more (*e.g.* 8, 10, 12, 14, 16, 18, 20, 25, 30, 35, 40, 50, 60, 70, 80, 90, 100, 150, 200, 250 or more). These NT121 proteins include variants of SEQ ID NO: 116. Preferred fragments of (b) comprise an epitope from SEQ ID NO: 116. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 116 while retaining at least one epitope of SEQ ID NO: 116. Other fragments omit one or more protein domains.

A NT121 antigen can be expressed with the native 26 N-terminal amino acids of NT121 (MKLTKTLLTTALLGASVFSFQSTAWA; SEQ ID NO: 120) or can be expressed with an alternative N-terminal sequence *e.g*. with a simple N-terminus methionine, or Met-Ala-, or a leader peptide which targets or traffics the expressed protein in a desired fashion.

### NT122 antigen

This antigen has been annotated as NTHI1667 hypothetical protein in 86-028NP strain.

Useful NT122 antigens can elicit an antibody (*e.g*. when administered to a human) that recognises SEQ ID NO: 117 and/or may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 117; and/or (b) comprising a fragment of at least 'n' consecutive amino acids of SEQ ID NO: 117, wherein 'n' is 7 or more *(e.g.* 8, 10, 12, 14, 16, 18, 20, 25, 30, 35, 40, 50, 60, 70, 80, 90, 100, 150, 200, 250 or more). These NT122 proteins include variants of SEQ ID NO: 117. Preferred fragments of (b) comprise an epitope from SEQ ID NO: 117. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 117 while retaining at least one epitope of SEQ ID NO: 117. Other fragments omit one or more protein domains.

A NT122 antigen can be expressed with the native 23 N-terminal amino acids of NT122 (MEKIMKKLTLALVLGSALAVTGC; SEQ ID NO: 121) or can be expressed with an alternative N-terminal sequence *e.g*. with a simple N-terminus methionine, or Met-Ala-, or a leader peptide which targets or traffics the expressed protein in a desired fashion.

### NT123 antigen

This antigen has been annotated as zinc protease NTHI1796 in 86-026NP strain.

Useful NT123 antigens can elicit an antibody (*e.g*. when administered to a human) that recognises SEQ ID NO: 44 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 44; and/or (b) comprising a fragment of at least 'n' consecutive amino acids of SEQ ID NO: 44, wherein 'n' is 7 or more (*e.g.* 8, 10, 12, 14, 16, 18, 20, 25, 30, 35, 40, 50, 60, 70, 80, 90, 100, 150, 200, 250 or more). These NT123 proteins include variants of SEQ ID NO: 44. Preferred fragments of (b) comprise an epitope from SEQ ID NO: 44. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 44 while retaining at least one epitope of SEQ ID NO: 44. Other fragments omit one or more protein domains.

A NT123 antigen can be expressed with the native 17 N-terminal amino acids of NT123 (MKKTTALFLLIFSLIAC; SEQ ID NO: 111) or can be expressed with an alternative N-terminal sequence *e.g*. with a simple N-terminus methionine, or Met-Ala-, or a leader peptide which targets or traffics the expressed protein in a desired fashion.

### NT124 antigen

This antigen has been annotated as hypothetical protein NTHI1930 in 86-026NP strain.

Useful NT124 antigens can elicit an antibody (*e.g*. when administered to a human) that recognises SEQ ID NO: 45 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 45; and/or (b) comprising a fragment of at least 'n' consecutive amino acids of SEQ ID NO: 45, wherein 'n' is 7 or more (*e.g.* 8, 10, 12, 14, 16, 18, 20, 25, 30, 35, 40, 50, 60, 70, 80, 90, 100, 150, 200, 250 or more). These NT124 proteins include variants of SEQ ID NO: 45. Preferred fragments of (b) comprise an epitope from SEQ ID NO: 45. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 45 while retaining at least one epitope of SEQ ID NO: 45. Other fragments omit one or more protein domains.

A NT124 antigen can be expressed with the native 22 N-terminal amino acids of NT124 (MKKSKIAAGVVISLAAVWCAGA; SEQ ID NO: 89) or can be expressed with an alternative N-terminal sequence *e.g*. with a simple N-terminus methionine, or Met-Ala-, or a leader peptide which targets or traffics the expressed protein in a desired fashion.

### NT061 antigen

This antigen has been annotated as survival protein SurA-like protein NTHI0588 in 86-026NP strain.

Useful NT061 antigens antigens can elicit an antibody (*e.g*. when administered to a human) that recognises SEQ ID NO: 128 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 128; and/or (b) comprising a fragment of at least 'n' consecutive amino acids of SEQ ID NO: 128, wherein 'n' is 7 or more (*e.g.* 8, 10, 12, 14, 16, 18, 20, 25, 30, 35, 40, 50, 60, 70, 80, 90, 100, 150, 200, 250 or more). These NT061 proteins include variants of SEQ ID NO: 128. Preferred fragments of (b) comprise an epitope from SEQ ID NO: 128. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 128 while retaining at least one epitope of SEQ ID NO: 128. Other fragments omit one or more protein domains.

A NT061 antigen can be expressed with the native 27 N-terminal amino acids of NT061 (MKMKKFILKSFLLATLGCVAFTSMAQA; SEQ ID NO: 129) or can be expressed with an alternative N-terminal sequence *e.g*. with a simple N-terminus methionine, or Met-Ala-, or a leader peptide which targets or traffics the expressed protein in a desired fashion.

### NT017 antigen

This antigen has been annotated as survival protein SurA-like protein NTHI0915 in 86-026NP strain.

Useful NT017 antigens antigens can elicit an antibody (*e.g*. when administered to a human) that recognises SEQ ID NO: 130 and/or may comprise an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more) to SEQ ID NO: 130; and/or (b) comprising a fragment of at least 'n' consecutive amino acids of SEQ ID NO: 130, wherein 'n' is 7 or more (*e.g.* 8, 10, 12, 14, 16, 18, 20, 25, 30, 35, 40, 50, 60, 70, 80, 90, 100, 150, 200, 250 or more). These NT017 proteins include variants of SEQ ID NO: 130. Preferred fragments of (b) comprise an epitope from SEQ ID NO: 130. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 130 while retaining at least one epitope of SEQ ID NO: 130. Other fragments omit one or more protein domains.

A NT017 antigen can be expressed with the native 20 N-terminal amino acids of NT017 (MLRFGVNQKTSLLLTALLSC; SEQ ID NO: 131) or can be expressed with an alternative N-terminal sequence *e.g*. with a simple N-terminus methionine, or Met-Ala-, or a leader peptide which targets or traffics the expressed protein in a desired fashion.

### Hybrid polypeptides

The polypeptides used may be expressed individually or independently on separate polypeptide chains. Alternatively, two or more of the polypeptides used may also be expressed as a single polypeptide chain (a 'hybrid' polypeptide). Hybrid polypeptides can be represented by the formula NH₂-A-{-X-L-}*ₙ*-B-COOH, wherein: A is an optional N-terminal amino acid sequence; B is an optional C-terminal amino acid sequence; *n* is an integer of 2 or more (*e.g.* 2, 3, 4, 5, 6, *etc*.); wherein at least one of the X*ₙ* is an amino acid sequence of an antigen disclosed herein (as described above); and L is an optional linker amino acid sequence. For example, each X*ₙ* may comprise the amino acid sequences of an antigen selected from the group consisting of: (1) NTHI0915 (NT018), (2) NTHI1416 (NT024), (3) NTHI2017 (NT032), (4) CGSHiGG_02400 (NT038, but this is not amongst preferred), (5) NTHI1292 (NT067), (6) NTHI0877 (NT001), (7) NTHI0266 (NT016), (8) CGSHiGG_00130 (NT052), (9) NTHI1627 (NT002), (10) NTHI1109 (NT026), (11) NTHI0821 (NT009), (12) NTHI0409 (NT025), (13) NTHI1954 (NT028), (14) NTHI0371 (NT029), (15) NTHI0509 (NT031), (16) NTHI0449 (NT015), (17) NTHI1473 (NT023), (18) gi-145633184 (NT100), (19) NTHI1110 (NT040), (20) gi-46129075 (NT048), (21) gi-145628236 (NT053), (22) NTHI1230 (NT066), (23) NTHI0522 (NT097), (24) P48 (NTHI0254 also defined as NT007), (25) HtrA (NTHI1905 also defined as NT006), (26) PE (NTHI0267 also defined as NT035), (27) P26 (NTHI0501 also defined as NT010), (28) PHiD (NTHI0811 also defined as NT080), (29) P6 (NTHI0501, also defined as NT081), (30) NT013, (31) NT106, (32) NT107, (33) NT108, (34) NT109, (35) NT110, (36) NT111, (37) NT112, (38) NT113, (39) NT114, (40) NT115, (41) NT116, (42) NT117, (43) NT118, (44) NT119, (45) NT120, (46) NT121, (47), NT122, (48) NT123, (49) NT124; (50) NT004; (51) NT014; (52) NT022 (also annotated as NTHI0830); (53) NT016 (also annotated as NTHI0266).

The X*ₙ* may comprise the amino acid sequences of two or more antigens selected from the group consisting of any of the antigen listed in the "First antigen group" and any of the antigen listed in the "Second antigen group". Each X*ₙ* may be an amino acid sequence of an antigen of an antigen combination described herein (as described above). In certain embodiments, *n* is 2. When *n* is 2, any combination of two of the antigens as described above may also be used as described herein. When *n* is 3, for example, any combination of three antigens as described above may be used. Generally, two or more of the X*ₙ* may be the same antigens or, when *n* is 2, 3, or 4, each X*ₙ* may be a different antigen. When two or more of the X*ₙ* are sequences of the same antigen), said two or more X*ₙ* may have the same polypeptide sequence or a different polypeptide sequence, *e.g*., may be different variants or fragments of the given antigen, as described above.

Where these antigens are defined in terms of (a) having 50% or more identity (e.g. 60%, 65%, 70%, 75%, 80%, 85%, 90%, 91%, 92%, 93%, 94%, 95%, 96%, 97%, 98%, 99%, 99.5% or more) to a given sequence; and/or (b) comprising a fragment of at least 'n' consecutive amino acids of a given sequence, wherein 'n' is 7 or more (e.g. 8, 10, 12, 14, 16, 18, 20, 25, 30, 35, 40, 50, 60, 70, 80, 90, 100, 150, 200, 250 or more), the level of identity in (a) and the value of 'n' in (b) may be the same for each X.

The leader peptide sequence in the wild-type form of each -X- moiety may be included or omitted in the hybrid protein. In some embodiments, the leader peptides will be deleted except for that of the - X- moiety located at the N-terminus of the hybrid protein *i.e.* the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker amino acid sequence -L- may be present or absent. For instance, when *n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc.* Linker amino acid sequence(s) -L- will typically be short (*e.g. 20* or fewer amino acids *i.e.* 20, 19, 18, 17, 16, 15, 14, 13, 12, 11, 10, 9, 8, 7, 6, 5, 4, 3, 2, 1). Examples comprise short peptide sequences which facilitate cloning, poly-glycine linkers *(i.e.* comprising Gly*ₙ* where *n* = 2, 3, 4, 5, 6, 7, 8, 9, 10 or more), and histidine tags (*i.e.* His*ₙ* where *n* = 3, 4, 5, 6, 7, 8, 9, 10 or more). Other suitable linker amino acid sequences will be apparent to those skilled in the art. A useful linker is GSGGGG (SEQ ID NO:46) or GSGSGGGG (SEQ ID NO:47), with the Gly-Ser dipeptide being formed from a *Bam*HI restriction site, thus aiding cloning and manipulation, and the (Gly)₄ tetrapeptide being a typical poly-glycine linker. Other suitable linkers, particularly for use as the final Lₙ are a Leu-Glu dipeptide.
- A- is an optional N-terminal amino acid sequence. This will typically be short (*e.g.* 40 or fewer amino acids *i.e.* 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g*. histidine tags *i.e.* His*ₙ* where *n* = 3, 4, 5, 6, 7, 8, 9, 10 or more) such as SEQ ID NO: 48. Other suitable N-terminal amino acid sequences will be apparent to those skilled in the art. If X₁ lacks its own N-terminus methionine, -A- is preferably an oligopeptide (*e.g*. with 1, 2, 3, 4, 5, 6, 7 or 8 amino acids) which provides a N-terminus methionine *e.g*. Met-Ala-Ser, or a single Met residue.
- B- is an optional C-terminal amino acid sequence. This will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g*. comprising histidine tags *i.e.* His*ₙ* where *n* = 3, 4, 5, 6, 7, 8, 9, 10 or more, such as SEQ ID NO: 68), or sequences which enhance protein stability. Other suitable C-terminal amino acid sequences will be apparent to those skilled in the art.

### Strains and variants

Antigens are defined above by reference to naming conventions from the literature *e.g*. the "NTHI" numbering (from the genome of strain 86-028NP) or CGSHiGG numbering (from the genome of strain PittGG). Such conventions are explained in more detail in reference 15 (particularly Table 1). Table V herein relates the existing nomenclature to the "NT" nomenclature used herein. Thus an exemplary amino acid and nucleotide sequence for any of the antigens disclosed herein can easily be found in public sequence databases for the indicated strains (together with additional information, such as functional annotations), but the disclosure is not limited to sequences from the 86-028NP, 3655 or PittGG strains. Genome sequences of several other NTHI strains are available (again, see Table 1 of reference 15). Standard search and alignment techniques can be used to identify in any of these (or other) further genome sequences the homolog of any particular sequence given herein. Moreover, the available sequences can be used to design primers for amplification of homologous sequences from other strains. Thus the disclosure is not limited to these specific strains, but rather encompasses such variants and homologs from other NTHI strains, as well as non-natural variants. In general, suitable variants of a particular SEQ ID NO include its allelic variants, its polymorphic forms, its homologs, its orthologs, its paralogs, its mutants, *etc.* For instance, SEQ ID Nos: 49, 52, 54, 55, 57-59, 64, 65 & 67 include mutations as described below.

Thus, for instance, polypeptides used may, compared to the SEQ ID NO herein, include one or more (*e.g.* 1, 2, 3, 4, 5, 6, 7, 8, 9, *etc.*) amino acid substitutions, such as conservative substitutions (*i.e.* substitutions of one amino acid with another which has a related side chain). Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may also include one or more (*e.g.* 1, 2, 3, 4, 5, 6, 7, 8, 9, *etc.*) single amino acid deletions relative to the SEQ ID NO sequences. The polypeptides may also include one or more (*e.g.* 1, 2, 3, 4, 5, 6, 7, 8, 9, *etc.*) insertions (*e.g.* each of 1, 2, 3, 4 or 5 amino acids) relative to the SEQ ID NO sequences.

Similarly, a polypeptide used may comprise an amino acid sequence that:
(a) is identical (*i.e.* 100% identical) to a sequence disclosed in the sequence listing;
(b) shares sequence identity (*e.g*. 80%, 85%, 90%, 91%, 92%, 93%, 94%, 95%, 96%, 97%, 98%, 99%, 99.5% or more) with a sequence disclosed in the sequence listing;
(c) has 1, 2, 3, 4, 5, 6, 7, 8, 9 or 10 (or more) single amino acid alterations (deletions, insertions, substitutions), which may be at separate locations or may be contiguous, as compared to the sequences of (a) or (b); and/or
(d) when aligned with a particular sequence from the sequence listing using a pairwise alignment algorithm, each moving window of x amino acids from N-terminus to C-terminus (such that for an alignment that extends to *p* amino acids, where *p*>*x*, there are *p-x*+*1* such windows) has at least *x·y* identical aligned amino acids, where: x is selected from 20, 25, 30, 35, 40, 45, 50, 60, 70, 80, 90, 100, 150, 200; y is selected from 0.50, 0.60, 0.70, 0.75, 0.80, 0.85, 0.90, 0.91, 0.92, 0.93, 0.94, 0.95, 0.96, 0.97, 0.98, 0.99; and if *x·y* is not an integer then it is rounded up to the nearest integer. The preferred pairwise alignment algorithm is the Needleman-Wunsch global alignment algorithm [16], using default parameters (*e.g*. with Gap opening penalty = 10.0, and with Gap extension penalty = 0.5, using the EBLOSUM62 scoring matrix). This algorithm is conveniently implemented in the *needle* tool in the EMBOSS package [17].

Where hybrid polypeptides are used, the individual antigens within the hybrid (*i.e.* individual -X-moieties) may be from one or more strains. Where *n*=2, for instance, X₂ may be from the same strain as X₁ or from a different strain. Where n=3, the strains might be (i) X₁=X₂=X₃ (ii) X₁=X₂≠X₃ (iii) X₁≠X₂=X₃ (iv) X₁≠X₂≠X₃ or (v) X₁=X₃≠X₂, *etc.*

Within group (c), deletions or substitutions may be at the N-terminus and/or C-terminus, or may be between the two termini. Thus a truncation is an example of a deletion. Truncations may involve deletion of up to 40 (or more) amino acids at the N-terminus and/or C-terminus. N-terminus truncation can remove leader peptides *e.g.* to facilitate recombinant expression in a heterologous host. C-terminus truncation can remove anchor sequences *e.g*. to facilitate recombinant expression in a heterologous host.

In general, when an antigen comprises a sequence that is not identical to a NTHI sequence from the sequence listing (*e.g*. when it comprises a sequence listing with <100% sequence identity thereto, or when it comprises a fragment thereof) it is preferred in each individual instance that the antigen can elicit an antibody which recognises the respective NTHI sequence from the sequence listing.

### Mutant bacteria

The present disclosure also provides a *NTHi* bacterium in which one or more of the antigens of the invention has/have been knocked out [18]. Techniques for producing knockout bacteria are well known, and knockout of genes from *NTHi* strains have been reported *i.e.* in Ref. 19. A knockout mutation may be situated in the coding region of the gene or may lie within its transcriptional control regions (*e.g*. within its promoter). A knockout mutation will reduce the level of mRNA encoding the antigen to <1% of that produced by the wild-type bacterium, preferably <0.5%, more preferably <0.1%, and most preferably to 0%.

The disclosure also provides a *NTHI* bacterium in which one or more of the antigens of the invention has a mutation which inhibits its activity. The gene encoding the antigen will have a mutation that changes the encoded amino acid sequence or abolishes its expression. Mutation may involve deletion, substitution, and/or insertion, any of which may be involve one or more amino acids.

One example provides deletions of one or more genes codying for antigens disclosed herein.

It was known in *E. coli* that two components of the division machinery with LytM domains (EnvC and NlpD) are direct regulators of the cell wall hydrolases (amidases) responsible for cell separation (AmiA, AmiB and AmiC) [20]. It is also known that LytM metalloproteases in E. coli are absolutely required for daughter cell separation.

Also disclosed herein are NTHI genes codifying for polypeptides that have the LytM catalytic domain. Generally metalloproteases are identified as containing HxH and HxxxD aminoacid domains in their catalytic domains. Preferably, these one or more genes are codifying for any one of NT013, NT022 or NT017.

Also disclosed is that the mutation or deletion of one or more genes encoding for polypeptides having in common the LytM catalytic domain results in a drastic change in the bacterial cell division and bacterial phenotype.

Inventors have also shown that said mutation or deletion results in the release of vesicles known as OMVs or outer membrane vesicles, whereas the same wild type NTHi strains do not normally release OMVs.

In one particularly preferred embodiment it is described that by deleting NT013 and/or NT022 not only the bacterial cell division is affected, but there is also a surprising production and release of outer membrane vesicles (OMVs) in NTHI strains, that normally do not release OMVs.

Preferred embodiments provide NTHI strains wherein the deletions of one or more genes codying for anyone of NT013 or NT022 or NT017. For instance, the genes deleted can be substituted with an antibiotic resistance cassette, such as the erytromycin resistance cassette. It has been found that all the above mentioned polypeptides have in common a LytM catalytic domain and are all metalloproteases.

It has been also found that the LytM domain in NT013 and NT022 is conserved. NT013 catalytic active site is represented by the following aminoacid motifs -HKGD- and -HLH- at the C-terminal portion, of NT022 catalytic active site is represented by the following aminoacid motifs -NKGID-and -KLH- at the C-terminal.

The disclsosure also provides a bacterium, such as a *NTHi* bacterium, which hyper-expresses an antigen disclosed herein.

The disclosure also provides a bacterium, such as a *NTHi* bacterium, that constitutively expresses an antigen disclosed herein. The disclosure also provides a *E. coli* comprising at least a gene encoding an antigen disclosed herein, wherein the gene is under the control of an inducible promoter.

### OMV based vaccine

Gram-negative bacteria are separated from the external medium by two successive layers of membrane structures. These structures, referred to as the cytoplasmic membrane and the outer membrane (OM), differ both structurally and functionally. The outer membrane plays an important role in the interaction of pathogenic bacteria with their respective hosts. Consequently, the surface exposed bacterial molecules represent important targets for the host immune response, making outer-membrane components attractive candidates in providing vaccine, diagnostic and therapeutics reagents.

Mutant bacteria disclosed herein are particularly useful for preparing bacterial outer membrane vesicles which include *NTHi* antigens *(e.g.* antigens disclosed herein), and which can be used as immunogens.

The disclosure also provides a bacterium, such as a *NTHi* bacterium, which hyper-expresses at least one antigen disclosed herein preferably by overproducing OMVs.

Up-regulation can be used to increase the levels of useful NTHi proteins in OMVs.

A method for producing a NTHi bacterium overproducing OMVs disclosed herein is also disclosed, which method comprises genetically modifying a Gram-negative bacterial strain by one or more of the following processes: (a) engineering the strain to downregulate expression of one or more *Tol* genes; and (b) mutating one or more gene(s) encoding a protein comprising a peptidoglycan-associated site to attenuate the peptidoglycan-binding activity of the protein(s); (c) by mutation or deletion of one or more genes encoding for polypeptides having in common the LytM catalytic domain. In one particularly preferred embodiment, the NTHi might not express active NT013, NT022 genes and/or any of Tol genes [19], [18].

The disclosure also provides a process for preparing a NTHi vesicle, comprising a step of treating a NTHi bacterium disclosed herein such that its outer membrane forms vesicles.

The disclosure also provides a process for preparing a NTHi vesicle, comprising a step of culturing a NTHi bacterium of disclosed herein under conditions in which its outer membrane spontaneously sheds vesicles.

The disclosure also provides a NTHi bacterium which overproduces OMVs and which also hyperexpresses the antigens disclosed herein.

### Polypeptides used with the disclosure

Polypeptides used with the disclosure can take various forms (*e.g*. native, fusions, glycosylated, non-glycosylated, lipidated, non-lipidated, phosphorylated, non-phosphorylated, myristoylated, non-myristoylated, monomeric, multimeric, particulate, denatured, *etc.*)*.*

Polypeptides can be prepared by various means (*e.g*. recombinant expression, purification from cell culture, chemical synthesis, *etc.*)*.* Recombinantly-expressed proteins are preferred, particularly for hybrid polypeptides.

Polypeptides are preferably provided in purified or substantially purified form *i.e.* substantially free from other polypeptides (*e.g*. free from naturally-occurring polypeptides), particularly from other *H. influenzae* or host cell polypeptides, and are generally at least about 50% pure (by weight), and usually at least about 90% pure *i.e.* less than about 50%, and more preferably less than about 10% (*e.g.* 5%) of a composition is made up of other expressed polypeptides. Thus the antigens in the compositions are separated from the whole organism with which the molecule is expressed.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labelling component. Also included are, for example, polypeptides containing one or more analogues of an amino acid (including, for example, unnatural amino acids, *etc.*), as well as other modifications known in the art. Polypeptides can occur as single chains or associated chains.

Polypeptides may comprise a sequence -P-Q- or -Q-P-, wherein: -P- is an amino acid sequence as defined above and -Q- is not a sequence as defined above *i.e.* may be provided as fusion proteins. Where the N-terminus codon of -P- is not ATG, but this codon is not present at the N-terminus of a polypeptide, it will be translated as the standard amino acid for that codon rather than as a Met. Where this codon is at the N-terminus of a polypeptide, however, it will be translated as Met. Examples of -Q- moieties include, but are not limited to, histidine tags (*i.e.* His*ₙ* where *n* = 3, 4, 5, 6, 7, 8, 9, 10 or more *e.g.* SEQ ID NO: 68), maltose-binding protein, or glutathione-S-transferase (GST).

Polypeptides may comprise sequence -P-Q- or -Q-P when initially expressed as a nascent protein, but in some embodiments the -Q- moiety may be absent from the protein at its point of use *e.g.* a leader peptide might be post-translationally cleaved.

A useful N-terminus sequence for expression is SEQ ID NO: 48.

Although expression of the polypeptides may take place in a *H. influenzae,* a heterologous host will usually be used for expression (recombinant expression). The heterologous host may be prokaryotic (*e.g.* a bacterium) or eukaryotic. It may be *E.coli*, but other suitable hosts include *Bacillus subtilis*, *Vibrio cholerae, Salmonella typhi, Salmonella typhimurium, Neisseria lactamica, Neisseria cinerea, Mycobacteria* (*e.g. M.tuberculosis*), yeasts, *etc.* Compared to the wild-type *H. influenzae* genes encoding polypeptides of the invention, it is helpful to change codons to optimise expression efficiency in such hosts without affecting the encoded amino acids.

Polypeptides may be synthesised by a process comprising a step of synthesising at least part of the polypeptide by chemical means.

### Nucleic acids

The disclosure also provides nucleic acids (*e.g*. combinations of nucleic acids, vectors, or vector combinations), encoding polypeptides used with the invention, combinations of polypeptides or hybrid polypeptides. It also provides nucleic acid comprising a nucleotide sequence that encodes one or more (*e.g*., 2, 3 or 4) polypeptides or hybrid polypeptides of the antigen combinations disclosed herein. A nucleic acid may be, *e.g.*, a vector (*e.g.* a cloning or expression vector).

Nucleotide sequences encoding polypeptides of the one or more (at least one) antigen and antigen combinations disclosed herein are either known (see *e.g.* references 6-9) or may be designed according to the genetic code. Thus, in the context of the present disclosure, such a nucleotide sequence may encode one or more of: (1) NTHI0915 (NT018), (2) NTHI1416 (NT024), (3) NTHI2017 (NT032), (4) CGSHiGG_02400 (NT038), (5) NTHI1292 (NT067), (6) NTHI0877 (NT001), (7) NTHI0266 (NT016), (8) CGSHiGG_00130 (NT052), (9) NTHI1627 (NT002), (10) NTHI1109 (NT026), (11) NTHI0821 (NT009), (12) NTHI0409 (NT025), (13) NTHI1954 (NT028), (14) NTHI0371 (NT029), (15) NTHI0509 (NT031), (16) NTHI0449 (NT015), (17) NTHI1473 (NT023), (18) gi-145633184 (NT100), (19) NTHI1110 (NT040), (20) gi-46129075 (NT048), (21) gi-145628236 (NT053), (22) NTHI1230 (NT066), (23) NTHI0522 (NT097) or a P48 antigen (such as SEQ ID NO: 24); an HtrA antigen (such as SEQ ID NO: 25); a PE antigen (such as SEQ ID NO: 26); P26 antigen (such as SEQ ID NO: 27); a PHiD antigen (such as SEQ ID NO: 28); a P6 antigen (such as SEQ ID NO: 29), (24) NT004, (25) NT014, (26) NT022 or one or more antigens from the "third antigen group" or may encode an amino acid sequence: (a) having 50% or more identity (*e.g*. 60%, 65%, 70%, 75%, 80%, 85%, 90%, 91%, 92%, 93%, 94%, 95%, 96%, 97%, 98%, 99%, 99.5% or more, *e.g.* 90% identity or more, or 95% identity or more, or 99% identity or more, to any of above mentioned polypeptides; and/or (b) comprising a fragment of at least 'n' consecutive amino acids of any of said polypeptides: 1, wherein 'n' is 7 or more (*e.g.* 8, 10, 12, 14, 16, 18, 20, 25, 30, 35, 40, 50, 60, 70, 80, 90, 100, 150, 200, 250 or more; *e.g.* 20 or more; or *e.g.* 50 or more; or *e.g.* 80 or more).

The invention also provides nucleic acid which can hybridize to these nucleic acids. Hybridization reactions can be performed under conditions of different "stringency". Conditions that increase stringency of a hybridization reaction of widely known and published in the art (*e.g.* page 752 of reference 121). Examples of relevant conditions include (in order of increasing stringency): incubation temperatures of 25°C, 37°C, 50°C, 55°C and 68°C; buffer concentrations of 10 x SSC, 6 x SSC, 1 x SSC, 0.1 x SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water. Hybridization techniques and their optimization are well known in the art (*e.g.* see refs, 21, 22 & 123).

A nucleic acid may hybridize to a target under low stringency conditions; in other embodiments it hybridizes under intermediate stringency conditions; in preferred embodiments, it hybridizes under high stringency conditions. An exemplary set of low stringency hybridization conditions is 50°C and 10 x SSC. An exemplary set of intermediate stringency hybridization conditions is 55°C and 1 x SSC. An exemplary set of high stringency hybridization conditions is 68°C and 0.1 x SSC.

The disclosure includes nucleic acid comprising sequences complementary to these sequences (*e.g*. for antisense or probing, or for use as primers).

Nucleic acid can take various forms (*e.g*. single-stranded, double-stranded, vectors, primers, probes, labelled *etc.*)*.* Nucleic acids may be circular or branched, but will generally be linear. Unless otherwise specified or required, any embodiment that utilizes a nucleic acid may utilize both the double-stranded form and each of two complementary single-stranded forms which make up the double-stranded form. Primers and probes are generally single-stranded, as are antisense nucleic acids.

Nucleic acids encoding antigens described herein are preferably provided in purified or substantially purified form *i.e.* substantially free from other nucleic acids (*e.g.* free from naturally-occurring nucleic acids), particularly from other *H. influenzae* or host cell nucleic acids, generally being at least about 50% pure (by weight), and usually at least about 90% pure. Nucleic acids are preferably *H. influenzae* nucleic acids.

Nucleic acids encoding antigens described herein may be prepared in many ways e.g. by chemical synthesis (*e.g*. phosphoramidite synthesis of DNA) in whole or in part, by digesting longer nucleic acids using nucleases (*e.g.* restriction enzymes), by joining shorter nucleic acids or nucleotides (*e.g.* using ligases or polymerases), from genomic or cDNA libraries, *etc.*

Nucleic acids may be attached to a solid support (*e.g.* a bead, plate, filter, film, slide, microarray support, resin, *etc*.)*.* Nucleic acids may be labelled *e.g.* with a radioactive or fluorescent label, or a biotin label. This is particularly useful where the nucleic acid is to be used in detection techniques *e.g*. where the nucleic acid is a primer or as a probe.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (*e.g*. peptide nucleic acids (PNAs) or phosphorothioates) or modified bases. Thus the disclosure includes mRNA, tRNA, rRNA, ribozymes, DNA, cDNA, recombinant nucleic acids, branched nucleic acids, plasmids, vectors, probes, primers, *etc..* Where nucleic acid takes the form of RNA, it may or may not have a 5' cap.

Nucleic acids encoding antigens described herein may be part of a vector *i.e.* part of a nucleic acid construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector. Preferred vectors are plasmids. A "host cell" includes an individual cell or cell culture which can be or has been a recipient of exogenous nucleic acid. Host cells include progeny of a single host cell, and the progeny may not necessarily be completely identical (in morphology or in total DNA complement) to the original parent cell due to natural, accidental, or deliberate mutation and/or change. Host cells include cells transfected or infected *in vivo* or *in vitro* with nucleic acid disclosed herein.

The term "complement" or "complementary" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) e.g. to complement pyrimidines (C or T).

Nucleic acids encoding antigens described herein can be used, for example: to produce polypeptides; as hybridization probes for the detection of nucleic acid in biological samples; to generate additional copies of the nucleic acids; to generate ribozymes or antisense oligonucleotides; as single-stranded DNA primers or probes; or as triple-strand forming oligonucleotides.

The disclosure provides a process for producing nucleic acid encoding antigens described herein, wherein the nucleic acid is synthesised in part or in whole using chemical means.

The disclosure provides vectors comprising nucleotide sequences encoding antigens described herein (*e.g.* cloning or expression vectors) and host cells transformed with such vectors.

For certain embodiments of the disclosure, nucleic acids are preferably at least 7 nucleotides in length (*e.g.* 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 nucleotides or longer).

For certain embodiments of the disclosure, nucleic acids are preferably at most 500 nucleotides in length (*e.g.* 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 nucleotides or shorter).

### Immunogenic compositions and medicaments

Immunogenic compositions of the invention may be useful as vaccines. Vaccines may either be prophylactic (*i.e.* to prevent infection) or therapeutic (*i.e.* to treat infection), but will typically be prophylactic.

Compositions may thus be pharmaceutically acceptable. They will usually include components in addition to the antigens *e.g*. they typically include one or more pharmaceutical carrier(s) and/or excipient(s). A thorough discussion of such components is available in reference 118.

Compositions will generally be administered to a mammal in aqueous form. Prior to administration, however, the composition may have been in a non-aqueous form. For instance, although some vaccines are manufactured in aqueous form, then filled and distributed and administered also in aqueous form, other vaccines are lyophilised during manufacture and are reconstituted into an aqueous form at the time of use. Thus a composition of the invention may be dried, such as a lyophilised formulation.

The composition may include preservatives such as thiomersal or 2-phenoxyethanol. It is preferred, however, that the vaccine should be substantially free from (*i.e.* less than 5µg/ml) mercurial material *e.g*. thiomersal-free. Vaccines containing no mercury are more preferred. Preservative-free vaccines are particularly preferred.

To improve thermal stability, a composition may include a temperature protective agent. Further details of such agents are provided below.

To control tonicity, it is preferred to include a physiological salt, such as a sodium salt. Sodium chloride (NaCl) is preferred, which may be present at between 1 and 20 mg/ml *e.g*. about 10±2mg/ml NaCl. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8.

The composition is preferably sterile. The composition is preferably non-pyrogenic *e.g*. containing <1 EU (endotoxin unit, a standard measure) per dose, and preferably <0.1 EU per dose. The composition is preferably gluten free.

The composition may include material for a single immunisation, or may include material for multiple immunisations (*i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Human vaccines are typically administered in a dosage volume of about 0.5ml, although a half dose (*i.e.* about 0.25ml) may be administered to children.

Immunogenic compositions of the invention can also comprise one or more immunoregulatory agents. Preferably, one or more of the immunoregulatory agents include one or more adjuvants. The adjuvants may include a TH1 adjuvant and/or a TH2 adjuvant, further discussed below.

Adjuvants which may be used in compositions of the invention include, but are not limited to:
- mineral salts, such as aluminium salts and calcium salts, including hydroxides (*e.g*. oxyhydroxides), phosphates (*e.g.* hydroxyphosphates, orthophosphates) and sulphates, *etc.* [*e.g.* see chapters 8 & 9 of ref. 23];
- oil-in-water emulsions, such as squalene-water emulsions, including MF59 (5% Squalene, 0.5% Tween 80, and 0.5% Span 85, formulated into submicron particles using a microfluidizer) (Chapter 10 of ref. 23; see also refs. 24-26, and chapter 12 of ref. 27], complete Freund's adjuvant (CFA) and incomplete Freund's adjuvant (IFA);
- saponin formulations [chapter 22 of ref. 23], such as QS21 [28] and ISCOMs [chapter 23 of ref. 23];
- virosomes and virus-like particles (VLPs) [29-35];
- bacterial or microbial derivatives, such as non-toxic derivatives of enterobacterial lipopolysaccharide (LPS), Lipid A derivatives [36, 37], immunostimulatory oligonucleotides [38-43], such as IC-31™ [44] (deoxynucleotide comprising 26-mer sequence 5'-(IC)₁₃-3' (SEQ ID NO: 112) and polycationic polymer peptide comprising 11-mer amino acid sequence KLKLLLLLKLK (SEQ ID NO: 113) and ADP-ribosylating toxins and detoxified derivatives thereof [45 - 54];
- human immunomodulators, including cytokines, such as interleukins (*e.g*. IL-1, IL-2, IL-4, IL-5, IL-6, IL-7, IL-12 [55, 56], interferons (*e.g*. interferon-γ), macrophage colony stimulating factor, and tumor necrosis factor;
- bioadhesives and mucoadhesives, such as chitosan and derivatives thereof, esterified hyaluronic acid microspheres [57] or mucoadhesives, such as cross-linked derivatives of poly(acrylic acid), polyvinyl alcohol, polyvinyl pyrollidone, polysaccharides and carboxymethylcellulose [58];
- microparticles (*i.e.* a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc.*);
- liposomes [Chapters 13 & 14 of ref. 23, ref. 59-61];
- polyoxyethylene ethers and polyoxyethylene esters [62];
- PCPP formulations [63 and 64];
- muramyl peptides, including N-acetyl-muramyl-L-threonyl-D-isoglutamine (thr-MDP), N-acetyl-normuramyl-1-alanyl-d-isoglutamine (nor-MDP), and N-acetylmuramyl-1-alanyl-d-isoglutaminyl-1-alanine-2-(1'-2'-dipalmitoyl-*sn*-glycero-3-hydroxyphosphoryloxy)-ethylamine MTP-PE); and
- imidazoquinolone compounds, including Imiquamod and its homologues (*e.g*. "Resiquimod 3M") [65 and 66].

Immunogenic compositions and vaccines of the invention may also comprise combinations of aspects of one or more of the adjuvants identified above. For example, the following adjuvant compositions may be used in the invention: (1) a saponin and an oil-in-water emulsion [67]; (2) a saponin (*e.g.* QS21) + a non-toxic LPS derivative (*e.g.* 3dMPL) [68]; (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) [69]; (5) combinations of 3dMPL with, for example, QS21 and/or oil-in-water emulsions [70]; (6) SAF, containing 10% squalne, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 23.

The use of an aluminium hydroxide and/or aluminium phosphate adjuvant is particularly preferred, and antigens are generally adsorbed to these salts. Calcium phosphate is another preferred adjuvant. Other preferred adjuvant combinations include combinations of Th1 and Th2 adjuvants such as CpG & alum or resiquimod & alum. A combination of aluminium phosphate and 3dMPL may be used (this has been reported as effective in pneumococcal immunisation [71]). The use of an MF59 adjuvant is preferred, in particular in case of IM (intramuscular) or IP (Intraperitoneal) immunization.

The compositions of the invention may elicit both a cell mediated immune response as well as a humoral immune response. This immune response will preferably induce long lasting (*e.g.* neutralising) antibodies and a cell mediated immunity that can quickly respond upon exposure to NTHI.

Two types of T cells, CD4 and CD8 cells, are generally thought necessary to initiate and/or enhance cell mediated immunity and humoral immunity. CD8 T cells can express a CD8 co-receptor and are commonly referred to as Cytotoxic T lymphocytes (CTLs). CD8 T cells are able to recognized or interact with antigens displayed on MHC Class I molecules.

CD4 T cells can express a CD4 co-receptor and are commonly referred to as T helper cells. CD4 T cells are able to recognize antigenic peptides bound to MHC class II molecules. Upon interaction with a MHC class II molecule, the CD4 cells can secrete factors such as cytokines. These secreted cytokines can activate B cells, cytotoxic T cells, macrophages, and other cells that participate in an immune response. Helper T cells or CD4+ cells can be further divided into two functionally distinct subsets: TH1 phenotype and TH2 phenotypes which differ in their cytokine and effector function.

Activated TH1 cells enhance cellular immunity (including an increase in antigen-specific CTL production) and are therefore of particular value in responding to intracellular infections. Activated TH1 cells may secrete one or more of IL-2, IFN-γ, and TNF-β. A TH1 immune response may result in local inflammatory reactions by activating macrophages, NK (natural killer) cells, and CD8 cytotoxic T cells (CTLs). A TH1 immune response may also act to expand the immune response by stimulating growth of B and T cells with IL-12. TH1 stimulated B cells may secrete IgG2a.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

An enhanced immune response may include one or more of an enhanced TH1 immune response and a TH2 immune response.

A TH1 immune response may include one or more of an increase in CTLs, an increase in one or more of the cytokines associated with a TH1 immune response (such as IL-2, IFN-γ, and TNF-β), an increase in activated macrophages, an increase in NK activity, or an increase in the production of IgG2a. Preferably, the enhanced TH1 immune response will include an increase in IgG2a production.

A TH1 immune response may be elicited using a TH1 adjuvant. A TH1 adjuvant will generally elicit increased levels of IgG2a production relative to immunization of the antigen without adjuvant. TH1 adjuvants suitable for use in the compositions of the invention may include for example saponin formulations, virosomes and virus like particles, non-toxic derivatives of enterobacterial lipopolysaccharide (LPS), immunostimulatory oligonucleotides. Immunostimulatory oligonucleotides, such as oligonucleotides containing a CpG motif, are preferred TH1 adjuvants for use in the compositions of the invention.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune response will include an increase in IgG1 production.

A TH2 immune response may be elicited using a TH2 adjuvant. A TH2 adjuvant will generally elicit increased levels of IgG1 production relative to immunization of the antigen without adjuvant. TH2 adjuvants suitable for use in the compositions of the invention include, for example, mineral containing compositions, oil-emulsions, and ADP-ribosylating toxins and detoxified derivatives thereof. Mineral containing compositions, such as aluminium salts are preferred TH2 adjuvants for use in the compositions of the invention.

Preferably, the compositions of the invention include a composition comprising a combination of a TH1 adjuvant and a TH2 adjuvant. Preferably, such a composition elicits an enhanced TH1 and an enhanced TH2 response, *i.e*., an increase in the production of both IgG1 and IgG2a production relative to immunization without an adjuvant. Still more preferably, the composition comprising a combination of a TH1 and a TH2 adjuvant elicits an increased TH1 and/or an increased TH2 immune response relative to immunization with a single adjuvant (*i.e.*, relative to immunization with a TH1 adjuvant alone or immunization with a TH2 adjuvant alone).

The immune response may be one or both of a TH1 immune response and a TH2 response. Preferably, immune response provides for one or both of an enhanced TH1 response and an enhanced TH2 response.

The enhanced immune response may be one or both of a systemic and a mucosal immune response. Preferably, the immune response provides for one or both of an enhanced systemic and an enhanced mucosal immune response. Preferably the mucosal immune response is a TH2 immune response. Preferably, the mucosal immune response includes an increase in the production of IgA.

*H. influenzae* infections can affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilised composition or a spray-freeze dried composition). The composition may be prepared for topical administration *e.g*. as an ointment, cream or powder. The composition may be prepared for oral administration *e.g*. as a tablet or capsule, as a spray, or as a syrup (optionally flavoured). The composition may be prepared for pulmonary administration *e.g*. as an inhaler, using a fine powder or a spray. The composition may be prepared as a suppository or pessary. The composition may be prepared for nasal, aural or ocular administration *e.g.* as drops. The composition may be in kit form, designed such that a combined composition is reconstituted just prior to administration to a patient. Such kits may comprise one or more antigens in liquid form and one or more lyophilised antigens.

Where a composition is to be prepared extemporaneously prior to use (*e.g*. where a component is presented in lyophilised form) and is presented as a kit, the kit may comprise two vials, or it may comprise one ready-filled syringe and one vial, with the contents of the syringe being used to reactivate the contents of the vial prior to injection.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Where more than one antigen is included in a composition then two antigens may be present at the same dose as each other or at different doses.

As mentioned above, a composition may include a temperature protective agent, and this component may be particularly useful in adjuvanted compositions (particularly those containing a mineral adjuvant, such as an aluminium salt). As described in reference 72, a liquid temperature protective agent may be added to an aqueous vaccine composition to lower its freezing point *e.g.* to reduce the freezing point to below 0°C. Thus the composition can be stored below 0°C, but above its freezing point, to inhibit thermal breakdown. The temperature protective agent also permits freezing of the composition while protecting mineral salt adjuvants against agglomeration or sedimentation after freezing and thawing, and may also protect the composition at elevated temperatures *e.g*. above 40°C. A starting aqueous vaccine and the liquid temperature protective agent may be mixed such that the liquid temperature protective agent forms from 1-80% by volume of the final mixture. Suitable temperature protective agents should be safe for human administration, readily miscible/soluble in water, and should not damage other components (*e.g*. antigen and adjuvant) in the composition. Examples include glycerin, propylene glycol, and/or polyethylene glycol (PEG). Suitable PEGs may have an average molecular weight ranging from 200-20,000 Da. In a preferred embodiment, the polyethylene glycol can have an average molecular weight of about 300 Da ('PEG-300').

Compositions of the invention may be formed by mixing (i) an aqueous composition comprising two or more (*e.g. 1, 2, 3*, *4*) antigen(s) of the antigen combinations of the invention with (ii) a temperature protective agent. The mixture may then be stored e.g. below 0°C, from 0-20°C, from 20-35°C, from 35-55°C, or higher. It may be stored in liquid or frozen form. The mixture may be lyophilised. The composition may alternatively be formed by mixing (i) a dried composition comprising two or more (*e.g. 1,* 2, *3*, *4*) antigen(s) of the antigen combinations of the invention, with (ii) a liquid composition comprising the temperature protective agent. Thus component (ii) can be used to reconstitute component (i).

### Methods of treatment, and administration of the vaccine

The compositions of the invention may be used in a method for raising an immune response in a mammal comprising the step of administering an effective amount of a composition of the invention, or one or more steps of administering at least one or more (*e.g.* 1, 2, 3, 4, 5, 6, 7, 8, 9 or 10) antigens of the invention. The immune response is protective and preferably involves antibodies and/or cell-mediated immunity. The method may raise a booster response.

The disclosure also provides at least one or more antigens of the invention for combined use as a medicament *e.g*. for use in raising an immune response in a mammal.

The disclosure also provides the use of at least one or more antigens of the invention in the manufacture of a medicament for raising an immune response in a mammal.

In the methods and uses disclosed herein, at least one or more (*e.g. 1, 2*, *3*, *4*) antigens of the invention may be administered simultaneously, separately or sequentially.

By raising an immune response in the mammal by these uses and methods, the mammal can be protected against *H. influenzae* infection. The invention is effective against *H. influenzae* of various different serotypes, but can be particularly useful in protecting against disease resulting from infection by non-typeable *H. influenzae* (NTHI). An infection may be associated with a disease or condition selected from, for instance, otitis media (including acute otitis media), bronchitis, conjunctivitis, sinusitis, a urinary tract infection, pneumonia, bacteremia, septic arthritis, epiglottitis, pneumonia, empyema, pericarditis, cellulitis, osteomyelitis, lower respiratory tract infection or meningitis. The invention is particularly useful for treating or preventing inflammation of the middle ear or for treating or preventing COPD diseases, by eliciting an immune response that prevents bacteria from moving from the throat to the middle ear via the eustachian tube, where the middle ear is then colonised.

The disclosure also provides a kit comprising a first component and a second component wherein neither the first component nor the second component is a composition of disclosed herein, but wherein the first component and the second component can be combined to provide a composition disclosed herein. The kit may further include a third component comprising one or more of the following: instructions, syringe or other delivery device, adjuvant, or pharmaceutically acceptable formulating solution.

The disclosure also provides a delivery device pre-filled with an immunogenic composition of the invention.

The mammal is preferably a human, *e.g*. human patient. Where the vaccine is for prophylactic use, the human is preferably a child (*e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.* A mammal (*e.g.* human, *e.g.* a patient) may either be at risk from the disease themselves or may be a pregnant female, *e.g*. woman ('maternal immunisation').

One way of checking efficacy of therapeutic treatment involves monitoring *H. influenzae* infection after administration of the compositions of the invention. One way of checking efficacy of prophylactic treatment involves monitoring immune responses, systemically (such as monitoring the level of IgG1 and IgG2a production) and/or mucosally (such as monitoring the level of IgA production), against the antigens in the compositions of the invention after administration of the composition. Immunogenicity of compositions of the invention can be determined by administering them to test subjects (*e.g*. children 12-16 months age, or animal models such as a chinchilla model

) and then determining standard parameters including ELISA titres (GMT) of IgG. These immune responses will generally be determined around 4 weeks after administration of the composition, and compared to values determined before administration of the composition. Where more than one dose of the composition is administered, more than one post-administration determination may be made. Typically, antigen-specific serum antibody responses are determined post-immunisation but pre-challenge whereas antigen-specific mucosal antibody responses are determined post-immunisation and post-challenge.

Another way of assessing the immunogenicity of the compositions of the present invention is to express the proteins recombinantly for screening patient sera or mucosal secretions by immunoblot and/or microarrays. A positive reaction between the protein and the patient sample indicates that the patient has mounted an immune response to the protein in question. This method may also be used to identify immunodominant antigens and/or epitopes within antigens.

The efficacy of vaccine compositions can also be determined *in vivo* by immunization studies in animal models of *H. influenzae* infection, *e.g.*, guinea pigs Chinchillas, or mice, with the vaccine compositions. One such model is described in reference 74.

Other useful animal model to be used to determine *in vivo* the efficacy of vaccine compositions of the invention is described in reference 75.

Compositions of the invention will generally be administered directly to a patient. Direct delivery may be accomplished by parenteral injection (*e.g*. subcutaneously, intraperitoneally, intravenously, intramuscularly, or to the interstitial space of a tissue), or mucosal, such as by rectal, oral, (*e.g.* tablet, spray), vaginal, topical, transdermal or transcutaneous, intranasal, ocular, aural, pulmonary or other mucosal administration.

The composition of the invention may be used to elicit systemic and/or mucosal immunity, preferably to elicit an enhanced systemic and/or mucosal immunity.

Preferably the enhanced systemic and/or mucosal immunity is reflected in an enhanced TH1 and/or TH2 immune response. Preferably, the enhanced immune response includes an increase in the production of IgG1 and/or IgG2a and/or IgA.

Dosage can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc*.)*.*

Vaccines prepared using the compositions of the invention may be used to treat both children and adults. Thus a human patient may be less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, or immunodeficient patients. The vaccines are not suitable solely for these groups, however, and may be used more generally in a population.

Vaccines produced using the compositions of the invention may be administered to patients at substantially the same time as (*e.g.* during the same medical consultation or visit to a healthcare professional or vaccination centre) other vaccines e.g. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H. influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, *etc.*

### Mucosal immunisation

The disclosure provides the antigens, antigen combinations, and compositions disclosed herein for mucosal immunisation. *E.g.*, the disclosure provides an immunogenic composition comprising (i) a polypeptide antigen combination of the invention, and (ii) a bacterial ADP-ribosylating toxin and or detoxified derivative thereof. The disclosure also provides a method for raising an immune response in a mammal comprising the step of administering an effective amount of such an immunogenic composition to the mammal. The composition is preferably administered via mucosa (to a mucosal surface) *e.g.* it may be administered intranasal.

The toxin of component (ii) may be, for example, derived from *E.coli* heat labile enterotoxin ("LT"). The derivative may have a detoxifying mutation in its A subunit *e.g*. it may be LT-K63 or LT-R72. In particular it may be LT-K63. In other embodiments, it is not LT-K63.

Intranasal administration of antigens or compositions disclosed herein and a LT-K63 adjuvant is preferred. This may decrease the *H. influenzae* bacterial load in the nasopharynx, lungs and blood, and increase survival rate of infected mammals.

### Further antigenic components of compositions of the invention

The invention also provides compositions further comprising at least one further non-typeable *H. influenzae* antigen.

The invention also provides compositions further comprising at least one antigen that is not a non-typeable *H. influenzae* antigen.

In particular, the invention also provides a composition comprising one or more polypeptides of the compositions of the invention and one or more of the following further antigens:
- an antigen from *N.meningitidis* serogroup A, B, C, W135 and/or Y.
- a saccharide or polypeptide antigen from *Streptococcus pneumoniae* [*e.g.* 76, 77, 78].
- an antigen from hepatitis A virus, such as inactivated virus [*e.g*. 79, 80].
- an antigen from hepatitis B virus, such as the surface and/or core antigens [*e.g*. 80, 81].
- a diphtheria antigen, such as a diphtheria toxoid [*e.g*. chapter 3 of ref. 82] or the CRM₁₉₇ mutant [*e.g.* 83].
- a tetanus antigen, such as a tetanus toxoid [*e.g*. chapter 4 of ref. 82].
- an antigen from *Bordetella pertussis*, such as pertussis holotoxin (PT) and filamentous haemagglutinin (FHA) from *B.pertussis*, optionally also in combination with pertactin and/or agglutinogens 2 and 3 [*e.g.* refs. 84 & 85].
- a whole cellular pertussis antigen
- a saccharide antigen from *Haemophilus influenzae* B [*e.g.* 86].
- polio antigen(s) [*e.g*. 87, 88] such as IPV.
- measles, mumps and/or rubella antigens [*e.g*. chapters 9, 10 & 11 of ref. 82].
- influenza antigen(s) [*e.g*. chapter 19 of ref. 82], such as the haemagglutinin and/or neuraminidase surface proteins.
- an antigen from *Moraxella catarrhalis* [*e.g.* 89].
- an protein antigen from *Streptococcus agalactiae* (group B streptococcus) [*e.g.* 90, 91].
- a saccharide antigen from *Streptococcus agalactiae* (group B streptococcus).
- an antigen from *Streptococcus pyogenes* (group A streptococcus) [*e.g.* 91, 92, 93].
- an antigen from *Staphylococcus aureus* [*e.g.* 94].
- an antigen from Respiratory Syncytial Virus, e.g. a recombinant protein F [e.g. 142]
- a vaccine composition comprising diphtheria (D), tetanus (T), pertussis (acellular, component) (Pa), hepatitis B (rDNA) (HBV), poliomyelitis (inactivated) (IPV) and *Haemophilus influenzae* type b (Hib) conjugate vaccine (adsorbed), e.g. Infanrix-hexa

The composition may comprise one or more of these further antigens. Combinations with a RSV vaccine and/or with a DTPa-containing vaccine are of particular interest.

Toxic protein antigens may be detoxified where necessary (*e.g*. detoxification of pertussis toxin by chemical and/or genetic means [85]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include diphtheria toxin, tetanus toxin, the *N. meningitidis* outer membrane protein [95], synthetic peptides [96,97], heat shock proteins [98,99], pertussis proteins [100,101], protein D from *H. influenzae* [102], cytokines [103], lymphokines [103], streptococcal proteins, hormones [103], growth factors [103], toxin A or B from *C.difficile* [104], iron-uptake proteins [105], *etc.* A preferred carrier protein is the CRM197 mutant of diphtheria toxin [106].

Antigens in the composition will typically be present at a concentration of at least 1µg/ml each. In general, the concentration of any given antigen will be sufficient to elicit an immune response against that antigen.

Antigens are preferably adsorbed to an aluminium salt.

### Antibodies

Antibodies against antigens disclosed herein can be used for passive immunisation [107]. Disclosed herein are antibodies specific to antigens disclosed herein for use in therapy. These antibodies may be used singly or in combination. Disclosed herein are immunogenic and pharmaceutical compositions comprising such antibodies.

The antibodies can be used in medicine and in therapy *e.g.* for passive immunisation against NTHI, or for clearing a NTHI infection. Disclosed herein is use of such antibodies in the manufacture of a medicament. Disclosed herein is a method for treating a mammal comprising the step of administering an effective amount of an antibody of the invention. As described above for immunogenic compositions, these methods and uses allow a mammal to be protected against NTHI infections. In particular, antibodies disclosed herein may be used in methods of treating or preventing infections by NTHI, comprising the step of administering to the mammal an effective amount of an antibody as described herein, or a composition comprising such an antibody.

The term "antibody" includes intact immunoglobulin molecules (like palivizumab), as well as fragments thereof which are capable of binding a NTHI antigen. These include hybrid (chimeric) antibody molecules [108, 109]; F(ab')2 and F(ab) fragments and Fv molecules; non-covalent heterodimers [110, 111]; single-chain Fv molecules (sFv) [112]; dimeric and trimeric antibody fragment constructs; minibodies [113, 114]; humanized antibody molecules [115-117]; and any functional fragments obtained from such molecules, as well as antibodies obtained through nonconventional processes such as phage display. Preferably, the antibodies are monoclonal antibodies. Methods of obtaining monoclonal antibodies are well known in the art. Humanised or fully-human antibodies are preferred. Antibodies and antibody combinations of the disclosed herein may be purified or isolated.

### General

The practice of the present invention will employ, unless otherwise indicated, conventional methods of chemistry, biochemistry, molecular biology, immunology and pharmacology, within the skill of the art. Such techniques are explained fully in the literature. See, *e.g*., references 118-125, *etc.*

Where the disclosure concerns an "epitope", this epitope may be a B-cell epitope and/or a T-cell epitope. Such epitopes can be identified empirically (*e.g*. using PEPSCAN [126,127] or similar methods), or they can be predicted (e.g. using the Jameson-Wolf antigenic index [128], matrix-based approaches [129], MAPITOPE [130], TEPITOPE [131,132], neural networks [133], OptiMer & EpiMer [134, 135], ADEPT [136], Tsites [137], hydrophilicity [138], antigenic index [139] or the methods disclosed in references 140-144, *etc*.). Epitopes are the parts of an antigen that are recognised by and bind to the antigen binding sites of antibodies or T-cell receptors, and they may also be referred to as "antigenic determinants".

Where an antigen "domain" is omitted, this may involve omission of a signal peptide, of a cytoplasmic domain, of a transmembrane domain, of an extracellular domain, *etc*.

The term "comprising" encompasses "including" as well as "consisting" e.g. a composition "comprising" X may consist exclusively of X or may include something additional e.g. X + Y.

The term "about" in relation to a numerical value *x* is optional and means, for example, *x*±10%.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of ref. 22. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 145.

### BRIEF DESCRIPTION OF DRAWINGS

Figure 1 shows a mini-induction confirming strong expression of the antigens in BL21 (DE3)T1^{r} cells. (a): (LMWM: molecular weight standard markers)

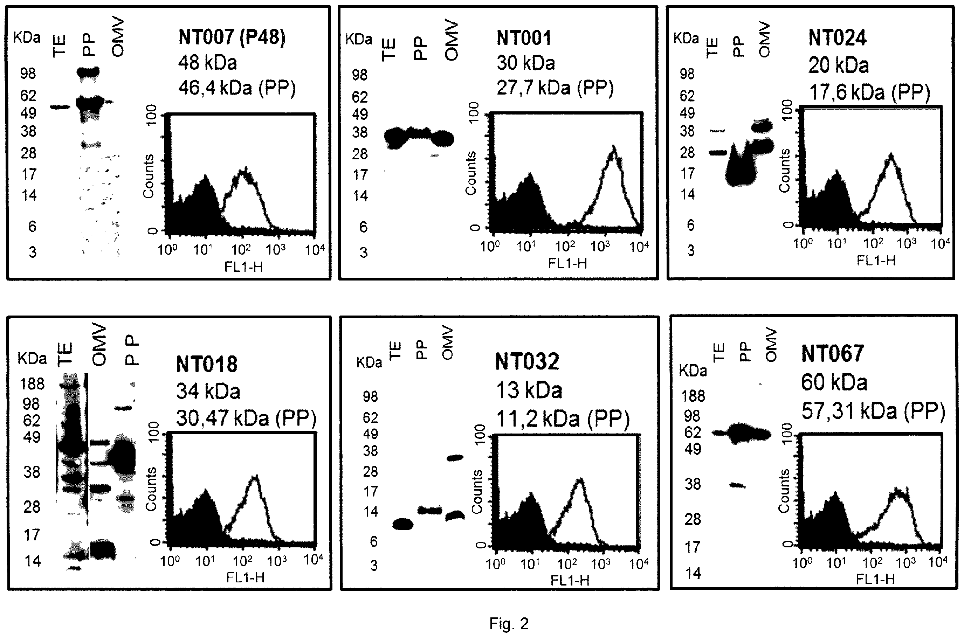

Figure 2 shows various results for NT001, NT007, NT018, NT024, NT032 and NT067. Similar expression results were obtained with the other preferred antigens, such as NT052, NT004, NT014, NT016 or NT022. Each panel shows western blot and FACS data. The western blots were performed using mouse sera, and lanes show reactivity with total bacterial extracts ("TE"), with vesicles prepared from NTHI outer membranes ("OMV"), or with purified recombination protein ("PP"). The FACS analyses follow incubation of inactivated bacteria with sera from mice immunized with various antigen compositions using only alum as negative control; pre-immune serum negative controls are shown as solid areas, and surface expression signal obtained with sample serum is shown as a single line.
Figure 3 shows the layout on a 96 well plate of a serum bactericidal assay to verify the capacity of antisera against antigens disclosed hereinto kill NTHI.

### EXAMPLES

### Overview

Antigens list all of them which were identified as conserved in a comparative analysis performed by the inventors of at least 86 different NTHI strains, were cloned and expressed. The proteins were purified and used to immunize mice. Antisera from the immunized mice were used to verify surface localization and protective capability of the proteins used in immunization (Table III and/or Table IV). The results show that immunization NT052, NT024, NT032, NT001, NT067, NT004, NT014, NT022, NT016 is highly protective against NTHI and they showed higher or at least comparable bacterial killing activity SBA (Serum bactericidal assay) titers even compared with the "second antigen group".

### Strains and variants

Inventors found that genes encoding NT022, NT016, NT014, NT018, NT024, NT032, NT067 and NT001 were present and conserved in all 86 genome sequences analysed.

The encoded NT018 sequences were 95-100% identical across the panel composed by the 15 complete genomes and the 32 strains from the Finnish otitis collection. The encoded NT024 sequences were 90-100% identical in the panel composed by the 15 complete genomes and the 32 strains from the Finnish otitis collection.

The encoded NT032 sequences were 95-100% identical in the panel composed by the 15 complete genomes and the 32 strains from the Finnish otitis collection; the encoded NT067 sequences were 95-100% identical in the panel composed by the 15 complete genomes and the 32 strains from the Finnish otitis collection. The encoded NT001 sequences were 95-100% identical in the panel composed by the 15 complete genomes and the 32 strains from the Finnish otitis collection.

Conservation in the encoded amino acid sequences are shown in Table I.

**Table I: antigen conservation (% identity) amongst Haemophilus genomes and strains**

| **Antigen** | NT018 | NT024 | NT032 | NT067 | NT001 |
|---|---|---|---|---|---|
| % | 95-100 | 90-100 | 95-100 | 95-100 | 95-100 |

For expression purposes, antigens belonging to the "first antigen group" and/or "second antigen group" were cloned from either strain Fi176 which is one strain isolated from a Finnish collection of strains obtained from patients with otitis media or from strain R2846 [146]. Most of the antigen selected and further tested in animal model are also found to be well conserved amongst strains, e.g. NT016, NT067, NT022, NT014.

In some cases mutations have been introduced into the wild-type sequences. These mutations are underlined in the sequence listing for NT018, NT067, NT001, NT016, NT002, NT026, NT009, NT015, NT023 and NT066 (see SEQ ID NOs: 49, 52, 54, 55, 57-59, 64, 65 & 67).

### Cloning and expression of NTHI recombinant proteins

Cloning and expression of antigens can be performed by standard methods [121].

ORFs for antigens from NTHI strain Fi176 or R2846 were PCR-amplified using specific oligonucleotides and NTHI chromosomal DNA as template. Resulting PCR products were cloned in pET15b (Novagen) using the PIPE method [147], consisting in the PCR amplification of the cloning vector (V-PCR) and in the PCR amplification of the insert (I-PCR). Then, 1 µl of V-PCR and 1 µl of I-PCR are mixed and transformed in chemically competent HK100 cells [148]. I-PCR reactions were set up containing 1 µM each of the forward and reverse primers, 1x Cloned Pfu DNA Polymerase Reaction Buffer, 2.5 units of Pfu Turbo DNA polymerase (Stratagene), 200 µM of each dNTP (Invitrogen) and 50 ng of genomic DNA template. The reactions were conducted as follows: initial denaturation for 2 min at 95 °C, then 25 cycles of 95 °C for 30 s, 55 °C for 45 s, and 68 °C for 3 min followed by a final cool down to 4 °C. V-PCR reactions were identical to the I-PCR reactions but the steps at 68 °C were lasting 14 min and 2 ng of pET15b plasmid were used as DNA template. Correct transformants where selected by PCR screening and DNA plasmid sequencing of the vector-insert junctions. The correct plasmid were then prepared from selected HK100 clones and used to transform BL21(DE3)T1^{r} cells (Sigma) in order to allow protein expression.

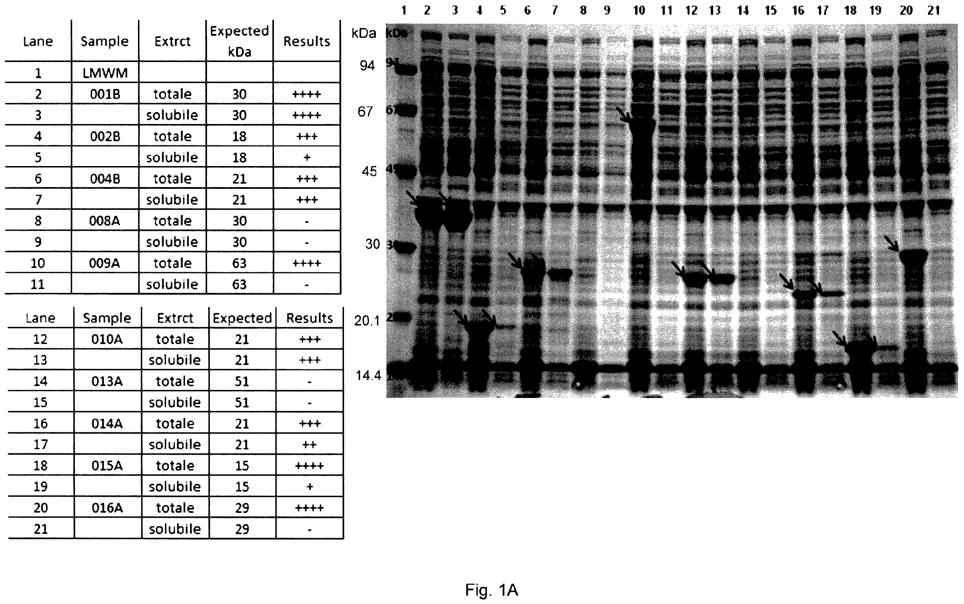

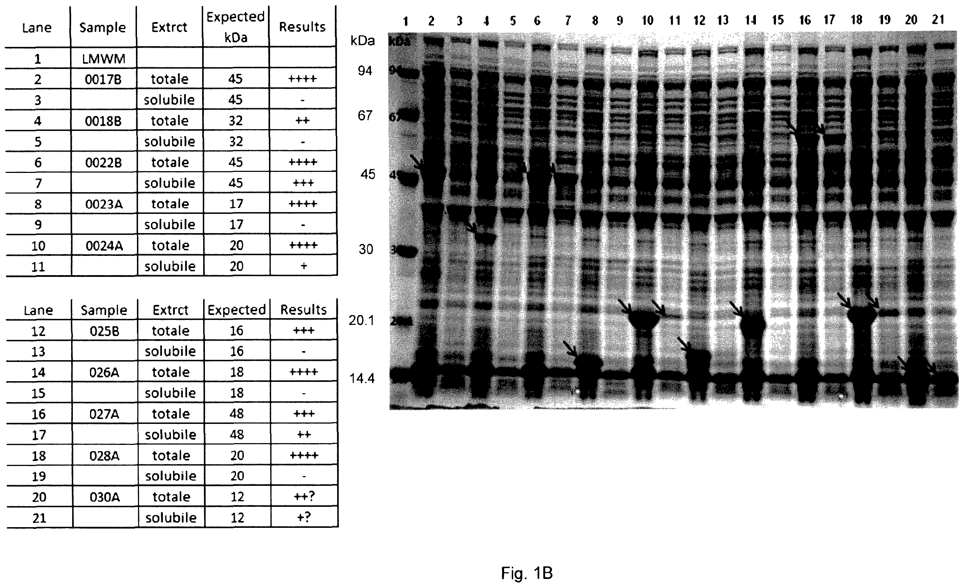

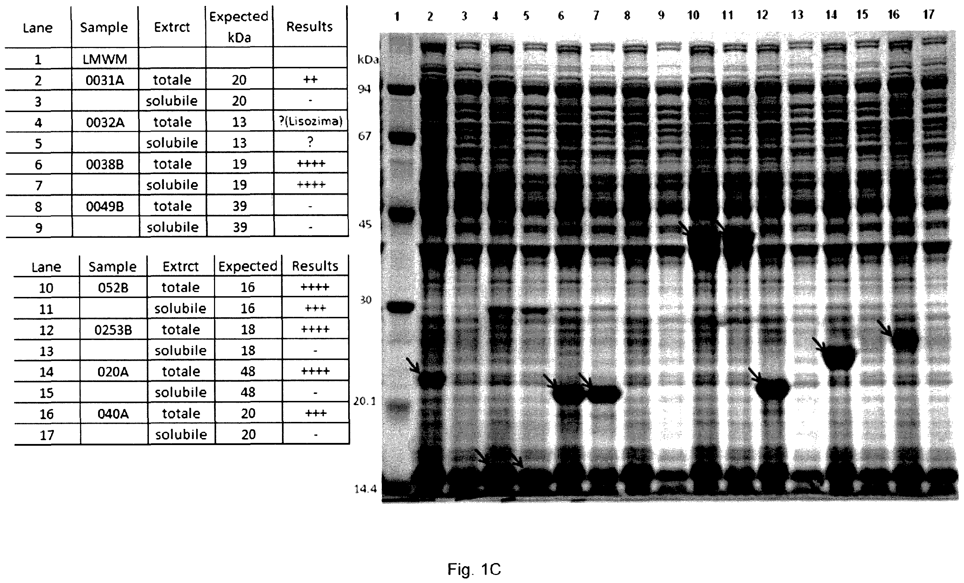

To express cloned proteins, BL21(DE3)T1^{r} clones containing pET15b constructs were grown in LB medium containing 100 µg/ml Ampicilin at 37 °C until OD6₀₀= 0.5. Protein expression was then induced by adding 1 mM IPTG and growing at the same temperature for additional 3 hrs. Conventional protein extractions and SDS-Page were performed to check protein expression. Figure 1 shows a mini-induction confirming good expression of the antigens.

### Protein purification

Proteins were purified by the following general procedure: BL21(DE3)T1 wet biomass is suspended in lysis buffer and clarified by centrifugation. For purification of soluble protein (), supernatants after lysis are applied on His Multitrap HP 50µl NiSepharose High Performance 96 well plates. For insoluble protein (HtrA, PE and P48), pellets containing the unsoluble fraction after lysis are solubilised with 6M Guanidine-HCl and re-centrifuged, and the supernatants applied to His Multitrap HP 50ml NiSepharose High Performance 96 well plates.

Flow-through is collected and all wells washed with buffer containing 20 mM imidazole. His fusion proteins are then eluted with 250mM imidazole. The procedure is performed using a vacuum system. Purified antigens are used in the immunisation schemes described herein.

The following protocol was followed:
1) Resuspend BL21(DE3)T1 pellet (1g) in 1,5ml B-PER™ (PIERCE) buffer, add 15µl of lysozyme, 7.5µl DNAse and 3 µl of MgCl₂ 1M
2) Incubate for 30 min for lysis
3) Centrifuge at 20000 rpm at 4°C for 30 minute; for purification of any insoluble protein, solubilise pellets containing the unsoluble fraction after lysis with 6M Guanidine-HCl and re-centrifuge
4) Recover supernatant and filter (pore of 0.8µm).
5) Use His Multitrap HP 50µl NiSepharose High Performance 96wells, connected to a vacuum system
   Buffer A: 50mM NaPPi, 300mM NaCl, pH8
   Buffer B: 50mM NaPPi, 300mM NaCl, 250mM Imidazole, pH8
   Buffer C: 50mM NaPPi, 300mM NaCl, 20mM Imidazole, pH8
      1st Step: remove ethanol from the plate.
      2nd Step: wash the plate with 400 µl of milliQ H2O.
      3rd Step: equilibrate the plate with 400 µl of Buffer A
      4th Step: load 600µl of starting material for each protein in one of the 12 columns. If the volume is larger, repeat until all the material is fully loaded.
         Recover the flow through.
      5th Step: Wash Step: 4 washes with 400 µl of Buffer C. Discard the flow through.
      6th Step: Elution: 2 x 300µl Buffer B (2 elution steps).

Activate vacuum 15 minutes after adding the buffer.

1µl of total extract, 1µl of starting material, 1 µl of flow through and 10µl of elution volume (for each protein) are analysed by SDS-PAGE.

For insoluble protein, buffer B is replaced by 10mM tris, 50mM Na₂HPO₄, 8M urea, 250mM imidazole, 40% glycerol.

### LAL Test

The LAL test is a test that measures the endotoxin concentration in a vaccine sample using the endosafe®-PTS™ Charles River technology.

### Test Technology

The PTS utilizes LAL kinetic chromogenic methodology to measure color intensity directly related to the endotoxin concentration in a sample. Each cartridge contains precise amounts of licensed LAL reagent, chromogenic substrate, and control standard endotoxin (CSE). The cartridges are manufactured according to rigid quality control procedures to ensure test accuracy and product stability.

**Table II: Purification of preferred antigens**

| | | | | | | | | | |
|---|---|---|---|---|---|---|---|---|---|
| **Internal** | **Annotation** | **kDa** | **kDa** | **Soluble** | | **Purity %** | | | **LAL** |

| **ID** | | **(expected)** | **(SE estimated)** | | **densitometry** | **RP-HPLC** | **SE-UPLC** | **SE-HPLC** | **Test EU/µg** |
|---|---|---|---|---|---|---|---|---|---|
| **nt001** | NTHI0877 | 30 | 36 | yes | 97 | 84 | | monomer | 0,47 |
| **nt018** | NTHI0915 | 34 | 46 | yes | 80 | 88 | | monomer | 0,18 |
| **nt024** | NTHI1416 | 20 | 20 | yes | 81 | 85 | 97 | monomer | 3,77 |
| **nt032** | NTHI2017 | 13 | 16 | yes | 91 | 76 | | monomer | 0,82 |
| **nt067** | NTHI1292 | 60 | 50 | yes | 88 | 78 | | monomer | 0,06 |
| **nt052** | CGSHiGG_00130 | 34 | 46 | yes | 88 | 88 | | monomer | 0,18 |
| **nt004** | CGSHiGG_08215 | 20 | 34 | yes | 95 | | 95 | monomer | 0,09 |
| **nt014** | HI1658 | 20 | 17 | yes | 89 | | 87 | monomer | 0,10 |
| **nt022** | NTHI0830 | 43 | 77 | yes | 93 | | 93 | monomer | 0,10 |
| **nt016** | NTHI0266 | 29 | 30 | yes | 98% | | | monomer | 0.13 |

### Immunisation of mice and production of antisera

Five weeks old CD1 mice (8 for each antigen) were immunized by 3 intraperitoneal injections (every two weeks) of 10 micrograms of purified protein antigens with Freund's adjuvant (200 microliters per mouse) or with Alum (aluminium hydroxide adjuvant; 2 mg/ml). Sera were collected two weeks after the third injection and stocked at -20°C. Controls were injected with Freund's adjuvant only or alum only.

### FACS analysis

A surface labeling assay by FACS was performed in order to examine the surface exposure of the selected antigens and the levels of expression in different strains. NTHI were incubated with sera derived from mice immunized with recombinant proteins or negative controls, and analysed by FACS. The results are shown in Figure 2. In Figure 2, pre-immune serum negative controls are shown as solid areas, and the signal obtained with sample serum is shown as a single line. The results of FACS analyses of antigens P48, HtrA, PE, and P26 demonstrate that each of these antigens is exposed on the surface of the bacterium and thus accessible to antibody binding.

The following materials and methods were used in this analysis:

### Materials

1. 96 U-bottom well plates.
2. Blocking and Washing Buffer: PBS containing 1% (w/v) BSA.
3. Goat anti-mouse IgG-Fluorescein IsoThio Cyanate FITC.
4. PBS containing 0.5% (v/v) para-formaldehyde: dilute a stock solution of 4% (v/v) para-formaldehyde in PBS to 0.5% (v/v) fresh before the assay and filter sterilize (0.22µm filter).
5. PBS containing 1% (w/v) BSA. To prepare this solution, dissolve 1% (w/v) BSA in PBS, making at least 100 ml for each strain. Filter-sterilize the solution (0.22µm filter) and prepare fresh for use.
6. *FACScan* tubes (Becton Dickson).
7. *FACScalibur* flow cytometer (Becton Dickinson).

### Methods

1. Grow NTHI until an ODλ₆₀₀ nm value of 0.5 is reached, then transfer 1 ml of culture to a sterile 1.5 ml Eppendorf tube and centrifuge at 13000g in a micro-centrifuge for 3 minutes to pellet the bacteria. Discard the supernatant and suspend the pellet suspended in 1 ml of PBS containing 1%(w/v) BSA. Finally, dilute the bacterial suspension 1/50 in PBS containing 1% (w/v) BSA.
2. Add 50µl samples of sera diluted in Blocking Buffer (at 1/100, 1/200 and 1/400) in a 96 well plate. Include positive controls, such as anti-OMV antisera,
3. Add 50 µl of bacterial cells to each well and store the plate at 4C° for 2 h.
4. Centrifuge the cells for 5 minutes at 3500 g, discard the supernatant and wash the cells by adding 200µl/well of Washing Buffer.
5. Add 50µl of a 1/100 dilution of FITC-conjugated goat anti-mouse Ig to each well and store the plate at 4°C for 1 h.
6. Centrifuge the cells at 3500g for 5 min and wash the pellet with 200µl/well of PBS.
7. Repeat the centrifugation step, discard the supernatant and add 200µl/well of PBS containing 0.5% (v/v) para-formaldehyde, in order to fix the cells.
8. Transfer the fixed samples to individual FACScan tubes and analyse by flow cytometry, following the equipment manufacturer's instructions.

### Serum Bactericidal assay (SBA)

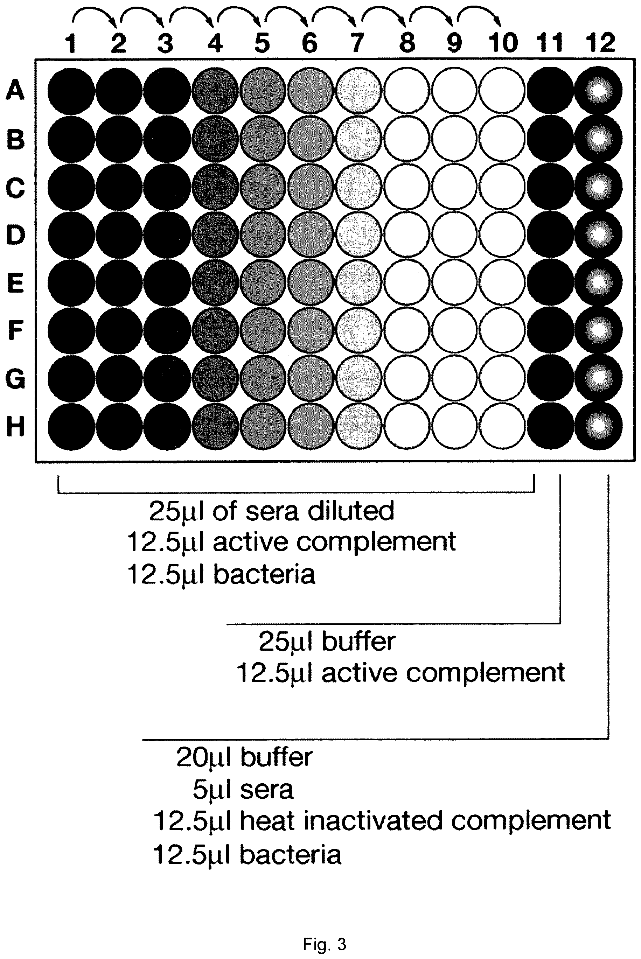

Antisera derived from mice immunized with recombinant proteins were tested in a serum bactericidal assay, to verify the presence of functional antibodies able to induce killing of NTHI. Pre-immune sera and sera from mice injected only with adjuvant were used as negative controls. NTHI (strain 176) culture (BHI+NAD and Haemin) was incubated at 37° C with shaking, until OD595 nm was 0.25-0.27. The bacterial cells were diluted in D-PBS buffer at the working dilution 1:50000. Sera were inactivated at 56° for 30 minutes and then serially diluted in D-PBS in a 96-well U-bottom plate (see Figure 3). Columns 11 and 12 of the plate shown in Figure 3 contain negative controls to assess the growth of the bacteria and to detect any non-complement mediated killing. Bacteria and a source of complement (Rabbit 7504, Cedarlane) were added to each well except in the complement control wells which received heat-inactivated complement.

As shown in Figure 3, wells in columns 1-10 contain 25µl diluted sera, 12.5 µl active complement, and 12.5 µl bacteria. Wells in column 11 contain 25µl buffer, 12.5 µl active complement, and 12.5 µl bacteria. Wells in column 12 contain 25µl buffer, 5 µl sera, 12.5 µl heat inactivated complement, and 12.5 µl bacteria.

10 µl of the time zero (T0) assay controls (column 11-12) were plated on agar chocolate plate (Biomerieux) by the spot and tilt method. Plates were incubated at 37°C, ON. The assay microtiter plates were incubated for 1 hour at 37°C. After this period (T60) 7 µl of each well were plated as spot on an agar chocolate plate (each well was plated in duplicate). The number of colonies (colony forming units, CFU) was counted using a colony counter or manually. A bactericidal effect was considered to be observed when the number of colonies was lower than 50% of T=0.

An overview of the results is provided in the following Table III and Table IV.

**Table III: Immunogenicity results**

| **Internal ID** | **Annotation** | **SEq ID NOs** | **kDa** | **SBA TITER Freund's adjuvant (176wt)** | **FACS** |
|---|---|---|---|---|---|
| NT001 | NTHI0877 | SEQ ID NO: 6 or SEQ ID NO: 54 | 30 | 2048-8192 | +++++ |
| NT016 | NTHI0266 | SEQ ID NO: 7 or SEQ ID NO: 55 | 29 | 2048-8192 | +++++ |
| NT024 | NTHI1416 | SEQ ID NO: 2 or SEQ ID NO: 50 | 20 | 2048-8192 | ++ |
| NT032 | NTHI2017 | SEQ ID NO: 3 or SEQ ID NO: 51 | 13 | 2048-8192 | + |
| NT018 | NTHI0915 | SEQ ID NO: 1 or SEQ ID NO: 49 | 34 | 2048-4096 | + |
| NT038 | CGSHiGG_0 2400 | SEQ ID NO: 4 or SEQ ID NO: 50 | 22 | 2048-4096 | ++ |
| NT052 | CGSHiGG_0 0130 | SEQ ID NO: 8 or SEQ ID NO: 56 | 44 | 2048-4096 | ++ |
| NT067 | NTHI1292 | SEQ ID NO: 5 or SEQ ID NO: 52 | 60 | 2048 | ++ |
| NT002 | NTHI1627 | SEQ ID NO: 9 or SEQ ID NO: 57 | 18 | 1024-2048 | +++ |
| NT026 | NTHI1109 | SEQ ID NO: 10 or SEQ IDNO: 58 | 19 | 4096-8192 | ++++ |
| NT009 | NTHI0821 | SEQ ID NO: 11 or SEQ ID NO:59 | 64 | 4096 | +++ |
| NT025 | NTHI0409 | SEQ ID NO: 12 or SEQ IDNO: 60 | 17 | 4096 | ++++ |
| NT028 | NTHI1954 | SEQ ID NO: 13 SEQ ID NO: 61 | 20 | 4096 | +++ |
| NT029 | NTHI0371 | SEQ ID NO: 14 SEQ ID NO: 62 | 101 | 4096 | +++ |
| NT031 | NTHI0509 | SEQ ID NO: 15 SEQ ID NO: 63 | 20 | 4096 | + |
| NT015 | NTHI0449 | SEQ ID NO: 16 SEQ ID NO: 64 | 15 | 2048-4096 | ++ |
| NT023 | NTHI1473 | SEQ ID NO: 17 SEQ ID NO: 65 | 17 | 2048-4096 | ++ |
| NT 100 | gi145633184 | SEQ ID NO: 18 SEQ ID NO: 66 | 34 | 2048-4096 | + |
| NT040 | NTHI1110 | SEQ ID NO: 19 | 26 | 1024-2048 | + |
| NT048 | gi-46129075 | SEQ ID NO: 20 | 71 | 1024-2048 | + |
| NT053 | gi145628236 | SEQ ID NO: 21 | 17 | 1024-2048 | + |
| NT066 | NTHI1230 | SEQ ID NO: 22 SEQ ID NO: 67 | 59 | 1024-2048 | + |
| NT097 | NTHI0522 | SEQ ID NO: 23 | 50 | 1024-2048 | ++ |
| NT006 (HtrA) | NTHI1905 | SEQ ID NO: 25 | 51 | 2048 | ++++ |
| NT035 (PE) | NTHI0267 | SEQ ID NO: 26 | 18 | 512-1024 | ++ |
| NT080 (PHiD) | NTHI0811 | SEQ ID NO: 28 | | 512 | |
| NT081(P6) | NTHI0501 | SEQ ID NO: 29 | | 512 | |
| NT010 (P26) | NTHI1083 | SEQ ID NO: 27 | 22 | 128-512 | +++ |
| NT007 (P48) | NTHI0254 | SEQ ID NO: 24 | 48 | 8192-16384 | +++++ |
| Unrelated antigen | | | | 16 | + |
| Freund's Adj. alone | | | | 512/1024 | |

These results show that antigens selected are highly effective in killing NTHI pathogens. In particular NT018, NT001, NT024, NT032, NT067, NT016 all show particularly strong protective effects.

**Table IV: Immunization experiments using compositions comprising NTHI antigens and Alum**

| **Protein** | **Purity** | **SBA** | **SBA (Alum)** | **FACS** | **FACS** | **Solubility** |
|---|---|---|---|---|---|---|
| **Antigen** | | **(Freund)** | | **Freund** | **alum** | |
| NT001 | 97% | 2048-8192 | 512-1024 | +++++ | ++ + | Yes |
| NT024 | 94% | 2048-8192 | 1024 | ++ | ++ + | Yes |
| NT038 | 97% | 2048-4096 | 64 | ++ | - | Yes |
| NT018 | 80% | 2048-4096 | 512-1024 | + | +++ | Yes |
| NT032 | 99% | 2048-8192 | 64-128 | + | + | Yes |
| NT067 | 88% | 2048 | 512-2048 | ++ | +++ | Yes |
| NT025 | 94% | 4096 | 128-256 | ++++ | + | No |
| NT026 | 64% | 4096-8192 | 64 | ++++ | - | No |
| NT028 | 81% | 4096 | 128-256 | +++ | ++ | No |
| NT029 | 52% | 4096 | 128 | +++ | ++ | Yes |
| NT023 | 80% | 2048-4096 | 256-512 | ++ | + | Yes |
| NT015 | 78% | 2048-4096 | 128-256 | ++ | + | No |
| NT031 | 90% | 4096 | 128 | + | + | No |
| NT100 | 81% | 2048-4096 | 512 | + | + | Yes |
| NT081 (P6) | 88% | 2048 | 256 | + | + | No |
| NT080 (PHiD) | 92% | 1024 | 128 | + | + | |
| NT006 (HtrA) | 57% | 2048 | 256 | ++++ | ++++ | |
| NT007 (P48) | 79% | 8192-16384 | 256-512 | +++++ | +++++ | |
| NT052 | 88% | 2048-4096 | 512-1024 | + | +++ | Yes |
| NT014 | 87% | 1024 | 512-1024 | ++ | ++ | Yes |
| NT004 | 95% | 256-512 | 128-256 | ++ | + | Yes |
| NT022 | 93% | 64-256 | 1024 | +++ | + | Yes |
| NT016 | 98% | 2048-8192 | 128 | +++ | ++++ | Yes |
| NT106 | 82% | Not tested | 64-128 | ++ | +++ | Yes |
| NT113 | 92% | Not tested | 128 | ++ | +++ | Yes |
| NT061 | 83% | Not tested | 128 | +++ | +++ | Yes |
| Freund's | | 512 | NA | | | |
| Alum | | NA | 4-8 | | | |

These results further confirmed that antigens selected are highly effective in killing NTHI pathogens also when used in immunogenic compositions with alum as adjuvant.

In particular NT016, NT052, NT018, NT001, NT024, NT032, NT067, NT014, NT022 all confirm particularly strong protective effects as measured in serum bactericidal assay (SBA).

Particularly preferred antigens were NT067, NT014, NT016, NT022. These antigens have been also tested in an *in vivo* animal model according to the protocol described in Ref. 75.

### In vivo vaccine efficacy testing

Individual antigens as listed in Table IV can be tested for their ability to protect against an otitis media (OM) infection using an *in vivo* model such as Junbo and Jeff mouse mutants [75].

The vaccine efficacy in the *in vivo* protection experiment is performed using 3 administrations (at day 0, 21, 35) of 10 micrograms/mouse of purified recombinant protein antigens formulated with or without adjuvant, followed by intranasal inoculation with selected NTHI pathogenic strains.

Pre-immune sera, post-immunization sera, and terminal sera 7 days post-NTHI inoculation are collected and stored at -80°C. Controls are immunized with adjuvant or with an unrelated antigen as control. Middle ear bulla and nasopharyngeal (NP) washes samples are collected and plated to determine NTHi numbers; bulla infection and nasopharingeal carriage rates, and bulla NTHi titres are then calculated.

**Table V: Nomenclature cross-reference with representative strains**

| | | **86-028NP** | **3655 Strain** | **PittG Strain** |
|---|---|---|---|---|
| **SEQ ID NOs** | **Name** | **NTHI_#** | | |
| 1 or 49 | NT018 | NTHI0915 | | |
| 2 or 50 | NT024 | NTHI1416 | | |
| 3 or 51 | NT032 | NTHI2017 | | |
| 4 or 53 | NT038 | | | CGSHiGG_02400 |
| 5 or 52 | NT067 | NTHI1292 | | |
| 6 or 54 | NT001 | NTHI0877 | | |
| 7 or 55 | NT016 | NTHI0266 | | |
| 8 or 56 | NT052 | | | CGSHiGG_00130 |
| 9 or 57 | NT002 | NTHI1627 | | |
| 10 or 58 | NT026 | NTHI1109 | | |
| 11 or 59 | NT009 | NTHI0821 | | |
| 12 or 60 | NT025 | NTHI0409 | | |
| 13 or 61 | NT028 | NTHI1954 | | |
| 14 or 62 | NT029 | NTHI0371 | | |
| 15 or 63 | NT031 | NTHI0509 | | |
| 16 or 64 | NT015 | NTHI0449 | | |
| 17 or 65 | NT023 | NTHI1473 | | |
| 18 or 66 | NT100 | | gi-145633184 | |
| 19 | NT040 | NTHI1110 | | |
| 20 | NT048 | | gi-46129075 | |
| 21 | NT053 | | gi-145628236 | |
| 22 or 67 | NT066 | NTHI1230 | | |
| 23 | NT097 | NTHI0522 | | |
| 24 | NT007 | P48 | | |
| 25 | NT006 | HtrA | | |
| 26 | NT035 | PE | | |
| 27 | NT010 | P26 | | |
| 28 | NT080 | PHiD | | |
| 29 | NT081 | P6 | | |
| 30 | NT013 | NTHI0532 | | |
| 31 | NT106 | NTHI0363 | | |
| 32 | NT107 | NTHI0370 | | |
| 33 | NT108 | NTHI0205 | | |
| 34 | NT109 | NTHI0374 | | |
| 35 | NT110 | NTHI0579 | | |
| 36 | NT111 | NTHI0837 | | |
| 37 | NT112 | NTHI0849 | | |
| 38 | NT113 | NTHI0921 | | |
| 39 | NT114 | NTHI0995 | | |
| 40 | NT115 | NTHI1091 | | |
| 41 | NT116 | NTHI1169 | | |
| 42 | NT117 | NTHI1208 | | |
| 43 | NT118 | NTHI1318 | | |
| 44 | NT123 | NTHI1796 | | |
| 45 | NT124 | NTHI1930 | | |
| 114 | NT119 | NTHI1565 | | |
| 115 | NT120 | NTHI1569 | | |
| 116 | NT121 | NTHI1571 | | |
| 117 | NT122 | NTHI1667 | | |
| 122 | NT004 | | | CGSHiGG_08215 |
| 123 | NT014 | | gi-145629254 | |
| 124 | NT022 | NTHI0830 | | |
| 128 | NT061 | NTHI0588 | | |
| 130 | NT017 | NTHI0915 | | |

### REFERENCES

[1] Fleischmann et al. (1995) Science 269:496-512.
[2] Li et al. (2003) Mol Microbiol 47:1101 -1111.
[3] GenBank accession NC_000907.
[4] WO2005/111066
[5] Murphy et al. Current Infectious Disease report (2009)11:177-182.
[6] Webb DC et al. - Investigation of the potential of a 48 kDa protein as a vaccine candidate for infection against nontypable Haemophilus influenzae. Vaccine. 2007 May 16;25(20):4012-9.
[7] Hallström T et al. - Nontypeable Haemophilus influenzae protein E binds vitronectin and is important for serum resistance. J Immunol. 2009 Aug 15;183(4):2593-601.
[8] Loosmore SM et al. - The Haemophilus influenzae HtrA protein is a protective antigen. Infect Immun. 1998 Mar;66(3):899-906.
[9] Kyd JM et al. - Potential of a novel protein, OMP26, from nontypeable Haemophilus influenzae to enhance pulmonary clearance in a rat model. Infect Immun. 1998 May;66(5):2272-8.
[10] Ronander E, The Journal of Infectious Diseases (2009): 199 p522-530
[11] WO00/55191.
[12] WO02/24729.
[13] Chanyangam M. et al., (1991) Infection and Immunity, Vol. 59 (2), 600-608
[14] Munson R.S.; Granoff D.M (1985) Infection and Immunity 49 (3):544 - 549
[15] Hogg et al. (2007) Genome Biology 8:R103.
[16] Needleman & Wunsch (1970) J. Mol. Biol. 48, 443-453.
[17] Rice et al. (2000) Trends Genet 16:276-277.
[18] Bernadac A., et al. (1998) Journal of Bacteriology 180 (18) : 4872-4878
[19] WO2002/062378
[20] Uehara T. et al., The EMBO Journal (2010) 29, 1412-1422
[21] US patent 5,707,829
[22] Current Protocols in Molecular Biology (F.M. Ausubel et al. eds., 1987) Supplement 30.
[23] Vaccine Design (1995) eds. Powell & Newman. ISBN: 030644867X. Plenum.
[24] WO90/14837.
[25] Podda & Del Giudice (2003) Expert Rev Vaccines 2:197-203.
[26] Podda (2001) Vaccine 19: 2673-2680.
[27] Vaccine Adjuvants: Preparation Methods and Research Protocols (Volume 42 of Methods in Molecular Medicine series). ISBN: 1-59259-083-7. Ed. O'Hagan.
[28] US 5,057,540.
[29] Niikura et al. (2002) Virology 293:273-280.
[30] Lenz et al. (2001) J Immunol 166:5346-5355.
[31] Pinto et al. (2003) J Infect Dis 188:327-338.
[32] Gerber et al. (2001) J Virol 75:4752-4760.
[33] WO03/024480.
[34] WO03/024481.
[35] Gluck et al. (2002) Vaccine 20:B10-B16.
[36] Meraldi et al. (2003) Vaccine 21:2485-2491.
[37] Pajak et al. (2003) Vaccine 21:836-842.
[38] Krieg (2003) Nature Medicine 9:831-835.
[39] McCluskie et al. (2002) FEMS Immunology and Medical Microbiology 32:179-185.
[40] WO98/40100.
[41] US 6,207,646.
[42] US 6,239,116.
[43] US 6,429,199.
[44] Schellack et al. (2006) Vaccine 24:5461-72.
[45] Johnson et al. (1999) Bioorg Med Chem Lett 9:2273-2278.
[46] Evans et al. (2003) Expert Rev Vaccines 2:219-229.
[47] Beignon et al. (2002) Infect Immun 70:3012-3019.
[48] Pizza et al. (2001) Vaccine 19:2534-2541.
[49] Pizza et al. (2000) Int J Med Microbiol 290:455-461.
[50] Scharton-Kersten et al. (2000) Infect Immun 68:5306-5313.
[51] Ryan et al. (1999) Infect Immun 67:6270-6280.
[52] Partidos et al. (1999) Immunol Lett 67:209-216.
[53] Peppoloni et al. (2003) Expert Rev Vaccines 2:285-293.
[54] Pine et al. (2002) J Control Release 85:263-270.
[55] WO99/40936.
[56] WO99/44636.
[57] Singh et all (2001) J Cont Release 70:267-276.
[58] WO99/27960.
[59] US 6,090,406.
[60] US 5,916,588.
[61] EP-A-0626169.
[62] WO99/52549.
[63] Andrianov et al. (1998) Biomaterials 19:109-115.
[64] Payne et al. (1998) Adv Drug Delivery Review 31:185-196.
[65] Stanley (2002) Clin Exp Dermatol 27:571-577.
[66] Jones (2003) Curr Opin Investig Drugs 4:214-218.
[67] WO99/11241.
[68] WO94/00153.
[69] WO98/57659.
[70] European patent applications 0835318, 0735898 and 0761231.
[71] Ogunniyi et al. (2001) Infect Immun 69:5997-6003.
[72] WO2006/110603.
[73] Mason et al. (2003) Infect Immun 71:3454-3462.
[74] Zwijnenburg et al. (2001) J Infect Dis 183:1143-6.
[75] Cheeseman M. T. et al. (2011) PLoS Genetics 7 (10) : e1002336.
[76] Watson (2000) Pediatr Infect Dis J 19:331-332.
[77] Rubin (2000) Pediatr Clin North Am 47:269-285, v.
[78] Jedrzejas (2001) Microbiol Mol Biol Rev 65:187-207.
[79] Bell (2000) Pediatr Infect Dis J 19:1187-1188.
[80] Iwarson (1995) APMIS 103:321-326.
[81] Gerlich et al. (1990) Vaccine 8 Suppl:S63-68 & 79-80.
[82] Vaccines (1988) eds. Plotkin & Mortimer. ISBN 0-7216-1946-0.
[83] Del Guidice et al. (1998) Molecular Aspects of Medicine 19:1-70.
[84] Gustafsson et al. (1996) N. Engl. J. Med. 334:349-355.
[85] Rappuoli et al. (1991) TIBTECH 9:232-238.
[86] Costantino et al. (1999) Vaccine 17:1251-1263.
[87] Sutter et al. (2000) Pediatr Clin North Am 47:287-308.
[88] Zimmerman & Spann (1999) Am Fam Physician 59:113-118, 125-126.
[89] McMichael (2000) Vaccine 19 Suppl 1:S101-107.
[90] Schuchat (1999) Lancet 353(9146):51-6.
[91] WO02/34771.
[92] Dale (1999) Infect Dis Clin North Am 13:227-43, viii.
[93] Ferretti et al. (2001) PNAS USA 98: 4658-4663.
[94] Kuroda et al. (2001) Lancet 357(9264):1225-1240; see also pages 1218-1219.
[95] EP-A-0372501
[96] EP-A-0378881
[97] EP-A-0427347
[98] WO93/17712
[99] WO94/03208
[100] WO98/58668
[101] EP-A-0471177
[102] WO00/56360
[103] WO91/01146
[104] WO00/61761
[105] WO01/72337
[106] Research Disclosure, 453077 (Jan 2002)
[107] Brandt et al. (2006) J Antimicrob Chemother. 58(6): 1291-4. Epub 2006 Oct 26
[108] Winter et al., (1991) Nature 349:293-99
[109] US 4,816,567.
[110] Inbar et al., (1972) Proc. Natl. Acad. Sci. U.S.A. 69:2659-62.
[111] Ehrlich et al., (1980) Biochem 19:4091-96.
[112] Huston et al., (1988) Proc. Natl. Acad. Sci. U.S.A. 85:5897-83.
[113] Pack et al., (1992) Biochem 31, 1579-84.
[114] Cumber et al., (1992) J. Immunology 149B, 120-26.
[115] Riechmann et al., (1988) Nature 332, 323-27.
[116] Verhoeyan et al., (1988) Science 239, 1534-36.
[117] GB 2,276,169.
[118] Gennaro (2000) Remington: The Science and Practice of Pharmacy. 20th edition, ISBN: 0683306472.
[119] Methods In Enzymology (S. Colowick and N. Kaplan, eds., Academic Press, Inc.)
[120] Handbook of Experimental Immunology, Vols. I-IV (D.M. Weir and C.C. Blackwell, eds, 1986, Blackwell Scientific Publications)
[121] Sambrook *et al.* (2001) *Molecular Cloning: A Laboratory Manual,* 3rd edition (Cold Spring Harbor Laboratory Press).
[122] Handbook of Surface and Colloidal Chemistry (Birdi, K.S. ed., CRC Press, 1997)
[123] Ausubel et al. (eds) (2002) Short protocols in molecular biology, 5th edition (Current Protocols).
[124] Molecular Biology Techniques: An Intensive Laboratory Course, (Ream et al., eds., 1998, Academic Press)
[125] PCR (Introduction to Biotechniques Series), 2nd ed. (Newton & Graham eds., 1997, Springer Verlag)
[126] Geysen et al. (1984) PNAS USA 81:3998-4002.
[127] Carter (1994) Methods Mol Biol 36:207-23.
[128] Jameson, BA et al. 1988, CABIOS 4(1):181-186.
[129] Raddrizzani & Hammer (2000) Brief Bioinform 1(2):179-89.
[130] Bublil et al. (2007) Proteins 68(1):294-304.
[131] De Lalla et al. (1999) J. Immunol. 163:1725-29.
[132] Kwok et al. (2001) Trends Immunol 22:583-88.
[133] Brusic et al. (1998) Bioinformatics 14(2):121-30
[134] Meister et al. (1995) Vaccine 13(6):581-91.
[135] Roberts et al. (1996) AIDS Res Hum Retroviruses 12(7):593-610.
[136] Maksyutov & Zagrebelnaya (1993) Comput Appl Biosci 9(3):291-7.
[137] Feller & de la Cruz (1991) Nature 349(6311):720-1.
[138] Hopp (1993) Peptide Research 6:183-190.
[139] Welling et al. (1985) FEBS Lett. 188:215-218.
[140] Davenport et al. (1995) Immunogenetics 42:392-297.
[141] Tsurui & Takahashi (2007) J Pharmacol Sci. 105(4):299-316.
[142] Tong et al. (2007) Brief Bioinform. 8(2):96-108.
[143] Schirle et al. (2001) J Immunol Methods. 257(1-2):1-16.
[144] Chen et al. (2007) Amino Acids 33(3):423-8.
[145] Smith & Waterman (1981) Adv. Appl. Math. 2: 482-489.
[146] Lundström et al. (2008) Biochemistry, 47 (22) :6025-38 Structural analysis of the lipopolysaccharide from nontypeable Haemophilus influenzae strain R2846.
[147] Klock, H.E., et al. (2008). Proteins 71:982-994
[148] Klock, H. E., et al. (2005) J. Struct. Funct. Genomics 6, 89-94

## Claims

1. An immunogenic composition comprising a NT067 non-typeable *H. influenzae* antigen for use in a method of raising a protective immune response against *H. influenzae* infection in a mammal, wherein said NT067 antigen is a polypeptide that comprises an amino acid sequence having 95% or more identity to SEQ ID NO: 5.

2. The composition for use of claim 1, wherein said NT067 antigen comprises the amino acid sequence of SEQ ID NO: 52.

3. The composition for use according to claim 1, further comprising one or more non-typeable *H. influenzae* antigens, selected from the group consisting of: NT052 non-typeable *H. influenzae* antigen, wherein said NT052 antigen is a polypeptide that comprises an amino acid sequence: (a) having 80% or more identity to SEQ ID NO: 8, and/or (b) that is a fragment of at least 10 consecutive amino acids, and comprises an epitope, of SEQ ID NO: 8; NT018 non-typeable *H. influenzae* antigen, wherein said NT018 antigen is a polypeptide that comprises an amino acid sequence: (a) having 80% or more identity to SEQ ID NO: 1, and/or (b) that is a fragment of at least 10 consecutive amino acids, and comprises an epitope, of SEQ ID NO: 1; NT024 non-typeable *H. influenzae* antigen, wherein said NT024 antigen is a polypeptide that comprises an amino acid sequence: (a) having 80% or more identity to SEQ ID NO: 2, and/or (b) that is a fragment of at least 10 consecutive amino acids, and comprises an epitope, of SEQ ID NO: 2; NT032 non-typeable *H. influenzae* antigen, wherein said NT032 antigen is a polypeptide that comprises an amino acid sequence: (a) having 80% or more identity to SEQ ID NO: 3, and/or (b) that is a fragment of at least 10 consecutive amino acids, and comprises an epitope, of SEQ ID NO: 3; NT038 non-typeable *H*. *influenzae* antigen, wherein said NT038 antigen is a polypeptide that comprises an amino acid sequence: (a) having 80% or more identity to SEQ ID NO: 4, and/or (b) that is a fragment of at least 10 consecutive amino acids, and comprises an epitope, of SEQ ID NO: 4; NT001 non-typeable *H. influenzae* antigen, wherein said NT001 antigen is a polypeptide that comprises an amino acid sequence: (a) having 80% or more identity to SEQ ID NO: 6, and/or (b) that is a fragment of at least 10 consecutive amino acids, and comprises an epitope, of SEQ ID NO: 6; NT016 non-typeable *H. influenzae* antigen, wherein said NT016 antigen is a polypeptide that comprises an amino acid sequence: (a) having 80% or more identity to SEQ ID NO: 7, and/or (b) that is a fragment of at least 10 consecutive amino acids, and comprises an epitope, of SEQ ID NO: 7; NT002 non-typeable *H. influenzae* antigen, wherein said NT002 antigen is a polypeptide that comprises an amino acid sequence: (a) having 80% or more identity to SEQ ID NO: 9, and/or (b) that is a fragment of at least 10 consecutive amino acids, and comprises an epitope, of SEQ ID NO: 9; NT026 non-typeable *H. influenzae* antigen, wherein said NT026 antigen is a polypeptide that comprises an amino acid sequence: (a) having 80% or more identity to SEQ ID NO: 10, and/or (b) that is a fragment of at least 10 consecutive amino acids, and comprises an epitope, of SEQ ID NO: 10; NT009 non-typeable *H. influenzae* antigen, wherein said NT009 antigen is a polypeptide that comprises an amino acid sequence: (a) having 80% or more identity to SEQ ID NO: 11, and/or (b) that is a fragment of at least 10 consecutive amino acids, and comprises an epitope, of SEQ ID NO: 11; NT025 non-typeable *H*. *influenzae* antigen, wherein said NT025 antigen is a polypeptide that comprises an amino acid sequence: (a) having 80% or more identity to SEQ ID NO: 12, and/or (b) that is a fragment of at least 10 consecutive amino acids, and comprises an epitope, of SEQ ID NO: 12; NT028 non-typeable *H. influenzae* antigen, wherein said NT028 antigen is a polypeptide that comprises an amino acid sequence: (a) having 80% or more identity to SEQ ID NO: 13, and/or (b) that is a fragment of at least 10 consecutive amino acids, and comprises an epitope, of SEQ ID NO: 13; NT029 non-typeable *H. influenzae* antigen, wherein said NT029 antigen is a polypeptide that comprises an amino acid sequence: (a) having 80% or more identity to SEQ ID NO: 14, and/or (b) that is a fragment of at least 10 consecutive amino acids, and comprises an epitope, of SEQ ID NO: 14; NT031 non-typeable *H. influenzae* antigen, wherein said NT031 antigen is a polypeptide that comprises an amino acid sequence: (a) having 80% or more identity to SEQ ID NO: 15, and/or (b) that is a fragment of at least 10 consecutive amino acids, and comprises an epitope, of SEQ ID NO: 15; NT015 non-typeable *H. influenzae* antigen, wherein said NT015 antigen is a polypeptide that comprises an amino acid sequence: (a) having 80% or more identity to SEQ ID NO: 16, and/or (b) that is a fragment of at least 10 consecutive amino acids, and comprises an epitope, of SEQ ID NO: 16; NT023 non-typeable *H. influenzae* antigen, wherein said NT023 antigen is a polypeptide that comprises an amino acid sequence: (a) having 80% or more identity to SEQ ID NO: 17, and/or (b) that is a fragment of at least 10 consecutive amino acids, and comprises an epitope, of SEQ ID NO: 17; NT100 non-typeable *H*. *influenzae* antigen, wherein said NT100 antigen is a polypeptide that comprises an amino acid sequence: (a) having 80% or more identity to SEQ ID NO: 18, and/or (b) that is a fragment of at least 10 consecutive amino acids, and comprises an epitope, of SEQ ID NO: 18; NT040 non-typeable *H. influenzae* antigen, wherein said NT040 antigen is a polypeptide that comprises an amino acid sequence: (a) having 80% or more identity to SEQ ID NO: 19, and/or (b) that is a fragment of at least 10 consecutive amino acids, and comprises an epitope, of SEQ ID NO: 19; NT048 non-typeable *H. influenzae* antigen, wherein said NT048 antigen is a polypeptide that comprises an amino acid sequence: (a) having 80% or more identity to SEQ ID NO: 20, and/or (b) that is a fragment of at least 10 consecutive amino acids, and comprises an epitope, of SEQ ID NO: 20; NT053 non-typeable *H. influenzae* antigen, wherein said NT053 antigen is a polypeptide that comprises an amino acid sequence: (a) having 80% or more identity to SEQ ID NO: 21, and/or (b) that is a fragment of at least 10 consecutive amino acids, and comprises an epitope, of SEQ ID NO: 21; NT066 non-typeable *H. influenzae* antigen, wherein said NT066 antigen is a polypeptide that comprises an amino acid sequence: (a) having 80% or more identity to SEQ ID NO: 22, and/or (b) that is a fragment of at least 10 consecutive amino acids, and comprises an epitope, of SEQ ID NO: 22; NT097 non-typeable *H. influenzae* antigen, wherein said NT097 antigen is a polypeptide that comprises an amino acid sequence: (a) having 80% or more identity to SEQ ID NO: 23, and/or (b) that is a fragment of at least 10 consecutive amino acids, and comprises an epitope, of SEQ ID NO: 23;NT 004 non-typeable *H*. *influenzae* antigen, wherein said NT004 antigen is a polypeptide that comprises an amino acid sequence: (a) having 80% or more identity to SEQ ID NO: 122, and/or (b) that is a fragment of at least 10 consecutive amino acids, and comprises an epitope, of SEQ ID NO: 122; NT014 non-typeable *H. influenzae* antigen, wherein said NT014 antigen is a polypeptide that comprises an amino acid sequence: (a) having 80% or more identity to SEQ ID NO: 123, and/or (b) that is a fragment of at least 10 consecutive amino acids, and comprises an epitope, of SEQ ID NO: 123; NT022 non-typeable *H. influenzae* antigen, wherein said NT022 antigen is a polypeptide that comprises an amino acid sequence: (a) having 80% or more identity to SEQ ID NO: 124, and/or (b) that is a fragment of at least 10 consecutive amino acids, and comprises an epitope, of SEQ ID NO: 124.

4. An immunogenic composition for use according to anyone of claims 1 to 3, further comprising at least one polypeptide selected from the group consisting of: NT007 which is a polypeptide that comprises an amino acid sequence: (a) having 80% or more identity to SEQ ID NO: 24, and/or (b) that is a fragment of at least 10 consecutive amino acids, and comprises an epitope, of SEQ ID NO: 24; NT006 which is a polypeptide that comprises an amino acid sequence: (a) having 80% or more identity to SEQ ID NO: 25, and/or (b) that is a fragment of at least 10 consecutive amino acids, and comprises an epitope, of SEQ ID NO: 25; NT035 which is a polypeptide that comprises an amino acid sequence: (a) having 80% or more identity to SEQ ID NO: 26, and/or (b) that is a fragment of at least 10 consecutive amino acids, and comprises an epitope, of SEQ ID NO: 26; NT010 which is a polypeptide that comprises an amino acid sequence: (a) having 80% or more identity to SEQ ID NO: 27, and/or (b) that is a fragment of at least 10 consecutive amino acids, and comprises an epitope, of SEQ ID NO: 27; NT080 which is a polypeptide that comprises an amino acid sequence: (a) having 80% or more identity to SEQ ID NO: 28, and/or (b) that is a fragment of at least 10 consecutive amino acids, and comprises an epitope, of SEQ ID NO: 28; NT081 which is a polypeptide that comprises an amino acid sequence: (a) having 80% or more identity to SEQ ID NO: 29, and/or (b) that is a fragment of at least 10 consecutive amino acids, and comprises an epitope, of SEQ ID NO: 29.

5. An immunogenic composition for use according to any preceding claim, further comprising at least one polypeptide selected from the group consisting of: NT013 which is a polypeptide that comprises an amino acid sequence: (a) having 80% or more identity to SEQ ID NO: 30, and/or (b) that is a fragment of at least 10 consecutive amino acids, and comprises an epitope, of SEQ ID NO: 30;NT106 which is a polypeptide that comprises an amino acid sequence: (a) having 80% or more identity to SEQ ID NO: 31, and/or (b) that is a fragment of at least 10 consecutive amino acids, and comprises an epitope, of SEQ ID NO: 31;NT107 which is a polypeptide that comprises an amino acid sequence: (a) having 80% or more identity to SEQ ID NO: 32, and/or (b) that is a fragment of at least 10 consecutive amino acids, and comprises an epitope, of SEQ ID NO: 32;NT108 which is a polypeptide that comprises an amino acid sequence: (a) having 80% or more identity to SEQ ID NO: 33, and/or (b) that is a fragment of at least 10 consecutive amino acids, and comprises an epitope, of SEQ ID NO: 33;NT109 which is a polypeptide that comprises an amino acid sequence: (a) having 80% or more identity to SEQ ID NO: 34, and/or (b) that is a fragment of at least 10 consecutive amino acids, and comprises an epitope, of SEQ ID NO: 34;NT110 which is a polypeptide that comprises an amino acid sequence: (a) having 80% or more identity to SEQ ID NO: 35, and/or (b) that is a fragment of at least 10 consecutive amino acids, and comprises an epitope, of SEQ ID NO: 35;NT111 which is a polypeptide that comprises an amino acid sequence: (a) having 80% or more identity to SEQ ID NO: 36, and/or (b) that is a fragment of at least 10 consecutive amino acids, and comprises an epitope, of SEQ ID NO: 36;NT112 which is a polypeptide that comprises an amino acid sequence: (a) having 80% or more identity to SEQ ID NO: 37, and/or (b) that is a fragment of at least 10 consecutive amino acids, and comprises an epitope, of SEQ ID NO: 37;NT113 which is a polypeptide that comprises an amino acid sequence: (a) having 80% or more identity to SEQ ID NO: 38, and/or (b) that is a fragment of at least 10 consecutive amino acids, and comprises an epitope, of SEQ ID NO: 38;NT114 which is a polypeptide that comprises an amino acid sequence: (a) having 80% or more identity to SEQ ID NO: 39, and/or (b) that is a fragment of at least 10 consecutive amino acids, and comprises an epitope, of SEQ ID NO: 39;NT115 which is a polypeptide that comprises an amino acid sequence: (a) having 80% or more identity to SEQ ID NO: 40, and/or (b) that is a fragment of at least 10 consecutive amino acids, and comprises an epitope, of SEQ ID NO: 40;NT116 which is a polypeptide that comprises an amino acid sequence: (a) having 80% or more identity to SEQ ID NO: 41, and/or (b) that is a fragment of at least 10 consecutive amino acids, and comprises an epitope, of SEQ ID NO: 41;NT117 which is a polypeptide that comprises an amino acid sequence: (a) having 80% or more identity to SEQ ID NO: 42, and/or (b) that is a fragment of at least 10 consecutive amino acids, and comprises an epitope, of SEQ ID NO: 42;NT118 which is a polypeptide that comprises an amino acid sequence: (a) having 80% or more identity to SEQ ID NO: 43, and/or (b) that is a fragment of at least 10 consecutive amino acids, and comprises an epitope, of 43;NT123 which is a polypeptide that comprises an amino acid sequence: (a) having 80% or more identity to SEQ ID NO: 44, and/or (b) that is a fragment of at least 10 consecutive amino acids, and comprises an epitope, of SEQ ID NO: 44;NT124 which is a polypeptide that comprises an amino acid sequence: (a) having 80% or more identity to SEQ ID NO: 45, and/or (b) that is a fragment of at least 10 consecutive amino acids, and comprises an epitope, of SEQ ID NO: 45;NT119 which is a polypeptide that comprises an amino acid sequence: (a) having 80% or more identity to SEQ ID NO: 114, and/or (b) that is a fragment of at least 10 consecutive amino acids, and comprises an epitope, of SEQ ID NO: 114;NT120 which is a polypeptide that comprises an amino acid sequence: (a) having 80% or more identity to SEQ ID NO: 115, and/or (b) that is a fragment of at least 10 consecutive amino acids, and comprises an epitope, of SEQ ID NO: 115;NT121 which is a polypeptide that comprises an amino acid sequence: (a) having 80% or more identity to SEQ ID NO: 116, and/or (b) that is a fragment of at least 10 consecutive amino acids, and comprises an epitope, of SEQ ID NO: 116;NT122 which is a polypeptide that comprises an amino acid sequence: (a) having 80% or more identity to SEQ ID NO: 117, and/or (b) that is a fragment of at least 10 consecutive amino acids, and comprises an epitope, of SEQ ID NO: 117; and/or NT061 which is a polypeptide that comprises an amino acid sequence: (a) having 80% or more identity to SEQ ID NO: 128, and/or (b) that is a fragment of at least 10 consecutive amino acids, and comprises an epitope, of SEQ ID NO: 128.

6. An immunogenic composition for use according to any one of claims 1 to 5 comprising a combination of two or more antigens.

7. The composition for use according to any one of claims 1 to 6, further comprising at least one vaccine antigen that is not a non-typeable *H. influenzae* antigen selected from any of:
- an antigen from *N.meningitidis* serogroup A, B, C, W135 and/or Y.
- a saccharide or polypeptide antigen from *Streptococcus pneumoniae*
- an antigen from hepatitis A virus
- an antigen from hepatitis B virus
- a diphtheria antigen, such as a diphtheria toxoid
- a tetanus antigen
- an antigen from *Bordetella pertussis*
- a whole cellular pertussis antigen
- a saccharide antigen from *Haemophilus influenzae B*
- polio antigen(s)
- measles, mumps and/or rubella antigens
- influenza antigen(s)
- an antigen from *Moraxella catarrhalis*
- an antigen from Respiratory Syncytial Virus
- a vaccine composition comprising diphtheria (D), tetanus (T), pertussis (acellular, component) (Pa), hepatitis B (rDNA) (HBV), poliomyelitis (inactivated) (IPV) and *Haemophilus influenzae* type b (Hib) conjugate vaccine (adsorbed), e.g. Infanrix-hexa

8. The composition for use according to any one of claims 1 to 7, wherein said method of raising a protective immune response against *H. influenzae* infection in a mammal is a method for protecting against otitis media.

9. The composition for use according to any one of claims 1 to 7, wherein said method of raising a protective immune response against *H. influenzae* infection in a mammal is a method for treatment or prevention of COPD diseases.

## Patentansprüche

1. Immunogene Zusammensetzung, umfassend ein nicht-typisierbares *H*. *influenzae*-NT067-Antigen, zur Verwendung in einem Verfahren zum Hervorrufen einer schützenden Immunantwort gegen *H. influenzae-Infektion* in einem Säugetier, wobei das NT067-Antigen ein Polypeptid ist, welches eine Aminosäuresequenz mit 95% oder mehr Identität zu SEQ ID NO: 5 umfasst.

2. Zusammensetzung zur Verwendung nach Anspruch 1, wobei das NT067-Antigen die Aminosäuresequenz von SEQ ID NO: 52 umfasst.

3. Zusammensetzung zur Verwendung gemäß Anspruch 1, weiterhin umfassend ein oder mehrere nicht-typisierbare *H. influenzae-Antigen(e),* ausgewählt aus der Gruppe, bestehend aus: nicht-typisierbarem *H. influenzae-NT052-*Antigen, wobei das NT052-Antigen ein Polypeptid ist, welches eine Aminosäuresequenz umfasst: (a) mit 80% oder mehr Identität zu SEQ ID NO: 8 und/oder (b) die ein Fragment von wenigstens 10 aufeinanderfolgenden Aminosäuren von SEQ ID NO: 8 ist und ein Epitop davon umfasst; nicht-typisierbarem *H. influenzae-NT018-*Antigen, wobei das NT018-Antigen ein Polypeptid ist, welches eine Aminosäuresequenz umfasst: (a) mit 80% oder mehr Identität zu SEQ ID NO: 1 und/oder (b) die ein Fragment von wenigstens 10 aufeinanderfolgenden Aminosäuren von SEQ ID NO: 1 ist und ein Epitop davon umfasst; nicht-typisierbarem *H. influenzae-NT024-*Antigen, wobei das NT024-Antigen ein Polypeptid ist, welches eine Aminosäuresequenz umfasst: (a) mit 80% oder mehr Identität zu SEQ ID NO: 2 und/oder (b) die ein Fragment von wenigstens 10 aufeinanderfolgenden Aminosäuren von SEQ ID NO: 2 ist und ein Epitop davon umfasst; nicht-typisierbarem *H. influenzae-NT032-*Antigen, wobei das NT032-Antigen ein Polypeptid ist, welches eine Aminosäuresequenz umfasst: (a) mit 80% oder mehr Identität zu SEQ ID NO: 3 und/oder (b) die ein Fragment von wenigstens 10 aufeinanderfolgenden Aminosäuren von SEQ ID NO: 3 ist und ein Epitop davon umfasst; nicht-typisierbarem *H. influenzae-NT038-*Antigen, wobei das NT038-Antigen ein Polypeptid ist, welches eine Aminosäuresequenz umfasst: (a) mit 80% oder mehr Identität zu SEQ ID NO: 4 und/oder (b) die ein Fragment von wenigstens 10 aufeinanderfolgenden Aminosäuren von SEQ ID NO: 4 ist und ein Epitop davon umfasst; nicht-typisierbarem *H. influenzae-*NT001-Antigen, wobei das NT001-Antigen ein Polypeptid ist, welches eine Aminosäuresequenz umfasst: (a) mit 80% oder mehr Identität zu SEQ ID NO: 6 und/oder (b) die ein Fragment von wenigstens 10 aufeinanderfolgenden Aminosäuren von SEQ ID NO: 6 ist und ein Epitop davon umfasst; nicht-typisierbarem *H. influenzae-NT016-*Antigen, wobei das NT016-Antigen ein Polypeptid ist, welches eine Aminosäuresequenz umfasst: (a) mit 80% oder mehr Identität zu SEQ ID NO: 7 und/oder (b) die ein Fragment von wenigstens 10 aufeinanderfolgenden Aminosäuren von SEQ ID NO: 7 ist und ein Epitop davon umfasst; nicht-typisierbarem *H. influenzae-NT002-*Antigen, wobei das NT002-Antigen ein Polypeptid ist, welches eine Aminosäuresequenz umfasst: (a) mit 80% oder mehr Identität zu SEQ ID NO: 9 und/oder (b) die ein Fragment von wenigstens 10 aufeinanderfolgenden Aminosäuren von SEQ ID NO: 9 ist und ein Epitop davon umfasst; nicht-typisierbarem *H. influenzae-NT026-*Antigen, wobei das NT026-Antigen ein Polypeptid ist, welches eine Aminosäuresequenz umfasst: (a) mit 80% oder mehr Identität zu SEQ ID NO: 10 und/oder (b) die ein Fragment von wenigstens 10 aufeinanderfolgenden Aminosäuren von SEQ ID NO: 10 ist und ein Epitop davon umfasst; nicht-typisierbarem *H. influenzae-NT009-*Antigen, wobei das NT009-Antigen ein Polypeptid ist, welches eine Aminosäuresequenz umfasst: (a) mit 80% oder mehr Identität zu SEQ ID NO: 11 und/oder (b) die ein Fragment von wenigstens 10 aufeinanderfolgenden Aminosäuren von SEQ ID NO: 11 ist und ein Epitop davon umfasst; nicht-typisierbarem *H. influenzae-*NT025-Antigen, wobei das NT025-Antigen ein Polypeptid ist, welches eine Aminosäuresequenz umfasst: (a) mit 80% oder mehr Identität zu SEQ ID NO: 12 und/oder (b) die ein Fragment von wenigstens 10 aufeinanderfolgenden Aminosäuren von SEQ ID NO: 12 ist und ein Epitop davon umfasst; nicht-typisierbarem *H. influenzae-NT028-*Antigen, wobei das NT028-Antigen ein Polypeptid ist, welches eine Aminosäuresequenz umfasst: (a) mit 80% oder mehr Identität zu SEQ ID NO: 13 und/oder (b) die ein Fragment von wenigstens 10 aufeinanderfolgenden Aminosäuren von SEQ ID NO: 13 ist und ein Epitop davon umfasst; nicht-typisierbarem *H. influenzae-NT029-*Antigen, wobei das NT029-Antigen ein Polypeptid ist, welches eine Aminosäuresequenz umfasst: (a) mit 80% oder mehr Identität zu SEQ ID NO: 14 und/oder (b) die ein Fragment von wenigstens 10 aufeinanderfolgenden Aminosäuren von SEQ ID NO: 14 ist und ein Epitop davon umfasst; nicht-typisierbarem *H. influenzae-NT031-*Antigen, wobei das NT031-Antigen ein Polypeptid ist, welches eine Aminosäuresequenz umfasst: (a) mit 80% oder mehr Identität zu SEQ ID NO: 15 und/oder (b) die ein Fragment von wenigstens 10 aufeinanderfolgenden Aminosäuren von SEQ ID NO: 15 ist und ein Epitop davon umfasst; nicht-typisierbarem *H. influenzae-NT015-*Antigen, wobei das NT015-Antigen ein Polypeptid ist, welches eine Aminosäuresequenz umfasst: (a) mit 80% oder mehr Identität zu SEQ ID NO: 16 und/oder (b) die ein Fragment von wenigstens 10 aufeinanderfolgenden Aminosäuren von SEQ ID NO: 16 ist und ein Epitop davon umfasst; nicht-typisierbarem *H. influenzae-NT023-*Antigen, wobei das NT023-Antigen ein Polypeptid ist, welches eine Aminosäuresequenz umfasst: (a) mit 80% oder mehr Identität zu SEQ ID NO: 17 und/oder (b) die ein Fragment von wenigstens 10 aufeinanderfolgenden Aminosäuren von SEQ ID NO: 17 ist und ein Epitop davon umfasst; nicht-typisierbarem *H. influenzaei*-NT100-Antigen, wobei das NT100-Antigen ein Polypeptid ist, welches eine Aminosäuresequenz umfasst: (a) mit 80% oder mehr Identität zu SEQ ID NO: 18 und/oder (b) die ein Fragment von wenigstens 10 aufeinanderfolgenden Aminosäuren von SEQ ID NO: 18 ist und ein Epitop davon umfasst; nicht-typisierbarem *H. influenzae-NT040-*Antigen, wobei das NT040-Antigen ein Polypeptid ist, welches eine Aminosäuresequenz umfasst: (a) mit 80% oder mehr Identität zu SEQ ID NO: 19 und/oder (b) die ein Fragment von wenigstens 10 aufeinanderfolgenden Aminosäuren von SEQ ID NO: 19 ist und ein Epitop davon umfasst; nicht-typisierbarem *H. influenzae-NT048-*Antigen, wobei das NT048-Antigen ein Polypeptid ist, welches eine Aminosäuresequenz umfasst: (a) mit 80% oder mehr Identität zu SEQ ID NO: 20 und/oder (b) die ein Fragment von wenigstens 10 aufeinanderfolgenden Aminosäuren von SEQ ID NO: 20 ist und ein Epitop davon umfasst; nicht-typisierbarem *H. influenzae-NT053-*Antigen, wobei das NT053-Antigen ein Polypeptid ist, welches eine Aminosäuresequenz umfasst: (a) mit 80% oder mehr Identität zu SEQ ID NO: 21 und/oder (b) die ein Fragment von wenigstens 10 aufeinanderfolgenden Aminosäuren von SEQ ID NO: 21 ist und ein Epitop davon umfasst; nicht-typisierbarem *H. influenzae-NT066-*Antigen, wobei das NT066-Antigen ein Polypeptid ist, welches eine Aminosäuresequenz umfasst: (a) mit 80% oder mehr Identität zu SEQ ID NO: 22 und/oder (b) die ein Fragment von wenigstens 10 aufeinanderfolgenden Aminosäuren von SEQ ID NO: 22 ist und ein Epitop davon umfasst; nicht-typisierbarem *H. influenzae*-NT097-Antigen, wobei das NT097-Antigen ein Polypeptid ist, welches eine Aminosäuresequenz umfasst: (a) mit 80% oder mehr Identität zu SEQ ID NO: 23 und/oder (b) die ein Fragment von wenigstens 10 aufeinanderfolgenden Aminosäuren von SEQ ID NO: 23 ist und ein Epitop davon umfasst; nicht-typisierbarem *H. influenzae-NT* 004-Antigen, wobei das NT004-Antigen ein Polypeptid ist, welches eine Aminosäuresequenz umfasst: (a) mit 80% oder mehr Identität zu SEQ ID NO: 122 und/oder (b) die ein Fragment von wenigstens 10 aufeinanderfolgenden Aminosäuren von SEQ ID NO: 122 ist und ein Epitop davon umfasst; nicht-typisierbarem *H. influenzae-NT014-*Antigen, wobei das NT014-Antigen ein Polypeptid ist, welches eine Aminosäuresequenz umfasst: (a) mit 80% oder mehr Identität zu SEQ ID NO: 123 und/oder (b) die ein Fragment von wenigstens 10 aufeinanderfolgenden Aminosäuren von SEQ ID NO: 123 ist und ein Epitop davon umfasst; nicht-typisierbarem *H. influenzae-NT022-*Antigen, wobei das NT022-Antigen ein Polypeptid ist, welches eine Aminosäuresequenz umfasst: (a) mit 80% oder mehr Identität zu SEQ ID NO: 124 und/oder (b) die ein Fragment von wenigstens 10 aufeinanderfolgenden Aminosäuren von SEQ ID NO: 124 ist und ein Epitop davon umfasst.

4. Immunogene Zusammensetzung zur Verwendung gemäß irgendeinem der Ansprüche 1 bis 3, weiterhin umfassend wenigstens ein Polypeptid, ausgewählt aus der Gruppe, bestehend aus: NT007, welches ein Polypeptid ist, das eine Aminosäuresequenz umfasst: (a) mit 80% oder mehr Identität zu SEQ ID NO: 24 und/oder (b) die ein Fragment von wenigstens 10 aufeinanderfolgenden Aminosäuren von SEQ ID NO: 24 ist und ein Epitop davon umfasst; NT006, welches ein Polypeptid ist, das eine Aminosäuresequenz umfasst: (a) mit 80% oder mehr Identität zu SEQ ID NO: 25 und/oder (b) die ein Fragment von wenigstens 10 aufeinanderfolgenden Aminosäuren von SEQ ID NO: 25 ist und ein Epitop davon umfasst; NT035, welches ein Polypeptid ist, das eine Aminosäuresequenz umfasst: (a) mit 80% oder mehr Identität zu SEQ ID NO: 26 und/oder (b) die ein Fragment von wenigstens 10 aufeinanderfolgenden Aminosäuren von SEQ ID NO: 26 ist und ein Epitop davon umfasst; NT010, welches ein Polypeptid ist, das eine Aminosäuresequenz umfasst: (a) mit 80% oder mehr Identität zu SEQ ID NO: 27 und/oder (b) die ein Fragment von wenigstens 10 aufeinanderfolgenden Aminosäuren von SEQ ID NO: 27 ist und ein Epitop davon umfasst; NT080, welches ein Polypeptid ist, das eine Aminosäuresequenz umfasst: (a) mit 80% oder mehr Identität zu SEQ ID NO: 28 und/oder (b) die ein Fragment von wenigstens 10 aufeinanderfolgenden Aminosäuren von SEQ ID NO: 28 ist und ein Epitop davon umfasst; NT081, welches ein Polypeptid ist, das eine Aminosäuresequenz umfasst: (a) mit 80% oder mehr Identität zu SEQ ID NO: 29 und/oder (b) die ein Fragment von wenigstens 10 aufeinanderfolgenden Aminosäuren von SEQ ID NO: 29 ist und ein Epitop davon umfasst.

5. Immunogene Zusammensetzung zur Verwendung gemäß irgendeinem vorangegangenen Anspruch, weiterhin umfassend wenigstens ein Polypeptid, ausgewählt aus der Gruppe, bestehend aus: NT013, welches ein Polypeptid ist, das eine Aminosäuresequenz umfasst: (a) mit 80% oder mehr Identität zu SEQ ID NO: 30 und/oder (b) die ein Fragment von wenigstens 10 aufeinanderfolgenden Aminosäuren von SEQ ID NO: 30 ist und ein Epitop davon umfasst; NT106, welches ein Polypeptid ist, das eine Aminosäuresequenz umfasst: (a) mit 80% oder mehr Identität zu SEQ ID NO: 31 und/oder (b) die ein Fragment von wenigstens 10 aufeinanderfolgenden Aminosäuren von SEQ ID NO: 31 ist und ein Epitop davon umfasst; NT107, welches ein Polypeptid ist, das eine Aminosäuresequenz umfasst: (a) mit 80% oder mehr Identität zu SEQ ID NO: 32 und/oder (b) die ein Fragment von wenigstens 10 aufeinanderfolgenden Aminosäuren von SEQ ID NO: 32 ist und ein Epitop davon umfasst; NT108, welches ein Polypeptid ist, das eine Aminosäuresequenz umfasst: (a) mit 80% oder mehr Identität zu SEQ ID NO: 33 und/oder (b) die ein Fragment von wenigstens 10 aufeinanderfolgenden Aminosäuren von SEQ ID NO: 33 ist und ein Epitop davon umfasst; NT109, welches ein Polypeptid ist, das eine Aminosäuresequenz umfasst: (a) mit 80% oder mehr Identität zu SEQ ID NO: 34 und/oder (b) die ein Fragment von wenigstens 10 aufeinanderfolgenden Aminosäuren von SEQ ID NO: 34 ist und ein Epitop davon umfasst; NT110, welches ein Polypeptid ist, das eine Aminosäuresequenz umfasst: (a) mit 80% oder mehr Identität zu SEQ ID NO: 35 und/oder (b) die ein Fragment von wenigstens 10 aufeinanderfolgenden Aminosäuren von SEQ ID NO: 35 ist und ein Epitop davon umfasst; NT111, welches ein Polypeptid ist, das eine Aminosäuresequenz umfasst: (a) mit 80% oder mehr Identität zu SEQ ID NO: 36 und/oder (b) die ein Fragment von wenigstens 10 aufeinanderfolgenden Aminosäuren von SEQ ID NO: 36 ist und ein Epitop davon umfasst; NT112, welches ein Polypeptid ist, das eine Aminosäuresequenz umfasst: (a) mit 80% oder mehr Identität zu SEQ ID NO: 37 und/oder (b) die ein Fragment von wenigstens 10 aufeinanderfolgenden Aminosäuren von SEQ ID NO: 37 ist und ein Epitop davon umfasst; NT113, welches ein Polypeptid ist, das eine Aminosäuresequenz umfasst: (a) mit 80% oder mehr Identität zu SEQ ID NO: 38 und/oder (b) die ein Fragment von wenigstens 10 aufeinanderfolgenden Aminosäuren von SEQ ID NO: 38 ist und ein Epitop davon umfasst; NT114, welches ein Polypeptid ist, das eine Aminosäuresequenz umfasst: (a) mit 80% oder mehr Identität zu SEQ ID NO: 39 und/oder (b) die ein Fragment von wenigstens 10 aufeinanderfolgenden Aminosäuren von SEQ ID NO: 39 ist und ein Epitop davon umfasst; NT115, welches ein Polypeptid ist, das eine Aminosäuresequenz umfasst: (a) mit 80% oder mehr Identität zu SEQ ID NO: 40 und/oder (b) die ein Fragment von wenigstens 10 aufeinanderfolgenden Aminosäuren von SEQ ID NO: 40 ist und ein Epitop davon umfasst; NT116, welches ein Polypeptid ist, das eine Aminosäuresequenz umfasst: (a) mit 80% oder mehr Identität zu SEQ ID NO: 41 und/oder (b) die ein Fragment von wenigstens 10 aufeinanderfolgenden Aminosäuren von SEQ ID NO: 41 ist und ein Epitop davon umfasst; NT117, welches ein Polypeptid ist, das eine Aminosäuresequenz umfasst: (a) mit 80% oder mehr Identität zu SEQ ID NO: 42 und/oder (b) die ein Fragment von wenigstens 10 aufeinanderfolgenden Aminosäuren von SEQ ID NO: 42 ist und ein Epitop davon umfasst; NT118, welches ein Polypeptid ist, das eine Aminosäuresequenz umfasst: (a) mit 80% oder mehr Identität zu SEQ ID NO: 43 und/oder (b) die ein Fragment von wenigstens 10 aufeinanderfolgenden Aminosäuren von SEQ ID NO: 43 ist und ein Epitop davon umfasst; NT123, welches ein Polypeptid ist, das eine Aminosäuresequenz umfasst: (a) mit 80% oder mehr Identität zu SEQ ID NO: 44 und/oder (b) die ein Fragment von wenigstens 10 aufeinanderfolgenden Aminosäuren von SEQ ID NO: 44 ist und ein Epitop davon umfasst; NT124, welches ein Polypeptid ist, das eine Aminosäuresequenz umfasst: (a) mit 80% oder mehr Identität zu SEQ ID NO: 45 und/oder (b) die ein Fragment von wenigstens 10 aufeinanderfolgenden Aminosäuren von SEQ ID NO: 45 ist und ein Epitop davon umfasst; NT119, welches ein Polypeptid ist, das eine Aminosäuresequenz umfasst: (a) mit 80% oder mehr Identität zu SEQ ID NO: 114 und/oder (b) die ein Fragment von wenigstens 10 aufeinanderfolgenden Aminosäuren von SEQ ID NO: 114 ist und ein Epitop davon umfasst; NT120, welches ein Polypeptid ist, das eine Aminosäuresequenz umfasst: (a) mit 80% oder mehr Identität zu SEQ ID NO: 115 und/oder (b) die ein Fragment von wenigstens 10 aufeinanderfolgenden Aminosäuren von SEQ ID NO: 115 ist und ein Epitop davon umfasst; NT121, welches ein Polypeptid ist, das eine Aminosäuresequenz umfasst: (a) mit 80% oder mehr Identität zu SEQ ID NO: 116 und/oder (b) die ein Fragment von wenigstens 10 aufeinanderfolgenden Aminosäuren von SEQ ID NO: 116 ist und ein Epitop davon umfasst; NT122, welches ein Polypeptid ist, das eine Aminosäuresequenz umfasst: (a) mit 80% oder mehr Identität zu SEQ ID NO: 117 und/oder (b) die ein Fragment von wenigstens 10 aufeinanderfolgenden Aminosäuren von SEQ ID NO: 117 ist und ein Epitop davon umfasst; und/oder NT061, welches ein Polypeptid ist, das eine Aminosäuresequenz umfasst: (a) mit 80% oder mehr Identität zu SEQ ID NO: 128 und/oder (b) die ein Fragment von wenigstens 10 aufeinanderfolgenden Aminosäuren von SEQ ID NO: 128 ist und ein Epitop davon umfasst.

6. Immunogene Zusammensetzung zur Verwendung gemäß irgendeinem der Ansprüche 1 bis 5, welche eine Kombination von zwei oder mehr Antigenen umfasst.

7. Zusammensetzung zur Verwendung gemäß irgendeinem der Ansprüche 1 bis 6, weiterhin umfassend wenigstens ein Impfstoffantigen, welches kein nicht-typisierbares *H*. *influenzae-Antigen* ist, ausgewählt aus irgendeinem von:
- einem Antigen von *N*. *meningitidis* Serogruppe A, B, C, W135 und/oder Y.
- einem Saccharid- oder Polypeptidantigen von *Streptococcus pneumoniae*
- einem Antigen von Hepatitis A-Virus
- einem Antigen von Hepatitis B-Virus
- einem Diphtherieantigen, wie beispielsweise einem Diphtherietoxoid
- einem Tetanusantigen
- einem Antigen von *Bordetella pertussis*
- einem ganzen zellulären Pertussisantigen
- einem Saccharidantigen von *Haemophilus influenzae B*
- Polioantigen(en)
- Masern-, Mumps- und/oder Rötelnantigenen
- Influenzaantigen(en)
- einem Antigen von *Moraxella catarrhalis*
- einem Antigen von respiratorischem Synzytialvirus
- einer Impfstoffzusammensetzung, umfassend Diphtherie (D), Tetanus (T), Pertussis (azellulär, Komponente) (Pa), Hepatitis B (rDNA) (HBV), Poliomyelitis (inaktiviert) (IPV) und *Haemophilus influenzae* Typ B (Hib)-Konjugatimpfstoff (adsorbiert), z. B. Infanrix-hexa.

8. Zusammensetzung zur Verwendung gemäß irgendeinem der Ansprüche 1 bis 7, wobei das Verfahren zum Hervorrufen einer schützenden Immunantwort gegen *H. influenzae-*Infektion in einem Säugetier ein Verfahren zum Schützen vor Otitis media ist.

9. Zusammensetzung zur Verwendung gemäß irgendeinem der Ansprüche 1 bis 7, wobei das Verfahren zum Hervorrufen einer schützenden Immunantwort gegen *H. influenzae-*Infektion ein Verfahren zur Behandlung oder Prävention von COPD-Erkrankungen ist.

## Revendications

1. Composition immunogène comprenant un antigène de *H*. *influenzae* non typable NT067 pour une utilisation dans un procédé de déclenchement d'une réponse immunitaire protectrice contre une infection par *H*. *influenzae* chez un mammifère, dans laquelle ledit antigène NT067 est un polypeptide qui comprend une séquence d'acides aminés présentant 95 % d'identité ou plus avec la SEQ ID N° : 5.

2. Composition pour une utilisation selon la revendication 1, dans laquelle ledit antigène NT067 comprend la séquence d'acides aminés de SEQ ID N° : 52.

3. Composition pour une utilisation selon la revendication 1, comprenant en outre un ou plusieurs antigènes de *H. influenzae* non typable, choisis dans le groupe consistant en : antigène de *H. influenzae* non typable NT052, dans laquelle ledit antigène NT052 est un polypeptide qui comprend une séquence d'acides aminés : (a) présentant 80 % d'identité ou plus avec la SEQ ID N° : 8, et/ou (b) qui est un fragment d'au moins 10 acides aminés consécutifs, et comprend un épitope, de SEQ ID N° : 8 ; antigène de *H. influenzae* non typable NT018, dans laquelle ledit antigène NT018 est un polypeptide qui comprend une séquence d'acides aminés : (a) présentant 80 % d'identité ou plus avec la SEQ ID N° : 1, et/ou (b) qui est un fragment d'au moins 10 acides aminés consécutifs, et comprend un épitope, de SEQ ID N° : 1 ; antigène de *H. influenzae* non typable NT024, dans laquelle ledit antigène NT024 est un polypeptide qui comprend une séquence d'acides aminés : (a) présentant 80 % d'identité ou plus avec la SEQ ID N° : 2, et/ou (b) qui est un fragment d'au moins 10 acides aminés consécutifs, et comprend un épitope, de SEQ ID N° : 2 ; antigène de *H. influenzae* non typable NT032, dans laquelle ledit antigène NT032 est un polypeptide qui comprend une séquence d'acides aminés : (a) présentant 80 % d'identité ou plus avec la SEQ ID N° : 3, et/ou (b) qui est un fragment d'au moins 10 acides aminés consécutifs, et comprend un épitope, de SEQ ID N° : 3 ; antigène de *H. influenzae* non typable NT038, dans laquelle ledit antigène NT038 est un polypeptide qui comprend une séquence d'acides aminés : (a) présentant 80 % d'identité ou plus avec la SEQ ID N° : 4, et/ou (b) qui est un fragment d'au moins 10 acides aminés consécutifs, et comprend un épitope, de SEQ ID N° : 4 ; antigène de *H. influenzae* non typable NT001, dans laquelle ledit antigène NT001 est un polypeptide qui comprend une séquence d'acides aminés : (a) présentant 80 % d'identité ou plus avec la SEQ ID N° : 6, et/ou (b) qui est un fragment d'au moins 10 acides aminés consécutifs, et comprend un épitope, de SEQ ID N° : 6 ; antigène de *H. influenzae* non typable NT016, dans laquelle ledit antigène NT016 est un polypeptide qui comprend une séquence d'acides aminés : (a) présentant 80 % d'identité ou plus avec la SEQ ID N° : 7, et/ou (b) qui est un fragment d'au moins 10 acides aminés consécutifs, et comprend un épitope, de SEQ ID N° : 7 ; antigène de *H. influenzae* non typable NT002, dans laquelle ledit antigène NT002 est un polypeptide qui comprend une séquence d'acides aminés : (a) présentant 80 % d'identité ou plus avec la SEQ ID N° : 9, et/ou (b) qui est un fragment d'au moins 10 acides aminés consécutifs, et comprend un épitope, de SEQ ID N° : 9 ; antigène de *H. influenzae* non typable NT026, dans laquelle ledit antigène NT026 est un polypeptide qui comprend une séquence d'acides aminés : (a) présentant 80 % d'identité ou plus avec la SEQ ID N° : 10, et/ou (b) qui est un fragment d'au moins 10 acides aminés consécutifs, et comprend un épitope, de SEQ ID N° : 10 ; antigène de *H. influenzae* non typable NT009, dans laquelle ledit antigène NT009 est un polypeptide qui comprend une séquence d'acides aminés : (a) présentant 80 % d'identité ou plus avec la SEQ ID N° : 11, et/ou (b) qui est un fragment d'au moins 10 acides aminés consécutifs, et comprend un épitope, de SEQ ID N° : 11 ; antigène *de H. influenzae* non typable NT025, dans laquelle ledit antigène NT025 est un polypeptide qui comprend une séquence d'acides aminés : (a) présentant 80 % d'identité ou plus avec la SEQ ID N° : 12, et/ou (b) qui est un fragment d'au moins 10 acides aminés consécutifs, et comprend un épitope, de SEQ ID N° : 12 ; antigène de *H. influenzae* non typable NT028, dans laquelle ledit antigène NT028 est un polypeptide qui comprend une séquence d'acides aminés : (a) présentant 80 % d'identité ou plus avec la SEQ ID N° : 13, et/ou (b) qui est un fragment d'au moins 10 acides aminés consécutifs, et comprend un épitope, de SEQ ID N° : 13 ; antigène de *H. influenzae* non typable NT029, dans laquelle ledit antigène NT029 est un polypeptide qui comprend une séquence d'acides aminés : (a) présentant 80 % d'identité ou plus avec la SEQ ID N° : 14, et/ou (b) qui est un fragment d'au moins 10 acides aminés consécutifs, et comprend un épitope, de SEQ ID N° : 14 ; antigène de *H. influenzae* non typable NT031, dans laquelle ledit antigène NT031 est un polypeptide qui comprend une séquence d'acides aminés : (a) présentant 80 % d'identité ou plus avec la SEQ ID N° : 15, et/ou (b) qui est un fragment d'au moins 10 acides aminés consécutifs, et comprend un épitope, de SEQ ID N° : 15 ; antigène de *H. influenzae* non typable NT015, dans laquelle ledit antigène NT015 est un polypeptide qui comprend une séquence d'acides aminés : (a) présentant 80 % d'identité ou plus avec la SEQ ID N° : 16, et/ou (b) qui est un fragment d'au moins 10 acides aminés consécutifs, et comprend un épitope, de SEQ ID N° : 16 ; antigène de *H. influenzae* non typable NT023, dans laquelle ledit antigène NT023 est un polypeptide qui comprend une séquence d'acides aminés : (a) présentant 80 % d'identité ou plus avec la SEQ ID N° : 17, et/ou (b) qui est un fragment d'au moins 10 acides aminés consécutifs, et comprend un épitope, de SEQ ID N° : 17 ; antigène de *H. influenzae* non typable NT100, dans laquelle ledit antigène NT100 est un polypeptide qui comprend une séquence d'acides aminés : (a) présentant 80 % d'identité ou plus avec la SEQ ID N° : 18, et/ou (b) qui est un fragment d'au moins 10 acides aminés consécutifs, et comprend un épitope, de SEQ ID N° : 18 ; antigène de *H. influenzae* non typable NT040, dans laquelle ledit antigène NT040 est un polypeptide qui comprend une séquence d'acides aminés : (a) présentant 80 % d'identité ou plus avec la SEQ ID N° : 19, et/ou (b) qui est un fragment d'au moins 10 acides aminés consécutifs, et comprend un épitope, de SEQ ID N° : 19 ; antigène de *H. influenzae* non typable NT048, dans laquelle ledit antigène NT048 est un polypeptide qui comprend une séquence d'acides aminés : (a) présentant 80 % d'identité ou plus avec la SEQ ID N° : 20, et/ou (b) qui est un fragment d'au moins 10 acides aminés consécutifs, et comprend un épitope, de SEQ ID N° : 20 ; antigène de *H. influenzae* non typable NT053, dans laquelle ledit antigène NT053 est un polypeptide qui comprend une séquence d'acides aminés : (a) présentant 80 % d'identité ou plus avec la SEQ ID N° : 21, et/ou (b) qui est un fragment d'au moins 10 acides aminés consécutifs, et comprend un épitope, de SEQ ID N° : 21 ; antigène de *H. influenzae* non typable NT066, dans laquelle ledit antigène NT066 est un polypeptide qui comprend une séquence d'acides aminés : (a) présentant 80 % d'identité ou plus avec la SEQ ID N° : 22, et/ou (b) qui est un fragment d'au moins 10 acides aminés consécutifs, et comprend un épitope, de SEQ ID N° : 22 ; antigène de *H. influenzae* non typable NT097, dans laquelle ledit antigène NT097 est un polypeptide qui comprend une séquence d'acides aminés : (a) présentant 80 % d'identité ou plus avec la SEQ ID N° : 23, et/ou (b) qui est un fragment d'au moins 10 acides aminés consécutifs, et comprend un épitope, de SEQ ID N° : 23 ; antigène de *H. influenzae* non typable NT004, dans laquelle ledit antigène NT004 est un polypeptide qui comprend une séquence d'acides aminés : (a) présentant 80 % d'identité ou plus avec la SEQ ID N° : 122, et/ou (b) qui est un fragment d'au moins 10 acides aminés consécutifs, et comprend un épitope, de SEQ ID N° : 122 ; antigène de *H. influenzae* non typable NT014, dans laquelle ledit antigène NT014 est un polypeptide qui comprend une séquence d'acides aminés : (a) présentant 80 % d'identité ou plus avec la SEQ ID N° : 123, et/ou (b) qui est un fragment d'au moins 10 acides aminés consécutifs, et comprend un épitope, de SEQ ID N° : 123 ; antigène de *H*. *influenzae* non typable NT022, dans laquelle ledit antigène NT022 est un polypeptide qui comprend une séquence d'acides aminés : (a) présentant 80 % d'identité ou plus avec la SEQ ID N° : 124, et/ou (b) qui est un fragment d'au moins 10 acides aminés consécutifs, et comprend un épitope, de SEQ ID N° : 124.

4. Composition immunogène pour une utilisation selon l'une quelconque des revendications 1 à 3, comprenant en outre au moins un polypeptide choisi dans le groupe consistant en : NT007 qui est un polypeptide qui comprend une séquence d'acides aminés : (a) présentant 80 % d'identité ou plus avec la SEQ ID N° : 24, et/ou (b) qui est un fragment d'au moins 10 acides aminés consécutifs, et comprend un épitope, de SEQ ID N° : 24 ; NT006 qui est un polypeptide qui comprend une séquence d'acides aminés : (a) présentant 80 % d'identité ou plus avec la SEQ ID N° : 25, et/ou (b) qui est un fragment d'au moins 10 acides aminés consécutifs, et comprend un épitope, de SEQ ID N° : 25 ; NT035 qui est un polypeptide qui comprend une séquence d'acides aminés : (a) présentant 80 % d'identité ou plus avec la SEQ ID N° : 26, et/ou (b) qui est un fragment d'au moins 10 acides aminés consécutifs, et comprend un épitope, de SEQ ID N° : 26 ; NT010 qui est un polypeptide qui comprend une séquence d'acides aminés : (a) présentant 80 % d'identité ou plus avec la SEQ ID N° : 27, et/ou (b) qui est un fragment d'au moins 10 acides aminés consécutifs, et comprend un épitope, de SEQ ID N° : 27 ; NT080 qui est un polypeptide qui comprend une séquence d'acides aminés : (a) présentant 80 % d'identité ou plus avec la SEQ ID N° : 28, et/ou (b) qui est un fragment d'au moins 10 acides aminés consécutifs, et comprend un épitope, de SEQ ID N° : 28 ; NT081 qui est un polypeptide qui comprend une séquence d'acides aminés : (a) présentant 80 % d'identité ou plus avec la SEQ ID N° : 29, et/ou (b) qui est un fragment d'au moins 10 acides aminés consécutifs, et comprend un épitope, de SEQ ID N° : 29.

5. Composition immunogène pour une utilisation selon une quelconque revendication précédente, comprenant en outre au moins un polypeptide choisi dans le groupe consistant en : NT013 qui est un polypeptide qui comprend une séquence d'acides aminés : (a) présentant 80 % d'identité ou plus avec la SEQ ID N° : 30, et/ou (b) qui est un fragment d'au moins 10 acides aminés consécutifs, et comprend un épitope, de SEQ ID N° : 30 ; NT106 qui est un polypeptide qui comprend une séquence d'acides aminés : (a) présentant 80 % d'identité ou plus avec la SEQ ID N° : 31, et/ou (b) qui est un fragment d'au moins 10 acides aminés consécutifs, et comprend un épitope, de SEQ ID N° : 31 ; NT107 qui est un polypeptide qui comprend une séquence d'acides aminés : (a) présentant 80 % d'identité ou plus avec la SEQ ID N° : 32, et/ou (b) qui est un fragment d'au moins 10 acides aminés consécutifs, et comprend un épitope, de SEQ ID N° : 32 ; NT108 qui est un polypeptide qui comprend une séquence d'acides aminés : (a) présentant 80 % d'identité ou plus avec la SEQ ID N° : 33, et/ou (b) qui est un fragment d'au moins 10 acides aminés consécutifs, et comprend un épitope, de SEQ ID N° : 33 ; NT109 qui est un polypeptide qui comprend une séquence d'acides aminés : (a) présentant 80 % d'identité ou plus avec la SEQ ID N° : 34, et/ou (b) qui est un fragment d'au moins 10 acides aminés consécutifs, et comprend un épitope, de SEQ ID N° : 34 ; NT110 qui est un polypeptide qui comprend une séquence d'acides aminés : (a) présentant 80 % d'identité ou plus avec la SEQ ID N° : 35, et/ou (b) qui est un fragment d'au moins 10 acides aminés consécutifs, et comprend un épitope, de SEQ ID N° : 35 ; NT111 qui est un polypeptide qui comprend une séquence d'acides aminés : (a) présentant 80 % d'identité ou plus avec la SEQ ID N° : 36, et/ou (b) qui est un fragment d'au moins 10 acides aminés consécutifs, et comprend un épitope, de SEQ ID N° : 36 ; NT112 qui est un polypeptide qui comprend une séquence d'acides aminés : (a) présentant 80 % d'identité ou plus avec la SEQ ID N° : 37, et/ou (b) qui est un fragment d'au moins 10 acides aminés consécutifs, et comprend un épitope, de SEQ ID N° : 37 ; NT113 qui est un polypeptide qui comprend une séquence d'acides aminés : (a) présentant 80 % d'identité ou plus avec la SEQ ID N° : 38, et/ou (b) qui est un fragment d'au moins 10 acides aminés consécutifs, et comprend un épitope, de SEQ ID N° : 38 ; NT114 qui est un polypeptide qui comprend une séquence d'acides aminés : (a) présentant 80 % d'identité ou plus avec la SEQ ID N° : 39, et/ou (b) qui est un fragment d'au moins 10 acides aminés consécutifs, et comprend un épitope, de SEQ ID N° : 39 ; NT115 qui est un polypeptide qui comprend une séquence d'acides aminés : (a) présentant 80 % d'identité ou plus avec la SEQ ID N° : 40, et/ou (b) qui est un fragment d'au moins 10 acides aminés consécutifs, et comprend un épitope, de SEQ ID N° : 40 ; NT116 qui est un polypeptide qui comprend une séquence d'acides aminés : (a) présentant 80 % d'identité ou plus avec la SEQ ID N° : 41, et/ou (b) qui est un fragment d'au moins 10 acides aminés consécutifs, et comprend un épitope, de SEQ ID N° : 41 ; NT117 qui est un polypeptide qui comprend une séquence d'acides aminés : (a) présentant 80 % d'identité ou plus avec la SEQ ID N° : 42, et/ou (b) qui est un fragment d'au moins 10 acides aminés consécutifs, et comprend un épitope, de SEQ ID N° : 42 ; NT118 qui est un polypeptide qui comprend une séquence d'acides aminés : (a) présentant 80 % d'identité ou plus avec la SEQ ID N° : 43, et/ou (b) qui est un fragment d'au moins 10 acides aminés consécutifs, et comprend un épitope, de 43 ; NT123 qui est un polypeptide qui comprend une séquence d'acides aminés : (a) présentant 80 % d'identité ou plus avec la SEQ ID N° : 44, et/ou (b) qui est un fragment d'au moins 10 acides aminés consécutifs, et comprend un épitope, de SEQ ID N° : 44 ; NT124 qui est un polypeptide qui comprend une séquence d'acides aminés : (a) présentant 80 % d'identité ou plus avec la SEQ ID N° : 45, et/ou (b) qui est un fragment d'au moins 10 acides aminés consécutifs, et comprend un épitope, de SEQ ID N° : 45 ; NT119 qui est un polypeptide qui comprend une séquence d'acides aminés : (a) présentant 80 % d'identité ou plus avec la SEQ ID N° : 114, et/ou (b) qui est un fragment d'au moins 10 acides aminés consécutifs, et comprend un épitope, de SEQ ID N° : 114 ; NT120 qui est un polypeptide qui comprend une séquence d'acides aminés : (a) présentant 80 % d'identité ou plus avec la SEQ ID N° : 115, et/ou (b) qui est un fragment d'au moins 10 acides aminés consécutifs, et comprend un épitope, de SEQ ID N° : 115 ; NT121 qui est un polypeptide qui comprend une séquence d'acides aminés : (a) présentant 80 % d'identité ou plus avec la SEQ ID N° : 116, et/ou (b) qui est un fragment d'au moins 10 acides aminés consécutifs, et comprend un épitope, de SEQ ID N° : 116 ; NT122 qui est un polypeptide qui comprend une séquence d'acides aminés : (a) présentant 80 % d'identité ou plus avec la SEQ ID N° : 117, et/ou (b) qui est un fragment d'au moins 10 acides aminés consécutifs, et comprend un épitope, de SEQ ID N° : 117 ; et/ou NT061 qui est un polypeptide qui comprend une séquence d'acides aminés : (a) présentant 80 % d'identité ou plus avec la SEQ ID N° : 128, et/ou (b) qui est un fragment d'au moins 10 acides aminés consécutifs, et comprend un épitope, de SEQ ID N° : 128.

6. Composition immunogène pour une utilisation selon l'une quelconque des revendications 1 à 5 comprenant une combinaison de deux antigènes ou plus.

7. Composition pour une utilisation selon l'une quelconque des revendications 1 à 6, comprenant en outre au moins un antigène vaccinal qui est n'est pas un antigène de *H. influenzae* non typable choisi parmi l'un quelconque de :
- un antigène de *N.meningitidis* de sérogroupe A, B, C, W135 et/ou Y.
- un antigène saccharidique ou polypeptidique de *Streptococcus pneumoniae*
- un antigène du virus de l'hépatite A
- un antigène du virus de l'hépatite B
- un antigène de la diphtérie, tel qu'une anatoxine diphtérique
- un antigène du tétanos
- un antigène de *Bordetella pertussis*
- un antigène de la coqueluche à cellules entières
- un antigène saccharidique de *Haemophilus influenzae B*
- un (des) antigène(s) de la poliomyélite
- des antigènes de la rougeole, de la rubéole et/ou des oreillons
- un (des) antigène(s) de la grippe
- un antigène de *Moraxella catarrhalis*
- un antigène du virus respiratoire syncytial
- une composition vaccinale comprenant un vaccin polyvalent contre la diphtérie (D), le tétanos (T), la coqueluche (acellulaire, composant) (Pa), l'hépatite B (ADNr) (VHB), la poliomyélite (inactivé) (VPI) et *Haemophilus influenzae* de type b (Hib) (adsorbé), par exemple Infanrix-hexa

8. Composition pour une utilisation selon l'une quelconque des revendications 1 à 7, dans laquelle ledit procédé de déclenchement d'une réponse immunitaire protectrice contre une infection par *H. influenzae* chez un mammifère est un procédé de protection contre une otite moyenne.

9. Composition pour une utilisation selon l'une quelconque des revendications 1 à 7, dans laquelle ledit procédé de déclenchement d'une réponse immunitaire protectrice contre une infection par *H. influenzae* chez un mammifère est un procédé de traitement ou de prévention de maladies de type BPCO.
